# EUROPEAN PATENT APPLICATION

(11) **EP 4 019 526 A1**
(43) Date of publication of application: **29.06.2022**
(21) Application number: 22157219.1
(22) Date of filing: 25.01.2019
(51) Int. Cl.: C07F 15/00, C09K 11/06, H01L 51/00

(54) **ORGANIC ELECTROLUMINESCENT MATERIALS AND DEVICES**

(30) Priority: 26.01.2018 US 201862622307 P; 14.01.2019 US 201916247032
(62) Divisional of application: 19153757.0
(71) Applicant: Universal Display Corporation, Ewing, NJ 08618 (US)
(72) Inventor: JI, Zhiqiang, Ewing, New Jersey 08618 (US); TSAI, Jui-Yi, Ewing, New Jersey 08618 (US); DYATKIN, Alexey Borisovich, Ewing, New Jersey 08618 (US); LIN, Chun, Ewing, New Jersey 08618 (US)
(74) Representative: Hansen, Norbert

(57) **Abstract**

A compound including a first ligand L_{A} of Formula I 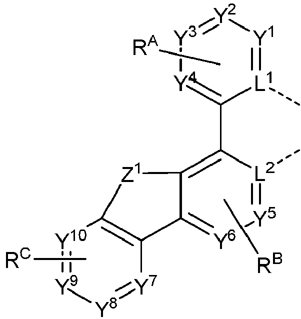 is disclosed. In the structure of Formula I, one of L¹ and L² is C, and the other is N; Y¹ to Y¹⁴ are each C or N; at least two adjacent Y⁷, Y⁸, Y⁹, and Y¹⁰ are carbon atoms that are fused to a structure of Formula II 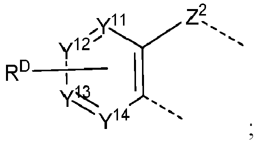 Z¹ and Z² are each O, S, Se, NR, CRR', or SiRR'; and each R, R', R^{A}, R^{B}, R^{C}, and R^{D} is hydrogen or a substituent; and any two substituents may be joined or fused together to form a ring. In the compound, L_{A} is complexed to a metal M by L¹ and L², and M has an atomic weight greater than 40. Organic light emitting devices and consumer products containing the compounds are also disclosed.

## Description

### CROSS-REFERENCE TO RELATED APPLICATIONS

This application claims priority under 35 U.S.C. § 119(e) to United States Provisional Application No. 62/622,307, filed January 26, 2018, the entire contents of which are incorporated herein by reference.

### FIELD

The present invention relates to compounds for use as emitters, and devices, such as organic light emitting diodes, including the same.

### BACKGROUND

Opto-electronic devices that make use of organic materials are becoming increasingly desirable for a number of reasons. Many of the materials used to make such devices are relatively inexpensive, so organic opto-electronic devices have the potential for cost advantages over inorganic devices. In addition, the inherent properties of organic materials, such as their flexibility, may make them well suited for particular applications such as fabrication on a flexible substrate. Examples of organic opto-electronic devices include organic light emitting diodes/devices (OLEDs), organic phototransistors, organic photovoltaic cells, and organic photodetectors. For OLEDs, the organic materials may have performance advantages over conventional materials. For example, the wavelength at which an organic emissive layer emits light may generally be readily tuned with appropriate dopants.

OLEDs make use of thin organic films that emit light when voltage is applied across the device. OLEDs are becoming an increasingly interesting technology for use in applications such as flat panel displays, illumination, and backlighting. Several OLED materials and configurations are described in U.S. Pat. Nos. 5,844,363, 6,303,238, and 5,707,745, which are incorporated herein by reference in their entirety.

One application for phosphorescent emissive molecules is a full color display. Industry standards for such a display call for pixels adapted to emit particular colors, referred to as "saturated" colors. In particular, these standards call for saturated red, green, and blue pixels. Alternatively the OLED can be designed to emit white light. In conventional liquid crystal displays emission from a white backlight is filtered using absorption filters to produce red, green and blue emission. The same technique can also be used with OLEDs. The white OLED can be either a single EML device or a stack structure. Color may be measured using CIE coordinates, which are well known to the art.

One example of a green emissive molecule is tris(2-phenylpyridine) iridium, denoted Ir(ppy)₃, which has the following structure:

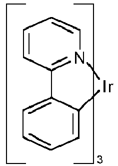

In this, and later figures herein, we depict the dative bond from nitrogen to metal (here, Ir) as a straight line.

As used herein, the term "organic" includes polymeric materials as well as small molecule organic materials that may be used to fabricate organic opto-electronic devices. "Small molecule" refers to any organic material that is not a polymer, and "small molecules" may actually be quite large. Small molecules may include repeat units in some circumstances. For example, using a long chain alkyl group as a substituent does not remove a molecule from the "small molecule" class. Small molecules may also be incorporated into polymers, for example as a pendent group on a polymer backbone or as a part of the backbone. Small molecules may also serve as the core moiety of a dendrimer, which consists of a series of chemical shells built on the core moiety. The core moiety of a dendrimer may be a fluorescent or phosphorescent small molecule emitter. A dendrimer may be a "small molecule," and it is believed that all dendrimers currently used in the field of OLEDs are small molecules.

As used herein, "top" means furthest away from the substrate, while "bottom" means closest to the substrate. Where a first layer is described as "disposed over" a second layer, the first layer is disposed further away from substrate. There may be other layers between the first and second layer, unless it is specified that the first layer is "in contact with" the second layer. For example, a cathode may be described as "disposed over" an anode, even though there are various organic layers in between.

As used herein, "solution processable" means capable of being dissolved, dispersed, or transported in and/or deposited from a liquid medium, either in solution or suspension form.

A ligand may be referred to as "photoactive" when it is believed that the ligand directly contributes to the photoactive properties of an emissive material. A ligand may be referred to as "ancillary" when it is believed that the ligand does not contribute to the photoactive properties of an emissive material, although an ancillary ligand may alter the properties of a photoactive ligand.

As used herein, and as would be generally understood by one skilled in the art, a first "Highest Occupied Molecular Orbital" (HOMO) or "Lowest Unoccupied Molecular Orbital" (LUMO) energy level is "greater than" or "higher than" a second HOMO or LUMO energy level if the first energy level is closer to the vacuum energy level. Since ionization potentials (IP) are measured as a negative energy relative to a vacuum level, a higher HOMO energy level corresponds to an IP having a smaller absolute value (an IP that is less negative). Similarly, a higher LUMO energy level corresponds to an electron affinity (EA) having a smaller absolute value (an EA that is less negative). On a conventional energy level diagram, with the vacuum level at the top, the LUMO energy level of a material is higher than the HOMO energy level of the same material. A "higher" HOMO or LUMO energy level appears closer to the top of such a diagram than a "lower" HOMO or LUMO energy level.

As used herein, and as would be generally understood by one skilled in the art, a first work function is "greater than" or "higher than" a second work function if the first work function has a higher absolute value. Because work functions are generally measured as negative numbers relative to vacuum level, this means that a "higher" work function is more negative. On a conventional energy level diagram, with the vacuum level at the top, a "higher" work function is illustrated as further away from the vacuum level in the downward direction. Thus, the definitions of HOMO and LUMO energy levels follow a different convention than work functions.

More details on OLEDs, and the definitions described above, can be found in U.S. Pat. No. 7,279,704, which is incorporated herein by reference in its entirety.

### SUMMARY

According to an aspect of the present disclosure, a compound comprising a first ligand L_{A} of Formula I 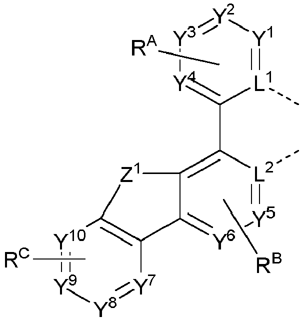 is disclosed. In the structure of Formula I:
one of L¹ and L² is C, and the other of L¹ and L² is N;
Y¹ to Y¹⁰ are each independently selected from the group consisting of C and N;
at least two adjacent Y⁷, Y⁸, Y⁹, and Y¹⁰ are carbon atoms that are fused to a structure of Formula II 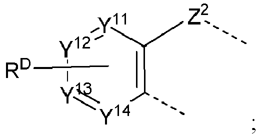
Y¹¹ to Y¹⁴ are each independently selected from the group consisting of C and N;
Z¹ and Z² are each independently selected from the group consisting of O, S, Se, NR, CRR', and SiRR';
R^{A}, R^{B}, and R^{D} represent mono to a maximum possible number of substitutions, or no substitution;
R^{C} represents di-, tri-, or tetra-substitution;
each R, R', R^{A}, R^{B}, R^{C}, and R^{D} is independently hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof;
any two substituents may be joined or fused together to form a ring;
L_{A} is complexed to a metal M by L¹ and L², and M has an atomic weight greater than 40;
M is optionally coordinated to other ligands; and
the ligand L_{A} is optionally linked with other ligands to comprise a tridentate, tetradentate, pentadentate, or hexadentate ligand.

An OLED comprising the compound of the present disclosure in an organic layer therein is also disclosed.

A consumer product comprising the OLED is also disclosed.

### BRIEF DESCRIPTION OF THE DRAWINGS

FIG. 1 shows an organic light emitting device.
FIG. 2 shows an inverted organic light emitting device that does not have a separate electron transport layer.

### DETAILED DESCRIPTION

Generally, an OLED comprises at least one organic layer disposed between and electrically connected to an anode and a cathode. When a current is applied, the anode injects holes and the cathode injects electrons into the organic layer(s). The injected holes and electrons each migrate toward the oppositely charged electrode. When an electron and hole localize on the same molecule, an "exciton," which is a localized electron-hole pair having an excited energy state, is formed. Light is emitted when the exciton relaxes via a photoemissive mechanism. In some cases, the exciton may be localized on an excimer or an exciplex. Non-radiative mechanisms, such as thermal relaxation, may also occur, but are generally considered undesirable.

The initial OLEDs used emissive molecules that emitted light from their singlet states ("fluorescence") as disclosed, for example, in U.S. Pat. No. 4,769,292, which is incorporated by reference in its entirety. Fluorescent emission generally occurs in a time frame of less than 10 nanoseconds.

More recently, OLEDs having emissive materials that emit light from triplet states ("phosphorescence") have been demonstrated. Baldo et al., "Highly Efficient Phosphorescent Emission from Organic Electroluminescent Devices," Nature, vol. 395, 151-154, 1998; ("Baldo-I") and Baldo et al., "Very high-efficiency green organic light-emitting devices based on electrophosphorescence," Appl. Phys. Lett., vol. 75, No. 3, 4-6 (1999) ("Baldo-II"), are incorporated by reference in their entireties. Phosphorescence is described in more detail in U.S. Pat. No. 7,279,704 at cols. 5-6, which are incorporated by reference.

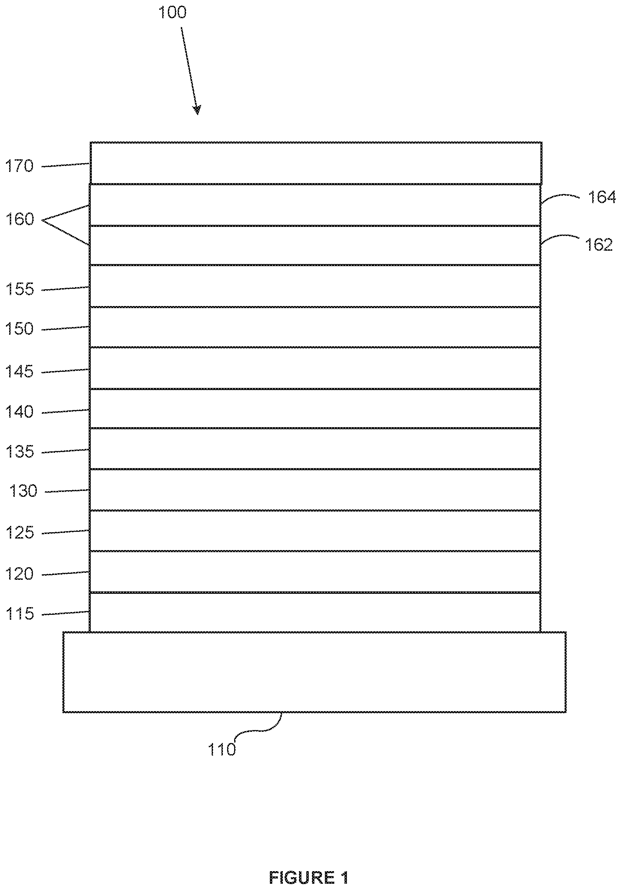

FIG. 1 shows an organic light emitting device 100. The figures are not necessarily drawn to scale. Device 100 may include a substrate 110, an anode 115, a hole injection layer 120, a hole transport layer 125, an electron blocking layer 130, an emissive layer 135, a hole blocking layer 140, an electron transport layer 145, an electron injection layer 150, a protective layer 155, a cathode 160, and a barrier layer 170. Cathode 160 is a compound cathode having a first conductive layer 162 and a second conductive layer 164. Device 100 may be fabricated by depositing the layers described, in order. The properties and functions of these various layers, as well as example materials, are described in more detail in US 7,279,704 at cols. 6-10, which are incorporated by reference.

More examples for each of these layers are available. For example, a flexible and transparent substrate-anode combination is disclosed in U.S. Pat. No. 5,844,363, which is incorporated by reference in its entirety. An example of a p-doped hole transport layer is m-MTDATA doped with F₄-TCNQ at a molar ratio of 50:1, as disclosed in U.S. Patent Application Publication No. 2003/0230980, which is incorporated by reference in its entirety. Examples of emissive and host materials are disclosed in U.S. Pat. No. 6,303,238 to Thompson et al., which is incorporated by reference in its entirety. An example of an n-doped electron transport layer is BPhen doped with Li at a molar ratio of 1:1, as disclosed in U.S. Patent Application Publication No. 2003/0230980, which is incorporated by reference in its entirety. U.S. Pat. Nos. 5,703,436 and 5,707,745, which are incorporated by reference in their entireties, disclose examples of cathodes including compound cathodes having a thin layer of metal such as Mg:Ag with an overlying transparent, electrically-conductive, sputter-deposited ITO layer. The theory and use of blocking layers is described in more detail in U.S. Pat. No. 6,097,147 and U.S. Patent Application Publication No. 2003/0230980, which are incorporated by reference in their entireties. Examples of injection layers are provided in U.S. Patent Application Publication No. 2004/0174116, which is incorporated by reference in its entirety. A description of protective layers may be found in U.S. Patent Application Publication No. 2004/0174116, which is incorporated by reference in its entirety.

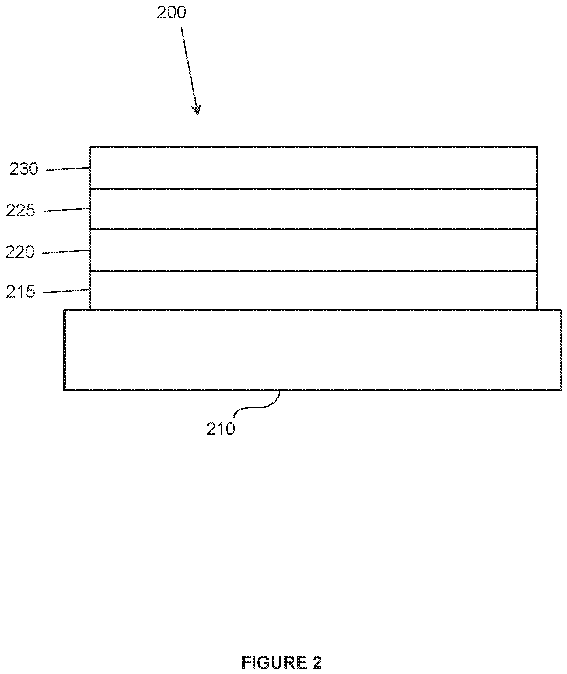

FIG. 2 shows an inverted OLED 200. The device includes a substrate 210, a cathode 215, an emissive layer 220, a hole transport layer 225, and an anode 230. Device 200 may be fabricated by depositing the layers described, in order. Because the most common OLED configuration has a cathode disposed over the anode, and device 200 has cathode 215 disposed under anode 230, device 200 may be referred to as an "inverted" OLED. Materials similar to those described with respect to device 100 may be used in the corresponding layers of device 200. FIG. 2 provides one example of how some layers may be omitted from the structure of device 100.

The simple layered structure illustrated in FIGS. 1 and 2 is provided by way of nonlimiting example, and it is understood that embodiments of the invention may be used in connection with a wide variety of other structures. The specific materials and structures described are exemplary in nature, and other materials and structures may be used. Functional OLEDs may be achieved by combining the various layers described in different ways, or layers may be omitted entirely, based on design, performance, and cost factors. Other layers not specifically described may also be included. Materials other than those specifically described may be used. Although many of the examples provided herein describe various layers as comprising a single material, it is understood that combinations of materials, such as a mixture of host and dopant, or more generally a mixture, may be used. Also, the layers may have various sublayers. The names given to the various layers herein are not intended to be strictly limiting. For example, in device 200, hole transport layer 225 transports holes and injects holes into emissive layer 220, and may be described as a hole transport layer or a hole injection layer. In one embodiment, an OLED may be described as having an "organic layer" disposed between a cathode and an anode. This organic layer may comprise a single layer, or may further comprise multiple layers of different organic materials as described, for example, with respect to FIGS. 1 and 2.

Structures and materials not specifically described may also be used, such as OLEDs comprised of polymeric materials (PLEDs) such as disclosed in U.S. Pat. No. 5,247,190 to Friend et al., which is incorporated by reference in its entirety. By way of further example, OLEDs having a single organic layer may be used. OLEDs may be stacked, for example as described in U.S. Pat. No. 5,707,745 to Forrest et al, which is incorporated by reference in its entirety. The OLED structure may deviate from the simple layered structure illustrated in FIGS. 1 and 2. For example, the substrate may include an angled reflective surface to improve out-coupling, such as a mesa structure as described in U.S. Pat. No. 6,091,195 to Forrest et al., and/or a pit structure as described in U.S. Pat. No. 5,834,893 to Bulovic et al., which are incorporated by reference in their entireties.

Unless otherwise specified, any of the layers of the various embodiments may be deposited by any suitable method. For the organic layers, preferred methods include thermal evaporation, ink-jet, such as described in U.S. Pat. Nos. 6,013,982 and 6,087,196, which are incorporated by reference in their entireties, organic vapor phase deposition (OVPD), such as described in U.S. Pat. No. 6,337,102 to Forrest et al., which is incorporated by reference in its entirety, and deposition by organic vapor jet printing (OVJP), such as described in U.S. Pat. No. 7,431,968, which is incorporated by reference in its entirety. Other suitable deposition methods include spin coating and other solution based processes. Solution based processes are preferably carried out in nitrogen or an inert atmosphere. For the other layers, preferred methods include thermal evaporation. Preferred patterning methods include deposition through a mask, cold welding such as described in U.S. Pat. Nos. 6,294,398 and 6,468,819, which are incorporated by reference in their entireties, and patterning associated with some of the deposition methods such as ink-jet and organic vapor jet printing (OVJP). Other methods may also be used. The materials to be deposited may be modified to make them compatible with a particular deposition method. For example, substituents such as alkyl and aryl groups, branched or unbranched, and preferably containing at least 3 carbons, may be used in small molecules to enhance their ability to undergo solution processing. Substituents having 20 carbons or more may be used, and 3-20 carbons is a preferred range. Materials with asymmetric structures may have better solution processability than those having symmetric structures, because asymmetric materials may have a lower tendency to recrystallize. Dendrimer substituents may be used to enhance the ability of small molecules to undergo solution processing.

Devices fabricated in accordance with embodiments of the present invention may further optionally comprise a barrier layer. One purpose of the barrier layer is to protect the electrodes and organic layers from damaging exposure to harmful species in the environment including moisture, vapor and/or gases, etc. The barrier layer may be deposited over, under or next to a substrate, an electrode, or over any other parts of a device including an edge. The barrier layer may comprise a single layer, or multiple layers. The barrier layer may be formed by various known chemical vapor deposition techniques and may include compositions having a single phase as well as compositions having multiple phases. Any suitable material or combination of materials may be used for the barrier layer. The barrier layer may incorporate an inorganic or an organic compound or both. The preferred barrier layer comprises a mixture of a polymeric material and a non-polymeric material as described in U.S. Pat. No. 7,968,146, PCT Pat. Application Nos. PCT/US2007/023098 and PCT/US2009/042829, which are herein incorporated by reference in their entireties. To be considered a "mixture", the aforesaid polymeric and non-polymeric materials comprising the barrier layer should be deposited under the same reaction conditions and/or at the same time. The weight ratio of polymeric to non-polymeric material may be in the range of 95:5 to 5:95. The polymeric material and the non-polymeric material may be created from the same precursor material. In one example, the mixture of a polymeric material and a non-polymeric material consists essentially of polymeric silicon and inorganic silicon.

Devices fabricated in accordance with embodiments of the invention can be incorporated into a wide variety of electronic component modules (or units) that can be incorporated into a variety of electronic products or intermediate components. Examples of such electronic products or intermediate components include display screens, lighting devices such as discrete light source devices or lighting panels, etc. that can be utilized by the end-user product manufacturers. Such electronic component modules can optionally include the driving electronics and/or power source(s). Devices fabricated in accordance with embodiments of the invention can be incorporated into a wide variety of consumer products that have one or more of the electronic component modules (or units) incorporated therein. A consumer product comprising an OLED that includes the compound of the present disclosure in the organic layer in the OLED is disclosed. Such consumer products would include any kind of products that include one or more light source(s) and/or one or more of some type of visual displays. Some examples of such consumer products include flat panel displays, curved displays, computer monitors, medical monitors, televisions, billboards, lights for interior or exterior illumination and/or signaling, heads-up displays, fully or partially transparent displays, flexible displays, rollable displays, foldable displays, stretchable displays, laser printers, telephones, mobile phones, tablets, phablets, personal digital assistants (PDAs), wearable devices, laptop computers, digital cameras, camcorders, viewfinders, micro-displays (displays that are less than 2 inches diagonal), 3-D displays, virtual reality or augmented reality displays, vehicles, video walls comprising multiple displays tiled together, theater or stadium screen, a light therapy device, and a sign. Various control mechanisms may be used to control devices fabricated in accordance with the present invention, including passive matrix and active matrix. Many of the devices are intended for use in a temperature range comfortable to humans, such as 18 degrees C. to 30 degrees C., and more preferably at room temperature (20-25 degrees C), but could be used outside this temperature range, for example, from -40 degree C to + 80 degree C.

The materials and structures described herein may have applications in devices other than OLEDs. For example, other optoelectronic devices such as organic solar cells and organic photodetectors may employ the materials and structures. More generally, organic devices, such as organic transistors, may employ the materials and structures.

The terms "halo," "halogen," and "halide" are used interchangeably and refer to fluorine, chlorine, bromine, and iodine.

The term "acyl" refers to a substituted carbonyl radical (C(O)-Rₛ).

The term "ester" refers to a substituted oxycarbonyl (-O-C(O)-Rₛ or -C(O)-O-Rₛ) radical.

The term "ether" refers to an -ORₛ radical.

The terms "sulfanyl" or "thio-ether" are used interchangeably and refer to a -SRₛ radical.

The term "sulfinyl" refers to a -S(O)-Rₛ radical.

The term "sulfonyl" refers to a -SO₂-Rₛ radical.

The term "phosphino" refers to a -P(Rₛ)₃ radical, wherein each Rₛ can be same or different.

The term "silyl" refers to a -Si(Rₛ)₃ radical, wherein each Rₛ can be same or different.

In each of the above, Rₛ can be hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, and combination thereof. Preferred Rₛ is selected from the group consisting of alkyl, cycloalkyl, aryl, heteroaryl, and combination thereof.

The term "alkyl" refers to and includes both straight and branched chain alkyl radicals. Preferred alkyl groups are those containing from one to fifteen carbon atoms and includes methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl, and the like. Additionally, the alkyl group is optionally substituted.

The term "cycloalkyl" refers to and includes monocyclic, polycyclic, and spiro alkyl radicals. Preferred cycloalkyl groups are those containing 3 to 12 ring carbon atoms and includes cyclopropyl, cyclopentyl, cyclohexyl, bicyclo[3.1.1]heptyl, spiro[4.5]decyl, spiro[5.5]undecyl, adamantyl, and the like. Additionally, the cycloalkyl group is optionally substituted.

The terms "heteroalkyl" or "heterocycloalkyl" refer to an alkyl or a cycloalkyl radical, respectively, having at least one carbon atom replaced by a heteroatom. Optionally the at least one heteroatom is selected from O, S, N, P, B, Si and Se, preferably, O, S or N. Additionally, the heteroalkyl or heterocycloalkyl group is optionally substituted.

The term "alkenyl" refers to and includes both straight and branched chain alkene radicals. Alkenyl groups are essentially alkyl groups that include at least one carbon-carbon double bond in the alkyl chain. Cycloalkenyl groups are essentially cycloalkyl groups that include at least one carbon-carbon double bond in the cycloalkyl ring. The term "heteroalkenyl" as used herein refers to an alkenyl radical having at least one carbon atom replaced by a heteroatom. Optionally the at least one heteroatom is selected from O, S, N, P, B, Si, and Se, preferably, O, S, or N. Preferred alkenyl, cycloalkenyl, or heteroalkenyl groups are those containing two to fifteen carbon atoms. Additionally, the alkenyl, cycloalkenyl, or heteroalkenyl group is optionally substituted.

The term "alkynyl" refers to and includes both straight and branched chain alkyne radicals. Preferred alkynyl groups are those containing two to fifteen carbon atoms. Additionally, the alkynyl group is optionally substituted.

The terms "aralkyl" or "arylalkyl" are used interchangeably and refer to an alkyl group that is substituted with an aryl group. Additionally, the aralkyl group is optionally substituted.

The term "heterocyclic group" refers to and includes aromatic and non-aromatic cyclic radicals containing at least one heteroatom. Optionally the at least one heteroatom is selected from O, S, N, P, B, Si, and Se, preferably, O, S, or N. Hetero-aromatic cyclic radicals may be used interchangeably with heteroaryl. Preferred hetero-non-aromatic cyclic groups are those containing 3 to 7 ring atoms which includes at least one hetero atom, and includes cyclic amines such as morpholino, piperidino, pyrrolidino, and the like, and cyclic ethers/thio-ethers, such as tetrahydrofuran, tetrahydropyran, tetrahydrothiophene, and the like. Additionally, the heterocyclic group may be optionally substituted.

The term "aryl" refers to and includes both single-ring aromatic hydrocarbyl groups and polycyclic aromatic ring systems. The polycyclic rings may have two or more rings in which two carbons are common to two adjoining rings (the rings are "fused") wherein at least one of the rings is an aromatic hydrocarbyl group, e.g., the other rings can be cycloalkyls, cycloalkenyls, aryl, heterocycles, and/or heteroaryls. Preferred aryl groups are those containing six to thirty carbon atoms, preferably six to twenty carbon atoms, more preferably six to twelve carbon atoms. Especially preferred is an aryl group having six carbons, ten carbons or twelve carbons. Suitable aryl groups include phenyl, biphenyl, triphenyl, triphenylene, tetraphenylene, naphthalene, anthracene, phenalene, phenanthrene, fluorene, pyrene, chrysene, perylene, and azulene, preferably phenyl, biphenyl, triphenyl, triphenylene, fluorene, and naphthalene. Additionally, the aryl group is optionally substituted.

The term "heteroaryl" refers to and includes both single-ring aromatic groups and polycyclic aromatic ring systems that include at least one heteroatom. The heteroatoms include, but are not limited to O, S, N, P, B, Si, and Se. In many instances, O, S, or N are the preferred heteroatoms. Hetero-single ring aromatic systems are preferably single rings with 5 or 6 ring atoms, and the ring can have from one to six heteroatoms. The hetero-polycyclic ring systems can have two or more rings in which two atoms are common to two adjoining rings (the rings are "fused") wherein at least one of the rings is a heteroaryl, e.g., the other rings can be cycloalkyls, cycloalkenyls, aryl, heterocycles, and/or heteroaryls. The hetero-polycyclic aromatic ring systems can have from one to six heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably dibenzothiophene, dibenzofuran, dibenzoselenophene, carbazole, indolocarbazole, imidazole, pyridine, triazine, benzimidazole, 1,2-azaborine, 1,3-azaborine, 1,4-azaborine, borazine, and aza-analogs thereof. Additionally, the heteroaryl group is optionally substituted.

Of the aryl and heteroaryl groups listed above, the groups of triphenylene, naphthalene, anthracene, dibenzothiophene, dibenzofuran, dibenzoselenophene, carbazole, indolocarbazole, imidazole, pyridine, pyrazine, pyrimidine, triazine, and benzimidazole, and the respective aza-analogs of each thereof are of particular interest.

The terms alkyl, cycloalkyl, heteroalkyl, heterocycloalkyl, alkenyl, cycloalkenyl, heteroalkenyl, alkynyl, aralkyl, heterocyclic group, aryl, and heteroaryl, as used herein, are independently unsubstituted, or independently substituted, with one or more general substituents.

In many instances, the general substituents are selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof.

In some instances, the preferred general substituents are selected from the group consisting of deuterium, fluorine, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof.

In some instances, the preferred general substituents are selected from the group consisting of deuterium, fluorine, alkyl, cycloalkyl, alkoxy, aryloxy, amino, silyl, aryl, heteroaryl, sulfanyl, and combinations thereof.

In yet other instances, the more preferred general substituents are selected from the group consisting of deuterium, fluorine, alkyl, cycloalkyl, aryl, heteroaryl, and combinations thereof.

The terms "substituted" and "substitution" refer to a substituent other than H that is bonded to the relevant position, e.g., a carbon or nitrogen. For example, when R¹ represents monosubstitution, then one R¹ must be other than H (i.e., a substitution). Similarly, when R¹ represents disubstitution, then two of R¹ must be other than H. Similarly, when R¹ represents no substitution, R¹, for example, can be a hydrogen for available valencies of ring atoms, as in carbon atoms for benzene and the nitrogen atom in pyrrole, or simply represents nothing for ring atoms with fully filled valencies, e.g., the nitrogen atom in pyridine. The maximum number of substitutions possible in a ring structure will depend on the total number of available valencies in the ring atoms.

As used herein, "combinations thereof' indicates that one or more members of the applicable list are combined to form a known or chemically stable arrangement that one of ordinary skill in the art can envision from the applicable list. For example, an alkyl and deuterium can be combined to form a partial or fully deuterated alkyl group; a halogen and alkyl can be combined to form a halogenated alkyl substituent; and a halogen, alkyl, and aryl can be combined to form a halogenated arylalkyl. In one instance, the term substitution includes a combination of two to four of the listed groups. In another instance, the term substitution includes a combination of two to three groups. In yet another instance, the term substitution includes a combination of two groups. Preferred combinations of substituent groups are those that contain up to fifty atoms that are not hydrogen or deuterium, or those which include up to forty atoms that are not hydrogen or deuterium, or those that include up to thirty atoms that are not hydrogen or deuterium. In many instances, a preferred combination of substituent groups will include up to twenty atoms that are not hydrogen or deuterium.

The "aza" designation in the fragments described herein, i.e. aza-dibenzofuran, aza-dibenzothiophene, etc. means that one or more of the C-H groups in the respective aromatic ring can be replaced by a nitrogen atom, for example, and without any limitation, azatriphenylene encompasses both dibenzo[*ƒ*,*h*]quinoxaline and dibenzo[*ƒ*,*h*]quinoline. One of ordinary skill in the art can readily envision other nitrogen analogs of the aza-derivatives described above, and all such analogs are intended to be encompassed by the terms as set forth herein.

As used herein, "deuterium" refers to an isotope of hydrogen. Deuterated compounds can be readily prepared using methods known in the art. For example, U.S. Pat. No. 8,557,400, Patent Pub. No. WO 2006/095951, and U.S. Pat. Application Pub. No. US 2011/0037057, which are hereby incorporated by reference in their entireties, describe the making of deuterium-substituted organometallic complexes. Further reference is made to Ming Yan, et al., Tetrahedron 2015, 71, 1425-30 and Atzrodt et al., Angew. Chem. Int. Ed. (Reviews) 2007, 46, 7744-65, which are incorporated by reference in their entireties, describe the deuteration of the methylene hydrogens in benzyl amines and efficient pathways to replace aromatic ring hydrogens with deuterium, respectively.

It is to be understood that when a molecular fragment is described as being a substituent or otherwise attached to another moiety, its name may be written as if it were a fragment (e.g. phenyl, phenylene, naphthyl, dibenzofuryl) or as if it were the whole molecule (e.g. benzene, naphthalene, dibenzofuran). As used herein, these different ways of designating a substituent or attached fragment are considered to be equivalent.

According to an aspect of the present disclosure, a compound comprising a first ligand L_{A} of Formula I 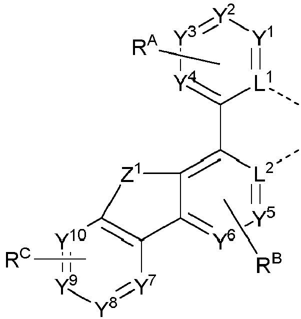 is disclosed. In the structure of Formula I: one of L¹ and L² is C, and the other of L¹ and L² is N;
Y¹ to Y¹⁰ are each independently selected from the group consisting of C and N;
at least two adjacent Y⁷, Y⁸, Y⁹, and Y¹⁰ are carbon atoms that are fused to a structure of Formula II 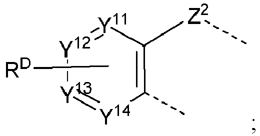
Y¹¹ to Y¹⁴ are each independently selected from the group consisting of C and N;
Z¹ and Z² are each independently selected from the group consisting of O, S, Se, NR, CRR', and SiRR';
R^{A}, R^{B}, and R^{D} represent mono to a maximum possible number of substitutions, or no substitution;
R^{C} represents di-, tri-, or tetra-substitution;
each R, R', R^{A}, R^{B}, R^{C}, and R^{D} is independently hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof;
any two substituents may be joined or fused together to form a ring;
L_{A} is complexed to a metal M by L¹ and L², and M has an atomic weight greater than 40;
M is optionally coordinated to other ligands; and
the ligand L_{A} is optionally linked with other ligands to comprise a tridentate, tetradentate, pentadentate, or hexadentate ligand.

In some embodiments, each R, R', R^{A}, R^{B}, R^{C}, and R^{D} is independently a hydrogen or a substituent selected from the group consisting of deuterium, fluorine, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof. In some embodiments, each R, R', R^{A}, R^{B}, R^{C}, and R^{D} is independently a hydrogen or a substituent selected from the group consisting of deuterium, fluorine, alkyl, cycloalkyl, alkoxy, aryloxy, amino, silyl, aryl, heteroaryl, sulfanyl, and combinations thereof. In other embodiments, each R, R', R^{A}, R^{B}, R^{C}, and R^{D} is independently a hydrogen or a substituent selected from the group consisting of deuterium, fluorine, alkyl, cycloalkyl, aryl, heteroaryl, and combinations thereof.

In some embodiments, M is selected from the group consisting of Ir, Rh, Re, Ru, Os, Pt, Au, and Cu. In some embodiments, M is Ir or Pt.

In some embodiments, the compound is homoleptic. In some embodiments, the compound is heteroleptic.

In some embodiments, Y¹ to Y¹⁴ are each C. In some embodiments, at least one of Y¹ to Y⁴ is N. In some embodiments, at least one of Y¹¹ to Y¹⁴ is N.

In some embodiments, Z¹ is O. In some embodiments, Z² is O. In some embodiments, both Z¹ and Z² are O.

In some embodiments, Z¹ is S. In some embodiments, Z² is S. In some embodiments, both Z¹ and Z² are S.

In some embodiments, the structure of Formula II is fused to Y⁹ and Y¹⁰. In some embodiments, the structure of Formula II is fused to Y⁸ and Y⁹. In some embodiments, the structure of Formula II is fused to Y⁷ and Y⁸.

In some embodiments, Y⁷ to Y¹⁰ are each C.

In some embodiments, L¹ is C and L² is N. In some embodiments, L¹ is N and L² is C.

In some embodiments, Z¹ and Z² are para with respect to one another. In other words, Z² is bonded directly to Y⁸.

In some embodiments, Z¹ and Z² are ortho with respect to one another. In other words, Z² is bonded directly to Y¹⁰.

In some embodiments, Z² is bonded directly to Y⁹ is a first meta orientation. In some embodiments, Z² is bonded directly to Y⁷ is a second meta orientation.

In some embodiments, the first ligand L_{A} is selected from the group consisting of:

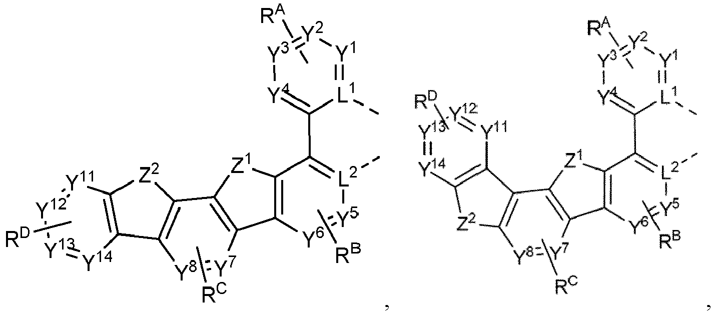

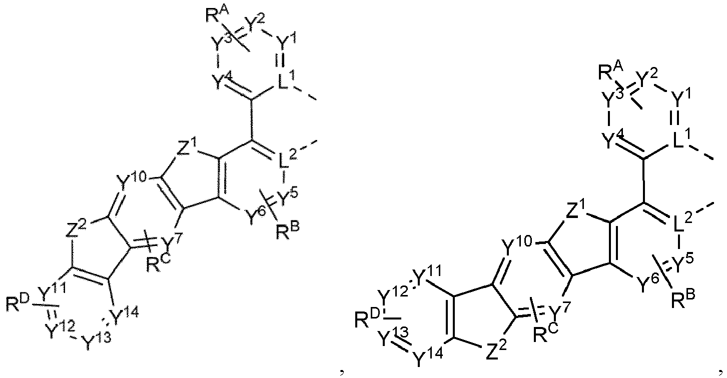

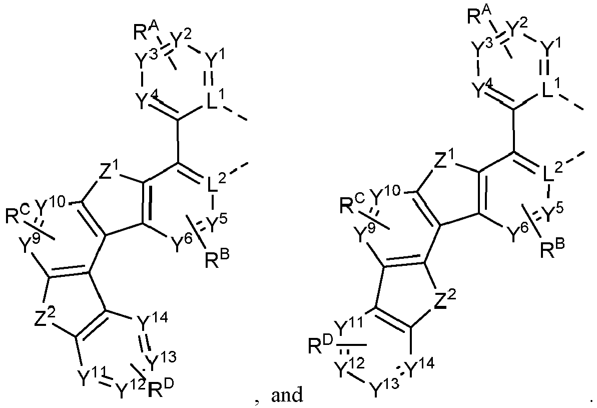

In some embodiments, the first ligand L_{A} is selected from the group consisting of: 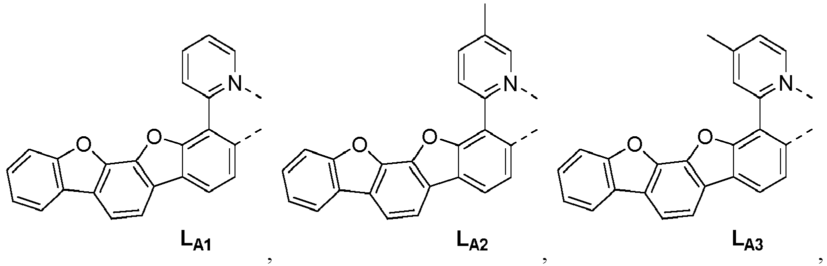 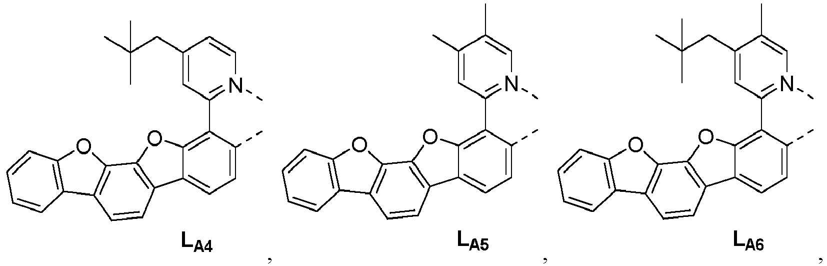 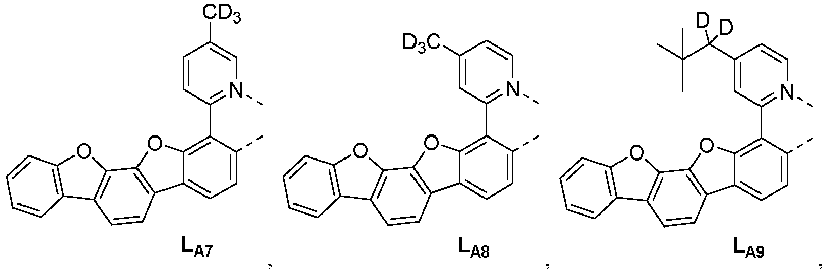 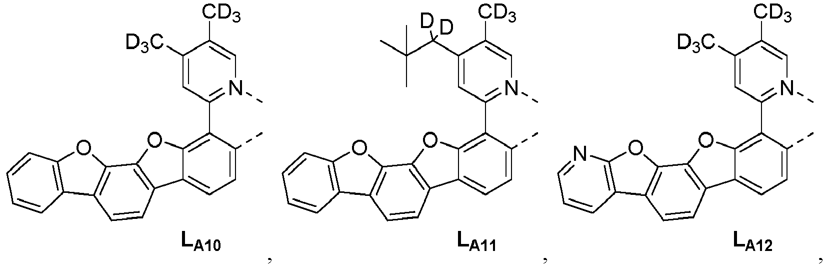 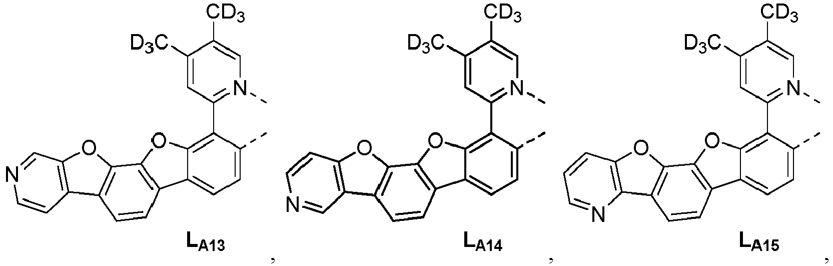 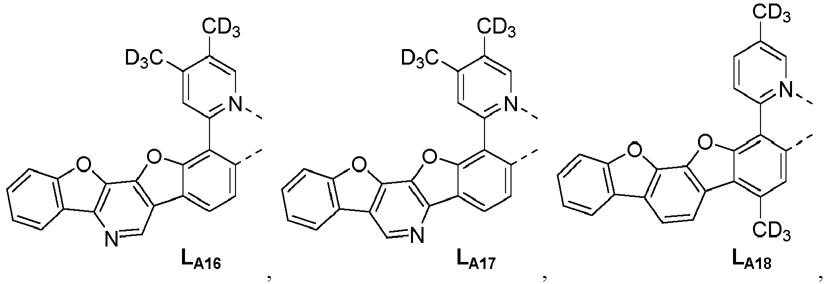 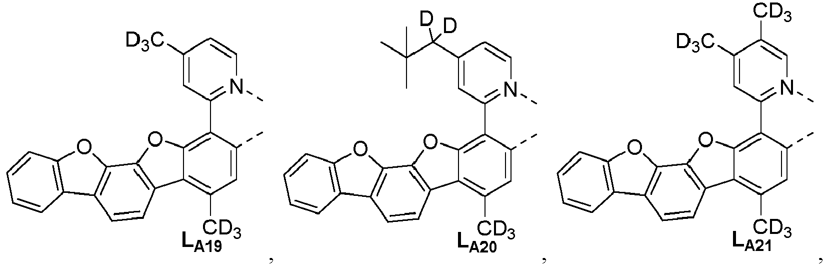 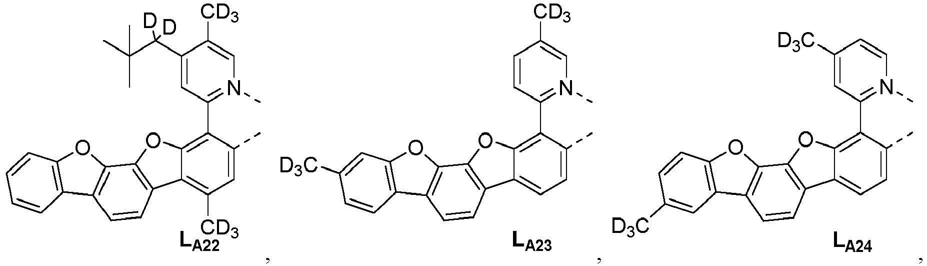 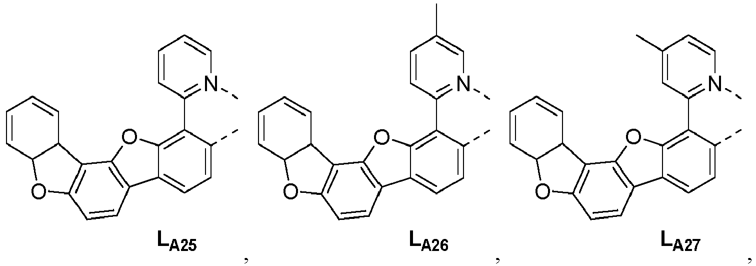 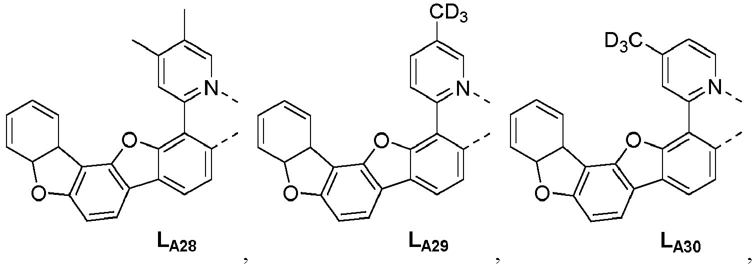 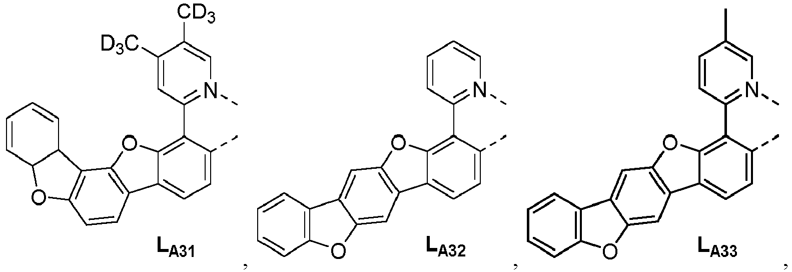 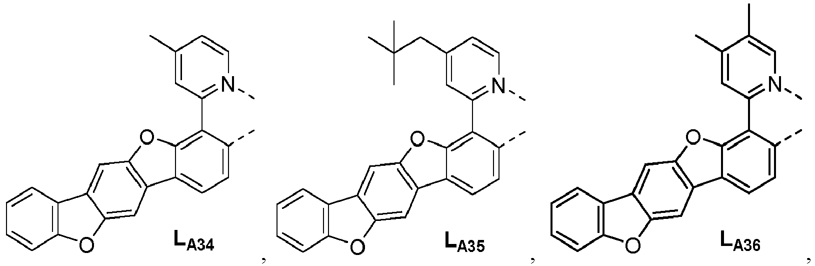 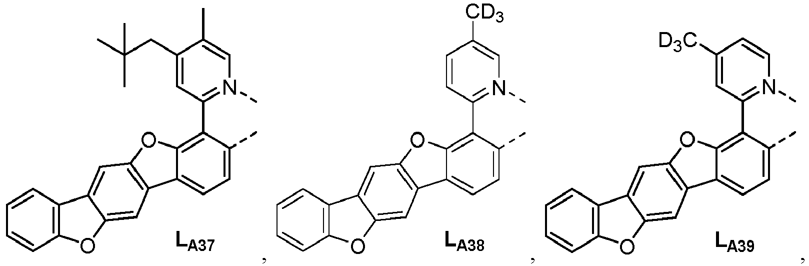 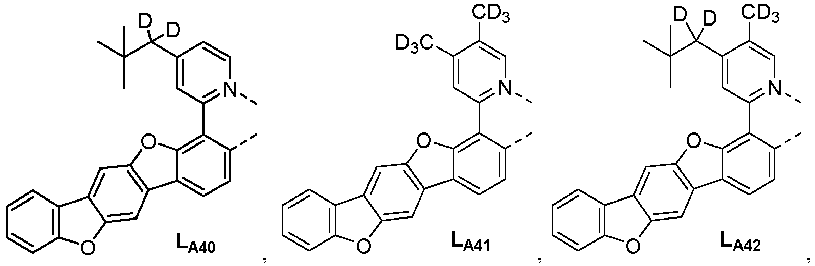 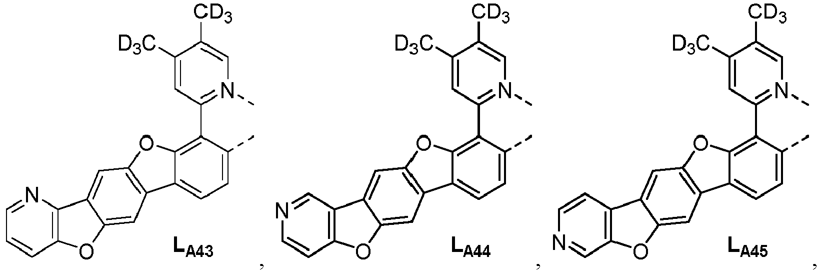 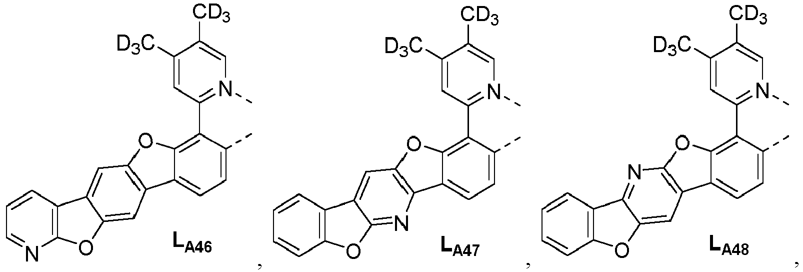 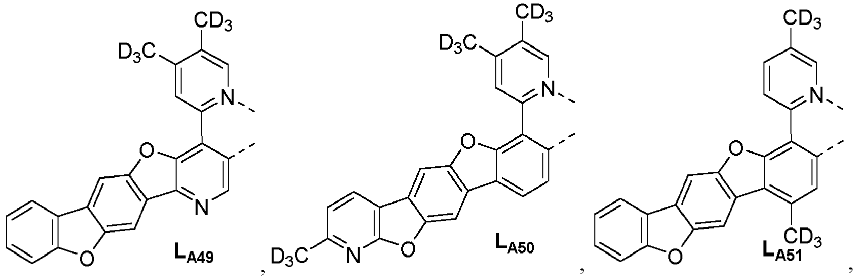 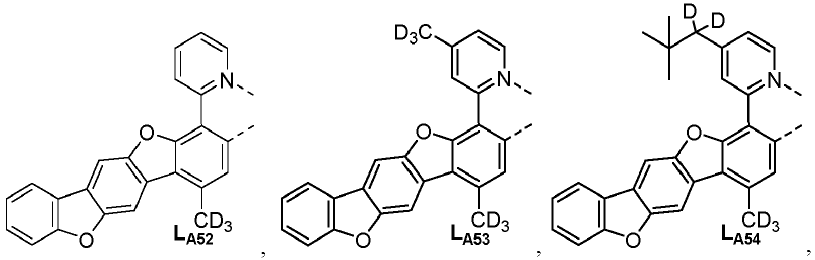 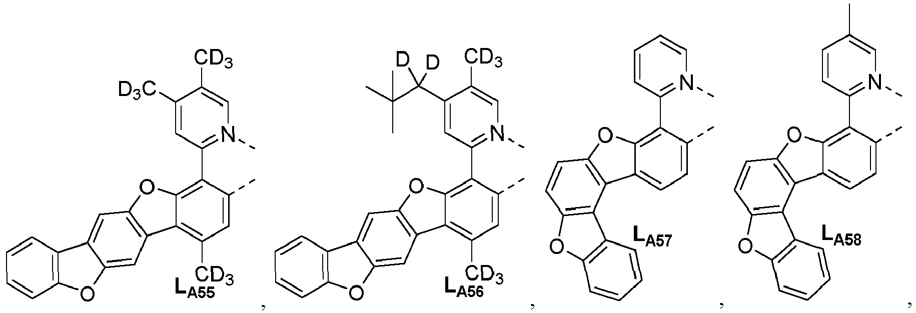 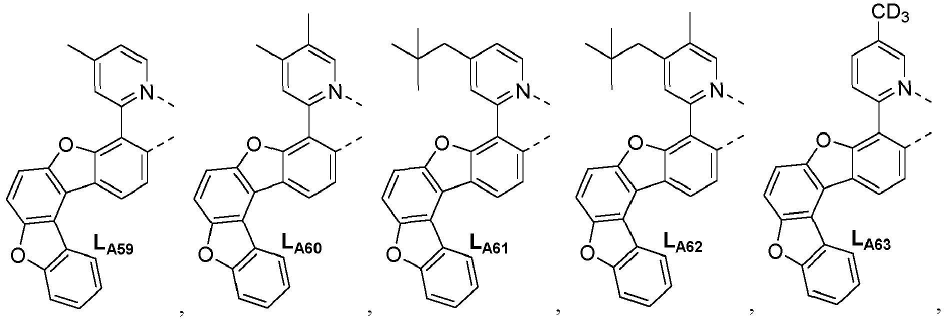 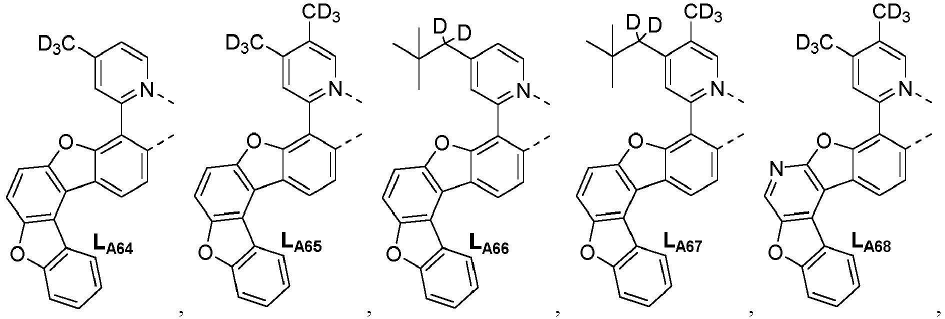 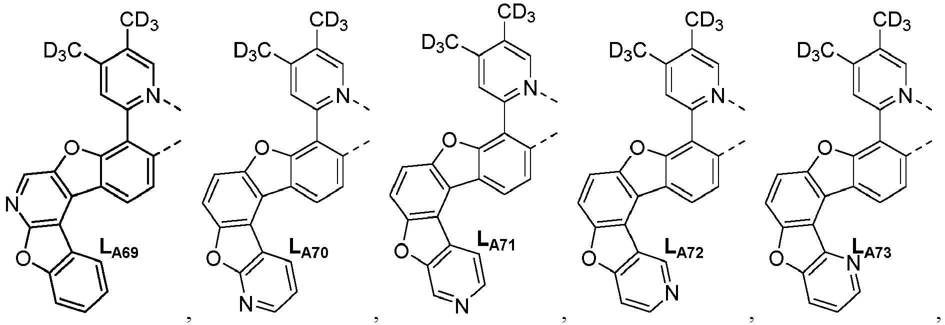 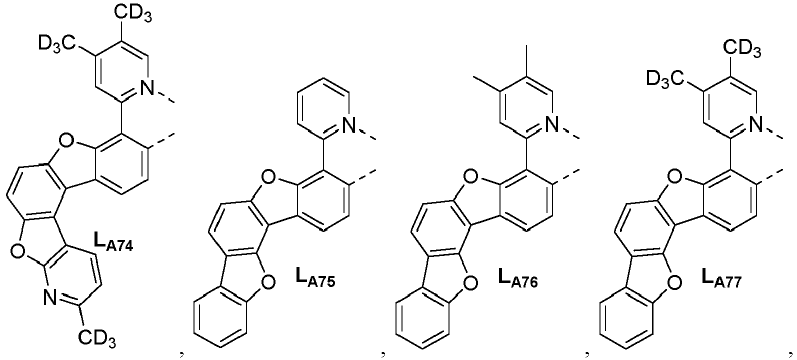 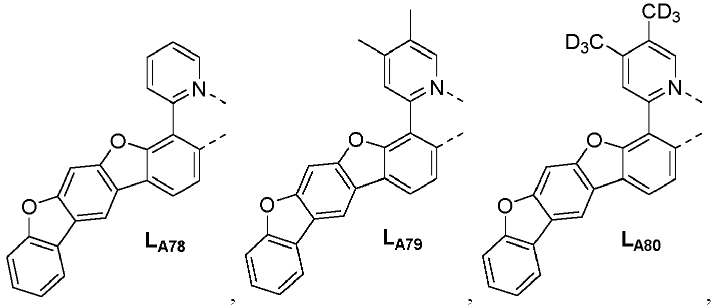 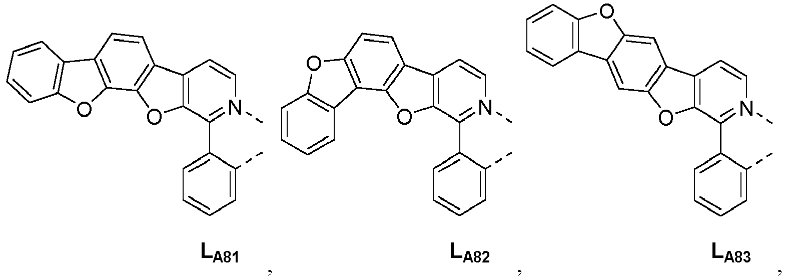 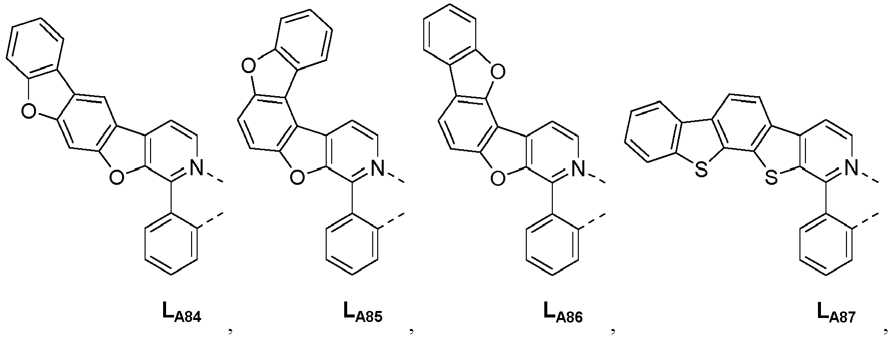 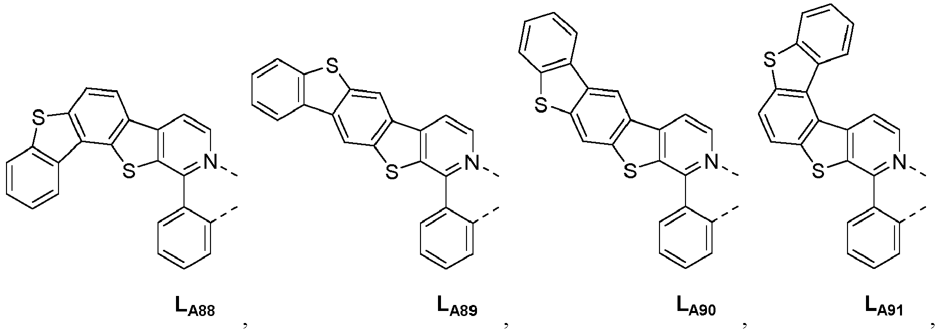 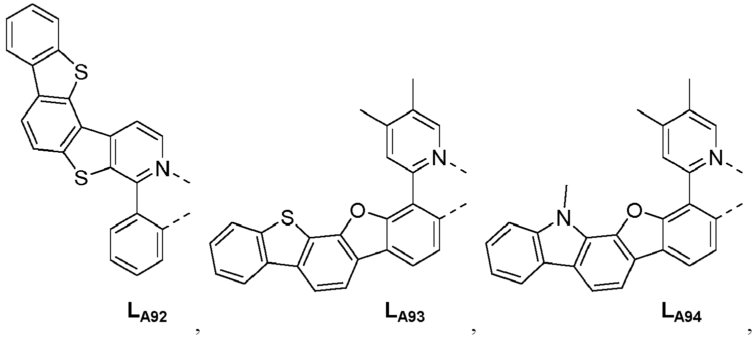 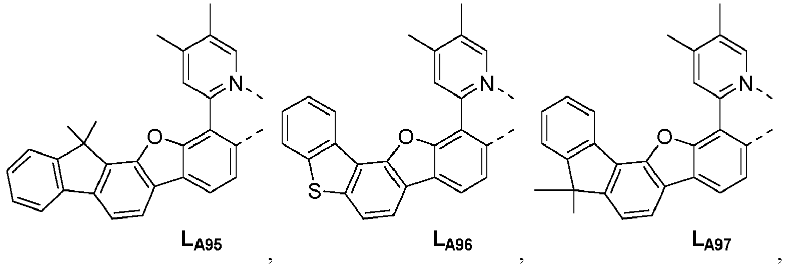 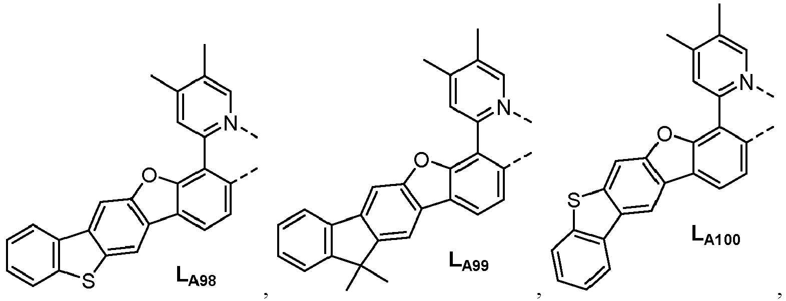 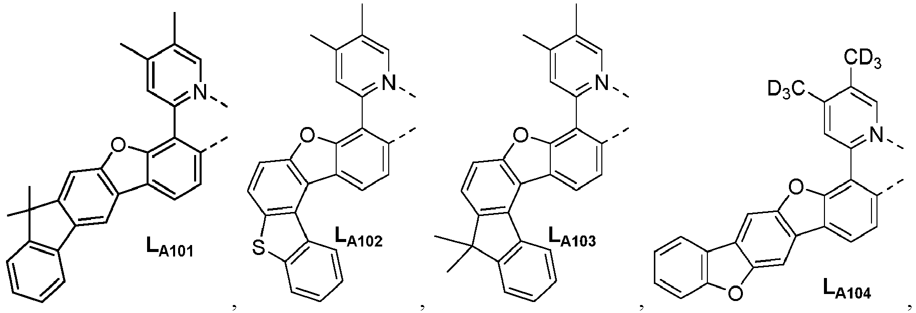 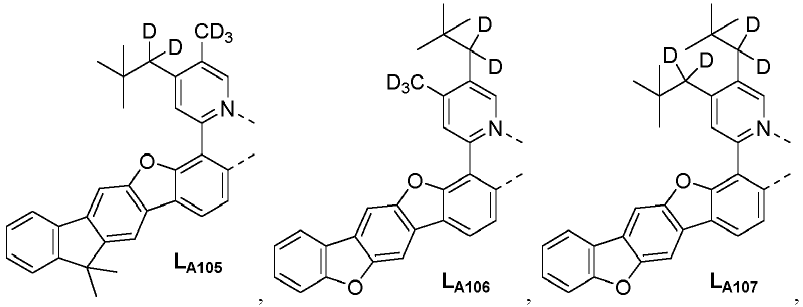 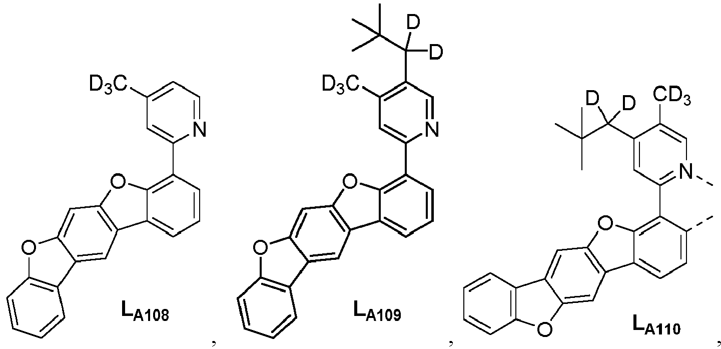 and

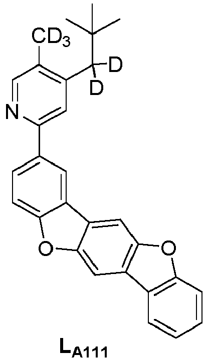

In some embodiments, the compound has a formula of M(L_{A})ₓ(L_{B})_{y}(L_{C})_{z} wherein L_{B} and L_{C} are each a different bidentate ligand; and wherein x is 1, 2, or 3; y is 0, 1, or 2; z is 0, 1, or 2; and x+y+z is the oxidation state of the metal M.

In some embodiments of formula of M(L_{A})ₓ(L_{B})_{y}(L_{C})_{z}, the compound has a formula selected from the group consisting of Ir(L_{A})₃, Ir(L_{A})(L_{B})₂, Ir(L_{A})2(L_{B}), Ir(L_{A})₂(L_{C}), and Ir(L_{A})(L_{B})(L_{C}); and wherein L_{A}, L_{B}, and L_{C} are different from each other.

In some embodiments of formula of M(L_{A})ₓ(L_{B})_{y}(L_{C})_{z}, the compound has a formula of Pt(L_{A})(L_{B}); and wherein L_{A} and L_{B} can be same or different.

In some embodiments of formula of M(L_{A})ₓ(L_{B})_{y}(L_{C})_{z}, ligands L_{A} and L_{B} are connected to form a tetradentate ligand.

In some embodiments of formula of M(L_{A})ₓ(L_{B})_{y}(L_{C})_{z}, ligands L_{A} and L_{B} are connected at two places to form a macrocyclic tetradentate ligand.

In some embodiments of formula of M(L_{A})ₓ(L_{B})_{y}(L_{C})_{z}, ligands L_{B} and L_{C} are each independently selected from the group consisting of: 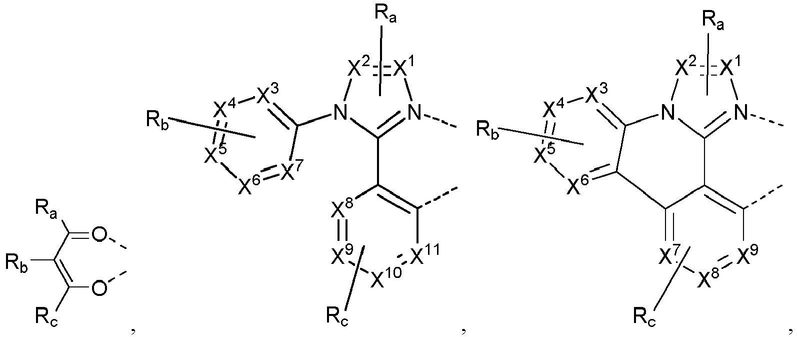 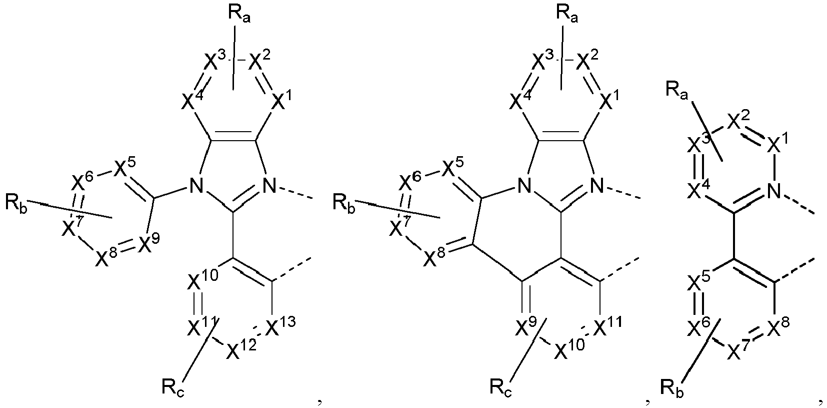 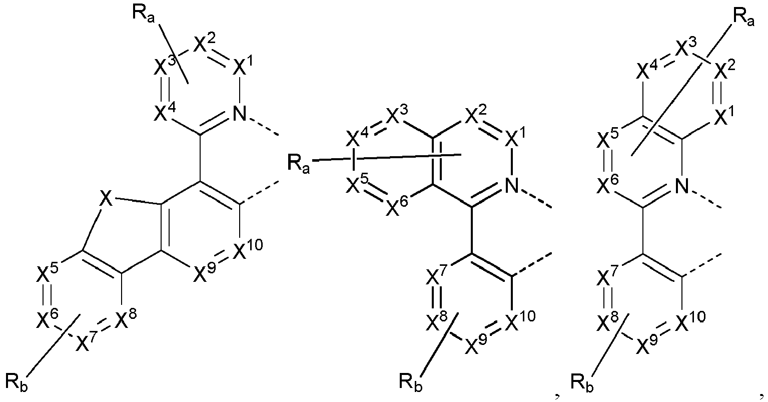 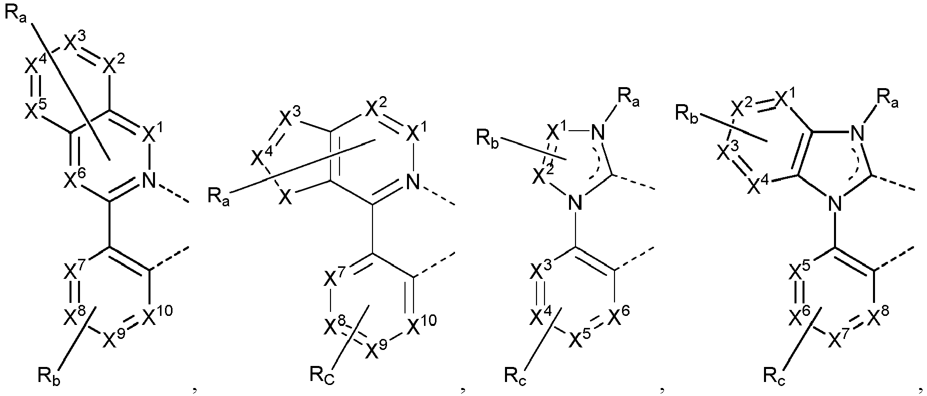 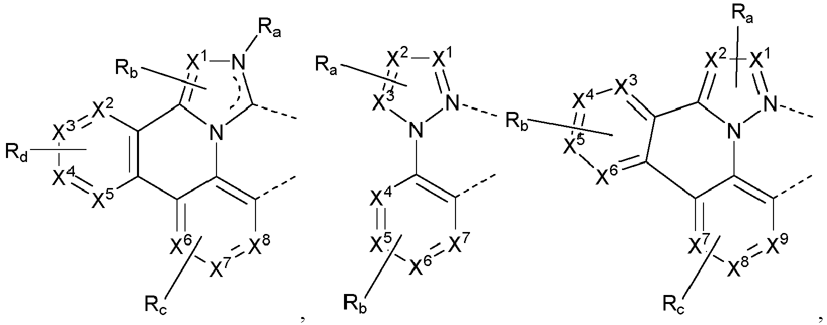 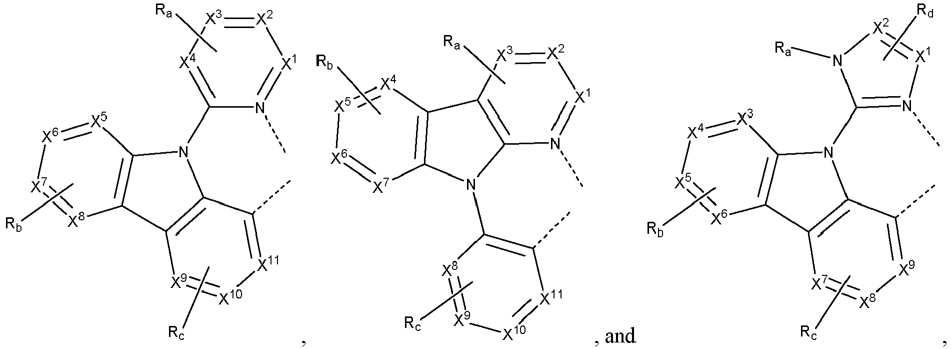 where:
each X¹ to X¹³ is independently selected from the group consisting of carbon and nitrogen;
X is selected from the group consisting of BR', NR', PR', O, S, Se, C=O, S=O, SO₂, CR'R", SiR'R", and GeR'R";
R' and R" are optionally fused or joined to form a ring;
each Rₐ, R_{b}, R_{c}, and R_{d} represents from mono substitution to a maximum possible number of substitutions, or no substitution;
R', R", Rₐ, R_{b}, R_{c}, and R_{d} are each independently a hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof; and
any two adjacent substituents of Rₐ, R_{b}, R_{c}, and R_{d} are optionally fused or joined to form a ring or form a multidentate ligand.

In some embodiments of formula of M(L_{A})ₓ(L_{B})_{y}(L_{C})_{z}, ligands L_{B} and L_{C} are each independently selected from the group consisting of:

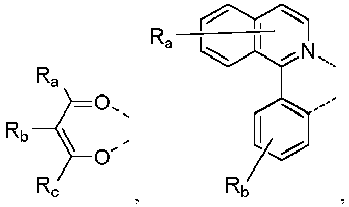

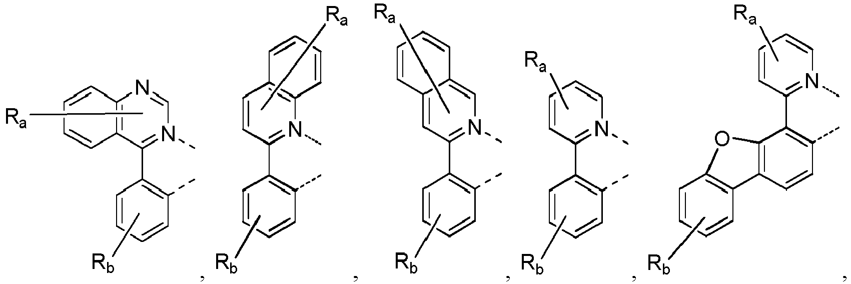

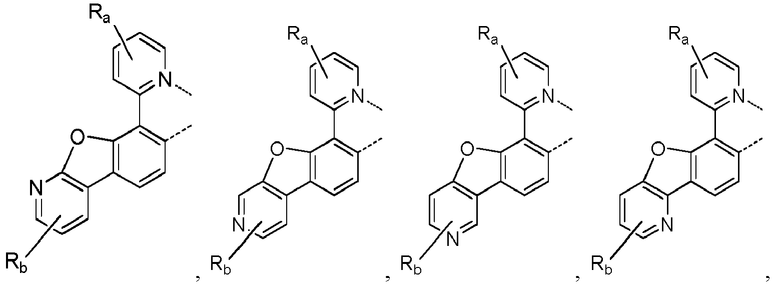

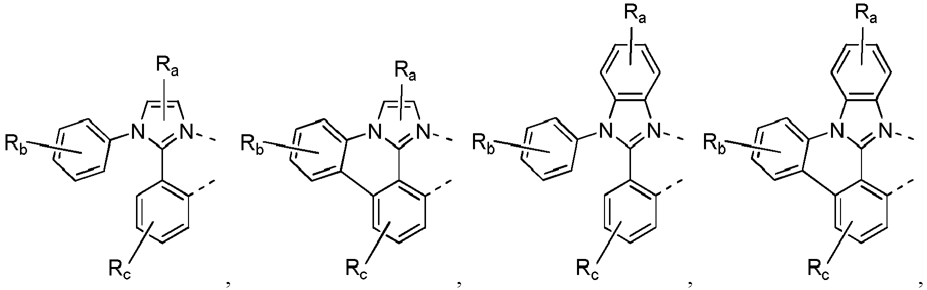

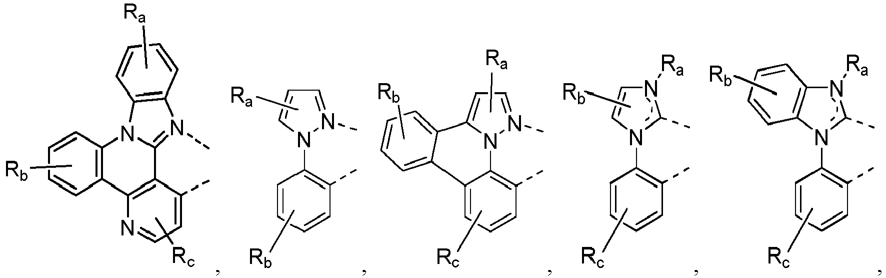

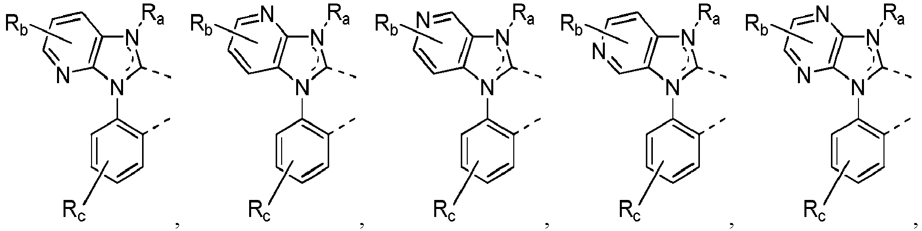

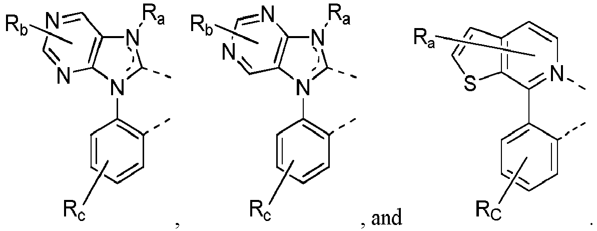

In some embodiments, the compound is Compound A*x* having the formula Ir(L_{A*i*})₃, Compound B*y* having the formula Ir(L_{A*i*})(L_{B*k*})₂, or Compound C*z* having the formula Ir(L_{A*i*})₂(L_{C*j*}). In Compounds Ax, By, and Cz, *x* = *i, y* = 468*i*+*k*-468, and *z* = 1260+*j*-1260. In Compounds Ax, By, and Cz, *i* is an integer from 1 to 111, *k* is an integer from 1 to 468, and *j* is an integer from 1 to 25. In Compounds Ax, By, and Cz, ligand L_{B*k*} has the following structures: 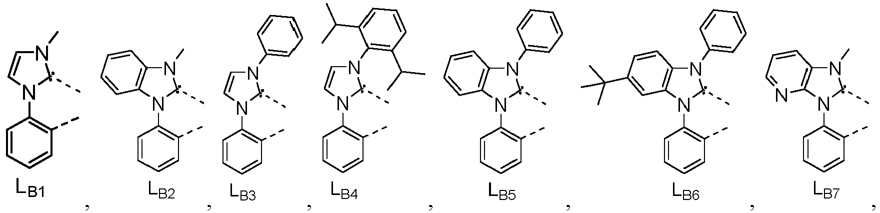 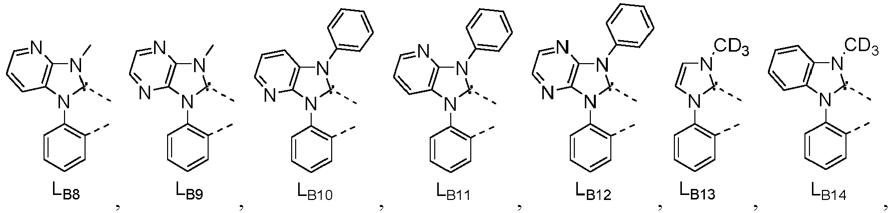 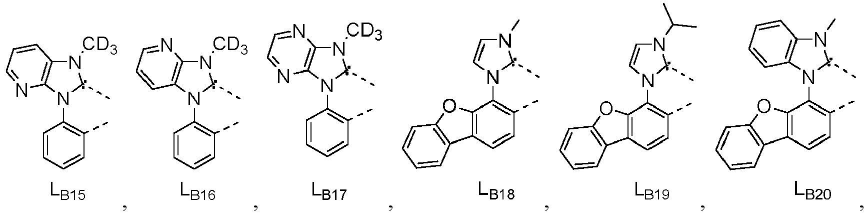 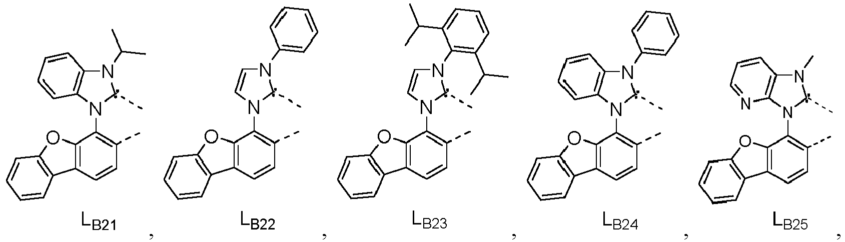 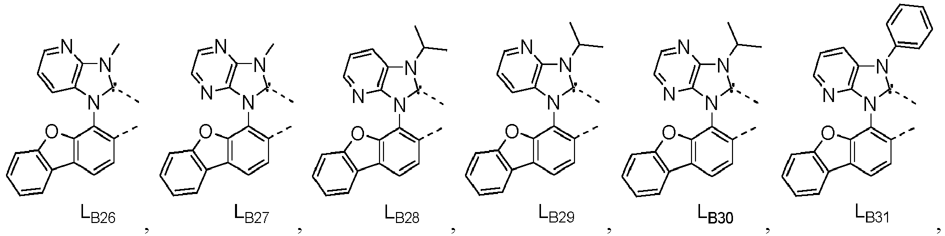 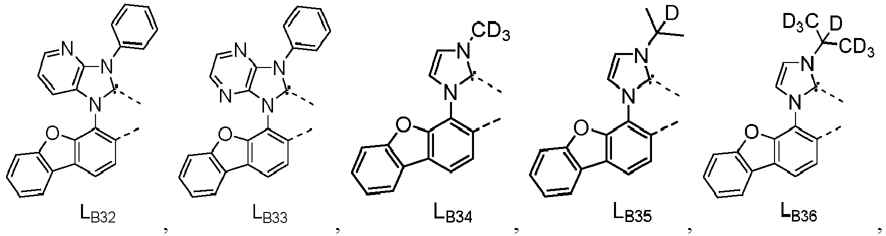 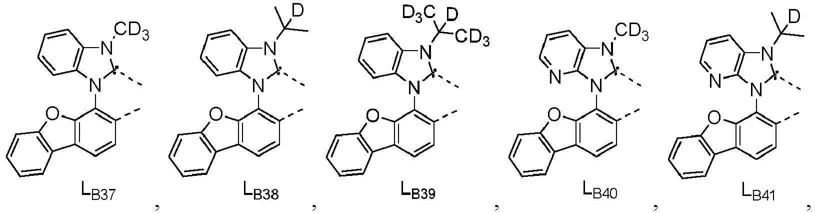 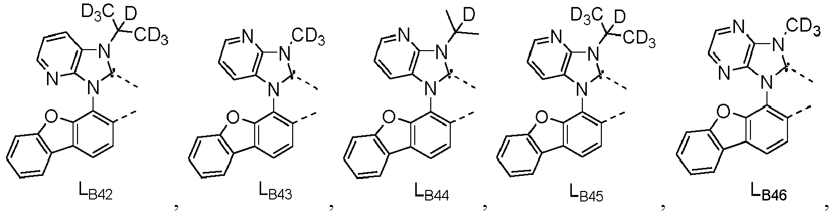 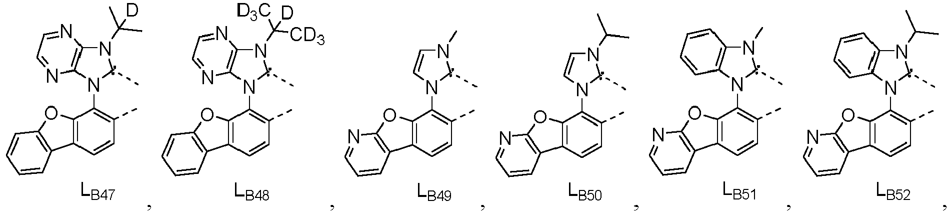 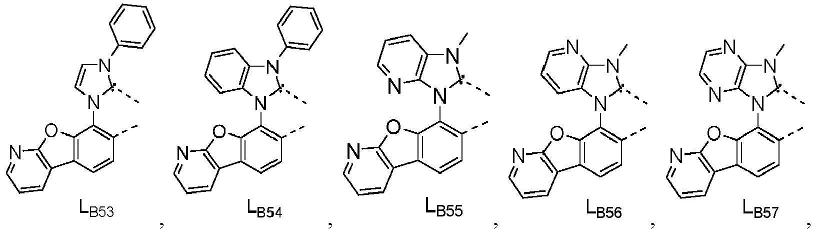 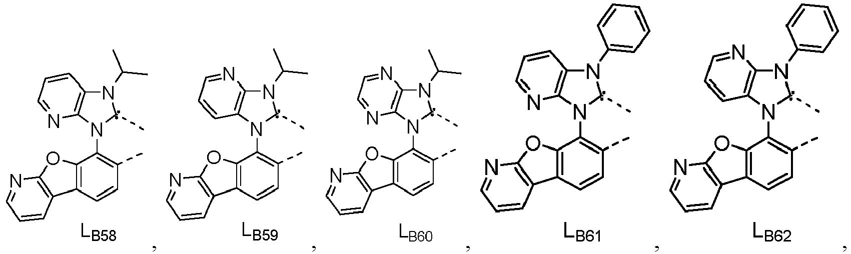 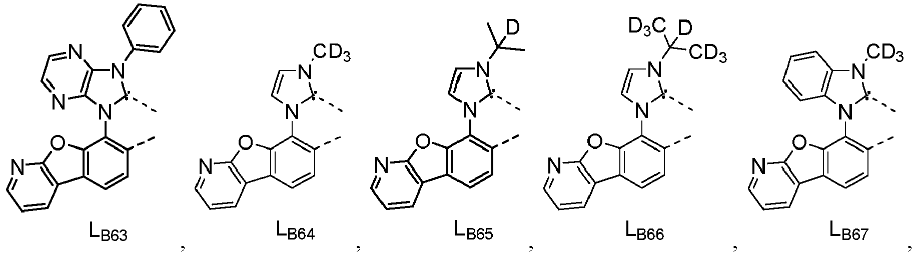 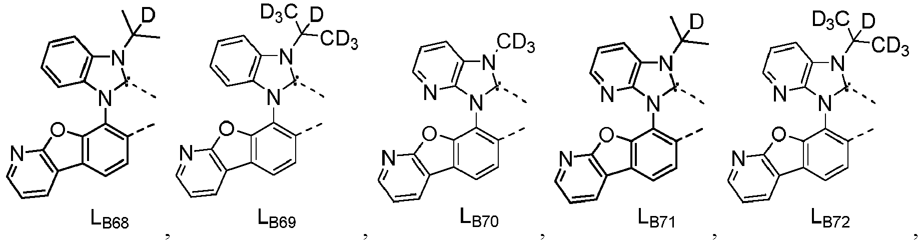 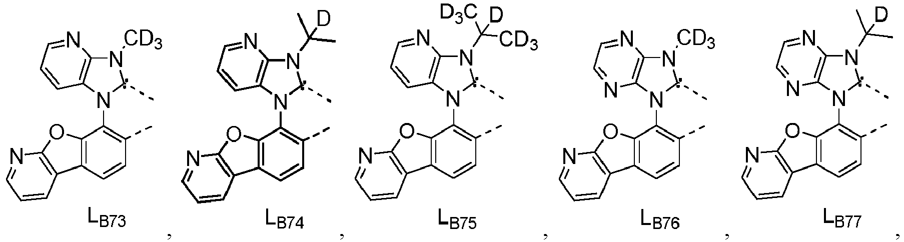 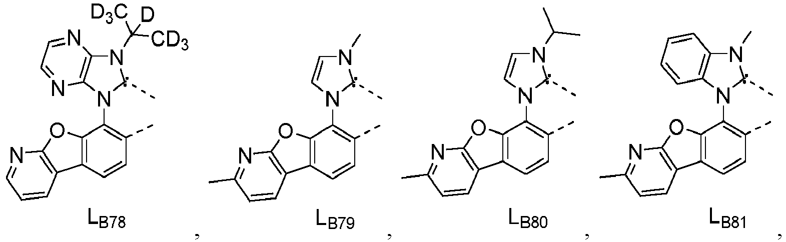 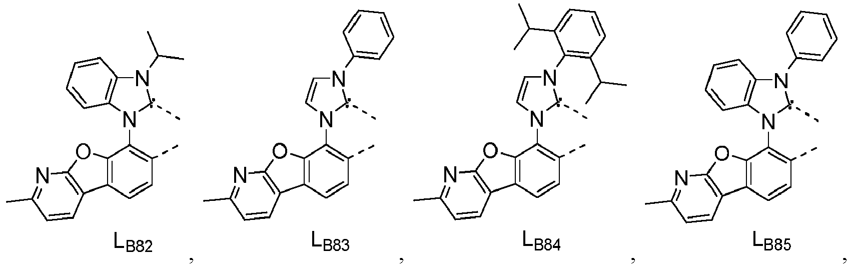 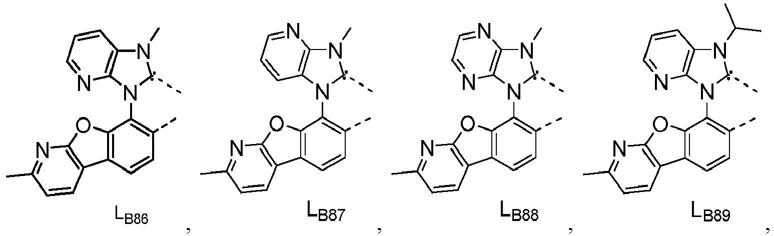 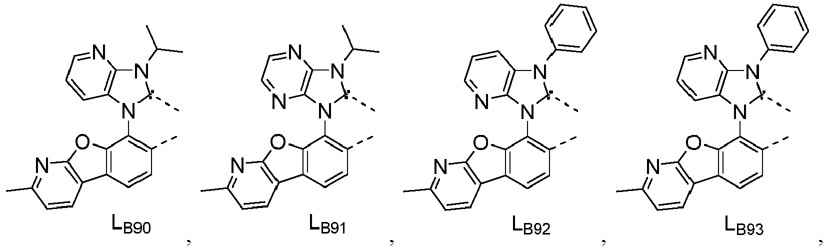 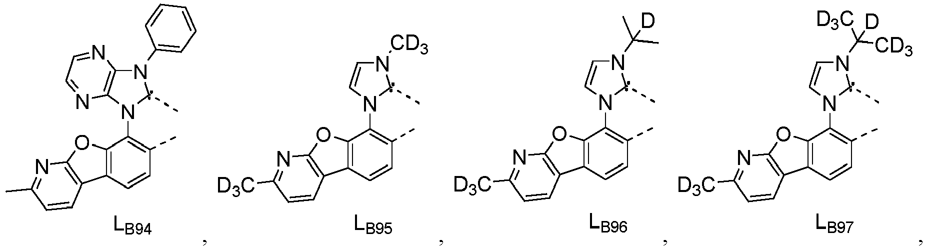 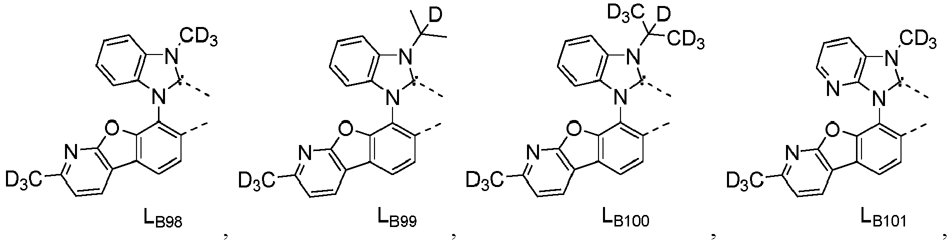 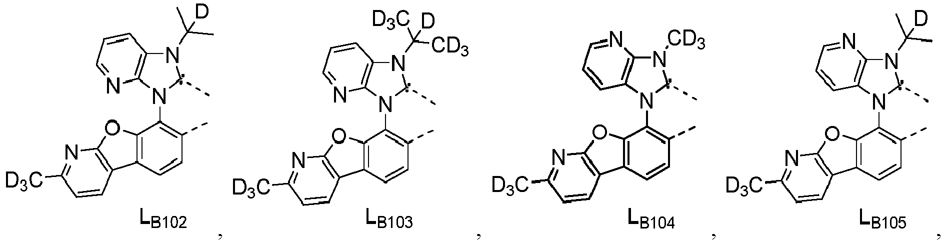 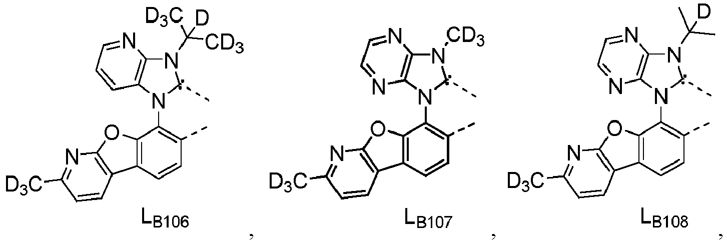 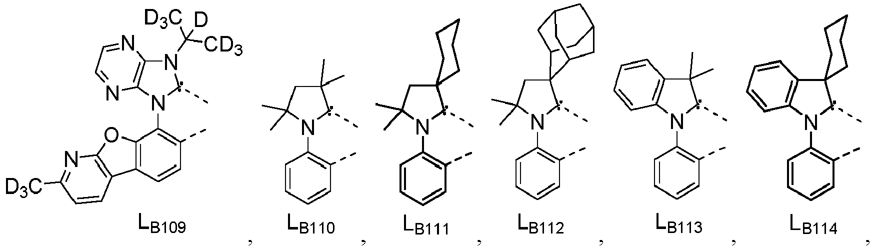 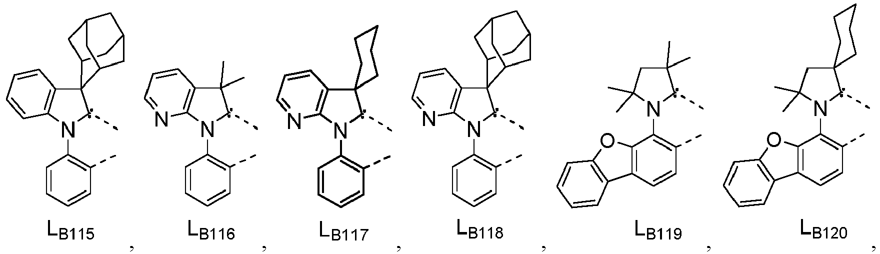 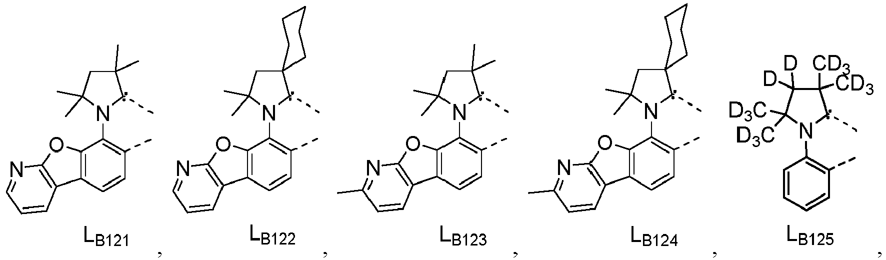 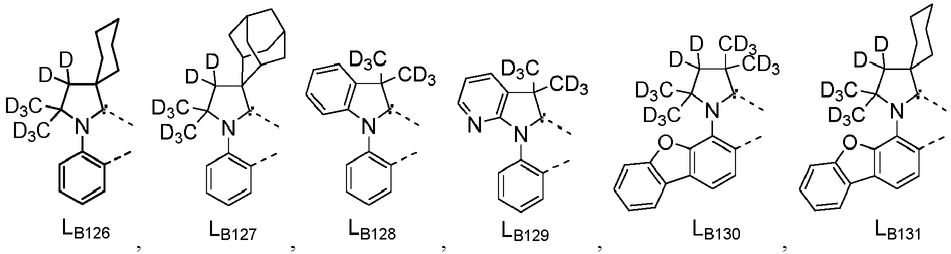 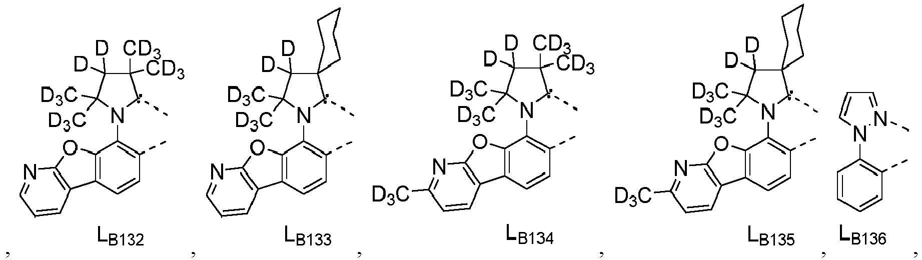 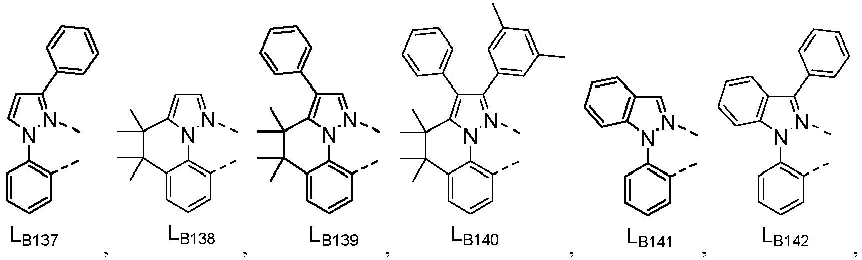 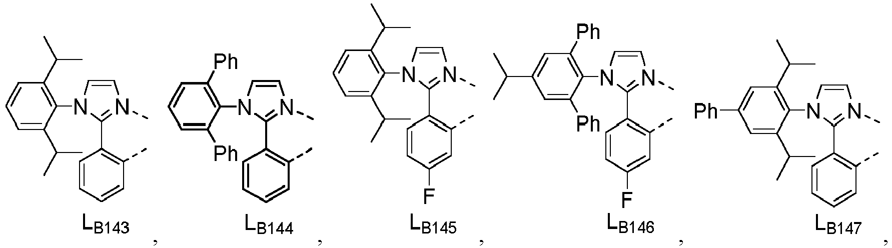 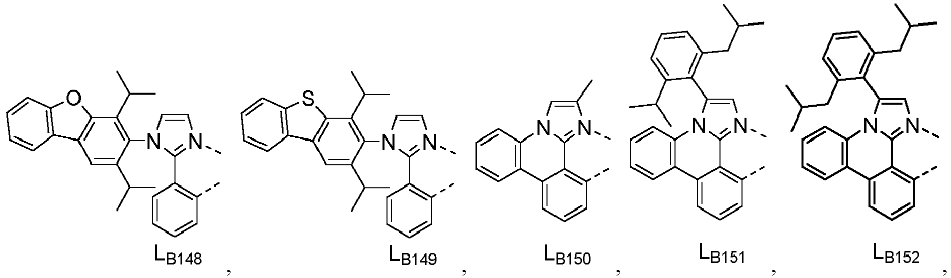 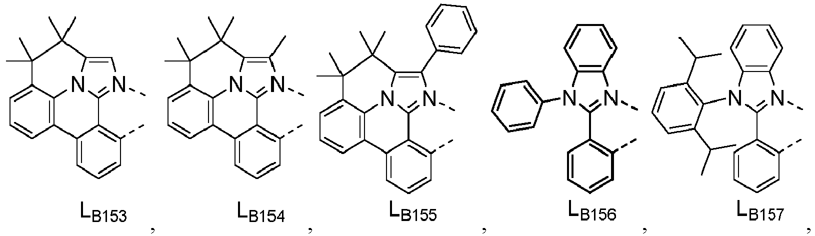 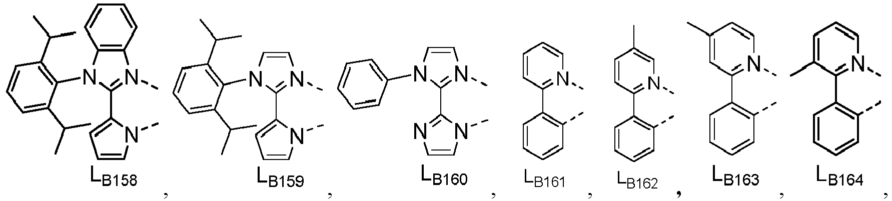 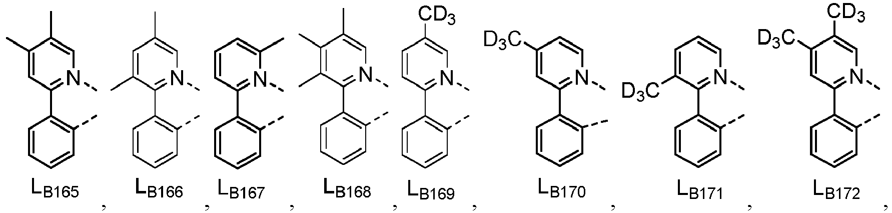 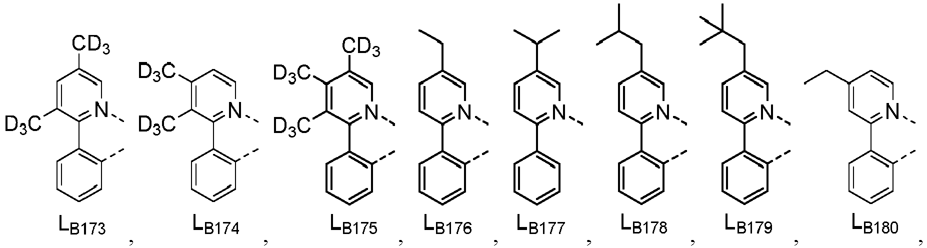 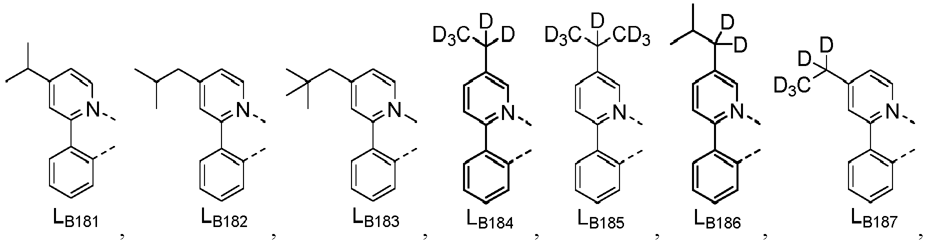 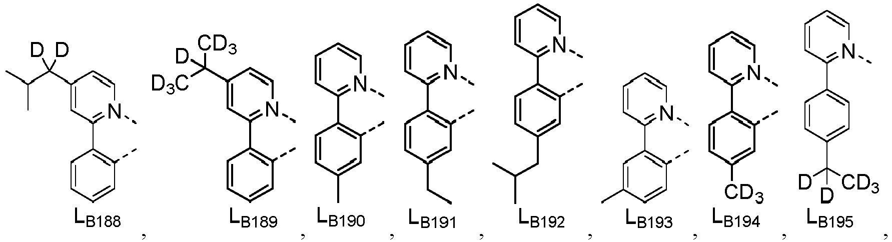 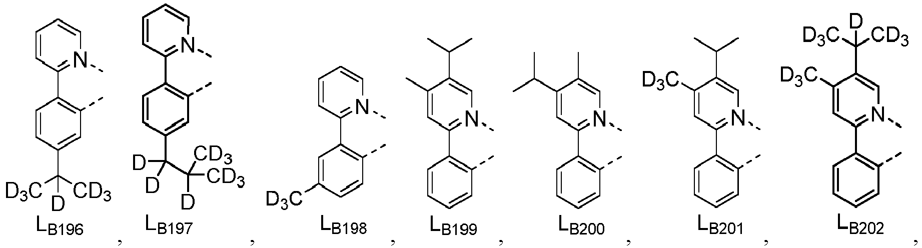 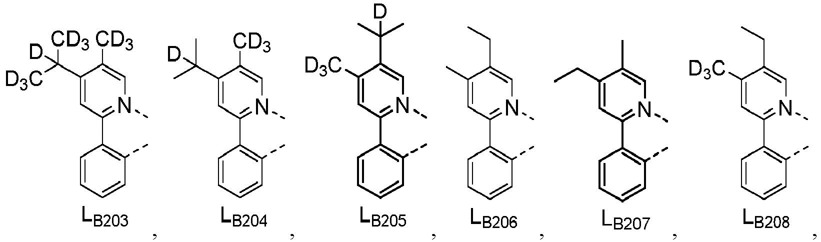 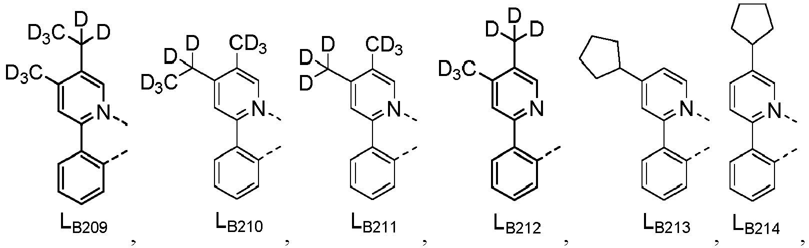 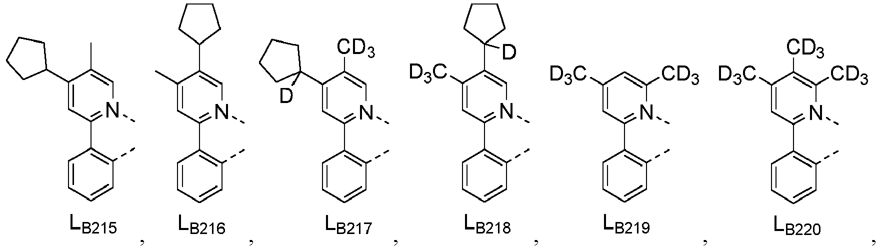 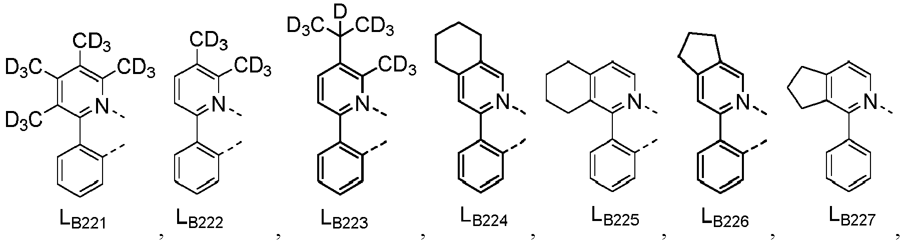 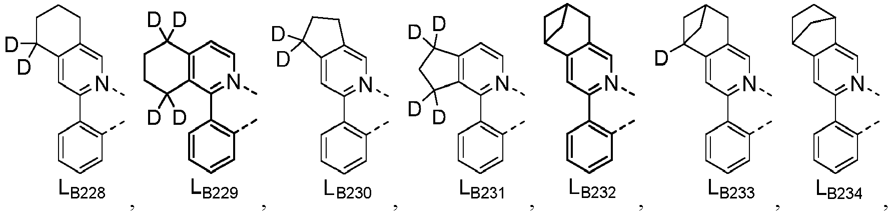 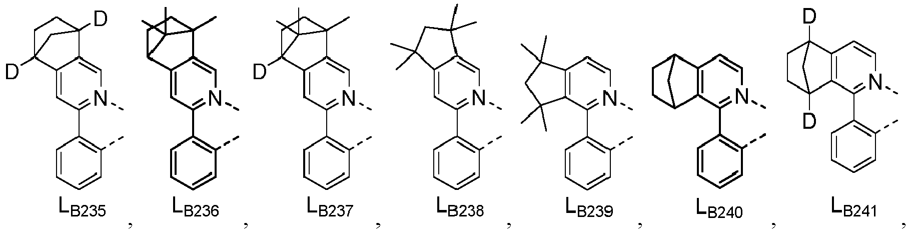 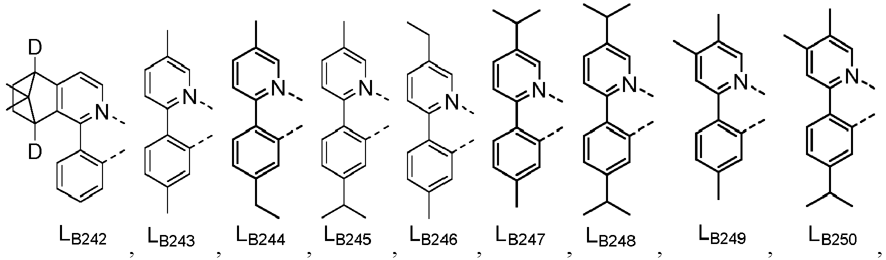 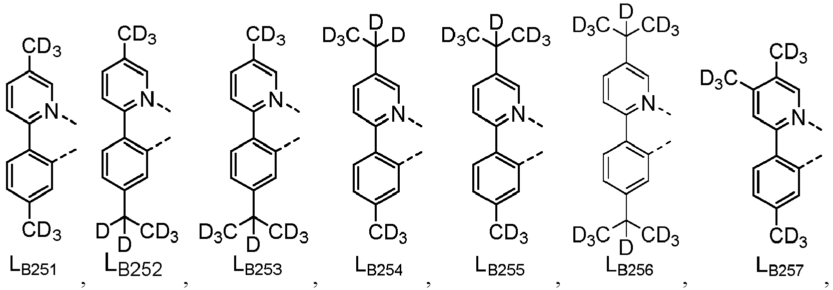 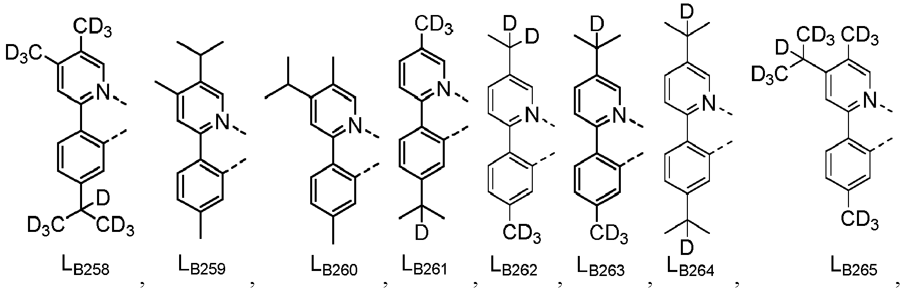 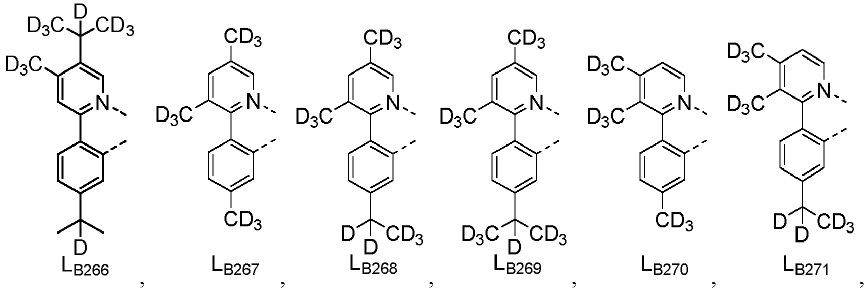 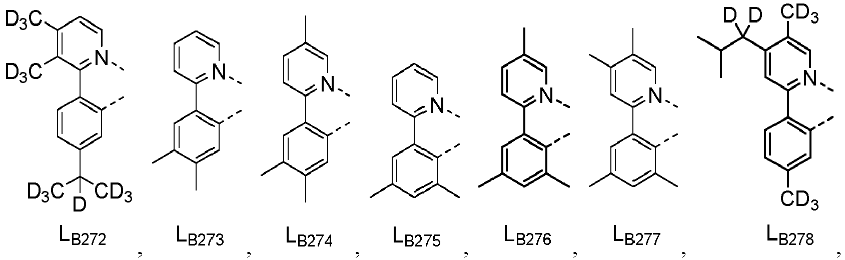 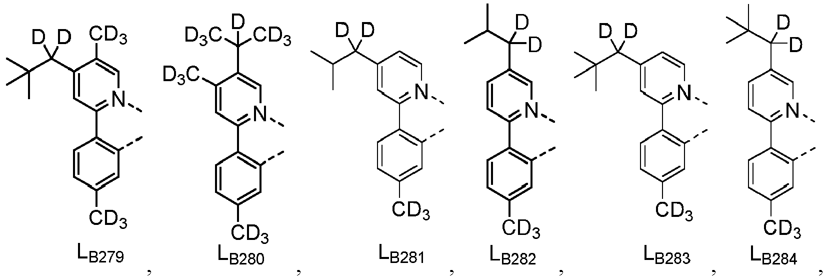 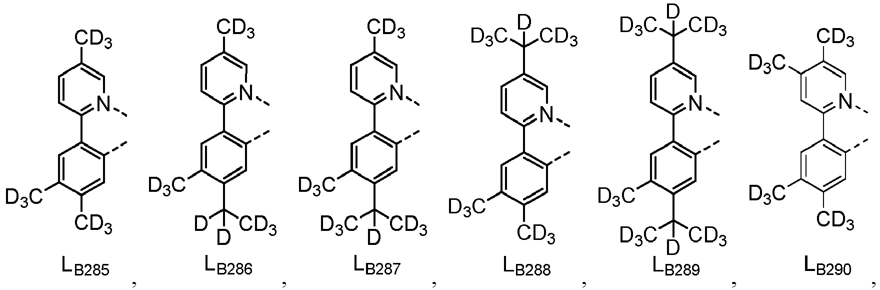 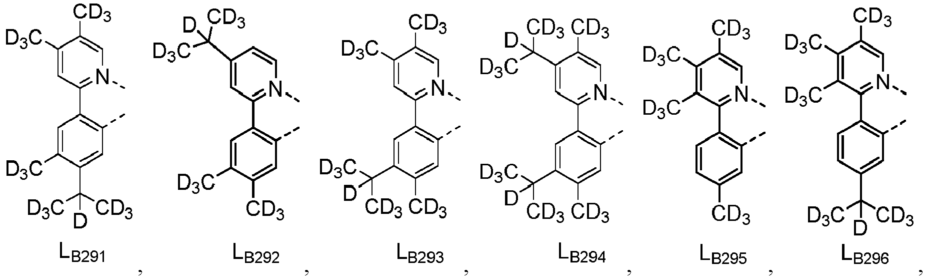 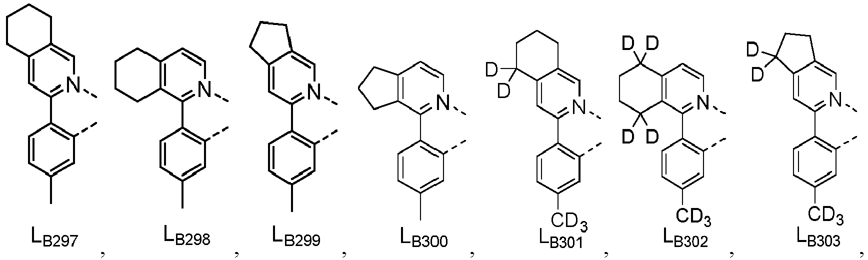 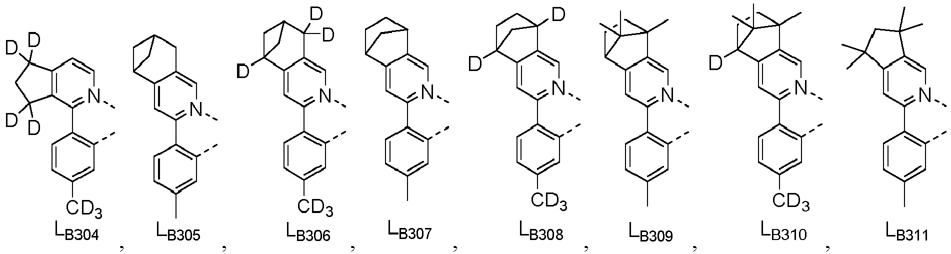 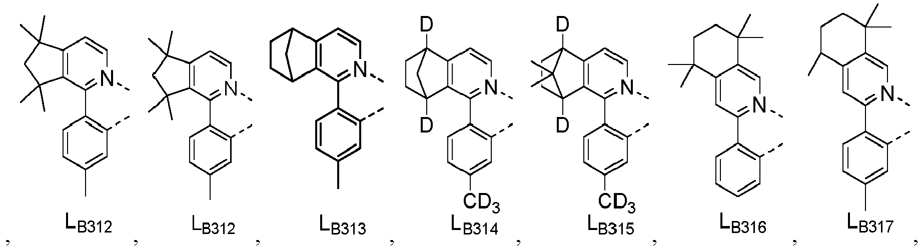 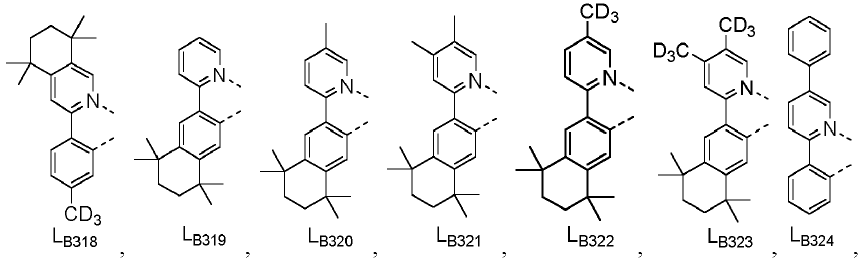 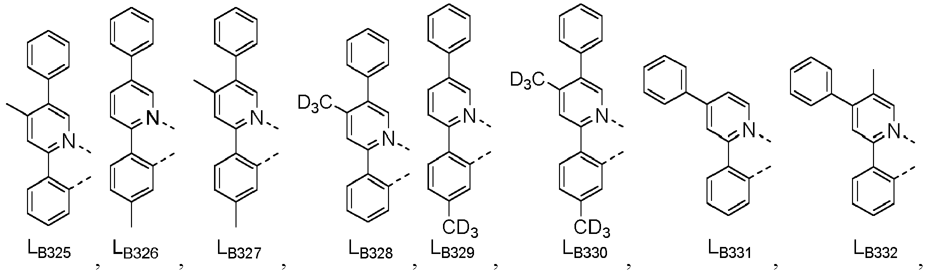 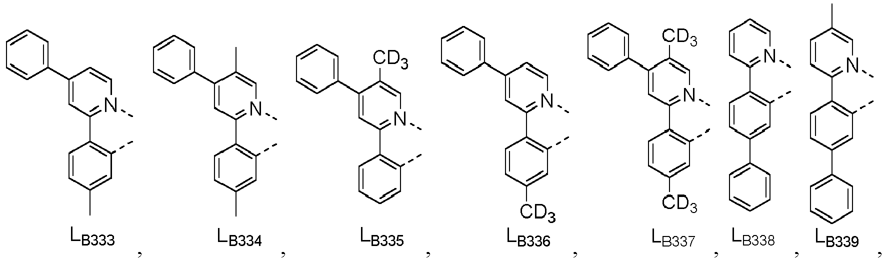 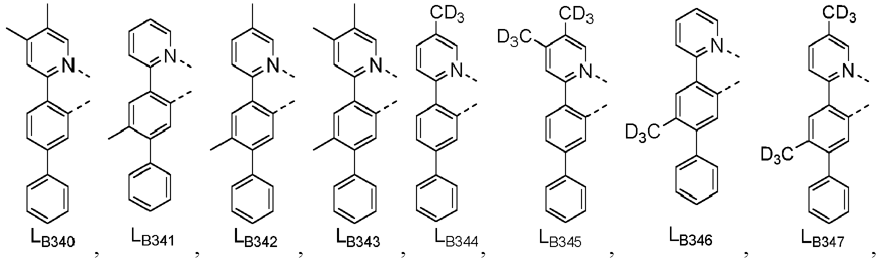 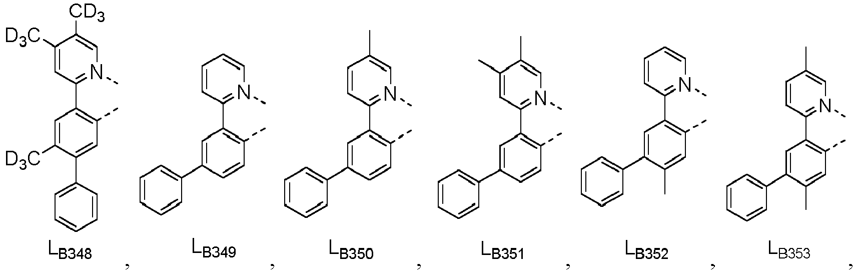 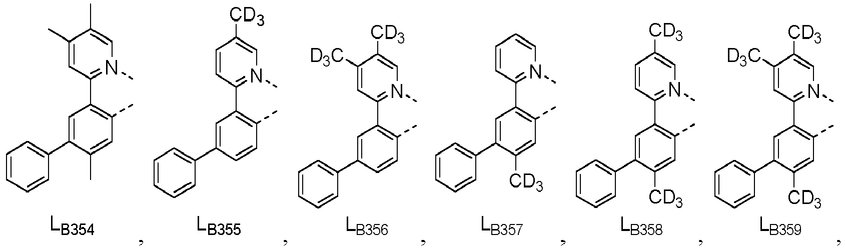 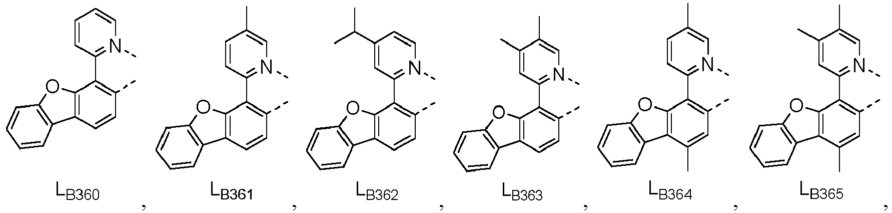 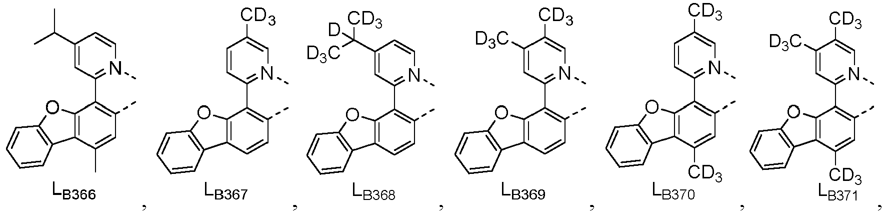 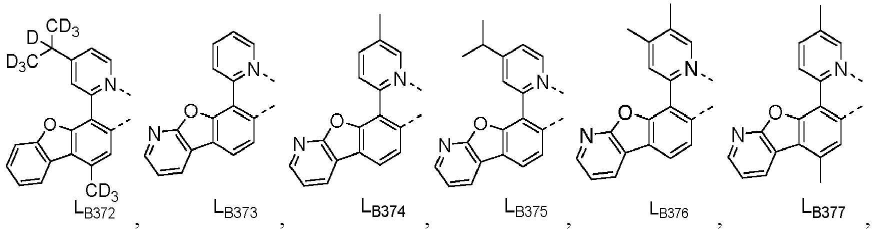 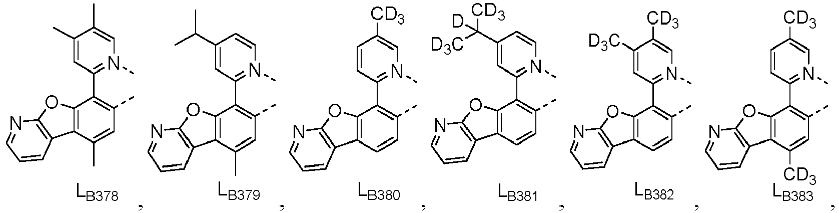 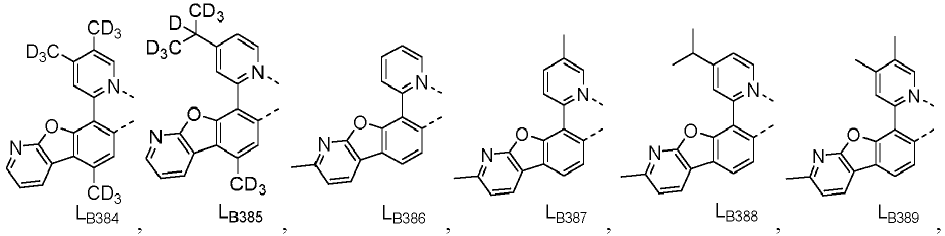 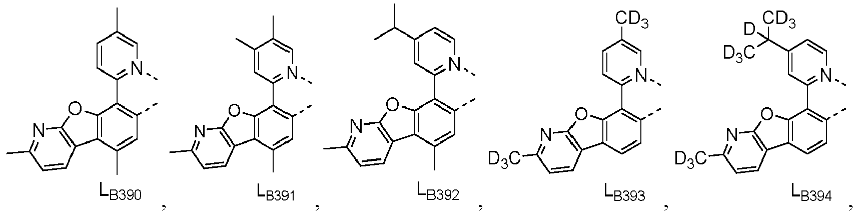 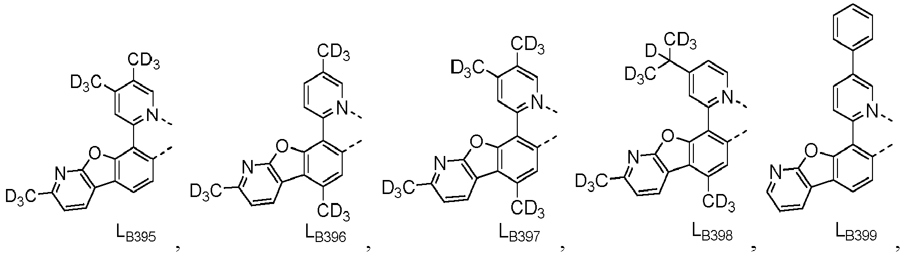 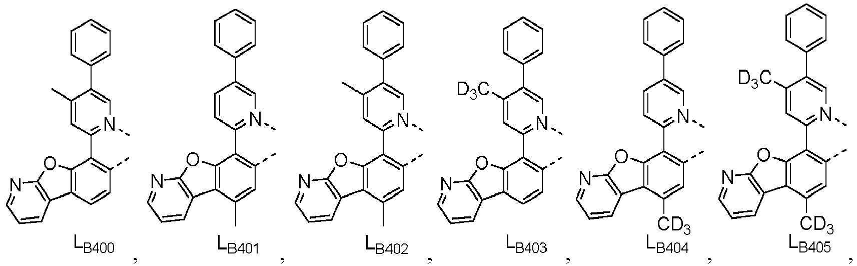 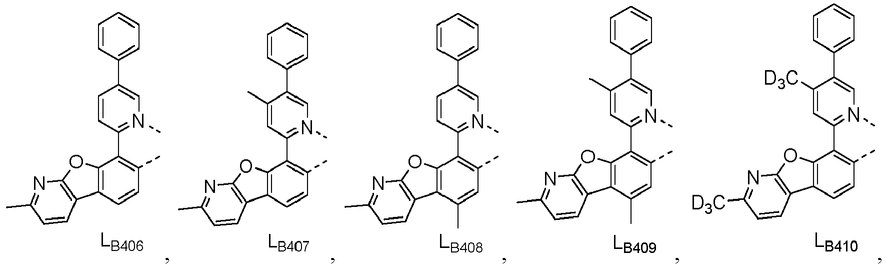 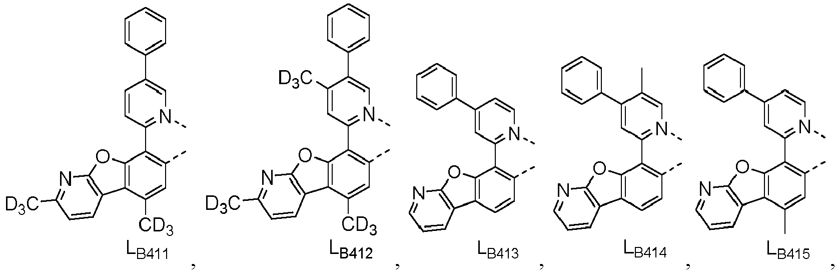 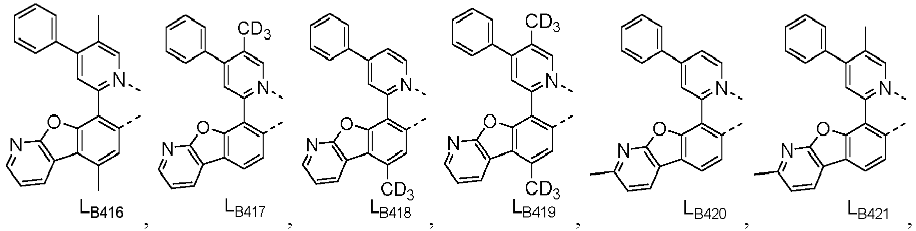 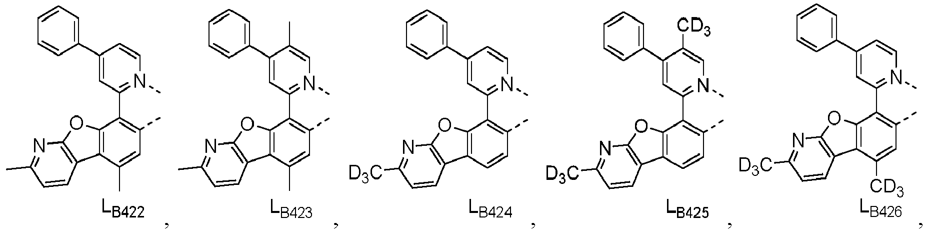 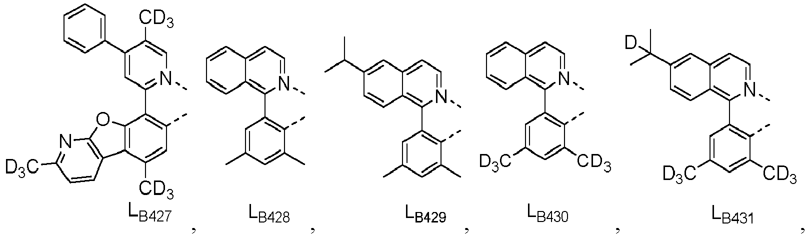 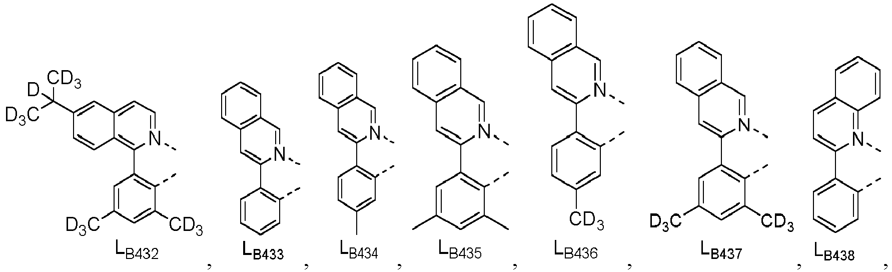 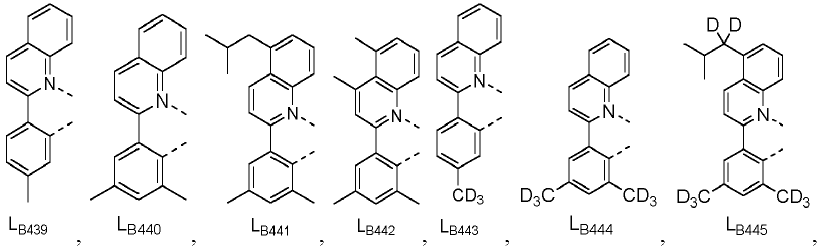 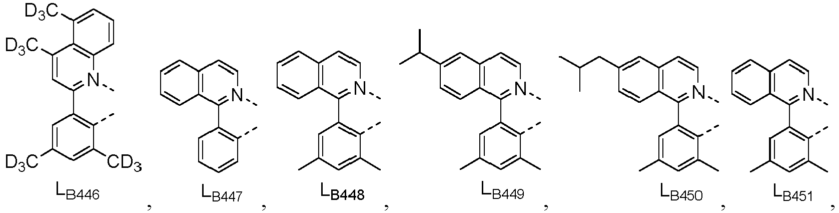 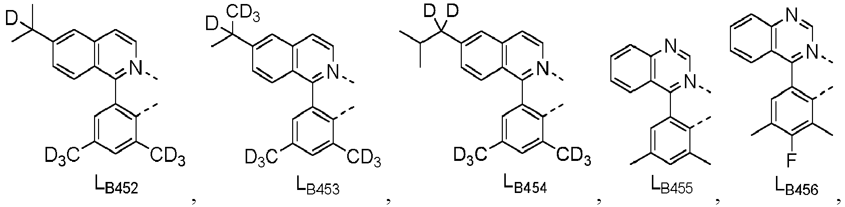 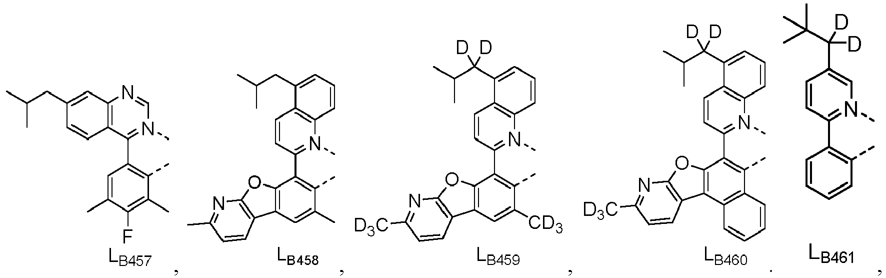 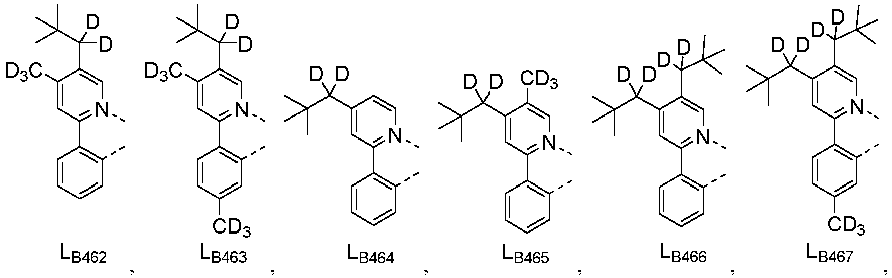 and 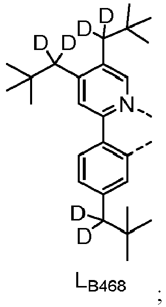 and L_{C} is selected from the group consisting of the following structures:
L_{C1} through L_{C1260} are based on a structure of Formula X 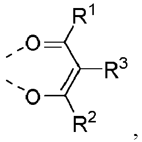 in which R¹, R², and R³ are defined as:

| Ligand | R¹ | R² | R³ | Ligand | R¹ | R² | R³ | Ligand | R¹ | R² | R³ |
|---|---|---|---|---|---|---|---|---|---|---|---|
| L_{C1} | R^{D1} | R^{D1} | H | L_{C421} | R^{D26} | R^{D21} | H | L_{C841} | R^{D7} | R^{D14} | R^{D1} |
| L_{C2} | R^{D2} | R^{D2} | H | L_{C422} | R^{D26} | R^{D23} | H | L_{C842} | R^{D7} | R^{D15} | R^{D1} |
| L_{C3} | R^{D3} | R^{D3} | H | L_{C423} | R^{D26} | R^{D24} | H | L_{C843} | R^{D7} | R^{D16} | R^{D1} |
| L_{C4} | R^{D4} | R^{D4} | H | L_{C424} | R^{D26} | R^{D25} | H | L_{C844} | R^{D7} | R^{D17} | R^{D1} |
| L_{C5} | R^{D5} | R^{D5} | H | L_{C425} | R^{D26} | R^{D27} | H | L_{C845} | R^{D7} | R^{D18} | R^{D1} |
| L_{C6} | R^{D6} | R^{D6} | H | L_{C426} | R^{D26} | R^{D28} | H | L_{C846} | R^{D7} | R^{D19} | R^{D1} |
| L_{C7} | R^{D7} | R^{D7} | H | L_{C427} | R^{D26} | R^{D29} | H | L_{C847} | R^{D7} | R^{D20} | R^{D1} |
| L_{C8} | R^{D8} | R^{D8} | H | L_{C428} | R^{D26} | R^{D30} | H | L_{C848} | R^{D7} | R^{D21} | R^{D1} |
| L_{C9} | R^{D9} | R^{D9} | H | L_{C429} | R^{D26} | R^{D31} | H | L_{C849} | R^{D7} | R^{D22} | R^{D1} |
| L_{C10} | R^{D10} | R^{D10} | H | L_{C430} | R^{D26} | R^{D32} | H | L_{C850} | R^{D7} | R^{D23} | R^{D1} |
| L_{C11} | R^{D11} | R^{D11} | H | L_{C431} | R^{D26} | R^{D33} | H | L_{C851} | R^{D7} | R^{D24} | R^{D1} |
| L_{C12} | R^{D12} | R^{D12} | H | L_{C432} | R^{D26} | R^{D34} | H | L_{C852} | R^{D7} | R^{D25} | R^{D1} |
| L_{C13} | R^{D13} | R^{D13} | H | L_{C433} | R^{D26} | R^{D35} | H | L_{C853} | R^{D7} | R^{D26} | R^{D1} |
| L_{C14} | R^{D14} | R^{D14} | H | L_{C434} | R^{D26} | R^{D40} | H | L_{C854} | R^{D7} | R^{D27} | R^{D1} |
| L_{C15} | R^{D15} | R^{D15} | H | L_{C435} | R^{D26} | R^{D41} | H | L_{C855} | R^{D7} | R^{D28} | R^{D1} |
| L_{C16} | R^{D16} | R^{D16} | H | L_{C436} | R^{D26} | R^{D42} | H | L_{C856} | R^{D7} | R^{D29} | R^{D1} |
| L_{C17} | R^{D17} | R^{D17} | H | L_{C437} | R^{D26} | R^{D64} | H | L_{C857} | R^{D7} | R^{D30} | R^{D1} |
| L_{C18} | R^{D18} | R^{D18} | H | L_{C438} | R^{D26} | R^{D66} | H | L_{C858} | R^{D7} | R^{D31} | R^{D1} |
| L_{C19} | R^{D19} | R^{D19} | H | L_{C439} | R^{D26} | R^{D68} | H | L_{C859} | R^{D7} | R^{D32} | R^{D1} |
| L_{C20} | R^{D20} | R^{D20} | H | L_{C440} | R^{D26} | R^{D76} | H | L_{C860} | R^{D7} | R^{D33} | R^{D1} |
| L_{C21} | R^{D21} | R^{D21} | H | L_{C441} | R^{D35} | R^{D5} | H | L_{C861} | R^{D7} | R^{D34} | R^{D1} |
| L_{C22} | R^{D22} | R^{D22} | H | L_{C442} | R^{D35} | R^{D6} | H | L_{C862} | R^{D7} | R^{D35} | R^{D1} |
| L_{C23} | R^{D23} | R^{D23} | H | L_{C443} | R^{D35} | R^{D9} | H | L_{C863} | R^{D7} | R^{D40} | R^{D1} |
| L_{C24} | R^{D24} | R^{D24} | H | L_{C444} | R^{D35} | R^{D10} | H | L_{C864} | R^{D7} | R^{D41} | R^{D1} |
| L_{C25} | R^{D25} | R^{D25} | H | L_{C445} | R^{D35} | R^{D12} | H | L_{C865} | R^{D7} | R^{D42} | R^{D1} |
| L_{C26} | R^{D26} | R^{D26} | H | L_{C446} | R^{D35} | R^{D15} | H | L_{C866} | R^{D7} | R^{D64} | R^{D1} |
| L_{C27} | R^{D27} | R^{D27} | H | L_{C447} | R^{D35} | R^{D16} | H | L_{C867} | R^{D7} | R^{D66} | R^{D1} |
| L_{C28} | R^{D28} | R^{D28} | H | L_{C448} | R^{D35} | R^{D17} | H | L_{C868} | R^{D7} | R^{D68} | R^{D1} |
| L_{C29} | R^{D29} | R^{D29} | H | L_{C449} | R^{D35} | R^{D18} | H | L_{C869} | R^{D7} | R^{D76} | R^{D1} |
| L_{C30} | R^{D30} | R^{D30} | H | L_{C450} | R^{D35} | R^{D19} | H | L_{C870} | R^{D8} | R^{D5} | R^{D1} |
| L_{C31} | R^{D31} | R^{D31} | H | L_{C451} | R^{D35} | R^{D20} | H | L_{C871} | R^{D8} | R^{D6} | R^{D1} |
| L_{C32} | R^{D32} | R^{D32} | H | L_{C452} | R^{D35} | R^{D21} | H | L_{C872} | R^{D8} | R^{D9} | R^{D1} |
| L_{C33} | R^{D33} | R^{D33} | H | L_{C453} | R^{D35} | R^{D23} | H | L_{C873} | R^{D8} | R^{D10} | R^{D1} |
| L_{C34} | R^{D34} | R^{D34} | H | L_{C454} | R^{D35} | R^{D24} | H | L_{C874} | R^{D8} | R^{D11} | R^{D1} |
| L_{C35} | R^{D35} | R^{D35} | H | L_{C455} | R^{D35} | R^{D25} | H | L_{C875} | R^{D8} | R^{D12} | R^{D1} |
| L_{C36} | R^{D40} | R^{D40} | H | L_{C456} | R^{D35} | R^{D27} | H | L_{C876} | R^{D8} | R^{D13} | R^{D1} |
| L_{C37} | R^{D41} | R^{D41} | H | L_{C457} | R^{D35} | R^{D28} | H | L_{C877} | R^{D8} | R^{D14} | R^{D1} |
| L_{C38} | R^{D42} | R^{D42} | H | L_{C458} | R^{D35} | R^{D29} | H | L_{C878} | R^{D8} | R^{D15} | R^{D1} |
| L_{C39} | R^{D64} | R^{D64} | H | L_{C459} | R^{D35} | R^{D30} | H | L_{C879} | R^{D8} | R^{D16} | R^{D1} |
| L_{C40} | R^{D66} | R^{D66} | H | L_{C460} | R^{D35} | R^{D31} | H | L_{C880} | R^{D8} | R^{D17} | R^{D1} |
| L_{C41} | R^{D68} | R^{D68} | H | L_{C461} | R^{D35} | R^{D32} | H | L_{C881} | R^{D8} | R^{D18} | R^{D1} |
| L_{C42} | R^{D76} | R^{D76} | H | L_{C462} | R^{D35} | R^{D33} | H | L_{C882} | R^{D8} | R^{D19} | R^{D1} |
| L_{C43} | R^{D1} | R^{D2} | H | L_{C463} | R^{D35} | R^{D34} | H | L_{C883} | R^{D8} | R^{D20} | R^{D1} |
| L_{C44} | R^{D1} | R^{D3} | H | L_{C464} | R^{D35} | R^{D40} | H | L_{C884} | R^{D8} | R^{D21} | R^{D1} |
| L_{C45} | R^{D1} | R^{D4} | H | L_{C465} | R^{D35} | R^{D41} | H | L_{C885} | R^{D8} | R^{D22} | R^{D1} |
| L_{C46} | R^{D1} | R^{D5} | H | L_{C466} | R^{D35} | R^{D42} | H | L_{C886} | R^{D8} | R^{D23} | R^{D1} |
| L_{C47} | R^{D1} | R^{D6} | H | L_{C467} | R^{D35} | R^{D64} | H | L_{C887} | R^{D8} | R^{D24} | R^{D1} |
| L_{C48} | R^{D1} | R^{D7} | H | L_{C468} | R^{D35} | R^{D66} | H | L_{C888} | R^{D8} | R^{D25} | R^{D1} |
| L_{C49} | R^{D1} | R^{D8} | H | L_{C469} | R^{D35} | R^{D68} | H | L_{C889} | R^{D8} | R^{D26} | R^{D1} |
| L_{C50} | R^{D1} | R^{D9} | H | L_{C470} | R^{D35} | R^{D76} | H | L_{C890} | R^{D8} | R^{D27} | R^{D1} |
| L_{C51} | R^{D1} | R^{D10} | H | L_{C471} | R^{D40} | R^{D5} | H | L_{C891} | R^{D8} | R^{D28} | R^{D1} |
| L_{C52} | R^{D1} | R^{D11} | H | L_{C472} | R^{D40} | R^{D6} | H | L_{C892} | R^{D8} | R^{D29} | R^{D1} |
| L_{C53} | R^{D1} | R^{D12} | H | L_{C473} | R^{D40} | R^{D9} | H | L_{C893} | R^{D8} | R^{D30} | R^{D1} |
| L_{C54} | R^{D1} | R^{D13} | H | L_{C474} | R^{D40} | R^{D10} | H | L_{C894} | R^{D8} | R^{D31} | R^{D1} |
| L_{C55} | R^{D1} | R^{D14} | H | L_{C475} | R^{D40} | R^{D12} | H | L_{C895} | R^{D8} | R^{D32} | R^{D1} |
| L_{C56} | R^{D1} | R^{D15} | H | L_{C476} | R^{D40} | R^{D15} | H | L_{C896} | R^{D8} | R^{D33} | R^{D1} |
| L_{C57} | R^{D1} | R^{D16} | H | L_{C477} | R^{D40} | R^{D16} | H | L_{C897} | R^{D8} | R^{D34} | R^{D1} |
| L_{C58} | R^{D1} | R^{D17} | H | L_{C478} | R^{D40} | R^{D17} | H | L_{C898} | R^{D8} | R^{D35} | R^{D1} |
| L_{C59} | R^{D1} | R^{D18} | H | L_{C479} | R^{D40} | R^{D18} | H | L_{C899} | R^{D8} | R^{D40} | R^{D1} |
| L_{C60} | R^{D1} | R^{D19} | H | L_{C480} | R^{D40} | R^{D19} | H | L_{C900} | R^{D8} | R^{D41} | R^{D1} |
| L_{C61} | R^{D1} | R^{D20} | H | L_{C481} | R^{D40} | R^{D20} | H | L_{C901} | R^{D8} | R^{D42} | R^{D1} |
| L_{C62} | R^{D1} | R^{D21} | H | L_{C482} | R^{D40} | R^{D21} | H | L_{C902} | R^{D8} | R^{D64} | R^{D1} |
| L_{C63} | R^{D1} | R^{D22} | H | L_{C483} | R^{D40} | R^{D23} | H | L_{C903} | R^{D8} | R^{D66} | R^{D1} |
| L_{C64} | R^{D1} | R^{D23} | H | L_{C484} | R^{D40} | R^{D24} | H | L_{C904} | R^{D8} | R^{D68} | R^{D1} |
| L_{C65} | R^{D1} | R^{D24} | H | L_{C485} | R^{D40} | R^{D25} | H | L_{C905} | R^{D8} | R^{D76} | R^{D1} |
| L_{C66} | R^{D1} | R^{D25} | H | L_{C486} | R^{D40} | R^{D27} | H | L_{C906} | R^{D11} | R^{D5} | R^{D1} |
| L_{C67} | R^{D1} | R^{D26} | H | L_{C487} | R^{D40} | R^{D28} | H | L_{C907} | R^{D11} | R^{D6} | R^{D1} |
| L_{C68} | R^{D1} | R^{D27} | H | L_{C488} | R^{D40} | R^{D29} | H | L_{C908} | R^{D11} | R^{D9} | R^{D1} |
| L_{C69} | R^{D1} | R^{D28} | H | L_{C489} | R^{D40} | R^{D30} | H | L_{C909} | R^{D11} | R^{D10} | R^{D1} |
| L_{C70} | R^{D1} | R^{D29} | H | L_{C490} | R^{D40} | R^{D31} | H | L_{C910} | R^{D11} | R^{D12} | R^{D1} |
| L_{C71} | R^{D1} | R^{D30} | H | L_{C491} | R^{D40} | R^{D32} | H | L_{C911} | R^{D11} | R^{D13} | R^{D1} |
| L_{C72} | R^{D1} | R^{D31} | H | L_{C492} | R^{D40} | R^{D33} | H | L_{C912} | R^{D11} | R^{D14} | R^{D1} |
| L_{C73} | R^{D1} | R^{D32} | H | L_{C493} | R^{D40} | R^{D34} | H | Lc₉₁₃ | R^{D11} | R^{D15} | R^{D1} |
| L_{C74} | R^{D1} | R^{D33} | H | L_{C494} | R^{D40} | R^{D41} | H | L_{C914} | R^{D11} | R^{D16} | R^{D1} |
| L_{C75} | R^{D1} | R^{D34} | H | L_{C495} | R^{D40} | R^{D42} | H | L_{C915} | R^{D11} | R^{D17} | R^{D1} |
| L_{C76} | R^{D1} | R^{D35} | H | L_{C496} | R^{D40} | R^{D64} | H | L_{C916} | R^{D11} | R^{D18} | R^{D1} |
| L_{C77} | R^{D1} | R^{D40} | H | L_{C497} | R^{D40} | R^{D66} | H | L_{C917} | R^{D11} | R^{D19} | R^{D1} |
| L_{C78} | R^{D1} | R^{D41} | H | L_{C498} | R^{D40} | R^{D68} | H | L_{C918} | R^{D11} | R^{D20} | R^{D1} |
| L_{C79} | R^{D1} | R^{D42} | H | L_{C499} | R^{D40} | R^{D76} | H | L_{C919} | R^{D11} | R^{D21} | R^{D1} |
| L_{C80} | R^{D1} | R^{D64} | H | L_{C500} | R^{D41} | R^{D5} | H | L_{C920} | R^{D11} | R^{D22} | R^{D1} |
| L_{C81} | R^{D1} | R^{D66} | H | L_{C501} | R^{D41} | R^{D6} | H | L_{C921} | R^{D11} | R^{D23} | R^{D1} |
| L_{C82} | R^{D1} | R^{D68} | H | L_{C502} | R^{D41} | R^{D9} | H | L_{C922} | R^{D11} | R^{D24} | R^{D1} |
| L_{C83} | R^{D1} | R^{D76} | H | L_{C503} | R^{D41} | R^{D10} | H | L_{C923} | R^{D11} | R^{D25} | R^{D1} |
| L_{C84} | R^{D2} | R^{D1} | H | L_{C504} | R^{D41} | R^{D12} | H | L_{C924} | R^{D11} | R^{D26} | R^{D1} |
| L_{C85} | R^{D2} | R^{D3} | H | L_{C505} | R^{D41} | R^{D15} | H | L_{C925} | R^{D11} | R^{D27} | R^{D1} |
| L_{C86} | R^{D2} | R^{D4} | H | L_{C506} | R^{D41} | R^{D16} | H | L_{C926} | R^{D11} | R^{D28} | R^{D1} |
| L_{C87} | R^{D2} | R^{D5} | H | L_{C507} | R^{D41} | R^{D17} | H | L_{C927} | R^{D11} | R^{D29} | R^{D1} |
| L_{C88} | R^{D2} | R^{D6} | H | L_{C508} | R^{D41} | R^{D18} | H | L_{C928} | R^{D11} | R^{D30} | R^{D1} |
| L_{C89} | R^{D2} | R^{D7} | H | L_{C509} | R^{D41} | R^{D19} | H | L_{C929} | R^{D11} | R^{D31} | R^{D1} |
| L_{C90} | R^{D2} | R^{D8} | H | L_{C510} | R^{D41} | R^{D20} | H | L_{C930} | R^{D11} | R^{D32} | R^{D1} |
| L_{C91} | R^{D2} | R^{D9} | H | L_{C511} | R^{D41} | R^{D21} | H | L_{C931} | R^{D11} | R^{D33} | R^{D1} |
| L_{C92} | R^{D2} | R^{D10} | H | L_{C512} | R^{D41} | R^{D23} | H | L_{C932} | R^{D11} | R^{D34} | R^{D1} |
| L_{C93} | R^{D2} | R^{D11} | H | L_{C513} | R^{D41} | R^{D24} | H | L_{C933} | R^{D11} | R^{D35} | R^{D1} |
| L_{C94} | R^{D2} | R^{D12} | H | L_{C514} | R^{D41} | R^{D25} | H | L_{C934} | R^{D11} | R^{D40} | R^{D1} |
| L_{C95} | R^{D2} | R^{D13} | H | L_{C515} | R^{D41} | R^{D27} | H | L_{C935} | R^{D11} | R^{D41} | R^{D1} |
| L_{C96} | R^{D2} | R^{D14} | H | L_{C516} | R^{D41} | R^{D28} | H | L_{C936} | R^{D11} | R^{D42} | R^{D1} |
| L_{C97} | R^{D2} | R^{D15} | H | L_{C517} | R^{D41} | R^{D29} | H | L_{C937} | R^{D11} | R^{D64} | R^{D1} |
| L_{C98} | R^{D2} | R^{D16} | H | L_{C518} | R^{D41} | R^{D30} | H | L_{C938} | R^{D11} | R^{D66} | R^{D1} |
| L_{C99} | R^{D2} | R^{D17} | H | L_{C519} | R^{D41} | R^{D31} | H | L_{C939} | R^{D11} | R^{D68} | R^{D1} |
| L_{C100} | R^{D2} | R^{D18} | H | L_{C520} | R^{D41} | R^{D32} | H | L_{C940} | R^{D11} | R^{D76} | R^{D1} |
| L_{C101} | R^{D2} | R^{D19} | H | L_{C521} | R^{D41} | R^{D33} | H | L_{C941} | R^{D13} | R^{D5} | R^{D1} |
| L_{C102} | R^{D2} | R^{D20} | H | L_{C522} | R^{D41} | R^{D34} | H | L_{C942} | R^{D13} | R^{D6} | R^{D1} |
| L_{C103} | R^{D2} | R^{D21} | H | L_{C523} | R^{D41} | R^{D42} | H | L_{C943} | R^{D13} | R^{D9} | R^{D1} |
| L_{C104} | R^{D2} | R^{D22} | H | L_{C524} | R^{D41} | R^{D64} | H | L_{C944} | R^{D13} | R^{D10} | R^{D1} |
| L_{C105} | R^{D2} | R^{D23} | H | L_{C525} | R^{D41} | R^{D66} | H | L_{C945} | R^{D13} | R^{D12} | R^{D1} |
| L_{C106} | R^{D2} | R^{D24} | H | L_{C526} | R^{D41} | R^{D68} | H | L_{C946} | R^{D13} | R^{D14} | R^{D1} |
| L_{C107} | R^{D2} | R^{D25} | H | L_{C527} | R^{D41} | R^{D76} | H | L_{C947} | R^{D13} | R^{D15} | R^{D1} |
| L_{C108} | R^{D2} | R^{D26} | H | L_{C528} | R^{D64} | R^{D5} | H | L_{C948} | R^{D13} | R^{D16} | R^{D1} |
| L_{C109} | R^{D2} | R^{D27} | H | L_{C529} | R^{D64} | R^{D6} | H | L_{C949} | R^{D13} | R^{D17} | R^{D1} |
| L_{C110} | R^{D2} | R^{D28} | H | L_{C530} | R^{D64} | R^{D9} | H | L_{C950} | R^{D13} | R^{D18} | R^{D1} |
| L_{C111} | R^{D2} | R^{D29} | H | L_{C531} | R^{D64} | R^{D10} | H | L_{C951} | R^{D13} | R^{D19} | R^{D1} |
| L_{C112} | R^{D2} | R^{D30} | H | L_{C532} | R^{D64} | R^{D12} | H | L_{C952} | R^{D13} | R^{D20} | R^{D1} |
| L_{C113} | R^{D2} | R^{D31} | H | L_{C533} | R^{D64} | R^{D15} | H | L_{C953} | R^{D13} | R^{D21} | R^{D1} |
| L_{C114} | R^{D2} | R^{D32} | H | L_{C534} | R^{D64} | R^{D16} | H | L_{C954} | R^{D13} | R^{D22} | R^{D1} |
| L_{C115} | R^{D2} | R^{D33} | H | L_{C535} | R^{D64} | R^{D17} | H | L_{C955} | R^{D13} | R^{D23} | R^{D1} |
| L_{C116} | R^{D2} | R^{D34} | H | L_{C536} | R^{D64} | R^{D18} | H | L_{C956} | R^{D13} | R^{D24} | R^{D1} |
| L_{C117} | R^{D2} | R^{D35} | H | L_{C537} | R^{D64} | R^{D19} | H | L_{C957} | R^{D13} | R^{D25} | R^{D1} |
| L_{C118} | R^{D2} | R^{D40} | H | L_{C538} | R^{D64} | R^{D20} | H | L_{C958} | R^{D13} | R^{D26} | R^{D1} |
| L_{C119} | R^{D2} | R^{D41} | H | L_{C539} | R^{D64} | R^{D21} | H | L_{C959} | R^{D13} | R^{D27} | R^{D1} |
| L_{C120} | R^{D2} | R^{D42} | H | L_{C540} | R^{D64} | R^{D23} | H | L_{C960} | R^{D13} | R^{D28} | R^{D1} |
| L_{C121} | R^{D2} | R^{D64} | H | L_{C541} | R^{D64} | R^{D24} | H | L_{C961} | R^{D13} | R^{D29} | R^{D1} |
| L_{C122} | R^{D2} | R^{D66} | H | L_{C542} | R^{D64} | R^{D25} | H | L_{C962} | R^{D13} | R^{D30} | R^{D1} |
| L_{C123} | R^{D2} | R^{D68} | H | L_{C543} | R^{D64} | R^{D27} | H | L_{C963} | R^{D13} | R^{D31} | R^{D1} |
| L_{C124} | R^{D2} | R^{D76} | H | L_{C544} | R^{D64} | R^{D28} | H | L_{C964} | R^{D13} | R^{D32} | R^{D1} |
| L_{C125} | R^{D3} | R^{D4} | H | L_{C545} | R^{D64} | R^{D29} | H | L_{C965} | R^{D13} | R^{D33} | R^{D1} |
| L_{C126} | R^{D3} | R^{D5} | H | L_{C546} | R^{D64} | R^{D30} | H | L_{C966} | R^{D13} | R^{D34} | R^{D1} |
| L_{C127} | R^{D3} | R^{D6} | H | L_{C547} | R^{D64} | R^{D31} | H | L_{C967} | R^{D13} | R^{D35} | R^{D1} |
| L_{C128} | R^{D3} | R^{D7} | H | L_{C548} | R^{D64} | R^{D32} | H | L_{C968} | R^{D13} | R^{D40} | R^{D1} |
| L_{C129} | R^{D3} | R^{D8} | H | L_{C549} | R^{D64} | R^{D33} | H | L_{C969} | R^{D13} | R^{D41} | R^{D1} |
| L_{C130} | R^{D3} | R^{D9} | H | L_{C550} | R^{D64} | R^{D34} | H | L_{C970} | R^{D13} | R^{D42} | R^{D1} |
| L_{C131} | R^{D3} | R^{D10} | H | L_{C551} | R^{D64} | R^{D42} | H | L_{C971} | R^{D13} | R^{D64} | R^{D1} |
| L_{C132} | R^{D3} | R^{D11} | H | L_{C552} | R^{D64} | R^{D64} | H | L_{C972} | R^{D13} | R^{D66} | R^{D1} |
| L_{C133} | R^{D3} | R^{D12} | H | L_{C553} | R^{D64} | R^{D66} | H | L_{C973} | R^{D13} | R^{D68} | R^{D1} |
| L_{C134} | R^{D3} | R^{D13} | H | L_{C554} | R^{D64} | R^{D68} | H | L_{C974} | R^{D13} | R^{D76} | R^{D1} |
| L_{C135} | R^{D3} | R^{D14} | H | L_{C555} | R^{D64} | R^{D76} | H | L_{C975} | R^{D14} | R^{D5} | R^{D1} |
| L_{C136} | R^{D3} | R^{D15} | H | L_{C556} | R^{D66} | R^{D5} | H | L_{C976} | R^{D14} | R^{D6} | R^{D1} |
| L_{C137} | R^{D3} | R^{D16} | H | L_{C557} | R^{D66} | R^{D6} | H | L_{C977} | R^{D14} | R^{D9} | R^{D1} |
| L_{C138} | R^{D3} | R^{D17} | H | L_{C558} | R^{D66} | R^{D9} | H | L_{C978} | R^{D14} | R^{D10} | R^{D1} |
| L_{C139} | R^{D3} | R^{D18} | H | L_{C559} | R^{D66} | R^{D10} | H | L_{C979} | R^{D14} | R^{D12} | R^{D1} |
| L_{C140} | R^{D3} | R^{D19} | H | L_{C560} | R^{D66} | R^{D12} | H | L_{C980} | R^{D14} | R^{D15} | R^{D1} |
| L_{C141} | R^{D3} | R^{D20} | H | L_{C561} | R^{D66} | R^{D15} | H | L_{C981} | R^{D14} | R^{D16} | R^{D1} |
| L_{C142} | R^{D3} | R^{D21} | H | L_{C562} | R^{D66} | R^{D16} | H | L_{C982} | R^{D14} | R^{D17} | R^{D1} |
| L_{C143} | R^{D3} | R^{D22} | H | L_{C563} | R^{D66} | R^{D17} | H | L_{C983} | R^{D14} | R^{D18} | R^{D1} |
| L_{C144} | R^{D3} | R^{D23} | H | L_{C564} | R^{D66} | R^{D18} | H | L_{C984} | R^{D14} | R^{D19} | R^{D1} |
| L_{C145} | R^{D3} | R^{D24} | H | L_{C565} | R^{D66} | R^{D19} | H | L_{C985} | R^{D14} | R^{D20} | R^{D1} |
| Lci46 | R^{D3} | R^{D25} | H | L_{C566} | R^{D66} | R^{D20} | H | L_{C986} | R^{D14} | R^{D21} | R^{D1} |
| Lci47 | R^{D3} | R^{D26} | H | L_{C567} | R^{D66} | R^{D21} | H | L_{C987} | R^{D14} | R^{D22} | R^{D1} |
| L_{C148} | R^{D3} | R^{D27} | H | L_{C568} | R^{D66} | R^{D23} | H | L_{C988} | R^{D14} | R^{D23} | R^{D1} |
| Lci49 | R^{D3} | R^{D28} | H | L_{C569} | R^{D66} | R^{D24} | H | L_{C989} | R^{D14} | R^{D24} | R^{D1} |
| L_{C150} | R^{D3} | R^{D29} | H | L_{C570} | R^{D66} | R^{D25} | H | L_{C990} | R^{D14} | R^{D25} | R^{D1} |
| L_{C151} | R^{D3} | R^{D30} | H | L_{C571} | R^{D66} | R^{D27} | H | L_{C991} | R^{D14} | R^{D26} | R^{D1} |
| Lci52 | R^{D3} | R^{D31} | H | L_{C572} | R^{D66} | R^{D28} | H | L_{C992} | R^{D14} | R^{D27} | R^{D1} |
| L_{C153} | R^{D3} | R^{D32} | H | L_{C573} | R^{D66} | R^{D29} | H | L_{C993} | R^{D14} | R^{D28} | R^{D1} |
| L_{C154} | R^{D3} | R^{D33} | H | L_{C574} | R^{D66} | R^{D30} | H | L_{C994} | R^{D14} | R^{D29} | R^{D1} |
| L_{C155} | R^{D3} | R^{D34} | H | L_{C575} | R^{D66} | R^{D31} | H | L_{C995} | R^{D14} | R^{D30} | R^{D1} |
| Lci56 | R^{D3} | R^{D35} | H | L_{C576} | R^{D66} | R^{D32} | H | L_{C996} | R^{D14} | R^{D31} | R^{D1} |
| Lci57 | R^{D3} | R^{D40} | H | L_{C577} | R^{D66} | R^{D33} | H | L_{C997} | R^{D14} | R^{D32} | R^{D1} |
| L_{C158} | R^{D3} | R^{D41} | H | L_{C578} | R^{D66} | R^{D34} | H | L_{C998} | R^{D14} | R^{D33} | R^{D1} |
| Lci59 | R^{D3} | R^{D42} | H | L_{C579} | R^{D66} | R^{D42} | H | L_{C999} | R^{D14} | R^{D34} | R^{D1} |
| L_{C160} | R^{D3} | R^{D64} | H | L_{C580} | R^{D66} | R^{D68} | H | L_{C1000} | R^{D14} | R^{D35} | R^{D1} |
| L_{C161} | R^{D3} | R^{D66} | H | L_{C581} | R^{D66} | R^{D76} | H | L_{C1001} | R^{D14} | R^{D40} | R^{D1} |
| L_{C162} | R^{D3} | R^{D68} | H | L_{C582} | R^{D68} | R^{D5} | H | L_{C1002} | R^{D14} | R^{D41} | R^{D1} |
| L_{C163} | R^{D3} | R^{D76} | H | L_{C583} | R^{D68} | R^{D6} | H | L_{C1003} | R^{D14} | R^{D42} | R^{D1} |
| L_{C164} | R^{D4} | R^{D5} | H | L_{C584} | R^{D68} | R^{D9} | H | L_{C1004} | R^{D14} | R^{D64} | R^{D1} |
| Lci65 | R^{D4} | R^{D6} | H | L_{C585} | R^{D68} | R^{D10} | H | L_{C1005} | R^{D14} | R^{D66} | R^{D1} |
| L_{C166} | R^{D4} | R^{D7} | H | L_{C586} | R^{D68} | R^{D12} | H | L_{C1006} | R^{D14} | R^{D68} | R^{D1} |
| L_{C167} | R^{D4} | R^{D8} | H | L_{C587} | R^{D68} | R^{D15} | H | L_{C1007} | R^{D14} | R^{D76} | R^{D1} |
| L_{C168} | R^{D4} | R^{D9} | H | L_{C588} | R^{D68} | R^{D16} | H | L_{C1008} | R^{D22} | R^{D5} | R^{D1} |
| L_{C169} | R^{D4} | R^{D10} | H | L_{C589} | R^{D68} | R^{D17} | H | L_{C1009} | R^{D22} | R^{D6} | R^{D1} |
| L_{C170} | R^{D4} | R^{D11} | H | L_{C590} | R^{D68} | R^{D18} | H | L_{C1010} | R^{D22} | R^{D9} | R^{D1} |
| L_{C171} | R^{D4} | R^{D12} | H | L_{C591} | R^{D68} | R^{D19} | H | L_{C1011} | R^{D22} | R^{D10} | R^{D1} |
| L_{C172} | R^{D4} | R^{D13} | H | L_{C592} | R^{D68} | R^{D20} | H | L_{C1012} | R^{D22} | R^{D12} | R^{D1} |
| L_{C173} | R^{D4} | R^{D14} | H | L_{C593} | R^{D68} | R^{D21} | H | L_{C1013} | R^{D22} | R^{D15} | R^{D1} |
| L_{C174} | R^{D4} | R^{D15} | H | L_{C594} | R^{D68} | R^{D23} | H | L_{C1014} | R^{D22} | R^{D16} | R^{D1} |
| L_{C175} | R^{D4} | R^{D16} | H | L_{C595} | R^{D68} | R^{D24} | H | L_{C1015} | R^{D22} | R^{D17} | R^{D1} |
| L_{C176} | R^{D4} | R^{D17} | H | L_{C596} | R^{D68} | R^{D25} | H | L_{C1016} | R^{D22} | R^{D18} | R^{D1} |
| L_{C177} | R^{D4} | R^{D18} | H | L_{C597} | R^{D68} | R^{D27} | H | L_{C1017} | R^{D22} | R^{D19} | R^{D1} |
| L_{C178} | R^{D4} | R^{D19} | H | L_{C598} | R^{D68} | R^{D28} | H | L_{C1018} | R^{D22} | R^{D20} | R^{D1} |
| L_{C179} | R^{D4} | R^{D20} | H | L_{C599} | R^{D68} | R^{D29} | H | L_{C1019} | R^{D22} | R^{D21} | R^{D1} |
| L_{C180} | R^{D4} | R^{D21} | H | L_{C600} | R^{D68} | R^{D30} | H | L_{C1020} | R^{D22} | R^{D23} | R^{D1} |
| L_{C181} | R^{D4} | R^{D22} | H | L_{C601} | R^{D68} | R^{D31} | H | L_{C1021} | R^{D22} | R^{D24} | R^{D1} |
| L_{C182} | R^{D4} | R^{D23} | H | L_{C602} | R^{D68} | R^{D32} | H | L_{C1022} | R^{D22} | R^{D25} | R^{D1} |
| L_{C183} | R^{D4} | R^{D24} | H | L_{C603} | R^{D68} | R^{D33} | H | L_{C1023} | R^{D22} | R^{D26} | R^{D1} |
| L_{C184} | R^{D4} | R^{D25} | H | L_{C604} | R^{D68} | R^{D34} | H | L_{C1024} | R^{D22} | R^{D27} | R^{D1} |
| L_{C185} | R^{D4} | R^{D26} | H | L_{C605} | R^{D68} | R^{D42} | H | L_{C1025} | R^{D22} | R^{D28} | R^{D1} |
| L_{C186} | R^{D4} | R^{D27} | H | L_{C606} | R^{D68} | R^{D76} | H | L_{C1026} | R^{D22} | R^{D29} | R^{D1} |
| Lci87 | R^{D4} | R^{D28} | H | L_{C607} | R^{D76} | R^{D5} | H | L_{C1027} | R^{D22} | R^{D30} | R^{D1} |
| L_{C188} | R^{D4} | R^{D29} | H | L_{C608} | R^{D76} | R^{D6} | H | L_{C1028} | R^{D22} | R^{D31} | R^{D1} |
| L_{C189} | R^{D4} | R^{D30} | H | L_{C609} | R^{D76} | R^{D9} | H | L_{C1029} | R^{D22} | R^{D32} | R^{D1} |
| L_{C190} | R^{D4} | R^{D31} | H | L_{C610} | R^{D76} | R^{D10} | H | L_{C1030} | R^{D22} | R^{D33} | R^{D1} |
| L_{C191} | R^{D4} | R^{D32} | H | L_{C611} | R^{D76} | R^{D12} | H | L_{C1031} | R^{D22} | R^{D34} | R^{D1} |
| L_{C192} | R^{D4} | R^{D33} | H | L_{C612} | R^{D76} | R^{D15} | H | L_{C1032} | R^{D22} | R^{D35} | R^{D1} |
| L_{C193} | R^{D4} | R^{D34} | H | L_{C613} | R^{D76} | R^{D16} | H | L_{C1033} | R^{D22} | R^{D40} | R^{D1} |
| Lci94 | R^{D4} | R^{D35} | H | L_{C614} | R^{D76} | R^{D17} | H | L_{C1034} | R^{D22} | R^{D41} | R^{D1} |
| Lci95 | R^{D4} | R^{D40} | H | L_{C615} | R^{D76} | R^{D18} | H | L_{C1035} | R^{D22} | R^{D42} | R^{D1} |
| L_{C196} | R^{D4} | R^{D41} | H | L_{C616} | R^{D76} | R^{D19} | H | L_{C1036} | R^{D22} | R^{D64} | R^{D1} |
| L_{C197} | R^{D4} | R^{D42} | H | L_{C617} | R^{D76} | R^{D20} | H | L_{C1037} | R^{D22} | R^{D66} | R^{D1} |
| L_{C198} | R^{D4} | R^{D64} | H | L_{C618} | R^{D76} | R^{D21} | H | L_{C1038} | R^{D22} | R^{D68} | R^{D1} |
| L_{C199} | R^{D4} | R^{D66} | H | L_{C619} | R^{D76} | R^{D23} | H | L_{C1039} | R^{D22} | R^{D76} | R^{D1} |
| L_{C200} | R^{D4} | R^{D68} | H | L_{C620} | R^{D76} | R^{D24} | H | L_{C1040} | R^{D26} | R^{D5} | R^{D1} |
| L_{C201} | R^{D4} | R^{D76} | H | L_{C621} | R^{D76} | R^{D25} | H | L_{C1041} | R^{D26} | R^{D6} | R^{D1} |
| L_{C202} | R^{D4} | R^{D1} | H | L_{C622} | R^{D76} | R^{D27} | H | L_{C1042} | R^{D26} | R^{D9} | R^{D1} |
| L_{C203} | R^{D7} | R^{D5} | H | L_{C623} | R^{D76} | R^{D28} | H | L_{C1043} | R^{D26} | R^{D10} | R^{D1} |
| L_{C204} | R^{D7} | R^{D6} | H | L_{C624} | R^{D76} | R^{D29} | H | L_{C1044} | R^{D26} | R^{D12} | R^{D1} |
| L_{C205} | R^{D7} | R^{D8} | H | L_{C625} | R^{D76} | R^{D30} | H | L_{C1045} | R^{D26} | R^{D15} | R^{D1} |
| L_{C206} | R^{D7} | R^{D9} | H | L_{C626} | R^{D76} | R^{D31} | H | L_{C1046} | R^{D26} | R^{D16} | R^{D1} |
| L_{C207} | R^{D7} | R^{D10} | H | L_{C627} | R^{D76} | R^{D32} | H | L_{C1047} | R^{D26} | R^{D17} | R^{D1} |
| L_{C208} | R^{D7} | R^{D11} | H | L_{C628} | R^{D76} | R^{D33} | H | L_{C1048} | R^{D26} | R^{D18} | R^{D1} |
| L_{C209} | R^{D7} | R^{D12} | H | L_{C629} | R^{D76} | R^{D34} | H | L_{C1049} | R^{D26} | R^{D19} | R^{D1} |
| L_{C210} | R^{D7} | R^{D13} | H | L_{C630} | R^{D76} | R^{D42} | H | L_{C1050} | R^{D26} | R^{D20} | R^{D1} |
| L_{C211} | R^{D7} | R^{D14} | H | L_{C631} | R^{D1} | R^{D1} | R^{D1} | L_{C1051} | R^{D26} | R^{D21} | R^{D1} |
| L_{C212} | R^{D7} | R^{D15} | H | L_{C632} | R^{D2} | R^{D2} | R^{D1} | L_{C1052} | R^{D26} | R^{D23} | R^{D1} |
| L_{C213} | R^{D7} | R^{D16} | H | L_{C633} | R^{D3} | R^{D3} | R^{D1} | L_{C1053} | R^{D26} | R^{D24} | R^{D1} |
| L_{C214} | R^{D7} | R^{D17} | H | L_{C634} | R^{D4} | R^{D4} | R^{D1} | L_{C1054} | R^{D26} | R^{D25} | R^{D1} |
| L_{C215} | R^{D7} | R^{D18} | H | L_{C635} | R^{D5} | R^{D5} | R^{D1} | L_{C1055} | R^{D26} | R^{D27} | R^{D1} |
| L_{C216} | R^{D7} | R^{D19} | H | L_{C636} | R^{D6} | R^{D6} | R^{D1} | L_{C1056} | R^{D26} | R^{D28} | R^{D1} |
| L_{C217} | R^{D7} | R^{D20} | H | L_{C637} | R^{D7} | R^{D7} | R^{D1} | L_{C1057} | R^{D26} | R^{D29} | R^{D1} |
| L_{C218} | R^{D7} | R^{D21} | H | L_{C638} | R^{D8} | R^{D8} | R^{D1} | L_{C1058} | R^{D26} | R^{D30} | R^{D1} |
| L_{C219} | R^{D7} | R^{D22} | H | L_{C639} | R^{D9} | R^{D9} | R^{D1} | L_{C1059} | R^{D26} | R^{D31} | R^{D1} |
| L_{C220} | R^{D7} | R^{D23} | H | L_{C640} | R^{D10} | R^{D10} | R^{D1} | Lc₁₀₆₀ | R^{D26} | R^{D32} | R^{D1} |
| L_{C221} | R^{D7} | R^{D24} | H | L_{C641} | R^{D11} | R^{D11} | R^{D1} | L_{C1061} | R^{D26} | R^{D33} | R^{D1} |
| L_{C222} | R^{D7} | R^{D25} | H | L_{C642} | R^{D12} | R^{D12} | R^{D1} | L_{C1062} | R^{D26} | R^{D34} | R^{D1} |
| L_{C223} | R^{D7} | R^{D26} | H | L_{C643} | R^{D13} | R^{D13} | R^{D1} | L_{C1063} | R^{D26} | R^{D35} | R^{D1} |
| L_{C224} | R^{D7} | R^{D27} | H | L_{C644} | R^{D14} | R^{D14} | R^{D1} | L_{C1064} | R^{D26} | R^{D40} | R^{D1} |
| L_{C225} | R^{D7} | R^{D28} | H | L_{C645} | R^{D15} | R^{D15} | R^{D1} | L_{C1065} | R^{D26} | R^{D41} | R^{D1} |
| L_{C226} | R^{D7} | R^{D29} | H | L_{C646} | R^{D16} | R^{D16} | R^{D1} | L_{C1066} | R^{D26} | R^{D42} | R^{D1} |
| L_{C227} | R^{D7} | R^{D30} | H | L_{C647} | R^{D17} | R^{D17} | R^{D1} | L_{C1067} | R^{D26} | R^{D64} | R^{D1} |
| L_{C228} | R^{D7} | R^{D31} | H | L_{C648} | R^{D18} | R^{D18} | R^{D1} | L_{C1068} | R^{D26} | R^{D66} | R^{D1} |
| L_{C229} | R^{D7} | R^{D32} | H | L_{C649} | R^{D19} | R^{D19} | R^{D1} | L_{C1069} | R^{D26} | R^{D68} | R^{D1} |
| L_{C230} | R^{D7} | R^{D33} | H | L_{C650} | R^{D20} | R^{D20} | R^{D1} | L_{C1070} | R^{D26} | R^{D76} | R^{D1} |
| L_{C231} | R^{D7} | R^{D34} | H | L_{C651} | R^{D21} | R^{D21} | R^{D1} | L_{C1071} | R^{D35} | R^{D5} | R^{D1} |
| L_{C232} | R^{D7} | R^{D35} | H | L_{C652} | R^{D22} | R^{D22} | R^{D1} | L_{C1072} | R^{D35} | R^{D6} | R^{D1} |
| L_{C233} | R^{D7} | R^{D40} | H | L_{C653} | R^{D23} | R^{D23} | R^{D1} | L_{C1073} | R^{D35} | R^{D9} | R^{D1} |
| L_{C234} | R^{D7} | R^{D41} | H | L_{C654} | R^{D24} | R^{D24} | R^{D1} | L_{C1074} | R^{D35} | R^{D10} | R^{D1} |
| L_{C235} | R^{D7} | R^{D42} | H | L_{C655} | R^{D25} | R^{D25} | R^{D1} | L_{C1075} | R^{D35} | R^{D12} | R^{D1} |
| L_{C236} | R^{D7} | R^{D64} | H | L_{C656} | R^{D26} | R^{D26} | R^{D1} | L_{C1076} | R^{D35} | R^{D15} | R^{D1} |
| L_{C237} | R^{D7} | R^{D66} | H | L_{C657} | R^{D27} | R^{D27} | R^{D1} | L_{C1077} | R^{D35} | R^{D16} | R^{D1} |
| L_{C238} | R^{D7} | R^{D68} | H | L_{C658} | R^{D28} | R^{D28} | R^{D1} | L_{C1078} | R^{D35} | R^{D17} | R^{D1} |
| L_{C239} | R^{D7} | R^{D76} | H | L_{C659} | R^{D29} | R^{D29} | R^{D1} | L_{C1079} | R^{D35} | R^{D18} | R^{D1} |
| L_{C240} | R^{D8} | R^{D5} | H | L_{C660} | R^{D30} | R^{D30} | R^{D1} | L_{C1080} | R^{D35} | R^{D19} | R^{D1} |
| L_{C241} | R^{D8} | R^{D6} | H | L_{C661} | R^{D31} | R^{D31} | R^{D1} | L_{C1081} | R^{D35} | R^{D20} | R^{D1} |
| L_{C242} | R^{D8} | R^{D9} | H | L_{C662} | R^{D32} | R^{D32} | R^{D1} | L_{C1082} | R^{D35} | R^{D21} | R^{D1} |
| L_{C243} | R^{D8} | R^{D10} | H | L_{C663} | R^{D33} | R^{D33} | R^{D1} | L_{C1083} | R^{D35} | R^{D23} | R^{D1} |
| L_{C244} | R^{D8} | R^{D11} | H | L_{C664} | R^{D34} | R^{D34} | R^{D1} | L_{C1084} | R^{D35} | R^{D24} | R^{D1} |
| L_{C245} | R^{D8} | R^{D12} | H | L_{C665} | R^{D35} | R^{D35} | R^{D1} | L_{C1085} | R^{D35} | R^{D25} | R^{D1} |
| L_{C246} | R^{D8} | R^{D13} | H | L_{C666} | R^{D40} | R^{D40} | R^{D1} | L_{C1086} | R^{D35} | R^{D27} | R^{D1} |
| L_{C247} | R^{D8} | R^{D14} | H | L_{C667} | R^{D41} | R^{D41} | R^{D1} | L_{C1087} | R^{D35} | R^{D28} | R^{D1} |
| L_{C248} | R^{D8} | R^{D15} | H | L_{C668} | R^{D42} | R^{D42} | R^{D1} | L_{C1088} | R^{D35} | R^{D29} | R^{D1} |
| L_{C249} | R^{D8} | R^{D16} | H | L_{C669} | R^{D64} | R^{D64} | R^{D1} | L_{C1089} | R^{D35} | R^{D30} | R^{D1} |
| L_{C250} | R^{D8} | R^{D17} | H | L_{C670} | R^{D66} | R^{D66} | R^{D1} | L_{C1090} | R^{D35} | R^{D31} | R^{D1} |
| L_{C251} | R^{D8} | R^{D18} | H | L_{C671} | R^{D68} | R^{D68} | R^{D1} | L_{C1091} | R^{D35} | R^{D32} | R^{D1} |
| L_{C252} | R^{D8} | R^{D19} | H | L_{C672} | R^{D76} | R^{D76} | R^{D1} | L_{C1092} | R^{D35} | R^{D33} | R^{D1} |
| L_{C253} | R^{D8} | R^{D20} | H | L_{C673} | R^{D1} | R^{D2} | R^{D1} | L_{C1093} | R^{D35} | R^{D34} | R^{D1} |
| L_{C254} | R^{D8} | R^{D21} | H | L_{C674} | R^{D1} | R^{D3} | R^{D1} | L_{C1094} | R^{D35} | R^{D40} | R^{D1} |
| L_{C255} | R^{D8} | R^{D22} | H | L_{C675} | R^{D1} | R^{D4} | R^{D1} | L_{C1095} | R^{D35} | R^{D41} | R^{D1} |
| L_{C256} | R^{D8} | R^{D23} | H | L_{C676} | R^{D1} | R^{D5} | R^{D1} | L_{C1096} | R^{D35} | R^{D42} | R^{D1} |
| L_{C257} | R^{D8} | R^{D24} | H | L_{C677} | R^{D1} | R^{D6} | R^{D1} | L_{C1097} | R^{D35} | R^{D64} | R^{D1} |
| L_{C258} | R^{D8} | R^{D25} | H | L_{C678} | R^{D1} | R^{D7} | R^{D1} | L_{C1098} | R^{D35} | R^{D66} | R^{D1} |
| L_{C259} | R^{D8} | R^{D26} | H | L_{C679} | R^{D1} | R^{D8} | R^{D1} | L_{C1099} | R^{D35} | R^{D68} | R^{D1} |
| L_{C260} | R^{D8} | R^{D27} | H | L_{C680} | R^{D1} | R^{D9} | R^{D1} | L_{C1100} | R^{D35} | R^{D76} | R^{D1} |
| L_{C261} | R^{D8} | R^{D28} | H | L_{C681} | R^{D1} | R^{D10} | R^{D1} | L_{C1101} | R^{D40} | R^{D5} | R^{D1} |
| L_{C262} | R^{D8} | R^{D29} | H | L_{C682} | R^{D1} | R^{D11} | R^{D1} | L_{C1102} | R^{D40} | R^{D6} | R^{D1} |
| L_{C263} | R^{D8} | R^{D30} | H | L_{C683} | R^{D1} | R^{D12} | R^{D1} | L_{C1103} | R^{D40} | R^{D9} | R^{D1} |
| L_{C264} | R^{D8} | R^{D31} | H | L_{C684} | R^{D1} | R^{D13} | R^{D1} | L_{C1104} | R^{D40} | R^{D10} | R^{D1} |
| L_{C265} | R^{D8} | R^{D32} | H | L_{C685} | R^{D1} | R^{D14} | R^{D1} | L_{C1105} | R^{D40} | R^{D12} | R^{D1} |
| L_{C266} | R^{D8} | R^{D33} | H | L_{C686} | R^{D1} | R^{D15} | R^{D1} | L_{C1106} | R^{D40} | R^{D15} | R^{D1} |
| L_{C267} | R^{D8} | R^{D34} | H | L_{C687} | R^{D1} | R^{D16} | R^{D1} | L_{C1107} | R^{D40} | R^{D16} | R^{D1} |
| L_{C268} | R^{D8} | R^{D35} | H | L_{C688} | R^{D1} | R^{D17} | R^{D1} | L_{C1108} | R^{D40} | R^{D17} | R^{D1} |
| L_{C269} | R^{D8} | R^{D40} | H | L_{C689} | R^{D1} | R^{D18} | R^{D1} | L_{C1109} | R^{D40} | R^{D18} | R^{D1} |
| L_{C270} | R^{D8} | R^{D41} | H | L_{C690} | R^{D1} | R^{D19} | R^{D1} | L_{C1110} | R^{D40} | R^{D19} | R^{D1} |
| L_{C271} | R^{D8} | R^{D42} | H | L_{C691} | R^{D1} | R^{D20} | R^{D1} | L_{C1111} | R^{D40} | R^{D20} | R^{D1} |
| L_{C272} | R^{D8} | R^{D64} | H | L_{C692} | R^{D1} | R^{D21} | R^{D1} | L_{C1112} | R^{D40} | R^{D21} | R^{D1} |
| L_{C273} | R^{D8} | R^{D66} | H | L_{C693} | R^{D1} | R^{D22} | R^{D1} | L_{C1113} | R^{D40} | R^{D23} | R^{D1} |
| L_{C274} | R^{D8} | R^{D68} | H | L_{C694} | R^{D1} | R^{D23} | R^{D1} | L_{C1114} | R^{D40} | R^{D24} | R^{D1} |
| L_{C275} | R^{D8} | R^{D76} | H | L_{C695} | R^{D1} | R^{D24} | R^{D1} | L_{C1115} | R^{D40} | R^{D25} | R^{D1} |
| L_{C276} | R^{D11} | R^{D5} | H | L_{C696} | R^{D1} | R^{D25} | R^{D1} | L_{C1116} | R^{D40} | R^{D27} | R^{D1} |
| L_{C277} | R^{D11} | R^{D6} | H | L_{C697} | R^{D1} | R^{D26} | R^{D1} | L_{C1117} | R^{D40} | R^{D28} | R^{D1} |
| L_{C278} | R^{D11} | R^{D9} | H | L_{C698} | R^{D1} | R^{D27} | R^{D1} | L_{C1118} | R^{D40} | R^{D29} | R^{D1} |
| L_{C279} | R^{D11} | R^{D10} | H | L_{C699} | R^{D1} | R^{D28} | R^{D1} | L_{C1119} | R^{D40} | R^{D30} | R^{D1} |
| L_{C280} | R^{D11} | R^{D12} | H | L_{C700} | R^{D1} | R^{D29} | R^{D1} | L_{C1120} | R^{D40} | R^{D31} | R^{D1} |
| L_{C281} | R^{D11} | R^{D13} | H | L_{C701} | R^{D1} | R^{D30} | R^{D1} | L_{C1121} | R^{D40} | R^{D32} | R^{D1} |
| L_{C282} | R^{D11} | R^{D14} | H | L_{C702} | R^{D1} | R^{D31} | R^{D1} | L_{C1122} | R^{D40} | R^{D33} | R^{D1} |
| L_{C283} | R^{D11} | R^{D15} | H | L_{C703} | R^{D1} | R^{D32} | R^{D1} | L_{C1123} | R^{D40} | R^{D34} | R^{D1} |
| L_{C284} | R^{D11} | R^{D16} | H | L_{C704} | R^{D1} | R^{D33} | R^{D1} | L_{C1124} | R^{D40} | R^{D41} | R^{D1} |
| L_{C285} | R^{D11} | R^{D17} | H | L_{C705} | R^{D1} | R^{D34} | R^{D1} | L_{C1125} | R^{D40} | R^{D42} | R^{D1} |
| L_{C286} | R^{D11} | R^{D18} | H | L_{C706} | R^{D1} | R^{D35} | R^{D1} | L_{C1126} | R^{D40} | R^{D64} | R^{D1} |
| L_{C287} | R^{D11} | R^{D19} | H | L_{C707} | R^{D1} | R^{D40} | R^{D1} | L_{C1127} | R^{D40} | R^{D66} | R^{D1} |
| L_{C288} | R^{D11} | R^{D20} | H | L_{C708} | R^{D1} | R^{D41} | R^{D1} | L_{C1128} | R^{D40} | R^{D68} | R^{D1} |
| L_{C289} | R^{D11} | R^{D21} | H | L_{C709} | R^{D1} | R^{D42} | R^{D1} | L_{C1129} | R^{D40} | R^{D76} | R^{D1} |
| L_{C290} | R^{D11} | R^{D22} | H | L_{C710} | R^{D1} | R^{D64} | R^{D1} | L_{C1130} | R^{D41} | R^{D5} | R^{D1} |
| L_{C291} | R^{D11} | R^{D23} | H | L_{C711} | R^{D1} | R^{D66} | R^{D1} | L_{C1131} | R^{D41} | R^{D6} | R^{D1} |
| L_{C292} | R^{D11} | R^{D24} | H | L_{C712} | R^{D1} | R^{D68} | R^{D1} | L_{C1132} | R^{D41} | R^{D9} | R^{D1} |
| L_{C293} | R^{D11} | R^{D25} | H | L_{C713} | R^{D1} | R^{D76} | R^{D1} | L_{C1133} | R^{D41} | R^{D10} | R^{D1} |
| L_{C294} | R^{D11} | R^{D26} | H | L_{C714} | R^{D2} | R^{D1} | R^{D1} | L_{C1134} | R^{D41} | R^{D12} | R^{D1} |
| L_{C295} | R^{D11} | R^{D27} | H | L_{C715} | R^{D2} | R^{D3} | R^{D1} | L_{C1135} | R^{D41} | R^{D15} | R^{D1} |
| L_{C296} | R^{D11} | R^{D28} | H | L_{C716} | R^{D2} | R^{D4} | R^{D1} | L_{C1136} | R^{D41} | R^{D16} | R^{D1} |
| L_{C297} | R^{D11} | R^{D29} | H | L_{C717} | R^{D2} | R^{D5} | R^{D1} | L_{C1137} | R^{D41} | R^{D17} | R^{D1} |
| L_{C298} | R^{D11} | R^{D30} | H | L_{C718} | R^{D2} | R^{D6} | R^{D1} | L_{C1138} | R^{D41} | R^{D18} | R^{D1} |
| L_{C299} | R^{D11} | R^{D31} | H | L_{C719} | R^{D2} | R^{D7} | R^{D1} | L_{C1139} | R^{D41} | R^{D19} | R^{D1} |
| L_{C300} | R^{D11} | R^{D32} | H | L_{C720} | R^{D2} | R^{D8} | R^{D1} | L_{C1140} | R^{D41} | R^{D20} | R^{D1} |
| L_{C301} | R^{D11} | R^{D33} | H | L_{C721} | R^{D2} | R^{D9} | R^{D1} | L_{C1141} | R^{D41} | R^{D21} | R^{D1} |
| L_{C302} | R^{D11} | R^{D34} | H | L_{C722} | R^{D2} | R^{D10} | R^{D1} | L_{C1142} | R^{D41} | R^{D23} | R^{D1} |
| L_{C303} | R^{D11} | R^{D35} | H | L_{C723} | R^{D2} | R^{D11} | R^{D1} | L_{C1143} | R^{D41} | R^{D24} | R^{D1} |
| L_{C304} | R^{D11} | R^{D40} | H | L_{C724} | R^{D2} | R^{D12} | R^{D1} | L_{C1144} | R^{D41} | R^{D25} | R^{D1} |
| L_{C305} | R^{D11} | R^{D41} | H | L_{C725} | R^{D2} | R^{D13} | R^{D1} | L_{C1145} | R^{D41} | R^{D27} | R^{D1} |
| L_{C306} | R^{D11} | R^{D42} | H | L_{C726} | R^{D2} | R^{D14} | R^{D1} | L_{C1146} | R^{D41} | R^{D28} | R^{D1} |
| L_{C307} | R^{D11} | R^{D64} | H | L_{C727} | R^{D2} | R^{D15} | R^{D1} | L_{C1147} | R^{D41} | R^{D29} | R^{D1} |
| L_{C308} | R^{D11} | R^{D66} | H | L_{C728} | R^{D2} | R^{D16} | R^{D1} | L_{C1148} | R^{D41} | R^{D30} | R^{D1} |
| L_{C309} | R^{D11} | R^{D68} | H | L_{C729} | R^{D2} | R^{D17} | R^{D1} | L_{C1149} | R^{D41} | R^{D31} | R^{D1} |
| L_{C310} | R^{D11} | R^{D76} | H | L_{C730} | R^{D2} | R^{D18} | R^{D1} | L_{C1150} | R^{D41} | R^{D32} | R^{D1} |
| L_{C311} | R^{D13} | R^{D5} | H | L_{C731} | R^{D2} | R^{D19} | R^{D1} | L_{C1151} | R^{D41} | R^{D33} | R^{D1} |
| L_{C312} | R^{D13} | R^{D6} | H | L_{C732} | R^{D2} | R^{D20} | R^{D1} | L_{C1152} | R^{D41} | R^{D34} | R^{D1} |
| L_{C313} | R^{D13} | R^{D9} | H | L_{C733} | R^{D2} | R^{D21} | R^{D1} | L_{C1153} | R^{D41} | R^{D42} | R^{D1} |
| L_{C314} | R^{D13} | R^{D10} | H | L_{C734} | R^{D2} | R^{D22} | R^{D1} | L_{C1154} | R^{D41} | R^{D64} | R^{D1} |
| L_{C315} | R^{D13} | R^{D12} | H | L_{C735} | R^{D2} | R^{D23} | R^{D1} | L_{C1155} | R^{D41} | R^{D66} | R^{D1} |
| L_{C316} | R^{D13} | R^{D14} | H | L_{C736} | R^{D2} | R^{D24} | R^{D1} | L_{C1156} | R^{D41} | R^{D68} | R^{D1} |
| L_{C317} | R^{D13} | R^{D15} | H | L_{C737} | R^{D2} | R^{D25} | R^{D1} | L_{C1157} | R^{D41} | R^{D76} | R^{D1} |
| L_{C318} | R^{D13} | R^{D16} | H | L_{C738} | R^{D2} | R^{D26} | R^{D1} | L_{C1158} | R^{D64} | R^{D5} | R^{D1} |
| L_{C319} | R^{D13} | R^{D17} | H | L_{C739} | R^{D2} | R^{D27} | R^{D1} | L_{C1159} | R^{D64} | R^{D6} | R^{D1} |
| L_{C320} | R^{D13} | R^{D18} | H | L_{C740} | R^{D2} | R^{D28} | R^{D1} | L_{C1160} | R^{D64} | R^{D9} | R^{D1} |
| L_{C321} | R^{D13} | R^{D19} | H | L_{C741} | R^{D2} | R^{D29} | R^{D1} | L_{C1161} | R^{D64} | R^{D10} | R^{D1} |
| L_{C322} | R^{D13} | R^{D20} | H | L_{C742} | R^{D2} | R^{D30} | R^{D1} | L_{C1162} | R^{D64} | R^{D12} | R^{D1} |
| L_{C323} | R^{D13} | R^{D21} | H | L_{C743} | R^{D2} | R^{D31} | R^{D1} | L_{C1163} | R^{D64} | R^{D15} | R^{D1} |
| L_{C324} | R^{D13} | R^{D22} | H | L_{C744} | R^{D2} | R^{D32} | R^{D1} | L_{C1164} | R^{D64} | R^{D16} | R^{D1} |
| L_{C325} | R^{D13} | R^{D23} | H | L_{C745} | R^{D2} | R^{D33} | R^{D1} | L_{C1165} | R^{D64} | R^{D17} | R^{D1} |
| L_{C326} | R^{D13} | R^{D24} | H | L_{C746} | R^{D2} | R^{D34} | R^{D1} | L_{C1166} | R^{D64} | R^{D18} | R^{D1} |
| L_{C327} | R^{D13} | R^{D25} | H | L_{C747} | R^{D2} | R^{D35} | R^{D1} | L_{C1167} | R^{D64} | R^{D19} | R^{D1} |
| L_{C328} | R^{D13} | R^{D26} | H | L_{C748} | R^{D2} | R^{D40} | R^{D1} | L_{C1168} | R^{D64} | R^{D20} | R^{D1} |
| L_{C329} | R^{D13} | R^{D27} | H | L_{C749} | R^{D2} | R^{D41} | R^{D1} | L_{C1169} | R^{D64} | R^{D21} | R^{D1} |
| L_{C330} | R^{D13} | R^{D28} | H | L_{C750} | R^{D2} | R^{D42} | R^{D1} | L_{C1170} | R^{D64} | R^{D23} | R^{D1} |
| L_{C331} | R^{D13} | R^{D29} | H | L_{C751} | R^{D2} | R^{D64} | R^{D1} | L_{C1171} | R^{D64} | R^{D24} | R^{D1} |
| L_{C332} | R^{D13} | R^{D30} | H | L_{C752} | R^{D2} | R^{D66} | R^{D1} | L_{C1172} | R^{D64} | R^{D25} | R^{D1} |
| L_{C333} | R^{D13} | R^{D31} | H | L_{C753} | R^{D2} | R^{D68} | R^{D1} | L_{C1173} | R^{D64} | R^{D27} | R^{D1} |
| L_{C334} | R^{D13} | R^{D32} | H | L_{C754} | R^{D2} | R^{D76} | R^{D1} | L_{C1174} | R^{D64} | R^{D28} | R^{D1} |
| L_{C335} | R^{D13} | R^{D33} | H | L_{C755} | R^{D3} | R^{D4} | R^{D1} | L_{C1175} | R^{D64} | R^{D29} | R^{D1} |
| L_{C336} | R^{D13} | R^{D34} | H | L_{C756} | R^{D3} | R^{D5} | R^{D1} | L_{C1176} | R^{D64} | R^{D30} | R^{D1} |
| L_{C337} | R^{D13} | R^{D35} | H | L_{C757} | R^{D3} | R^{D6} | R^{D1} | L_{C1177} | R^{D64} | R^{D31} | R^{D1} |
| L_{C338} | R^{D13} | R^{D40} | H | L_{C758} | R^{D3} | R^{D7} | R^{D1} | L_{C1178} | R^{D64} | R^{D32} | R^{D1} |
| L_{C339} | R^{D13} | R^{D41} | H | L_{C759} | R^{D3} | R^{D8} | R^{D1} | L_{C1179} | R^{D64} | R^{D33} | R^{D1} |
| L_{C340} | R^{D13} | R^{D42} | H | L_{C760} | R^{D3} | R^{D9} | R^{D1} | L_{C1180} | R^{D64} | R^{D34} | R^{D1} |
| L_{C341} | R^{D13} | R^{D64} | H | L_{C761} | R^{D3} | R^{D10} | R^{D1} | L_{C1181} | R^{D64} | R^{D42} | R^{D1} |
| L_{C342} | R^{D13} | R^{D66} | H | L_{C762} | R^{D3} | R^{D11} | R^{D1} | L_{C1182} | R^{D64} | R^{D64} | R^{D1} |
| L_{C343} | R^{D13} | R^{D68} | H | L_{C763} | R^{D3} | R^{D12} | R^{D1} | L_{C1183} | R^{D64} | R^{D66} | R^{D1} |
| L_{C344} | R^{D13} | R^{D76} | H | L_{C764} | R^{D3} | R^{D13} | R^{D1} | L_{C1184} | R^{D64} | R^{D68} | R^{D1} |
| L_{C345} | R^{D14} | R^{D5} | H | L_{C765} | R^{D3} | R^{D14} | R^{D1} | L_{C1185} | R^{D64} | R^{D76} | R^{D1} |
| L_{C346} | R^{D14} | R^{D6} | H | L_{C766} | R^{D3} | R^{D15} | R^{D1} | L_{C1186} | R^{D66} | R^{D5} | R^{D1} |
| L_{C347} | R^{D14} | R^{D9} | H | L_{C767} | R^{D3} | R^{D16} | R^{D1} | L_{C1187} | R^{D66} | R^{D6} | R^{D1} |
| L_{C348} | R^{D14} | R^{D10} | H | L_{C768} | R^{D3} | R^{D17} | R^{D1} | L_{C1188} | R^{D66} | R^{D9} | R^{D1} |
| L_{C349} | R^{D14} | R^{D12} | H | L_{C769} | R^{D3} | R^{D18} | R^{D1} | L_{C1189} | R^{D66} | R^{D10} | R^{D1} |
| L_{C350} | R^{D14} | R^{D15} | H | L_{C770} | R^{D3} | R^{D19} | R^{D1} | L_{C1190} | R^{D66} | R^{D12} | R^{D1} |
| L_{C351} | R^{D14} | R^{D16} | H | L_{C771} | R^{D3} | R^{D20} | R^{D1} | L_{C1191} | R^{D66} | R^{D15} | R^{D1} |
| L_{C352} | R^{D14} | R^{D17} | H | L_{C772} | R^{D3} | R^{D21} | R^{D1} | L_{C1192} | R^{D66} | R^{D16} | R^{D1} |
| L_{C353} | R^{D14} | R^{D18} | H | L_{C773} | R^{D3} | R^{D22} | R^{D1} | L_{C1193} | R^{D66} | R^{D17} | R^{D1} |
| L_{C354} | R^{D14} | R^{D19} | H | L_{C774} | R^{D3} | R^{D23} | R^{D1} | L_{C1194} | R^{D66} | R^{D18} | R^{D1} |
| L_{C355} | R^{D14} | R^{D20} | H | L_{C775} | R^{D3} | R^{D24} | R^{D1} | L_{C1195} | R^{D66} | R^{D19} | R^{D1} |
| L_{C356} | R^{D14} | R^{D21} | H | L_{C776} | R^{D3} | R^{D25} | R^{D1} | L_{C1196} | R^{D66} | R^{D20} | R^{D1} |
| L_{C357} | R^{D14} | R^{D22} | H | L_{C777} | R^{D3} | R^{D26} | R^{D1} | L_{C1197} | R^{D66} | R^{D21} | R^{D1} |
| L_{C358} | R^{D14} | R^{D23} | H | L_{C778} | R^{D3} | R^{D27} | R^{D1} | L_{C1198} | R^{D66} | R^{D23} | R^{D1} |
| L_{C359} | R^{D14} | R^{D24} | H | L_{C779} | R^{D3} | R^{D28} | R^{D1} | L_{C1199} | R^{D66} | R^{D24} | R^{D1} |
| L_{C360} | R^{D14} | R^{D25} | H | L_{C780} | R^{D3} | R^{D29} | R^{D1} | L_{C1200} | R^{D66} | R^{D25} | R^{D1} |
| L_{C361} | R^{D14} | R^{D26} | H | L_{C781} | R^{D3} | R^{D30} | R^{D1} | L_{C1201} | R^{D66} | R^{D27} | R^{D1} |
| L_{C362} | R^{D14} | R^{D27} | H | L_{C782} | R^{D3} | R^{D31} | R^{D1} | L_{C1202} | R^{D66} | R^{D28} | R^{D1} |
| L_{C363} | R^{D14} | R^{D28} | H | L_{C783} | R^{D3} | R^{D32} | R^{D1} | L_{C1203} | R^{D66} | R^{D29} | R^{D1} |
| L_{C364} | R^{D14} | R^{D29} | H | L_{C784} | R^{D3} | R^{D33} | R^{D1} | L_{C1204} | R^{D66} | R^{D30} | R^{D1} |
| L_{C365} | R^{D14} | R^{D30} | H | L_{C785} | R^{D3} | R^{D34} | R^{D1} | L_{C1205} | R^{D66} | R^{D31} | R^{D1} |
| L_{C366} | R^{D14} | R^{D31} | H | L_{C786} | R^{D3} | R^{D35} | R^{D1} | L_{C1206} | R^{D66} | R^{D32} | R^{D1} |
| L_{C367} | R^{D14} | R^{D32} | H | L_{C787} | R^{D3} | R^{D40} | R^{D1} | L_{C1207} | R^{D66} | R^{D33} | R^{D1} |
| L_{C368} | R^{D14} | R^{D33} | H | L_{C788} | R^{D3} | R^{D41} | R^{D1} | L_{C1208} | R^{D66} | R^{D34} | R^{D1} |
| L_{C369} | R^{D14} | R^{D34} | H | L_{C789} | R^{D3} | R^{D42} | R^{D1} | L_{C1209} | R^{D66} | R^{D42} | R^{D1} |
| L_{C370} | R^{D14} | R^{D35} | H | L_{C790} | R^{D3} | R^{D64} | R^{D1} | L_{C1210} | R^{D66} | R^{D68} | R^{D1} |
| L_{C371} | R^{D14} | R^{D40} | H | L_{C791} | R^{D3} | R^{D66} | R^{D1} | L_{C1211} | R^{D66} | R^{D76} | R^{D1} |
| L_{C372} | R^{D14} | R^{D41} | H | L_{C792} | R^{D3} | R^{D68} | R^{D1} | L_{C1212} | R^{D68} | R^{D5} | R^{D1} |
| L_{C373} | R^{D14} | R^{D42} | H | L_{C793} | R^{D3} | R^{D76} | R^{D1} | L_{C1213} | R^{D68} | R^{D6} | R^{D1} |
| L_{C374} | R^{D14} | R^{D64} | H | L_{C794} | R^{D4} | R^{D5} | R^{D1} | L_{C1214} | R^{D68} | R^{D9} | R^{D1} |
| L_{C375} | R^{D14} | R^{D66} | H | L_{C795} | R^{D4} | R^{D6} | R^{D1} | L_{C1215} | R^{D68} | R^{D10} | R^{D1} |
| L_{C376} | R^{D14} | R^{D68} | H | L_{C796} | R^{D4} | R^{D7} | R^{D1} | L_{C1216} | R^{D68} | R^{D12} | R^{D1} |
| L_{C377} | R^{D14} | R^{D76} | H | L_{C797} | R^{D4} | R^{D8} | R^{D1} | L_{C1217} | R^{D68} | R^{D15} | R^{D1} |
| L_{C378} | R^{D22} | R^{D5} | H | L_{C798} | R^{D4} | R^{D9} | R^{D1} | L_{C1218} | R^{D68} | R^{D16} | R^{D1} |
| L_{C379} | R^{D22} | R^{D6} | H | L_{C799} | R^{D4} | R^{D10} | R^{D1} | L_{C1219} | R^{D68} | R^{D17} | R^{D1} |
| L_{C380} | R^{D22} | R^{D9} | H | L_{C800} | R^{D4} | R^{D11} | R^{D1} | L_{C1220} | R^{D68} | R^{D18} | R^{D1} |
| L_{C381} | R^{D22} | R^{D10} | H | L_{C801} | R^{D4} | R^{D12} | R^{D1} | L_{C1221} | R^{D68} | R^{D19} | R^{D1} |
| L_{C382} | R^{D22} | R^{D12} | H | L_{C802} | R^{D4} | R^{D13} | R^{D1} | L_{C1222} | R^{D68} | R^{D20} | R^{D1} |
| L_{C383} | R^{D22} | R^{D15} | H | L_{C803} | R^{D4} | R^{D14} | R^{D1} | L_{C1223} | R^{D68} | R^{D21} | R^{D1} |
| L_{C384} | R^{D22} | R^{D16} | H | L_{C804} | R^{D4} | R^{D15} | R^{D1} | L_{C1224} | R^{D68} | R^{D23} | R^{D1} |
| L_{C385} | R^{D22} | R^{D17} | H | L_{C805} | R^{D4} | R^{D16} | R^{D1} | L_{C1225} | R^{D68} | R^{D24} | R^{D1} |
| L_{C386} | R^{D22} | R^{D18} | H | L_{C806} | R^{D4} | R^{D17} | R^{D1} | L_{C1226} | R^{D68} | R^{D25} | R^{D1} |
| L_{C387} | R^{D22} | R^{D19} | H | L_{C807} | R^{D4} | R^{D18} | R^{D1} | L_{C1227} | R^{D68} | R^{D27} | R^{D1} |
| L_{C388} | R^{D22} | R^{D20} | H | L_{C808} | R^{D4} | R^{D19} | R^{D1} | L_{C1228} | R^{D68} | R^{D28} | R^{D1} |
| L_{C389} | R^{D22} | R^{D21} | H | L_{C809} | R^{D4} | R^{D20} | R^{D1} | L_{C1229} | R^{D68} | R^{D29} | R^{D1} |
| L_{C390} | R^{D22} | R^{D23} | H | L_{C810} | R^{D4} | R^{D21} | R^{D1} | L_{C1230} | R^{D68} | R^{D30} | R^{D1} |
| L_{C391} | R^{D22} | R^{D24} | H | L_{C811} | R^{D4} | R^{D22} | R^{D1} | L_{C1231} | R^{D68} | R^{D31} | R^{D1} |
| L_{C392} | R^{D22} | R^{D25} | H | L_{C812} | R^{D4} | R^{D23} | R^{D1} | L_{C1232} | R^{D68} | R^{D32} | R^{D1} |
| L_{C393} | R^{D22} | R^{D26} | H | L_{C813} | R^{D4} | R^{D24} | R^{D1} | L_{C1233} | R^{D68} | R^{D33} | R^{D1} |
| L_{C394} | R^{D22} | R^{D27} | H | L_{C814} | R^{D4} | R^{D25} | R^{D1} | L_{C1234} | R^{D68} | R^{D34} | R^{D1} |
| L_{C395} | R^{D22} | R^{D28} | H | L_{C815} | R^{D4} | R^{D26} | R^{D1} | L_{C1235} | R^{D68} | R^{D42} | R^{D1} |
| L_{C396} | R^{D22} | R^{D29} | H | L_{C816} | R^{D4} | R^{D27} | R^{D1} | L_{C1236} | R^{D68} | R^{D76} | R^{D1} |
| L_{C397} | R^{D22} | R^{D30} | H | L_{C817} | R^{D4} | R^{D28} | R^{D1} | L_{C1237} | R^{D76} | R^{D5} | R^{D1} |
| L_{C398} | R^{D22} | R^{D31} | H | L_{C818} | R^{D4} | R^{D29} | R^{D1} | L_{C1238} | R^{D76} | R^{D6} | R^{D1} |
| L_{C399} | R^{D22} | R^{D32} | H | L_{C819} | R^{D4} | R^{D30} | R^{D1} | L_{C1239} | R^{D76} | R^{D9} | R^{D1} |
| L_{C400} | R^{D22} | R^{D33} | H | L_{C820} | R^{D4} | R^{D31} | R^{D1} | L_{C1240} | R^{D76} | R^{D10} | R^{D1} |
| L_{C401} | R^{D22} | R^{D34} | H | L_{C821} | R^{D4} | R^{D32} | R^{D1} | L_{C1241} | R^{D76} | R^{D12} | R^{D1} |
| L_{C402} | R^{D22} | R^{D35} | H | L_{C822} | R^{D4} | R^{D33} | R^{D1} | L_{C1242} | R^{D76} | R^{D15} | R^{D1} |
| L_{C403} | R^{D22} | R^{D40} | H | L_{C823} | R^{D4} | R^{D34} | R^{D1} | L_{C1243} | R^{D76} | R^{D16} | R^{D1} |
| L_{C404} | R^{D22} | R^{D41} | H | L_{C824} | R^{D4} | R^{D35} | R^{D1} | L_{C1244} | R^{D76} | R^{D17} | R^{D1} |
| L_{C405} | R^{D22} | R^{D42} | H | L_{C825} | R^{D4} | R^{D40} | R^{D1} | L_{C1245} | R^{D76} | R^{D18} | R^{D1} |
| L_{C406} | R^{D22} | R^{D64} | H | L_{C826} | R^{D4} | R^{D41} | R^{D1} | L_{C1246} | R^{D76} | R^{D19} | R^{D1} |
| L_{C407} | R^{D22} | R^{D66} | H | L_{C827} | R^{D4} | R^{D42} | R^{D1} | L_{C1247} | R^{D76} | R^{D20} | R^{D1} |
| L_{C408} | R^{D22} | R^{D68} | H | L_{C828} | R^{D4} | R^{D64} | R^{D1} | L_{C1248} | R^{D76} | R^{D21} | R^{D1} |
| L_{C409} | R^{D22} | R^{D76} | H | L_{C829} | R^{D4} | R^{D66} | R^{D1} | L_{C1249} | R^{D76} | R^{D23} | R^{D1} |
| L_{C410} | R^{D26} | R^{D5} | H | L_{C830} | R^{D4} | R^{D68} | R^{D1} | L_{C1250} | R^{D76} | R^{D24} | R^{D1} |
| L_{C411} | R^{D26} | R^{D6} | H | L_{C831} | R^{D4} | R^{D76} | R^{D1} | L_{C1251} | R^{D76} | R^{D25} | R^{D1} |
| L_{C412} | R^{D26} | R^{D9} | H | L_{C832} | R^{D4} | R^{D1} | R^{D1} | L_{C1252} | R^{D76} | R^{D27} | R^{D1} |
| L_{C413} | R^{D26} | R^{D10} | H | L_{C833} | R^{D7} | R^{D5} | R^{D1} | L_{C1253} | R^{D76} | R^{D28} | R^{D1} |
| L_{C414} | R^{D26} | R^{D12} | H | L_{C834} | R^{D7} | R^{D6} | R^{D1} | L_{C1254} | R^{D76} | R^{D29} | R^{D1} |
| L_{C415} | R^{D26} | R^{D15} | H | L_{C835} | R^{D7} | R^{D8} | R^{D1} | L_{C1255} | R^{D76} | R^{D30} | R^{D1} |
| L_{C416} | R^{D26} | R^{D16} | H | L_{C836} | R^{D7} | R^{D9} | R^{D1} | L_{C1256} | R^{D76} | R^{D31} | R^{D1} |
| L_{C417} | R^{D26} | R^{D17} | H | L_{C837} | R^{D7} | R^{D10} | R^{D1} | L_{C1257} | R^{D76} | R^{D32} | R^{D1} |
| L_{C418} | R^{D26} | R^{D18} | H | L_{C838} | R^{D7} | R^{D11} | R^{D1} | L_{C1258} | R^{D76} | R^{D33} | R^{D1} |
| L_{C419} | R^{D26} | R^{D19} | H | L_{C839} | R^{D7} | R^{D12} | R^{D1} | L_{C1259} | R^{D76} | R^{D34} | R^{D1} |
| L_{C420} | R^{D26} | R^{D20} | H | L_{C840} | R^{D7} | R^{D13} | R^{D1} | L_{C1260} | R^{D76} | R^{D42} | R^{D1} |

where R^{D1} to R^{D21} have the following structures:

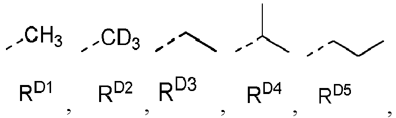

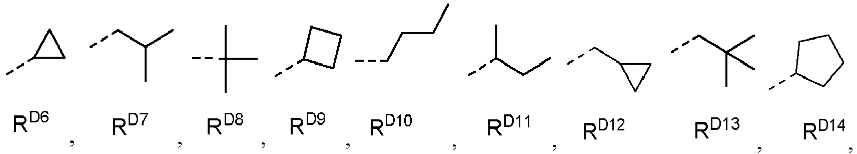

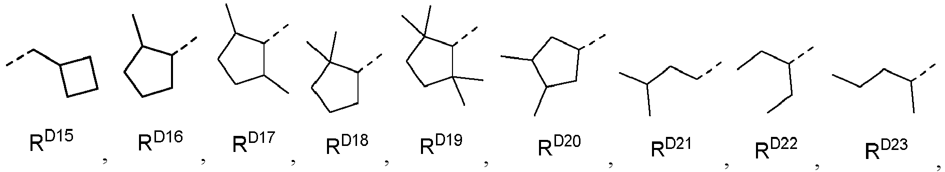

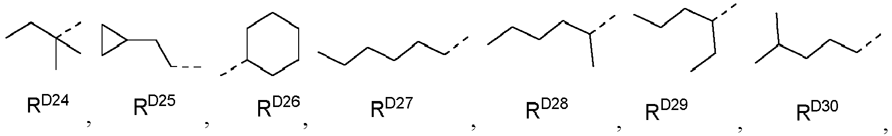

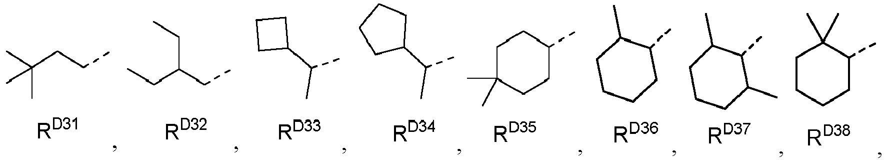

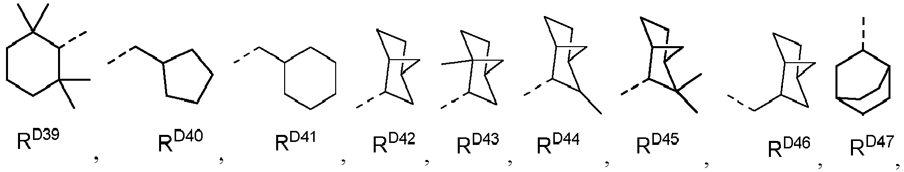

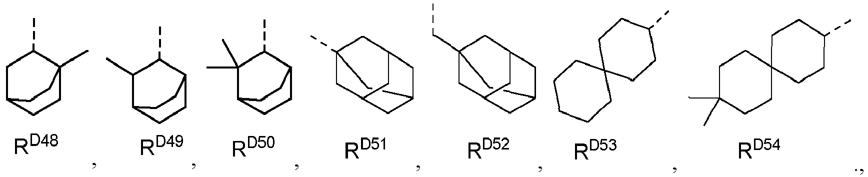

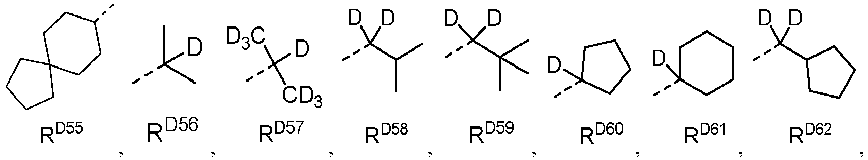

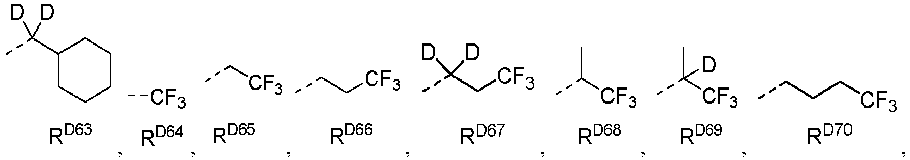

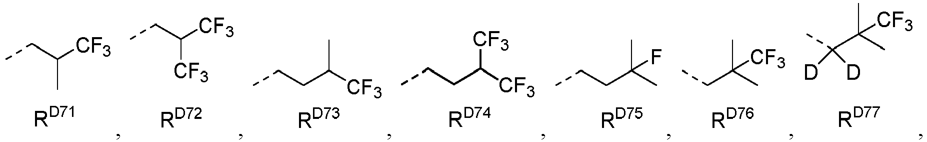

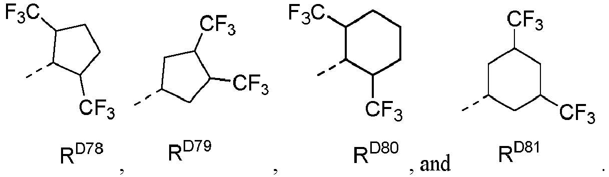

In some embodiments, an organic light emitting device (OLED) is described. The OLED can include an anode; a cathode; and an organic layer, disposed between the anode and the cathode, where the organic layer includes a compound comprising a first ligand L_{A} of Formula I as described herein.

In some embodiments, a consumer product comprising an OLED as described herein is described.

In some embodiments, the OLED has one or more characteristics selected from the group consisting of being flexible, being rollable, being foldable, being stretchable, and being curved. In some embodiments, the OLED is transparent or semi-transparent. In some embodiments, the OLED further comprises a layer comprising carbon nanotubes.

In some embodiments, the OLED further comprises a layer comprising a delayed fluorescent emitter. In some embodiments, the OLED comprises a RGB pixel arrangement or white plus color filter pixel arrangement. In some embodiments, the OLED is a mobile device, a hand held device, or a wearable device. In some embodiments, the OLED is a display panel having less than 10 inch diagonal or 50 square inch area. In some embodiments, the OLED is a display panel having at least 10 inch diagonal or 50 square inch area. In some embodiments, the OLED is a lighting panel.

According to another aspect, an emissive region in an OLED (e.g., the organic layer described herein) is disclosed. The emissive region comprises a compound comprising a first ligand L_{A} of Formula I as described herein. In some embodiments, the first compound in the emissive region is an emissive dopant or a non-emissive dopant. In some embodiments, the emissive dopant further comprises a host, wherein the host comprises at least one selected from the group consisting of metal complex, triphenylene, carbazole, dibenzothiophene, dibenzofuran, dibenzoselenophene, aza-triphenylene, azacarbazole, aza-dibenzothiophene, aza-dibenzofuran, and aza-dibenzoselenophene. In some embodiments, the emissive region further comprises a host, wherein the host is selected from the group consisting of: 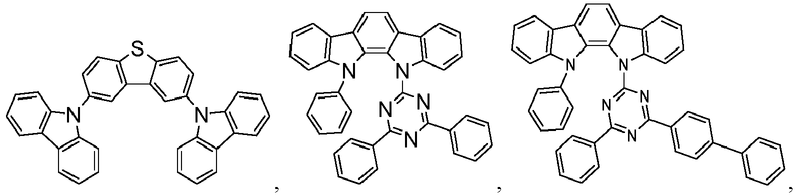 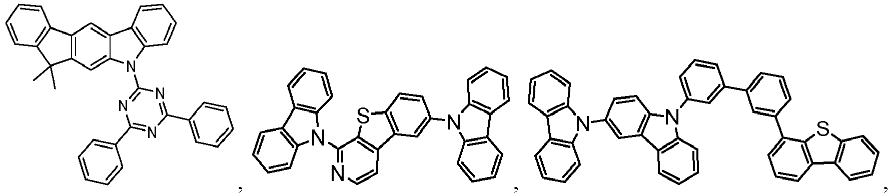 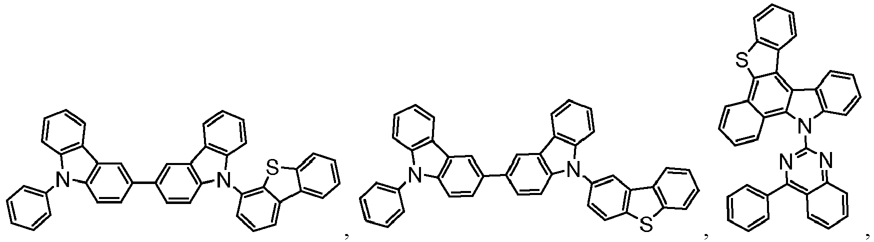 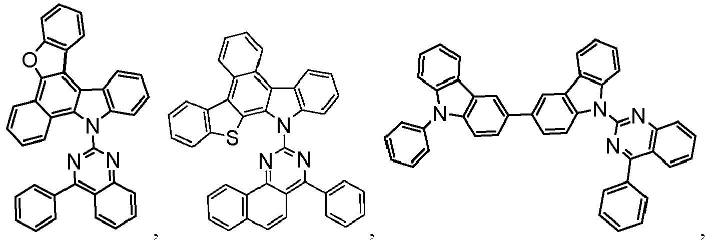 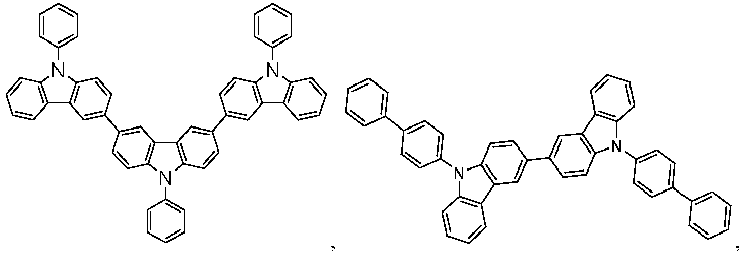 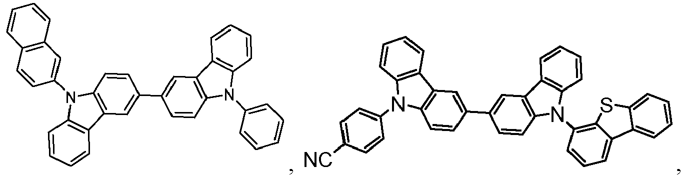 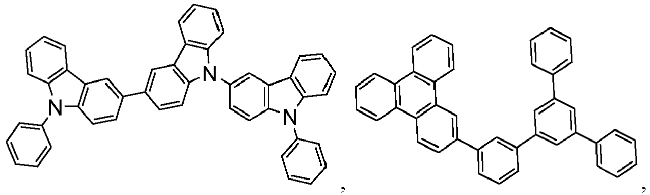 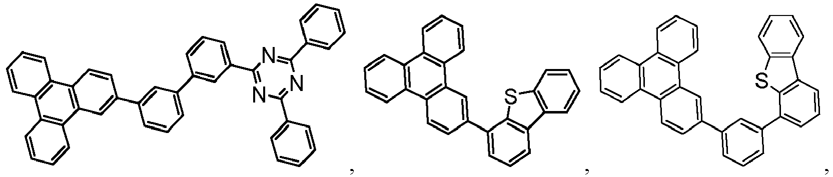 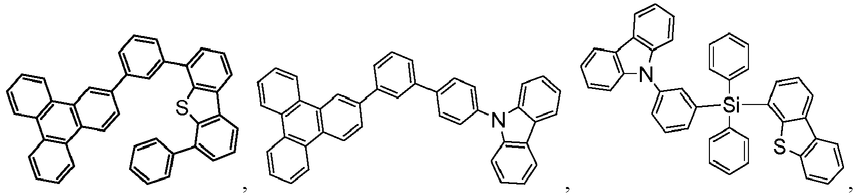 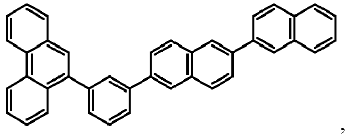 and combinations thereof.

In some embodiments, the compound can be an emissive dopant. In some embodiments, the compound can produce emissions via phosphorescence, fluorescence, thermally activated delayed fluorescence, i.e., TADF (also referred to as E-type delayed fluorescence; *see, e*.*g*., U.S. Application No. 15/700,352, which is hereby incorporated by reference in its entirety), triplet-triplet annihilation, or combinations of these processes. In some embodiments, the emissive dopant can be a racemic mixture, or can be enriched in one enantiomer.

According to another aspect, a formulation comprising the compound described herein is also disclosed.

The OLED disclosed herein can be incorporated into one or more of a consumer product, an electronic component module, and a lighting panel. The organic layer can be an emissive layer and the compound can be an emissive dopant in some embodiments, while the compound can be a non-emissive dopant in other embodiments.

The organic layer can also include a host. In some embodiments, two or more hosts are preferred. In some embodiments, the hosts used maybe a) bipolar, b) electron transporting, c) hole transporting or d) wide band gap materials that play little role in charge transport. In some embodiments, the host can include a metal complex. The host can be a triphenylene containing benzo-fused thiophene or benzo-fused furan. Any substituent in the host can be an unfused substituent independently selected from the group consisting of CₙH₂ₙ₊₁, OCₙH₂ₙ₊₁, OAr₁, N(CₙH₂ₙ₊₁)₂, N(Ar₁)(Ar₂), CH=CH-CₙH₂ₙ₊₁, C≡C-CₙH₂ₙ₊₁, Ar₁, Ar₁-Ar₂, and CₙH₂ₙ-Ar₁, or the host has no substitutions. In the preceding substituents n can range from 1 to 10; and Ar₁ and Ar₂ can be independently selected from the group consisting of benzene, biphenyl, naphthalene, triphenylene, carbazole, and heteroaromatic analogs thereof. The host can be an inorganic compound. For example a Zn containing inorganic material e.g. ZnS.

The host can be a compound comprising at least one chemical group selected from the group consisting of triphenylene, carbazole, dibenzothiophene, dibenzofuran, dibenzoselenophene, azatriphenylene, azacarbazole, aza-dibenzothiophene, aza-dibenzofuran, and aza-dibenzoselenophene. The host can include a metal complex. The host can be, but is not limited to, a specific compound selected from the group consisting of: 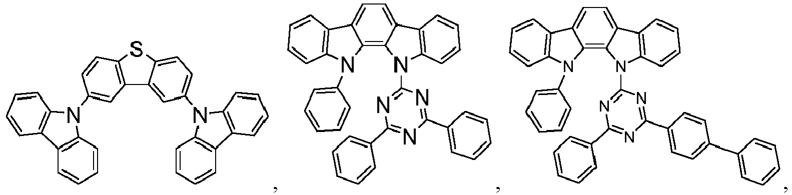 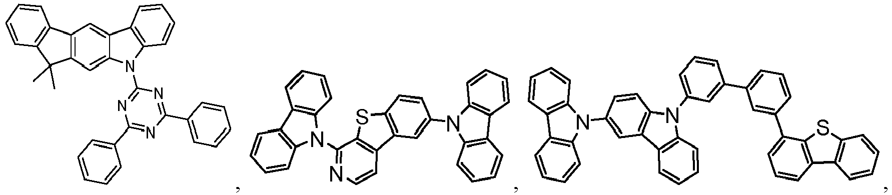 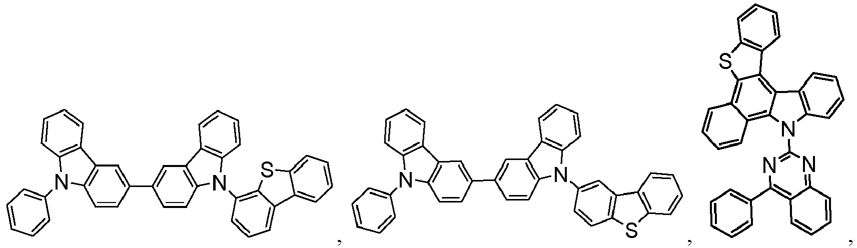 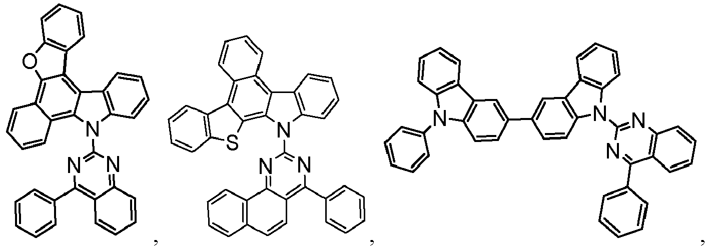 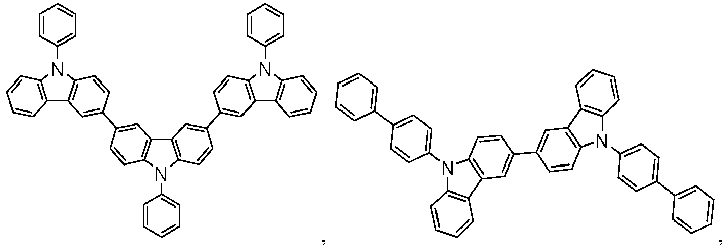 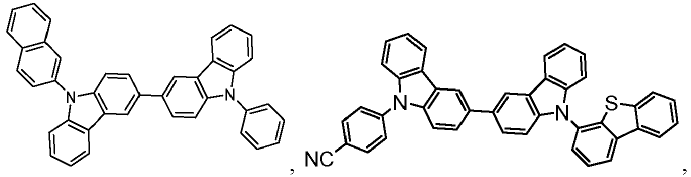 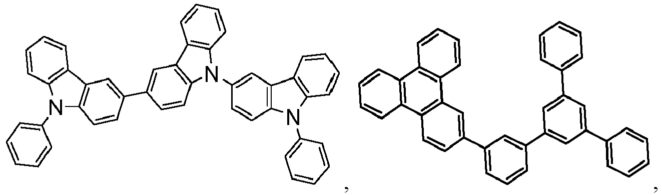 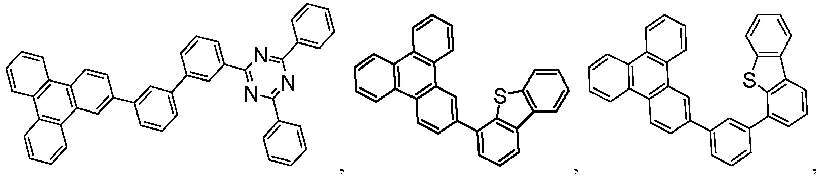 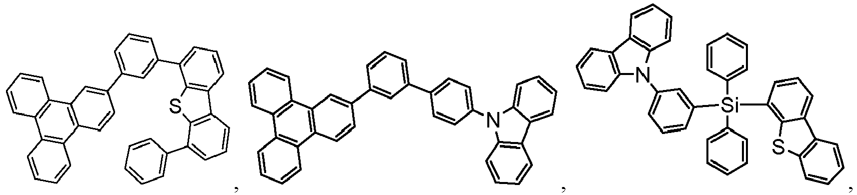 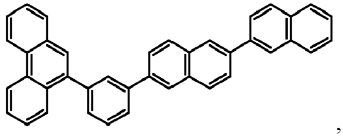 and combinations thereof.
Additional information on possible hosts is provided below.

In yet another aspect of the present disclosure, a formulation that comprises the novel compound disclosed herein is described. The formulation can include one or more components selected from the group consisting of a solvent, a host, a hole injection material, hole transport material, electron blocking material, hole blocking material, and an electron transport material, disclosed herein.

### COMBINATION WITH OTHER MATERIALS

The materials described herein as useful for a particular layer in an organic light emitting device may be used in combination with a wide variety of other materials present in the device. For example, emissive dopants disclosed herein may be used in conjunction with a wide variety of hosts, transport layers, blocking layers, injection layers, electrodes and other layers that may be present. The materials described or referred to below are non-limiting examples of materials that may be useful in combination with the compounds disclosed herein, and one of skill in the art can readily consult the literature to identify other materials that may be useful in combination.

### Conductivity Dopants:

A charge transport layer can be doped with conductivity dopants to substantially alter its density of charge carriers, which will in turn alter its conductivity. The conductivity is increased by generating charge carriers in the matrix material, and depending on the type of dopant, a change in the Fermi level of the semiconductor may also be achieved. Hole-transporting layer can be doped by p-type conductivity dopants and n-type conductivity dopants are used in the electron-transporting layer.

Non-limiting examples of the conductivity dopants that may be used in an OLED in combination with materials disclosed herein are exemplified below together with references that disclose those materials: EP01617493, EP01968131, EP2020694, EP2684932, US20050139810, US20070160905, US20090167167, US2010288362, WO06081780, WO2009003455, WO2009008277, WO2009011327, WO2014009310, US2007252140, US2015060804, US20150123047, and US2012146012.

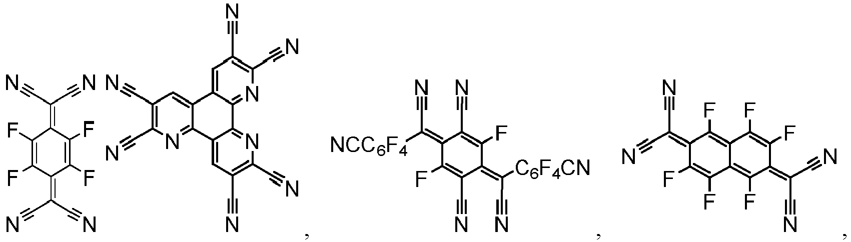

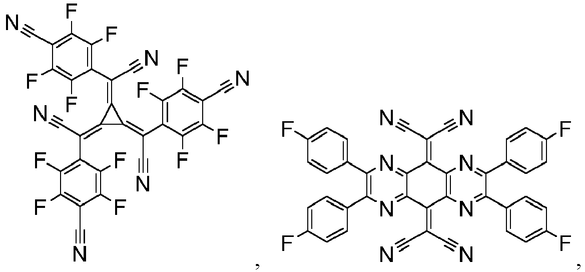

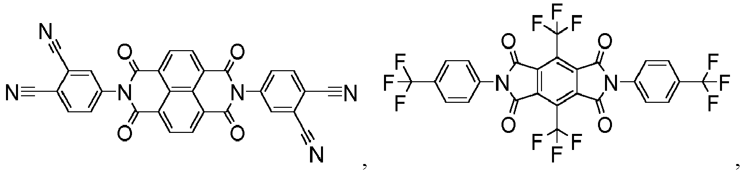

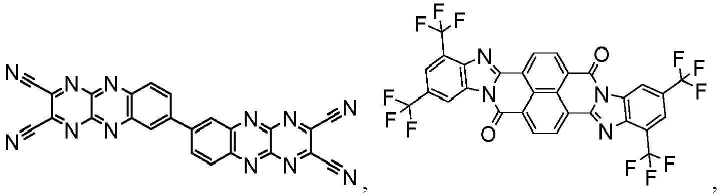

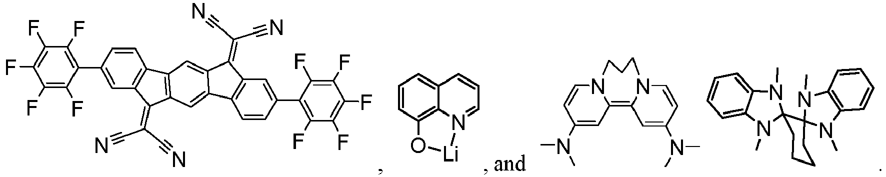

### HIL/HTL:

A hole injecting/transporting material to be used in the present invention is not particularly limited, and any compound may be used as long as the compound is typically used as a hole injecting/transporting material. Examples of the material include, but are not limited to: a phthalocyanine or porphyrin derivative; an aromatic amine derivative; an indolocarbazole derivative; a polymer containing fluorohydrocarbon; a polymer with conductivity dopants; a conducting polymer, such as PEDOT/PSS; a self-assembly monomer derived from compounds such as phosphonic acid and silane derivatives; a metal oxide derivative, such as MoOₓ; a p-type semiconducting organic compound, such as 1,4,5,8,9,12-Hexaazatriphenylenehexacarbonitrile; a metal complex, and a cross-linkable compounds.

Examples of aromatic amine derivatives used in HIL or HTL include, but not limit to the following general structures:

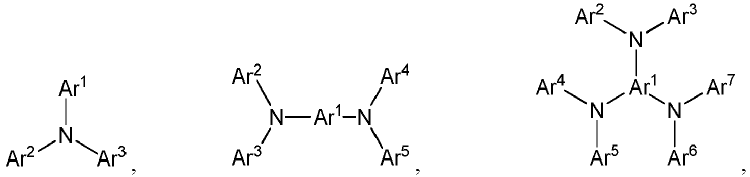

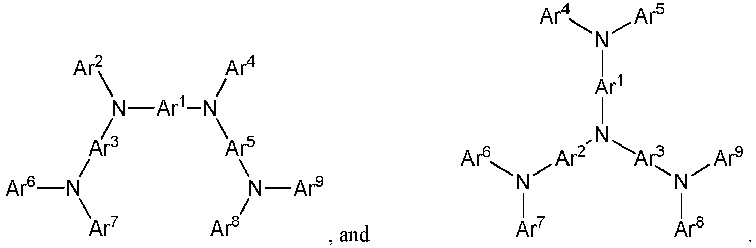

Each of Ar¹ to Ar⁹ is selected from the group consisting of aromatic hydrocarbon cyclic compounds such as benzene, biphenyl, triphenyl, triphenylene, naphthalene, anthracene, phenalene, phenanthrene, fluorene, pyrene, chrysene, perylene, and azulene; the group consisting of aromatic heterocyclic compounds such as dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine; and the group consisting of 2 to 10 cyclic structural units which are groups of the same type or different types selected from the aromatic hydrocarbon cyclic group and the aromatic heterocyclic group and are bonded to each other directly or via at least one of oxygen atom, nitrogen atom, sulfur atom, silicon atom, phosphorus atom, boron atom, chain structural unit and the aliphatic cyclic group. Each Ar may be unsubstituted or may be substituted by a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acids, ether, ester, nitrile, isonitrile, sulfanyl, sulfinyl, sulfonyl, phosphino, and combinations thereof.

In one aspect, Ar¹ to Ar⁹ is independently selected from the group consisting of: 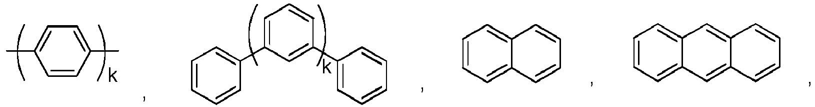 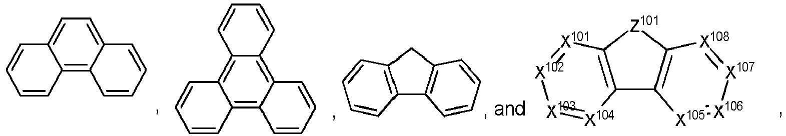 wherein k is an integer from 1 to 20; X¹⁰¹ to X¹⁰⁸ is C (including CH) or N; Z¹⁰¹ is NAr¹, O, or S; Ar¹ has the same group defined above.

Examples of metal complexes used in HIL or HTL include, but are not limited to the following general formula: 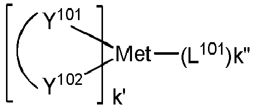 wherein Met is a metal, which can have an atomic weight greater than 40; (Y¹⁰¹-Y¹⁰²) is a bidentate ligand, Y¹⁰¹ and Y¹⁰² are independently selected from C, N, O, P, and S; L¹⁰¹ is an ancillary ligand; k' is an integer value from 1 to the maximum number of ligands that may be attached to the metal; and k'+k" is the maximum number of ligands that may be attached to the metal.

In one aspect, (Y¹⁰¹-Y¹⁰²) is a 2-phenylpyridine derivative. In another aspect, (Y¹⁰¹-Y¹⁰²) is a carbene ligand. In another aspect, Met is selected from Ir, Pt, Os, and Zn. In a further aspect, the metal complex has a smallest oxidation potential in solution vs. Fc⁺/Fc couple less than about 0.6 V.

Non-limiting examples of the HIL and HTL materials that may be used in an OLED in combination with materials disclosed herein are exemplified below together with references that disclose those materials: CN102702075, DE102012005215, EP01624500, EP01698613, EP01806334, EP01930964, EP01972613, EP01997799, EP02011790, EP02055700, EP02055701, EP1725079, EP2085382, EP2660300, EP650955, JP07-073529, JP2005112765, JP2007091719, JP2008021687, JP2014-009196, KR20110088898, KR20130077473, TW201139402, US06517957, US20020158242, US20030162053, US20050123751, US20060182993, US20060240279, US20070145888, US20070181874, US20070278938, US20080014464, US20080091025, US20080106190, US20080124572, US20080145707, US20080220265, US20080233434, US20080303417, US2008107919, US20090115320, US20090167161, US2009066235, US2011007385, US20110163302, US2011240968, US2011278551, US2012205642, US2013241401, US20140117329, US2014183517, US5061569, US5639914, WO05075451, WO07125714, WO08023550, WO08023759, WO2009145016, WO2010061824, WO2011075644, WO2012177006, WO2013018530, WO2013039073, WO2013087142, WO2013118812, WO2013120577, WO2013157367, WO2013175747, WO2014002873, WO2014015935, WO2014015937, WO2014030872, WO2014030921, WO2014034791, WO2014104514, WO2014157018. 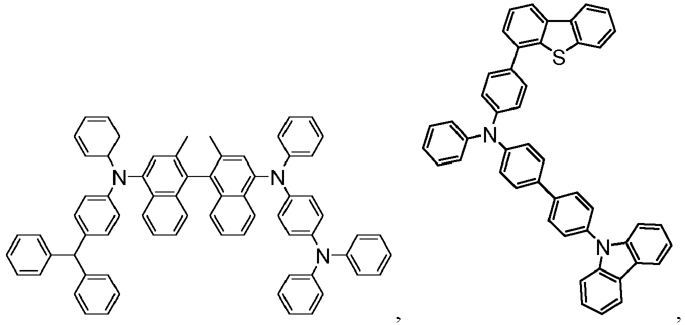 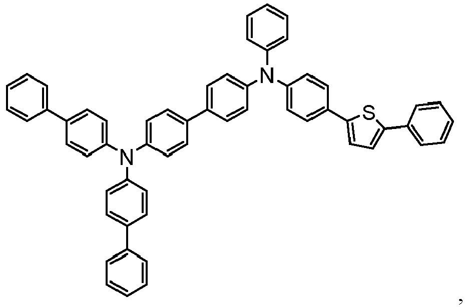 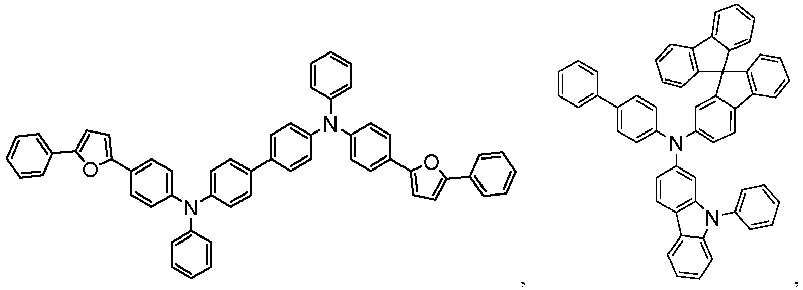 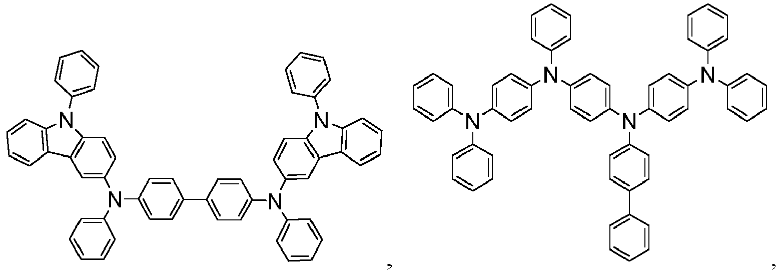 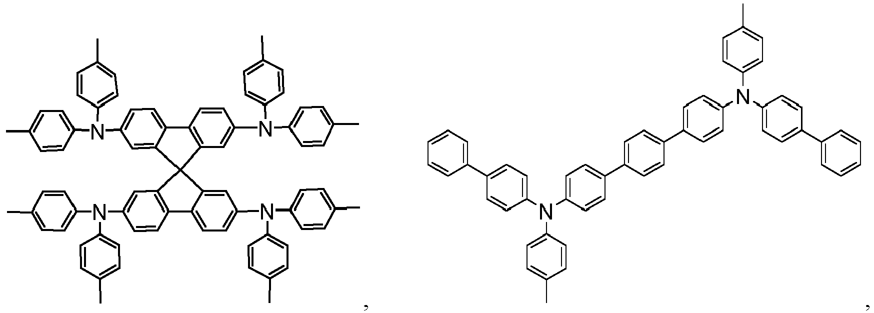 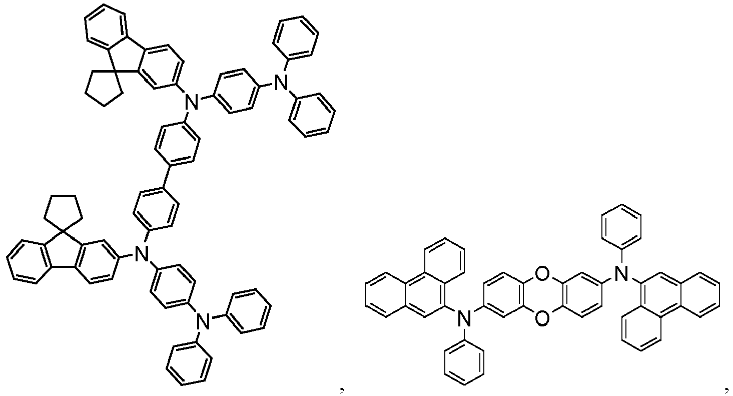 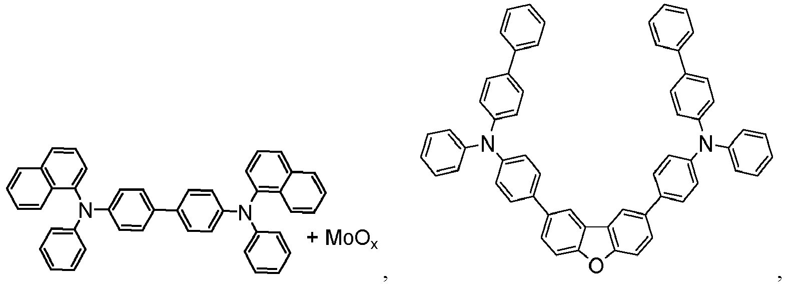 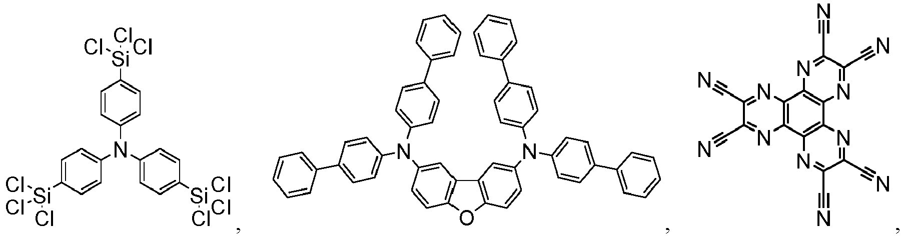 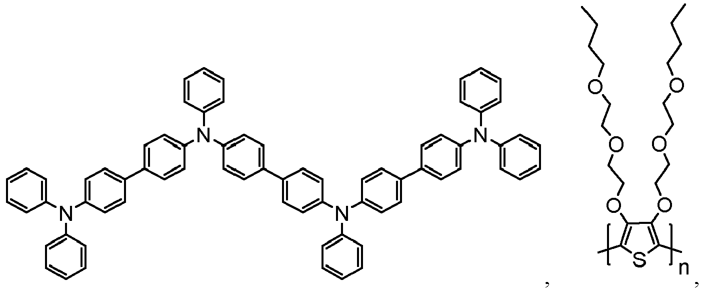 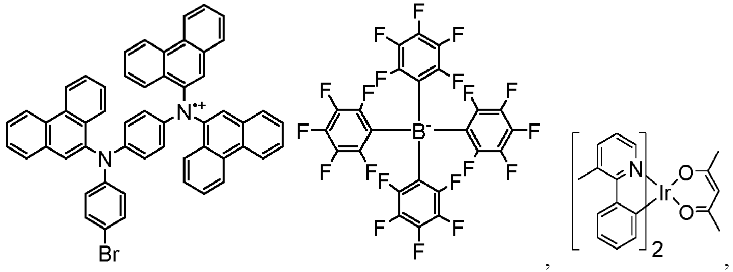 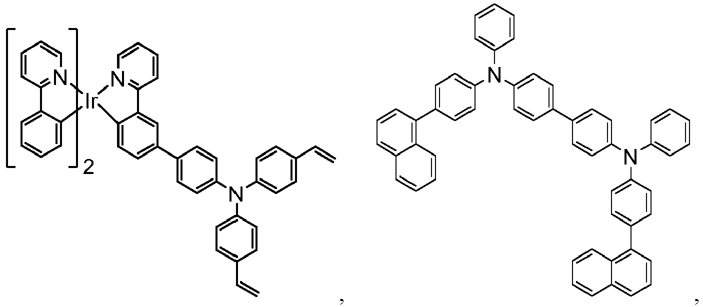 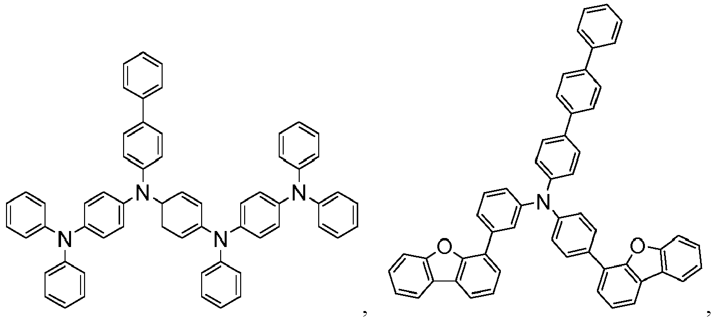 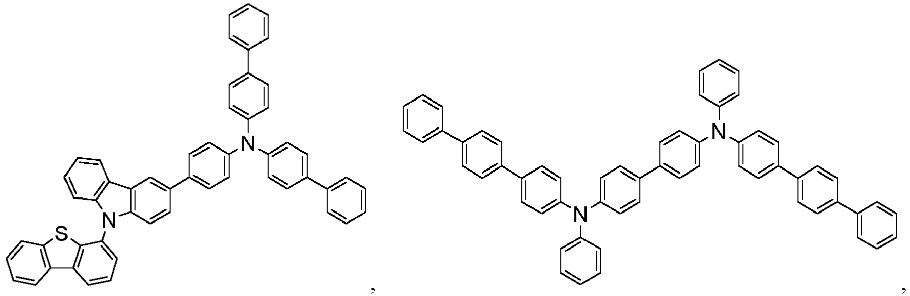 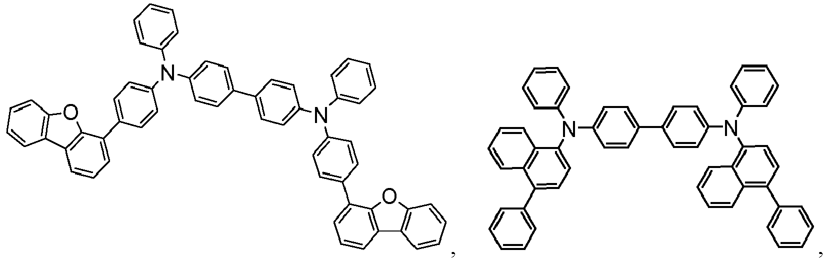 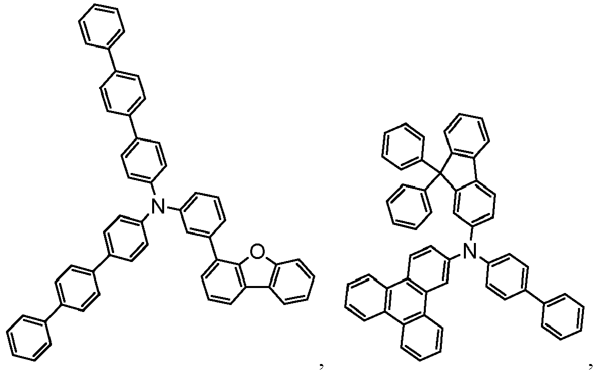 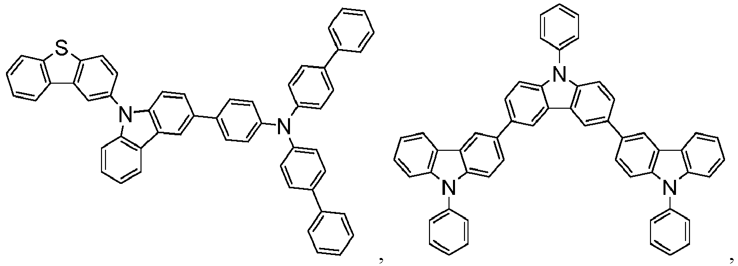 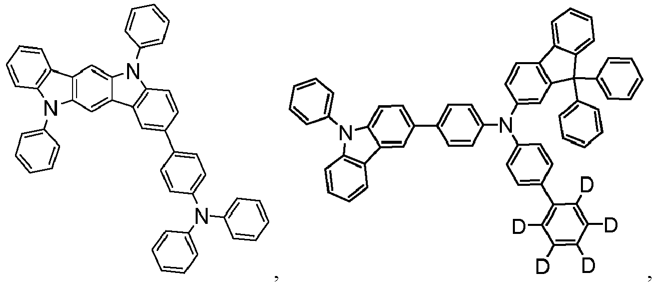 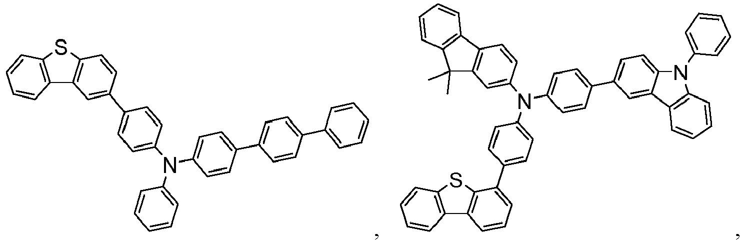 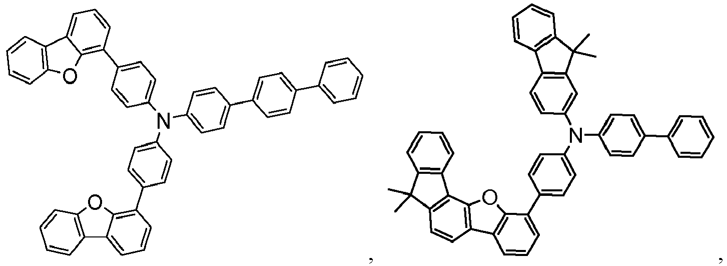 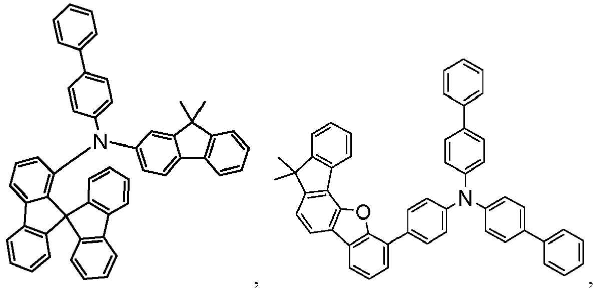 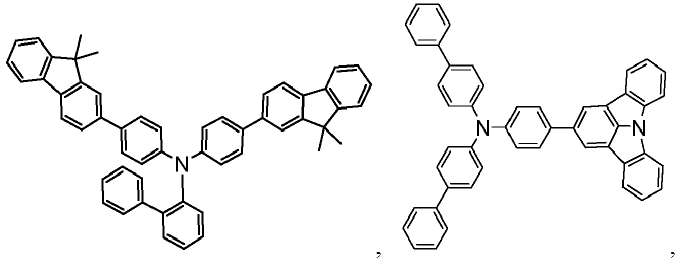 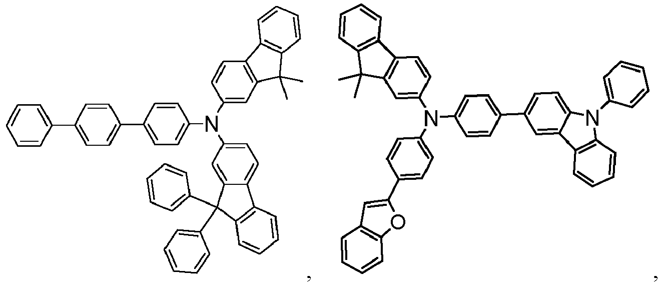 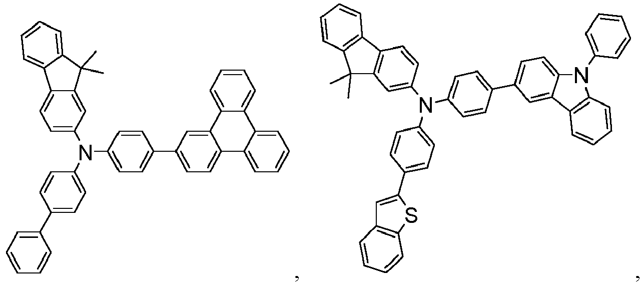 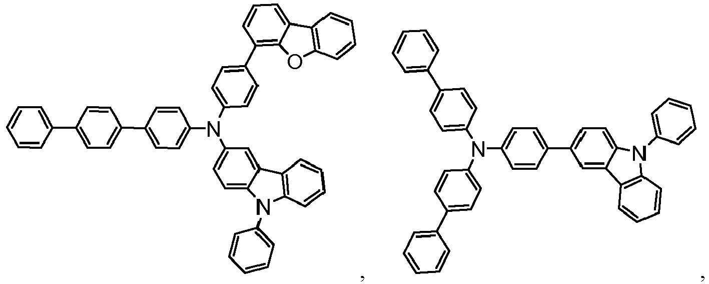 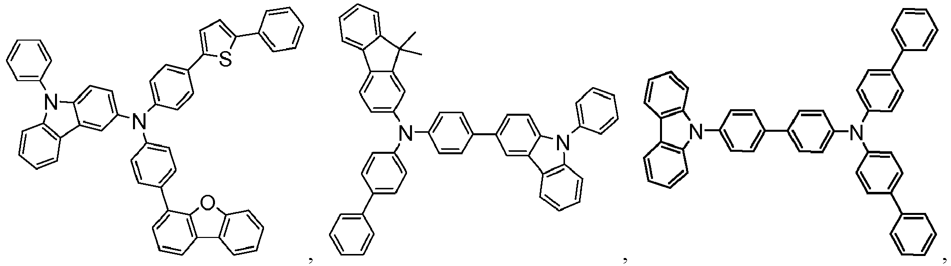 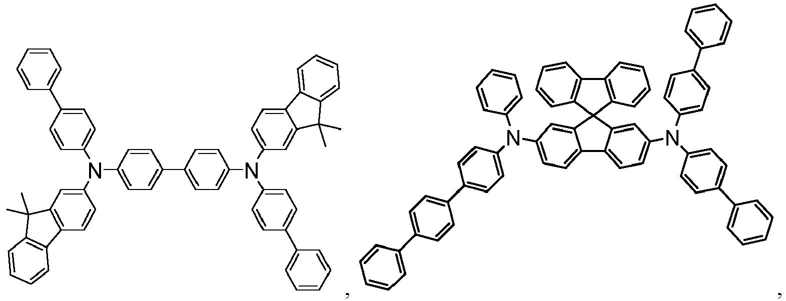 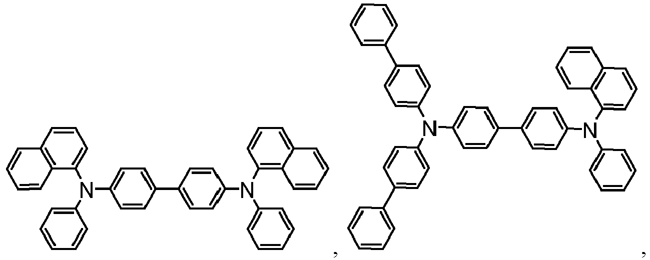 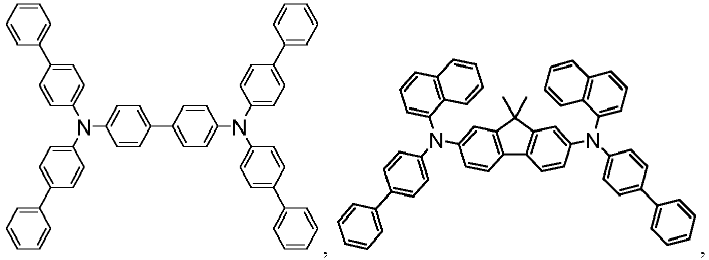 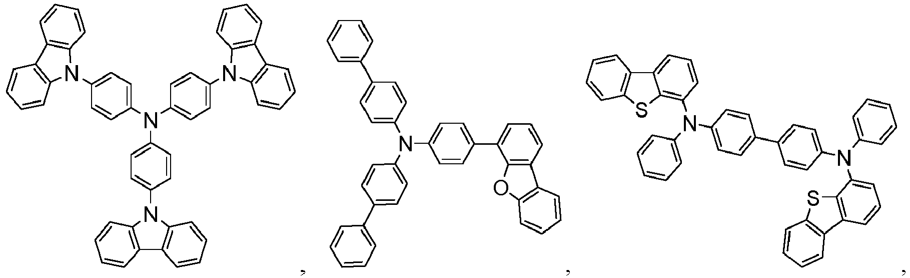 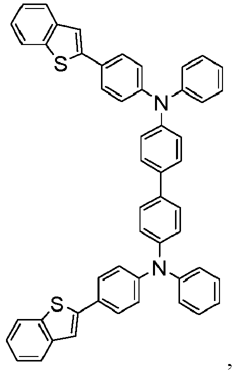 and

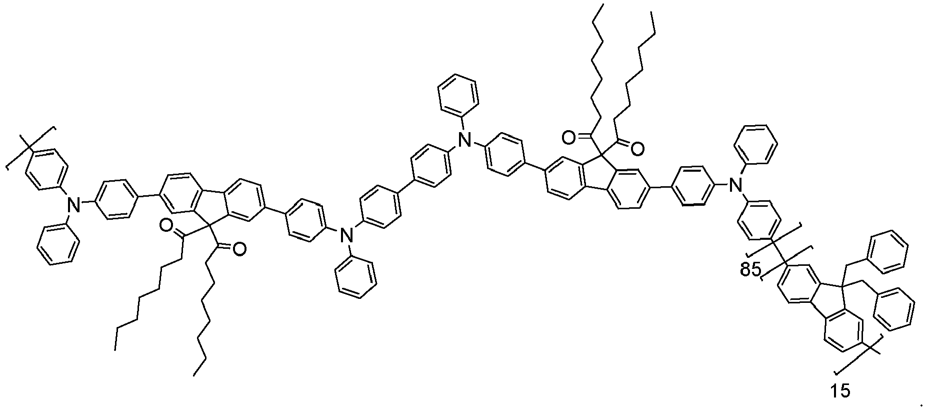

### EBL:

An electron blocking layer (EBL) may be used to reduce the number of electrons and/or excitons that leave the emissive layer. The presence of such a blocking layer in a device may result in substantially higher efficiencies, and/or longer lifetime, as compared to a similar device lacking a blocking layer. Also, a blocking layer may be used to confine emission to a desired region of an OLED. In some embodiments, the EBL material has a higher LUMO (closer to the vacuum level) and/or higher triplet energy than the emitter closest to the EBL interface. In some embodiments, the EBL material has a higher LUMO (closer to the vacuum level) and/or higher triplet energy than one or more of the hosts closest to the EBL interface. In one aspect, the compound used in EBL contains the same molecule or the same functional groups used as one of the hosts described below.

### Host:

The light emitting layer of the organic EL device of the present invention preferably contains at least a metal complex as light emitting material, and may contain a host material using the metal complex as a dopant material. Examples of the host material are not particularly limited, and any metal complexes or organic compounds may be used as long as the triplet energy of the host is larger than that of the dopant. Any host material may be used with any dopant so long as the triplet criteria is satisfied.

Examples of metal complexes used as host are preferred to have the following general formula: 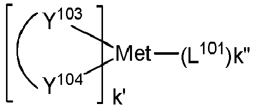 wherein Met is a metal; (Y¹⁰³-Y¹⁰⁴) is a bidentate ligand, Y¹⁰³ and Y¹⁰⁴ are independently selected from C, N, O, P, and S; L¹⁰¹ is an another ligand; k' is an integer value from 1 to the maximum number of ligands that may be attached to the metal; and k'+k" is the maximum number of ligands that may be attached to the metal.

In one aspect, the metal complexes are: 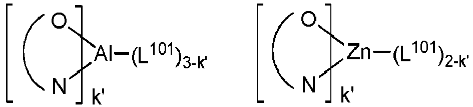 wherein (O-N) is a bidentate ligand, having metal coordinated to atoms O and N.

In another aspect, Met is selected from Ir and Pt. In a further aspect, (Y¹⁰³-Y¹⁰⁴) is a carbene ligand.

In one aspect, the host compound contains at least one of the following groups selected from the group consisting of aromatic hydrocarbon cyclic compounds such as benzene, biphenyl, triphenyl, triphenylene, tetraphenylene, naphthalene, anthracene, phenalene, phenanthrene, fluorene, pyrene, chrysene, perylene, and azulene; the group consisting of aromatic heterocyclic compounds such as dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine; and the group consisting of 2 to 10 cyclic structural units which are groups of the same type or different types selected from the aromatic hydrocarbon cyclic group and the aromatic heterocyclic group and are bonded to each other directly or via at least one of oxygen atom, nitrogen atom, sulfur atom, silicon atom, phosphorus atom, boron atom, chain structural unit and the aliphatic cyclic group. Each option within each group may be unsubstituted or may be substituted by a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acids, ether, ester, nitrile, isonitrile, sulfanyl, sulfinyl, sulfonyl, phosphino, and combinations thereof.

In one aspect, the host compound contains at least one of the following groups in the molecule: 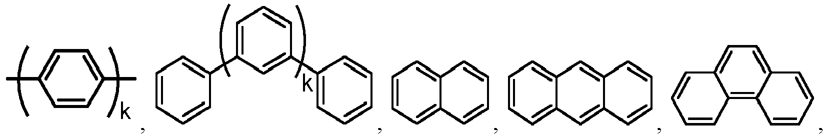 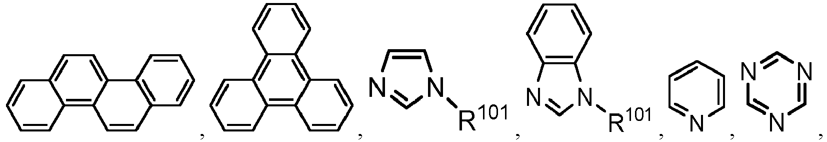 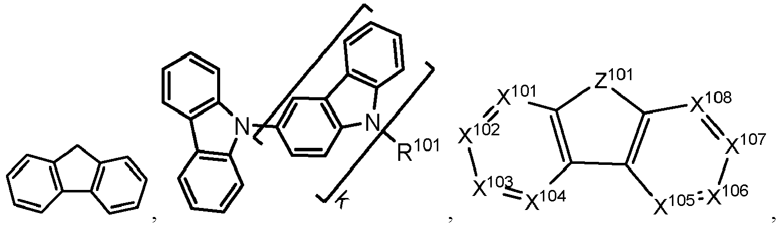 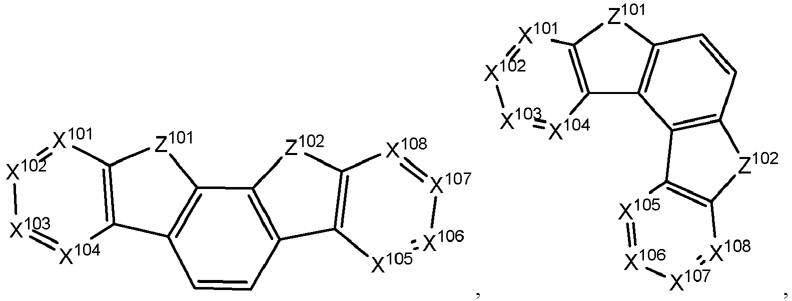 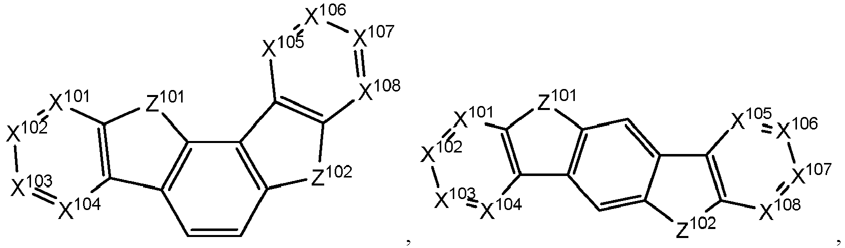 and 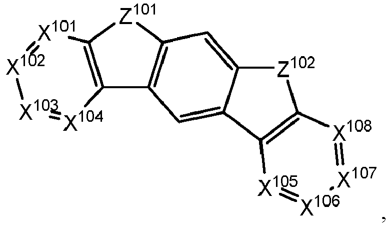 wherein R¹⁰¹ is selected from the group consisting of hydrogen, deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acids, ether, ester, nitrile, isonitrile, sulfanyl, sulfinyl, sulfonyl, phosphino, and combinations thereof, and when it is aryl or heteroaryl, it has the similar definition as Ar's mentioned above. k is an integer from 0 to 20 or 1 to 20. X¹⁰¹ to X¹⁰⁸ are independently selected from C (including CH) or N. Z¹⁰¹ and Z¹⁰² are independently selected from NR¹⁰¹, O, or S.

Non-limiting examples of the host materials that may be used in an OLED in combination with materials disclosed herein are exemplified below together with references that disclose those materials: EP2034538, EP2034538A, EP2757608, JP2007254297, KR20100079458, KR20120088644, KR20120129733, KR20130115564, TW201329200, US20030175553, US20050238919, US20060280965, US20090017330, US20090030202, US20090167162, US20090302743, US20090309488, US20100012931, US20100084966, US20100187984, US2010187984, US2012075273, US2012126221, US2013009543, US2013105787, US2013175519, US2014001446, US20140183503, US20140225088, US2014034914, US7154114, WO2001039234, WO2004093207, WO2005014551, WO2005089025, WO2006072002, WO2006114966, WO2007063754, WO2008056746, WO2009003898, WO2009021126, WO2009063833, WO2009066778, WO2009066779, WO2009086028, WO2010056066, WO2010107244, WO2011081423, WO2011081431, WO2011086863, WO2012128298, WO2012133644, WO2012133649, WO2013024872, WO2013035275, WO2013081315, WO2013191404, WO2014142472, US20170263869, US20160163995, US9466803,

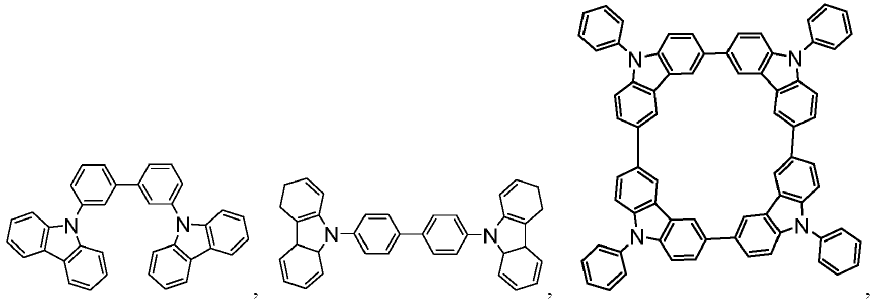

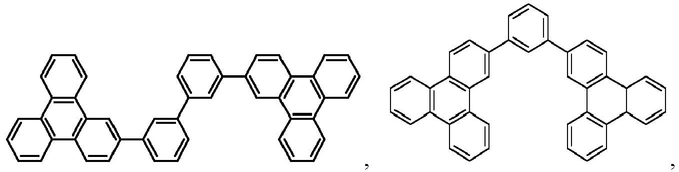

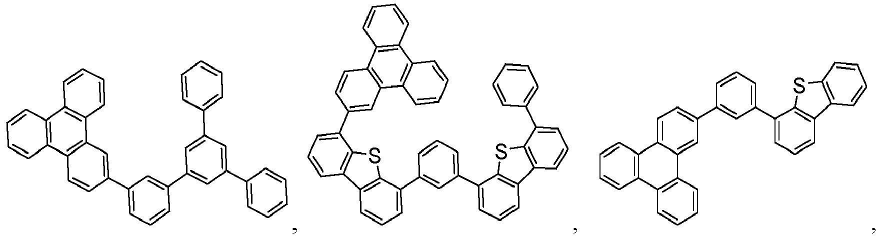

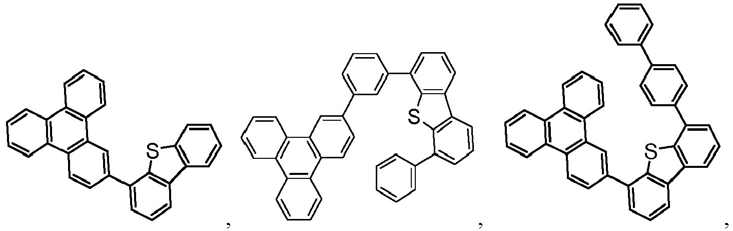

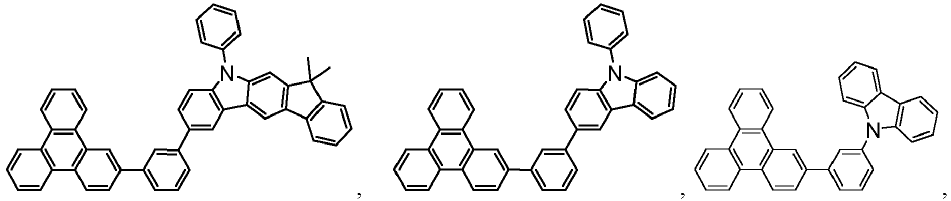

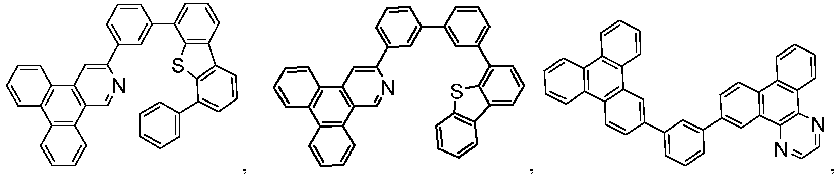

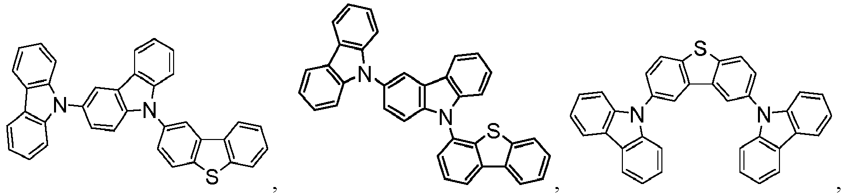

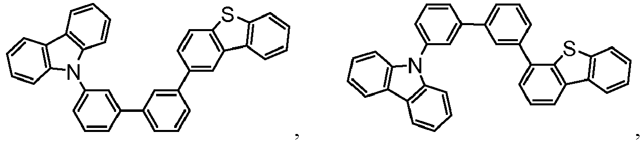

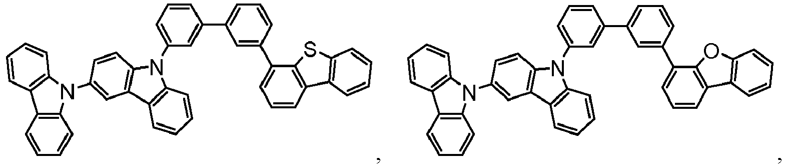

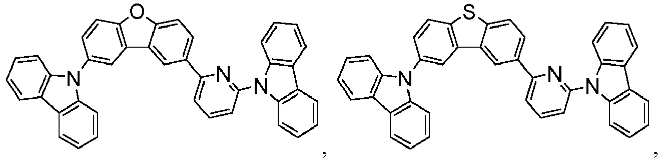

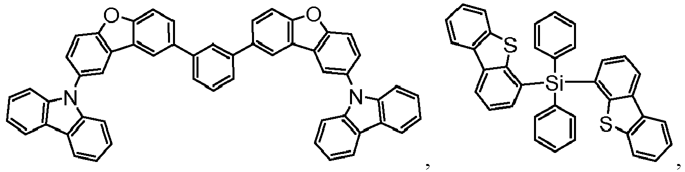

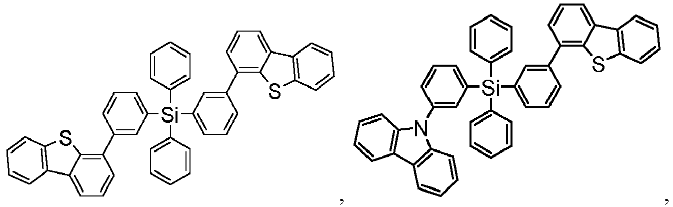

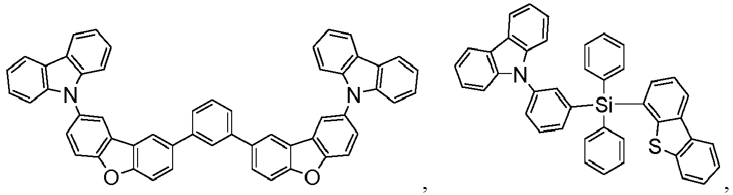

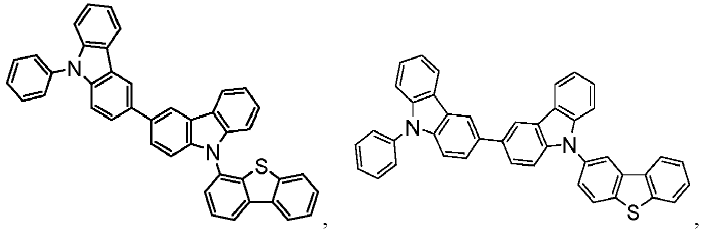

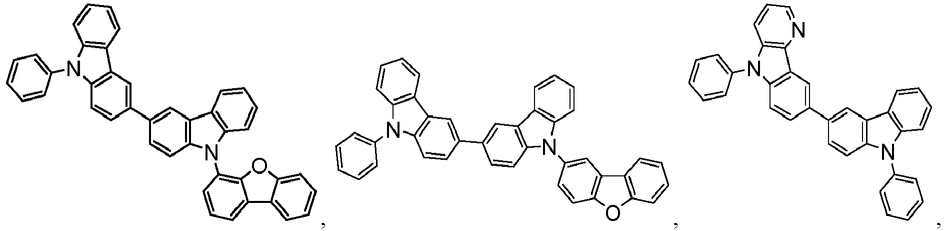

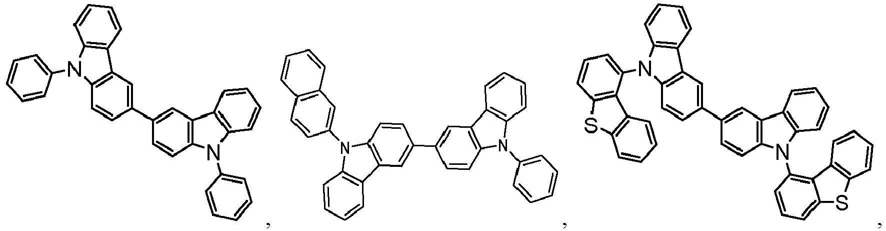

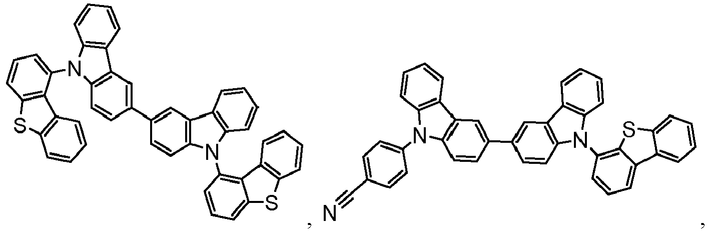

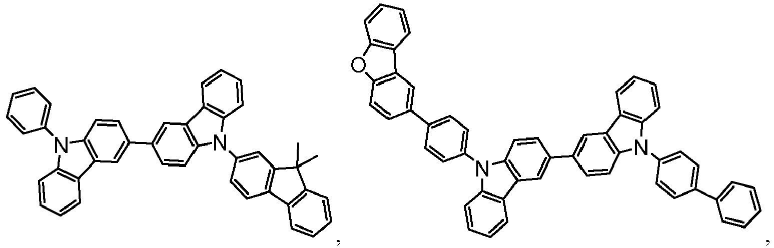

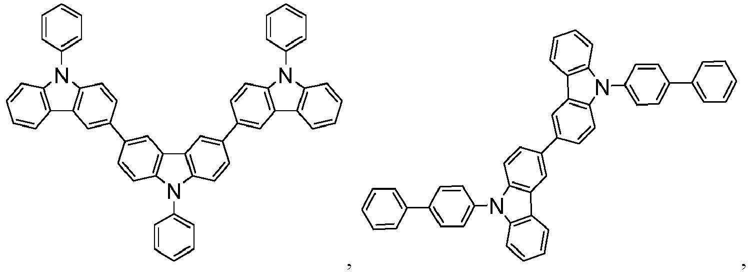

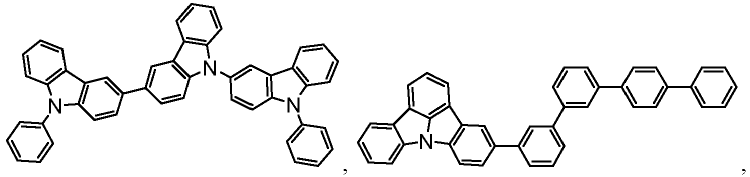

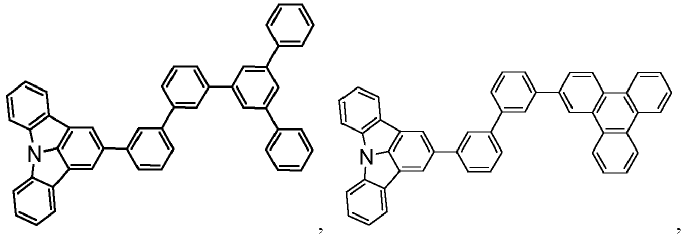

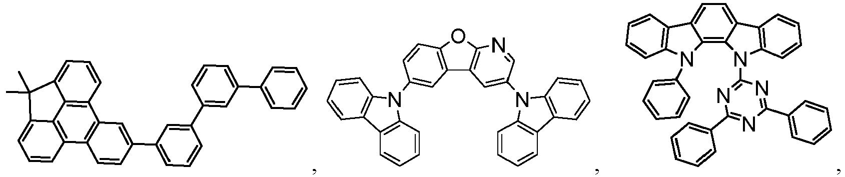

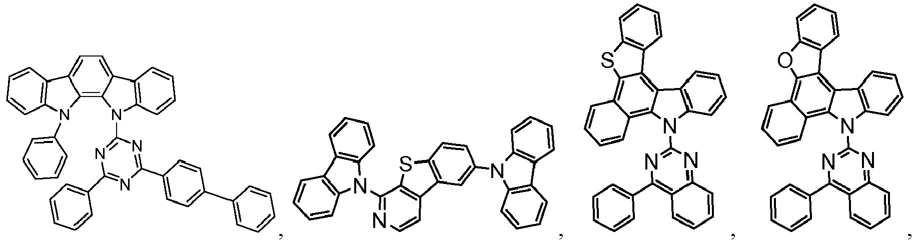

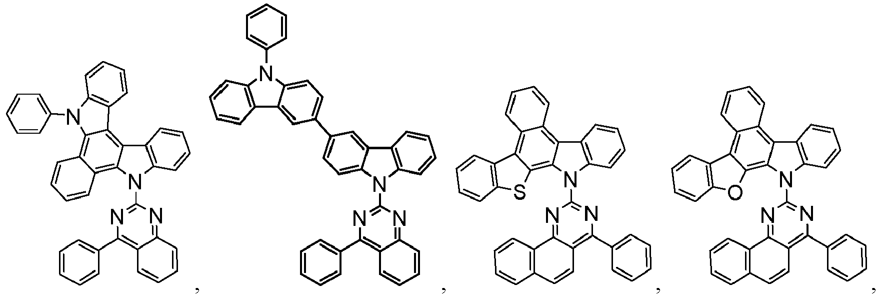

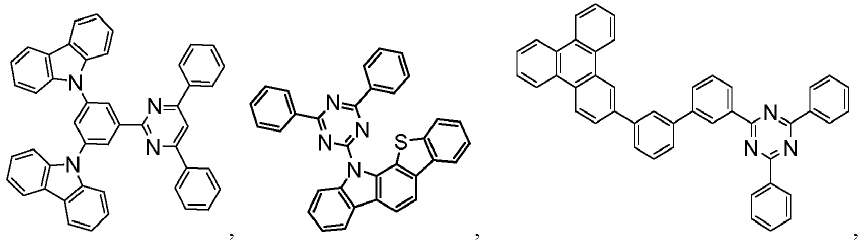

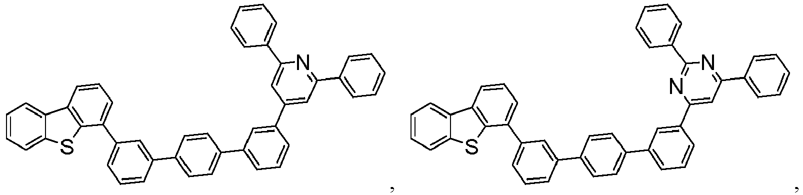

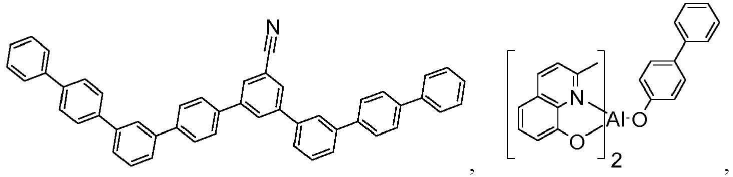

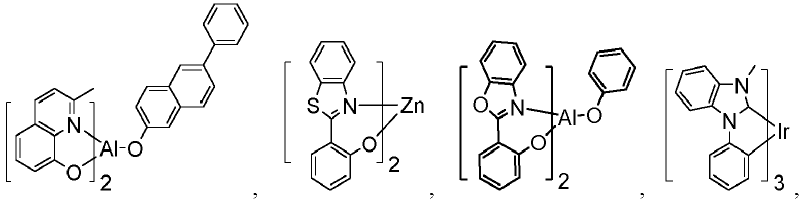

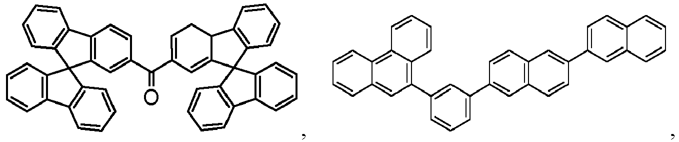

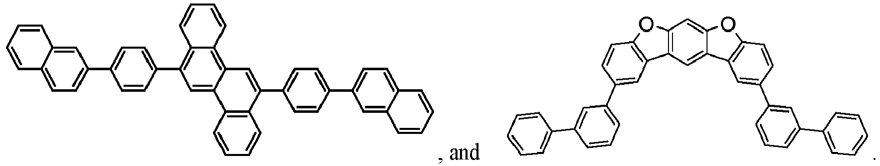

### Additional Emitters:

One or more additional emitter dopants may be used in conjunction with the compound of the present disclosure. Examples of the additional emitter dopants are not particularly limited, and any compounds may be used as long as the compounds are typically used as emitter materials. Examples of suitable emitter materials include, but are not limited to, compounds which can produce emissions via phosphorescence, fluorescence, thermally activated delayed fluorescence, i.e., TADF (also referred to as E-type delayed fluorescence), triplet-triplet annihilation, or combinations of these processes.

Non-limiting examples of the emitter materials that may be used in an OLED in combination with materials disclosed herein are exemplified below together with references that disclose those materials: CN103694277, CN1696137, EB01238981, EP01239526, EP01961743, EP1239526, EP1244155, EP1642951, EP1647554, EP1841834, EP1841834B, EP2062907, EP2730583, JP2012074444, JP2013110263, JP4478555, KR1020090133652, KR20120032054, KR20130043460, TW201332980, US06699599, US06916554, US20010019782, US20020034656, US20030068526, US20030072964, US20030138657, US20050123788, US20050244673, US2005123791, US2005260449, US20060008670, US20060065890, US20060127696, US20060134459, US20060134462, US20060202194, US20060251923, US20070034863, US20070087321, US20070103060, US20070111026, US20070190359, US20070231600, US2007034863, US2007104979, US2007104980, US2007138437, US2007224450, US2007278936, US20080020237, US20080233410, US20080261076, US20080297033, US200805851, US2008161567, US2008210930, US20090039776, US20090108737, US20090115322, US20090179555, US2009085476, US2009104472, US20100090591, US20100148663, US20100244004, US20100295032, US2010102716, US2010105902, US2010244004, US2010270916, US20110057559, US20110108822, US20110204333, US2011215710, US2011227049, US2011285275, US2012292601, US20130146848, US2013033172, US2013165653, US2013181190, US2013334521, US20140246656, US2014103305, US6303238, US6413656, US6653654, US6670645, US6687266, US6835469, US6921915, US7279704, US7332232, US7378162, US7534505, US7675228, US7728137, US7740957, US7759489, US7951947, US8067099, US8592586, US8871361, WO06081973, WO06121811, WO07018067, WO07108362, WO07115970, WO07115981, WO08035571, WO2002015645, WO2003040257, WO2005019373, WO2006056418, WO2008054584, WO2008078800, WO2008096609, WO2008101842, WO2009000673, WO2009050281, WO2009100991, WO2010028151, WO2010054731, WO2010086089, WO2010118029, WO2011044988, WO2011051404, WO2011107491, WO2012020327, WO2012163471, WO2013094620, WO2013107487, WO2013174471, WO2014007565, WO2014008982, WO2014023377, WO2014024131, WO2014031977, WO2014038456, WO2014112450.

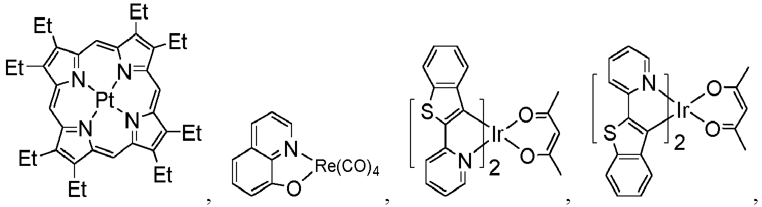

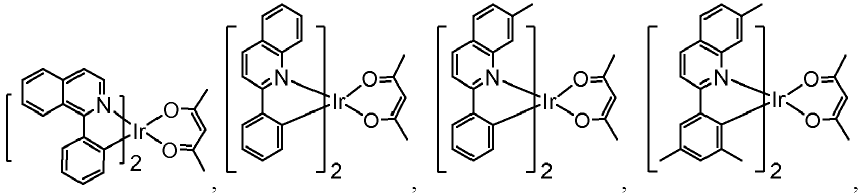

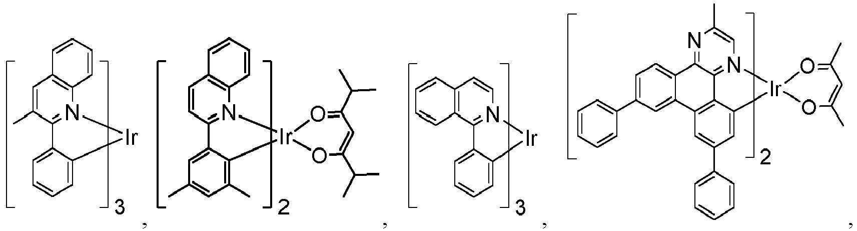

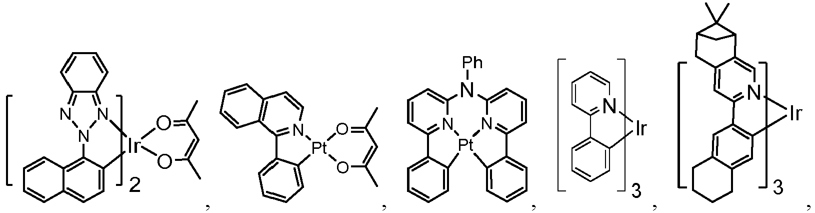

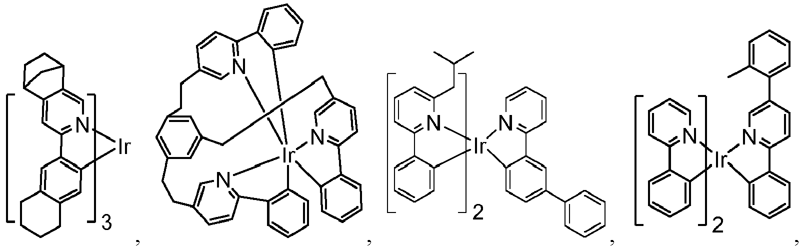

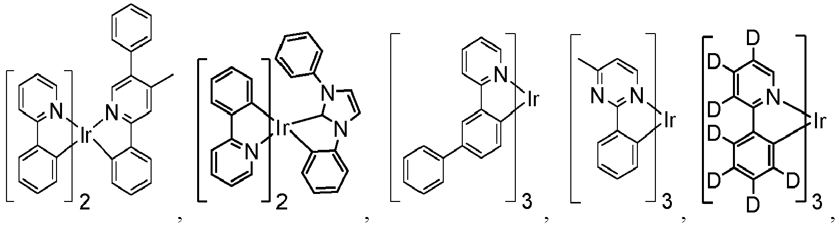

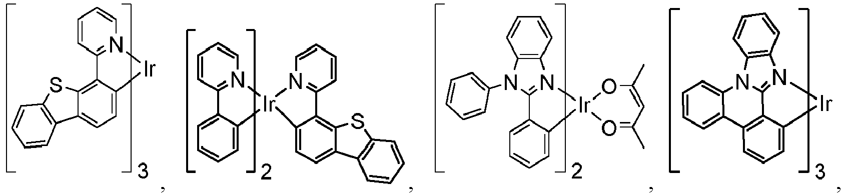

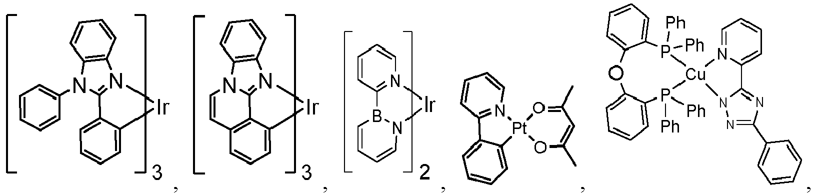

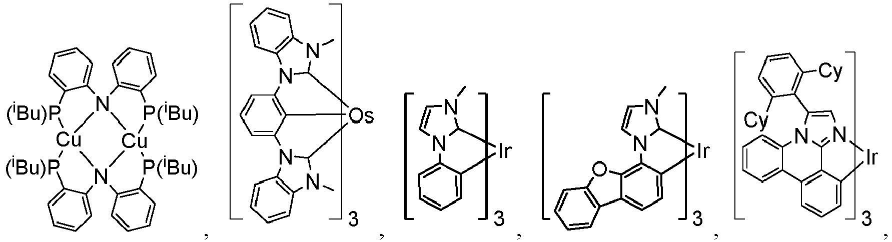

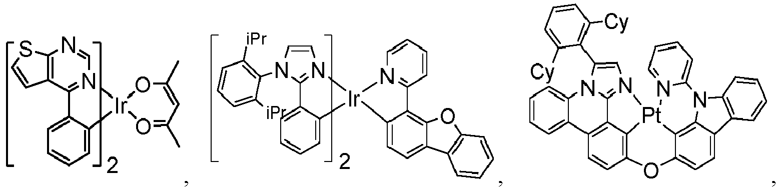

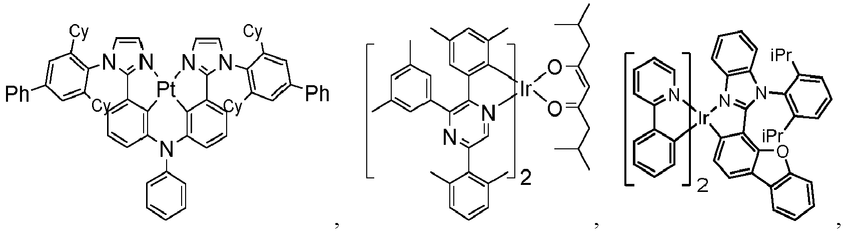

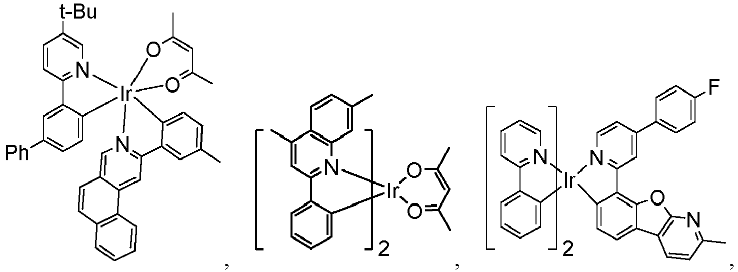

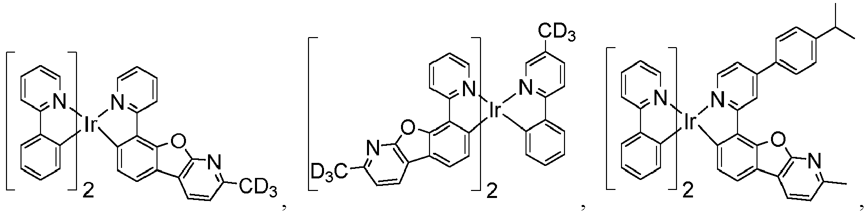

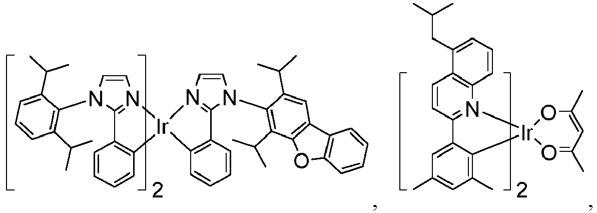

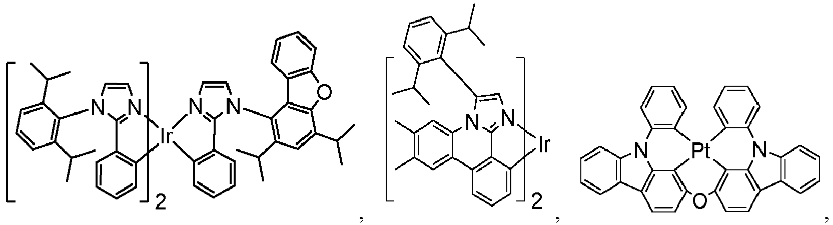

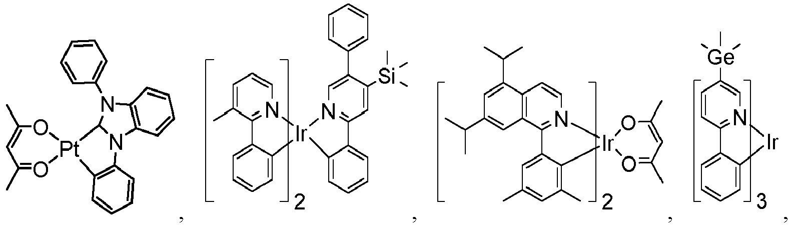

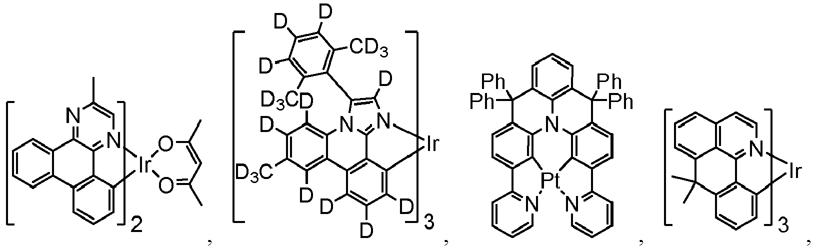

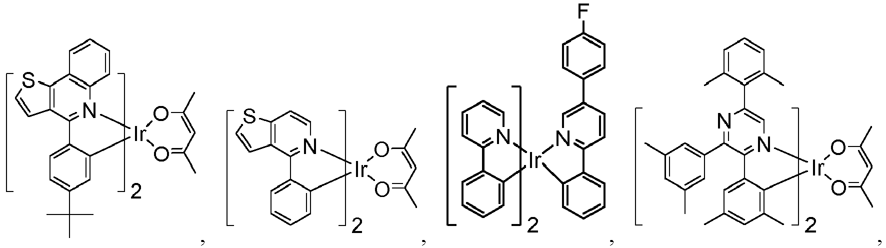

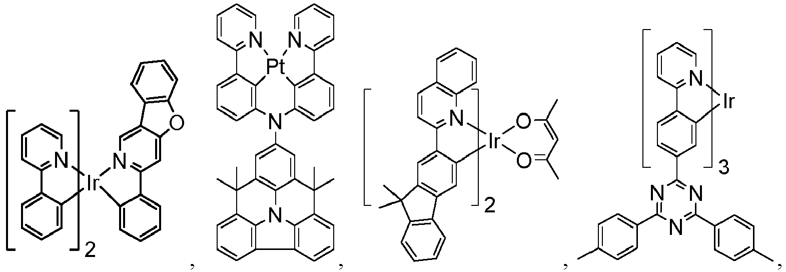

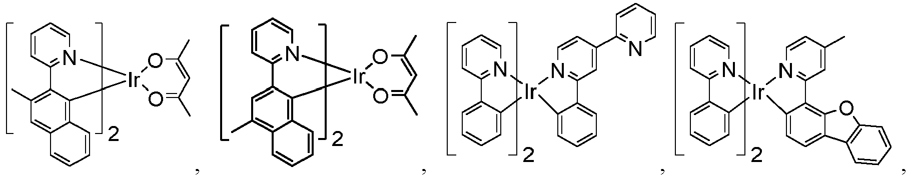

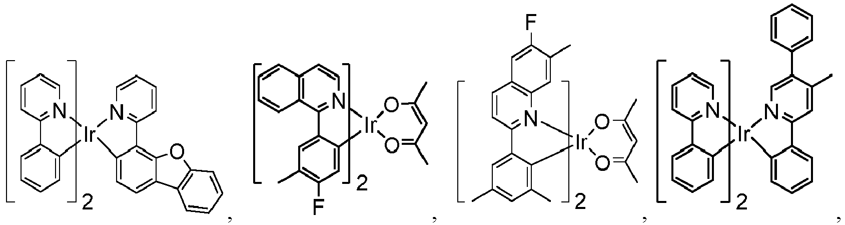

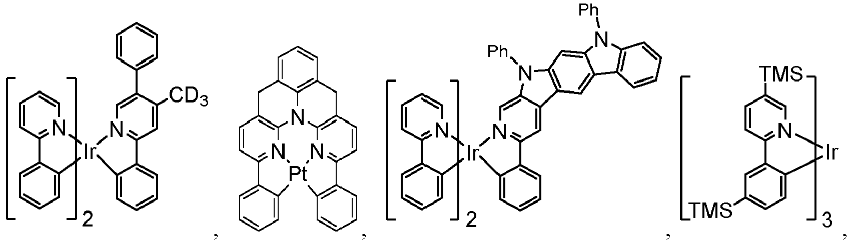

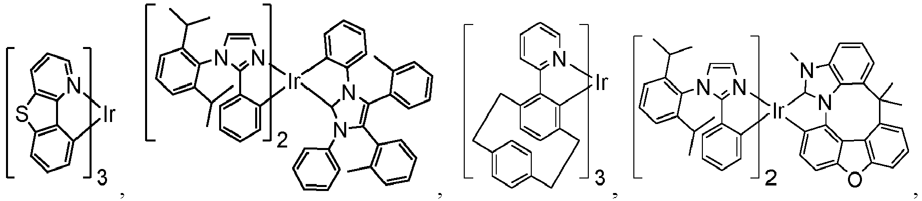

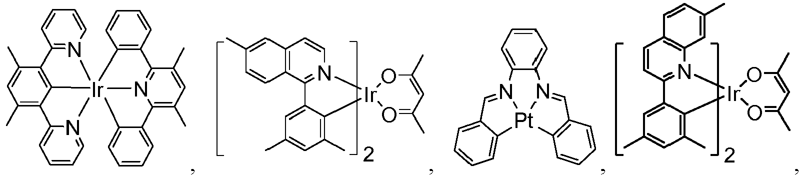

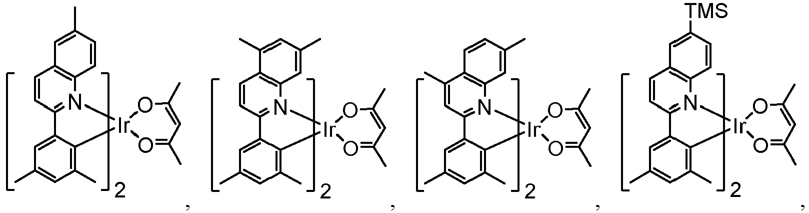

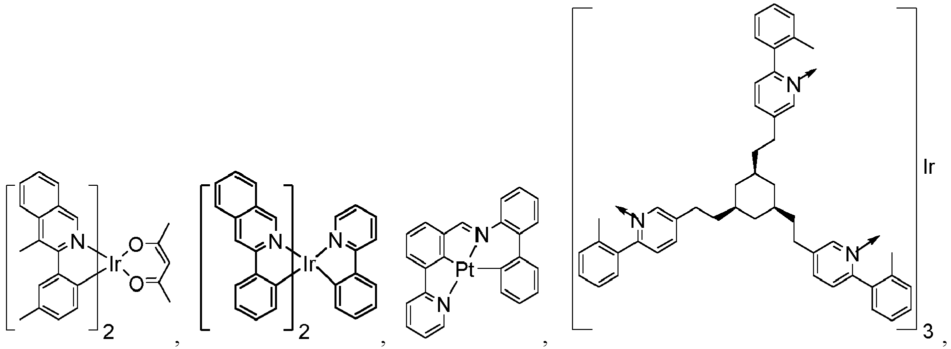

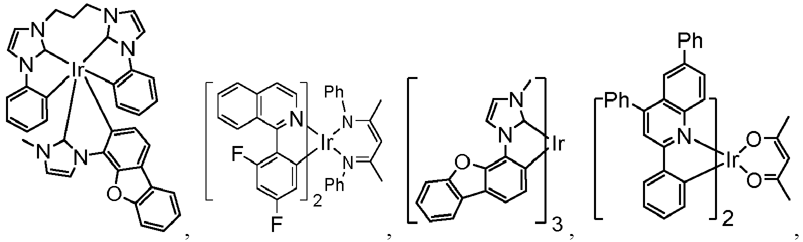

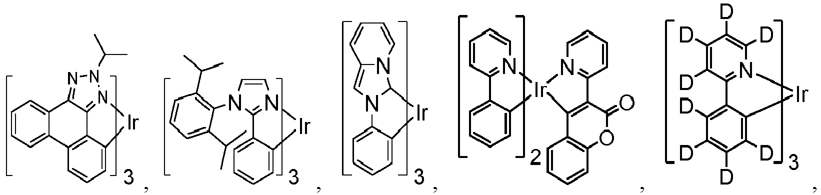

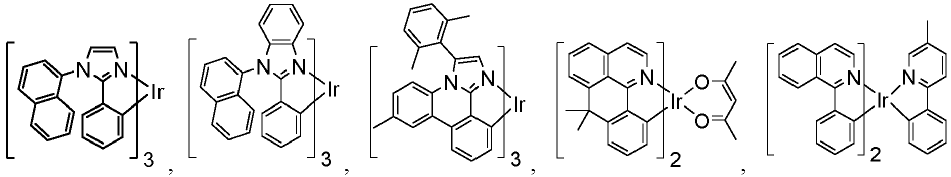

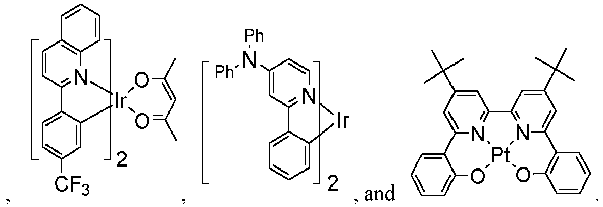

### HBL:

A hole blocking layer (HBL) may be used to reduce the number of holes and/or excitons that leave the emissive layer. The presence of such a blocking layer in a device may result in substantially higher efficiencies and/or longer lifetime as compared to a similar device lacking a blocking layer. Also, a blocking layer may be used to confine emission to a desired region of an OLED. In some embodiments, the HBL material has a lower HOMO (further from the vacuum level) and/or higher triplet energy than the emitter closest to the HBL interface. In some embodiments, the HBL material has a lower HOMO (further from the vacuum level) and/or higher triplet energy than one or more of the hosts closest to the HBL interface.

In one aspect, compound used in HBL contains the same molecule or the same functional groups used as host described above.

In another aspect, compound used in HBL contains at least one of the following groups in the molecule: 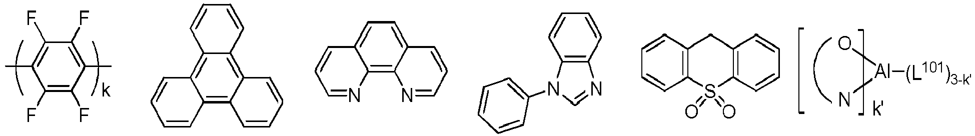 wherein k is an integer from 1 to 20; L¹⁰¹ is an another ligand, k' is an integer from 1 to 3.

### ETL:

Electron transport layer (ETL) may include a material capable of transporting electrons. Electron transport layer may be intrinsic (undoped), or doped. Doping may be used to enhance conductivity. Examples of the ETL material are not particularly limited, and any metal complexes or organic compounds may be used as long as they are typically used to transport electrons.

In one aspect, compound used in ETL contains at least one of the following groups in the molecule: 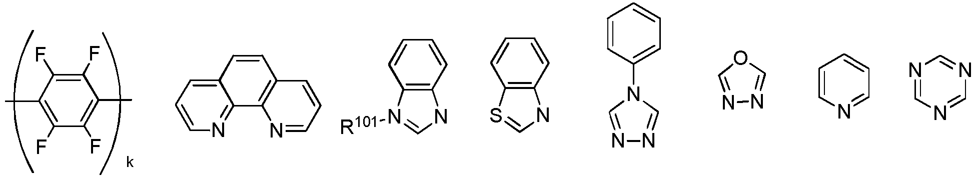 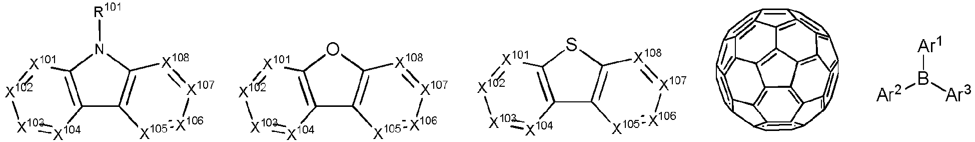 wherein R¹⁰¹ is selected from the group consisting of hydrogen, deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acids, ether, ester, nitrile, isonitrile, sulfanyl, sulfinyl, sulfonyl, phosphino, and combinations thereof, when it is aryl or heteroaryl, it has the similar definition as Ar's mentioned above. Ar¹ to Ar³ has the similar definition as Ar's mentioned above. k is an integer from 1 to 20. X¹⁰¹ to X¹⁰⁸ is selected from C (including CH) or N.

In another aspect, the metal complexes used in ETL contains, but not limit to the following general formula: 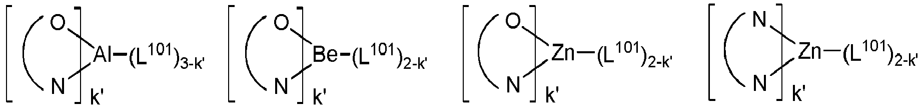 wherein (O-N) or (N-N) is a bidentate ligand, having metal coordinated to atoms O, N or N, N; L¹⁰¹ is another ligand; k' is an integer value from 1 to the maximum number of ligands that may be attached to the metal.

Non-limiting examples of the ETL materials that may be used in an OLED in combination with materials disclosed herein are exemplified below together with references that disclose those materials: CN103508940, EP01602648, EP01734038, EP01956007, JP2004-022334, JP2005149918, JP2005-268199, KR0117693, KR20130108183, US20040036077, US20070104977, US2007018155, US20090101870, US20090115316, US20090140637, US20090179554, US2009218940, US2010108990, US2011156017, US2011210320, US2012193612, US2012214993, US2014014925, US2014014927, US20140284580, US6656612, US8415031, WO2003060956, WO2007111263, WO2009148269, WO2010067894, WO2010072300, WO2011074770, WO2011105373, WO2013079217, WO2013145667, WO2013180376, WO2014104499, WO2014104535,

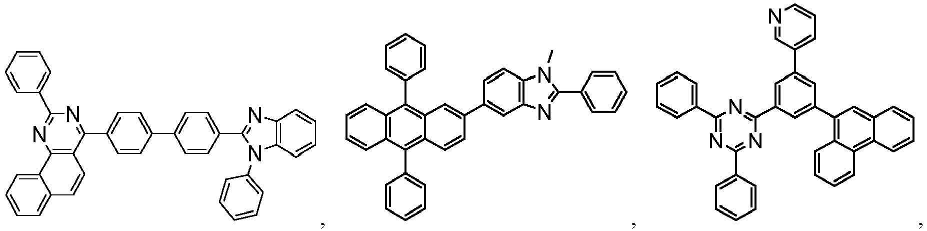

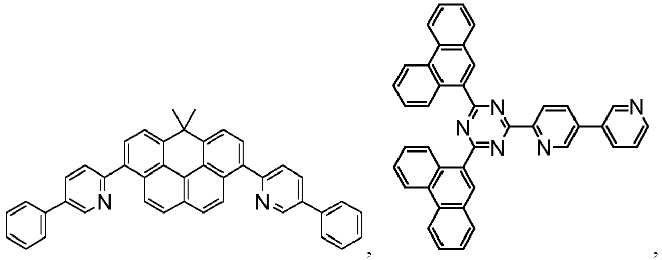

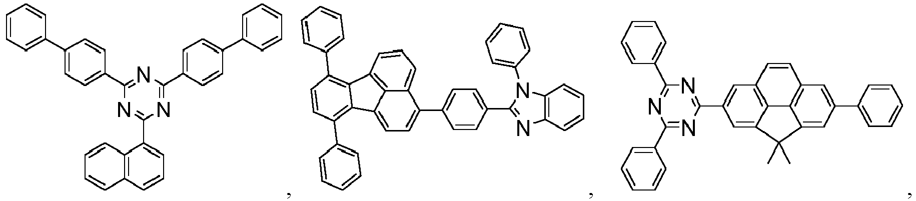

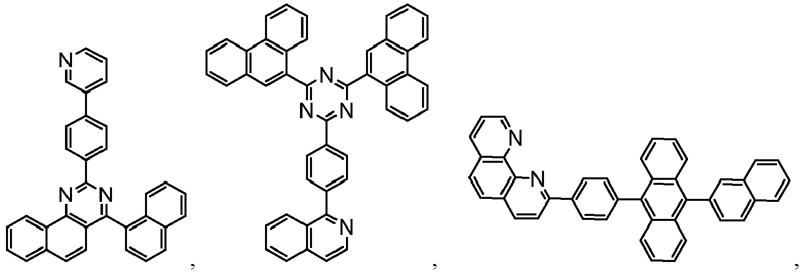

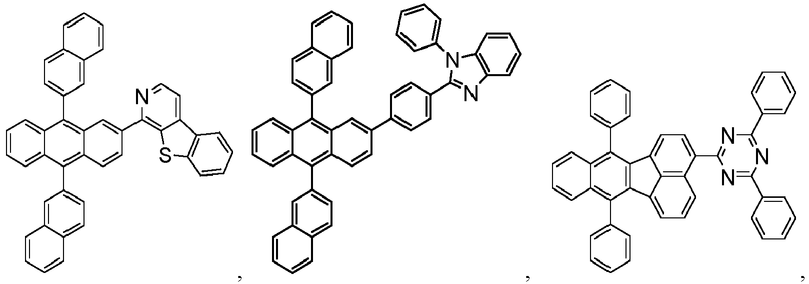

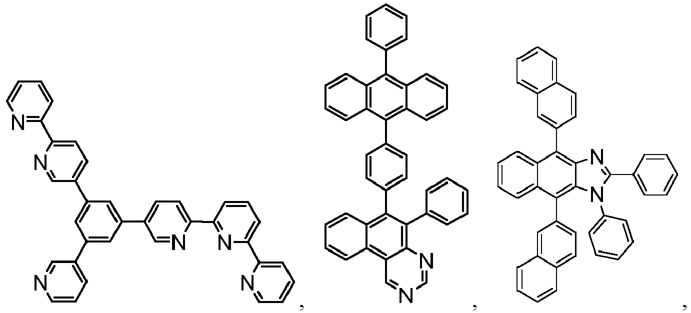

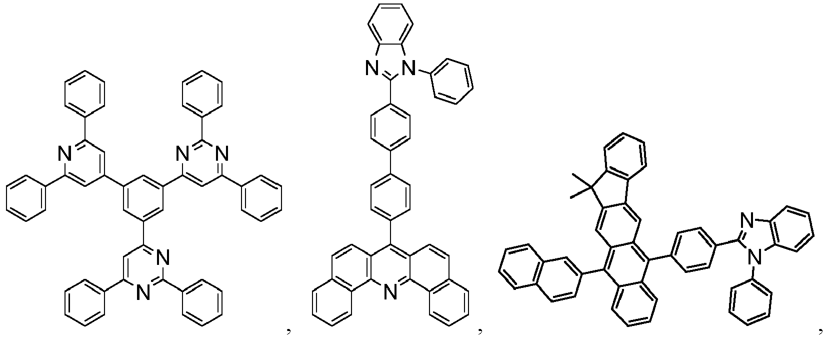

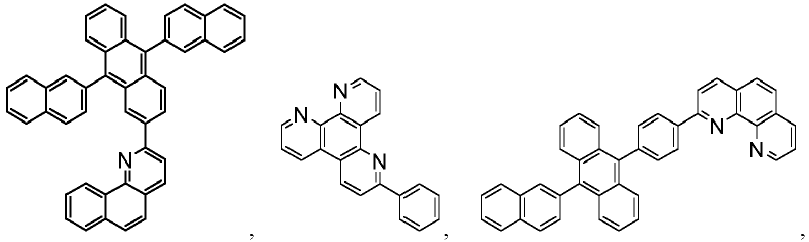

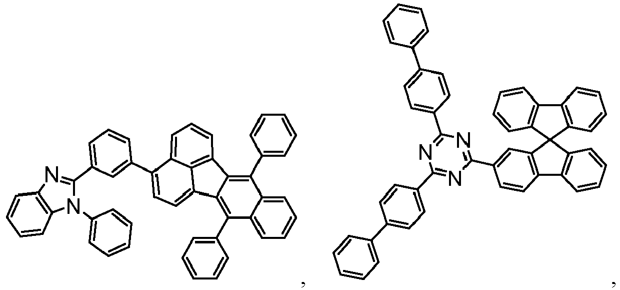

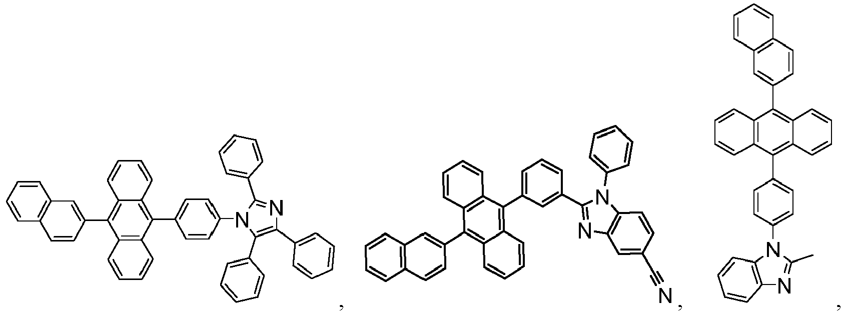

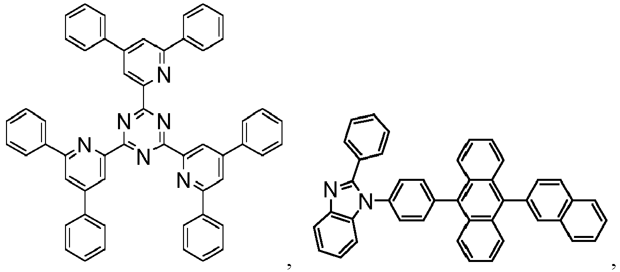

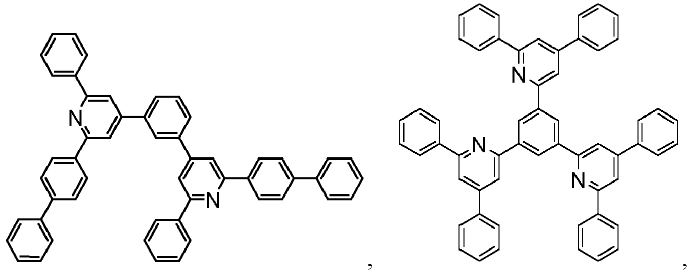

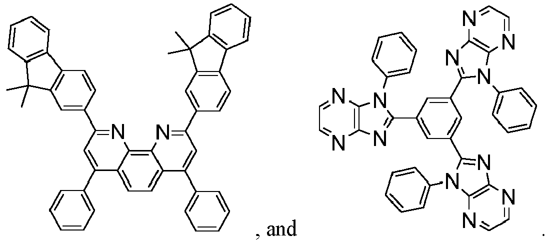

### Charge generation layer (CGL)

In tandem or stacked OLEDs, the CGL plays an essential role in the performance, which is composed of an n-doped layer and a p-doped layer for injection of electrons and holes, respectively. Electrons and holes are supplied from the CGL and electrodes. The consumed electrons and holes in the CGL are refilled by the electrons and holes injected from the cathode and anode, respectively; then, the bipolar currents reach a steady state gradually. Typical CGL materials include n and p conductivity dopants used in the transport layers.

In any above-mentioned compounds used in each layer of the OLED device, the hydrogen atoms can be partially or fully deuterated. Thus, any specifically listed substituent, such as, without limitation, methyl, phenyl, pyridyl, etc. may be undeuterated, partially deuterated, and fully deuterated versions thereof. Similarly, classes of substituents such as, without limitation, alkyl, aryl, cycloalkyl, heteroaryl, etc. also may be undeuterated, partially deuterated, and fully deuterated versions thereof.

### EXPERIMENTAL

### Synthesis Section

### Synthesis of IrL_{A108}(L_{B257})₂

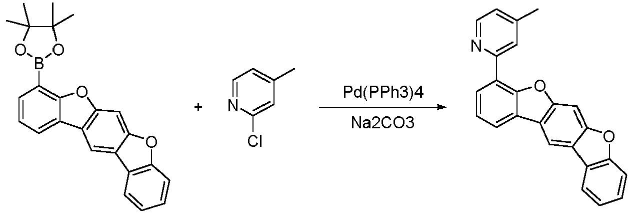

Boronic ester (8 g, 20.82 mmol), 2-chloro-4-methylpyridine (2.66 g, 20.82 mmol) and sodium carbonate (6.62 g, 62.5 mmol) were dissolved in dimethyoxyethane (DME)/water mixture (100ml/25 ml). The reaction mixture was degassed and tetrakis(triphenylphosphine)palladium(0) ("tetrakis" or Pd(PPh₃)₄) (0.722 g, 0.625 mmol) was added. The mixture was heated under nitrogen at 100 °C overnight. After the reaction was cooled to room temperature (~ 22 °C), it was diluted with water and extracted with ethylacetate (EtOAc). The combined organic phase was washed with brine and solvent was evaporated. The residue purified by chromatography on a silica gel column eluted with 2% EtOAc in dichloromethane (DCM) to yield target compound as white solid (6.3 g, 87% yield).

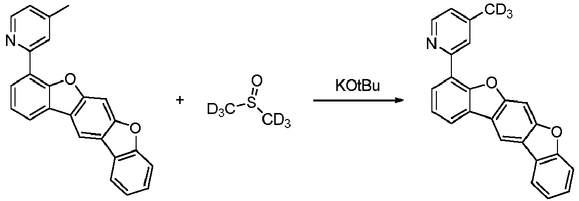

The polycyclic compound from the previous step (6.3 g, 18.03 mmol) was dissolved in ((methyl-d3)sulfinyl)methane-d3 (38.3 ml, 541 mmol). The mixture was heated at 40 °C under N₂ and potassium 2-methylpropan-2-olate (KOtBu)(1.01, 9.02 mmol) was added. The mixture was heated under N₂ at 65 °C for 18 h. After the reaction was cooled to room temperature, D₂O (20 mL) was added, followed by excess of water. The mixture was extracted with DCM. The combined organic phase was washed with brine. The solvent was evaporated and the residue was purified on a silica gel column eluted with 10% EtOAc in DCM to yield the deuterated product (5.0 g, 79% yield).

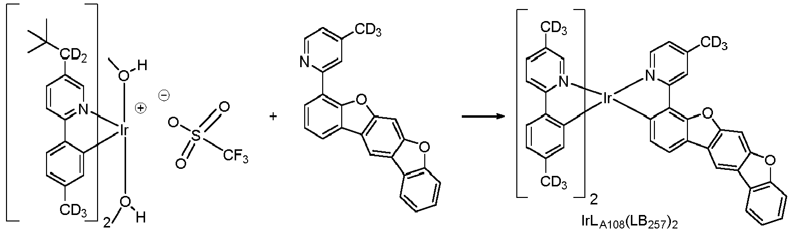

The iridium complex triflic salt (1.6 g) and ligand from the previous step (1.5 g, 4.30 mmol) were added to 2-ethoxyethanol (40 ml) and dimethylformamide (DMF) (60.00 ml). The mixture was degassed for 20 min and heated to 130 °C under nitrogen for 18 h. After the reaction was cooled to room temperature, then the solvent was evaporated. The residue was dissolved in DCM and was filtered through a short silica gel plug. The solvent was evaporated, and the residue was subjected to column chromatography on a silica gel column, eluted with a mixture of DCM/heptane 7/3 mixture (v/v) to yield target material **IrL_{A108}(L**_{B257}**)₂** (0.4 g, 22% yield).

### Synthesis of IrL_{A104}(L_{B461})₂

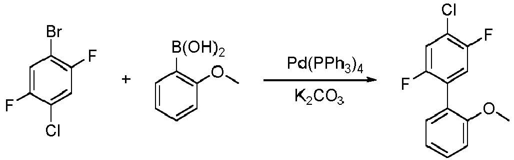

1-Bromo-4-chloro-2,5-difluorobenzene (12 g, 52.8 mmol), (2-methoxyphenyl)boronic acid (8.42 g, 55.4 mmol), and potassium carbonate (14.58 g, 106 mmol) were dissolved in a toluene (100 ml)/water (20 ml) mixture under nitrogen to give a colorless suspension. Tetrakis(triphenylphosphine)palladium(0) (0.544 g, 0.528 mmol) was added to the reaction mixture in one portion. The reaction mixture was degassed and heated to reflux under nitrogen for 16 h. Based on the results of gas chromatography-mass spectroscopy (GCMS) analysis the reaction was not complete. Thus, 7 g more boronic acid, 1 g of K₂CO₃, and 0.6 g of Pd(PPh₃)₄ as catalyst was added, and the resulting mixture was degassed and refluxed under nitrogen for 14 h. The reaction mixture was then cooled down and the organic phase was separated, evaporated, and purified by column chromatography on silica gel, eluted with heptanes to yield 4-chloro-2,5-difluoro-2'-methoxy-1,1'-biphenyl (11.0 g, 82 % yield) as yellow oil.

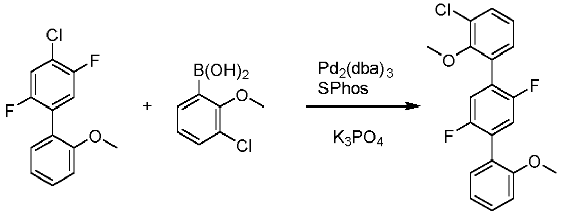

In a 500 mL two-necked round-bottomed flask, 4-chloro-2,5-difluoro-2'-methoxy-1,1'-biphenyl (7.51 g, 29.5 mmol), (3-chloro-2-methoxyphenyl)boronic acid (5.5 g, 29.5 mmol), and potassium phosphate tribasic hydrate (13.59 g, 59.0 mmol) were dissolved in DME (120 mL) and water (5 mL) under nitrogen to give a colorless suspension. Tris(dibenzylideneacetone)dipalladium(0) (Pd₂(dba)₃) (0.540 g, 0.590 mmol) and dicyclohexyl(2',6'-dimethoxy-[1,1'-biphenyl]-2-yl)phosphane ("SPhos", 0.969 g, 2.361 mmol) were added to the reaction mixture as one portion. The reaction mixture was degassed and heated to 100 °C for 14 h. The reaction mixture was then cooled down to room temperature, diluted with EtOAc and washed with water. The organic extract was evaporated and the solid residue was subjected to column chromatography on silica gel and eluted with heptanes/EtOAc gradient mixture to yield 3-chloro-2',5'-difluoro-2,2"-dimethoxy-1,1':4',1"-terphenyl (9.00 g, 24.95 mmol, 85 % yield) as a white solid.

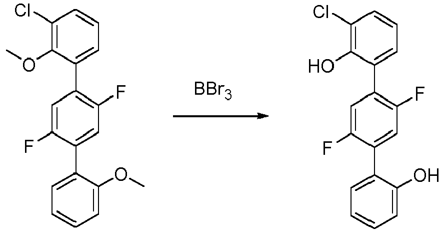

In a 500 mL round-bottomed flask 3-chloro-2',5'-difluoro-2,2"-dimethoxy-1,1':4',1"-terphenyl (15 g, 41.6 mmol) was dissolved in DCM (120 ml) open to air to give a colorless solution. The reaction mixture was cooled in the ice bath and a IN tribromoborane solution in DCM (87 ml, 87 mmol) was added dropwise. The resulting mixture was stirred for 3 h at 0 °C, then allowed to warm up to 20 °C and stirred for 16 h. The reaction mixture was quenched with water, diluted with water, and extracted with EtOAc. The combined organic extracts were dried over anhydrous sodium sulfate, filtered, and evaporated. The crude product was added to a silica gel column and was eluted with heptanes/EtOAc 1/1 (v/v) to give 3-chloro-2',5'-difluoro-[1,1':4',1"-terphenyl]-2,2"-diol (11.1 g, 33.4 mmol, 80 % yield) as a colorless solid.

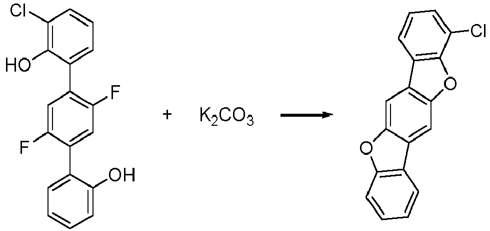

In an oven-dried 250 mL round-bottomed flask 3-chloro-2',5'-difluoro-[1,1':4',1"-terphenyl]-2,2"-diol (12.8 g, 38.5 mmol) and potassium carbonate (15.95 g, 115 mmol) were dissolved in N-methyl-2-pyyolidone (NMP)(120 ml) under nitrogen atmosphere to give a dark suspension. The reaction mixture was heated to 130 °C for 14 h and solvent was distilled off in vacuum. The reaction mixture was diluted with ethyl acetate (3x), stirred and the resulting precipitate was filtered. This precipitate was washed with water, ethanol, heptanes and dried to produce the desired product (9.0 g, 80% yield).

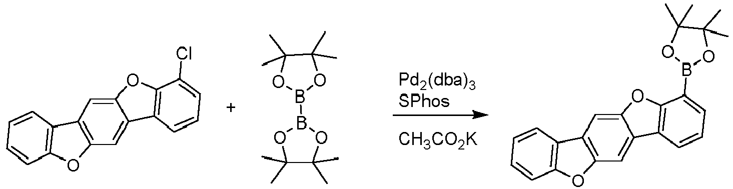

The polycyclic chloride from the previous step (3.7 g, 12.6 mmol), 4,4,4',4',5,5,5',5'-octamethyl-2,2'-bi(1,3,2-dioxaborolane) (6.42 g, 25.2 mmol), potassium acetate (3.1 g, 31.6 mmol), tris(dibenzylideneacetone)dipalladium(0) (1 mol%) and SPhos (4 mol%) were suspended in dioxane (100 mL). The reaction mixture was degassed and heated to 100 °C for 14 h under nitrogen. The reaction mixture was cooled down to room temperature, diluted with water, and extracted with EtOAc. The organic extract was passed through a short silica plug and concentrated. The target boronic ester formed white precipitate and was filtered off (white solid, 3.1 g, 68% yield).

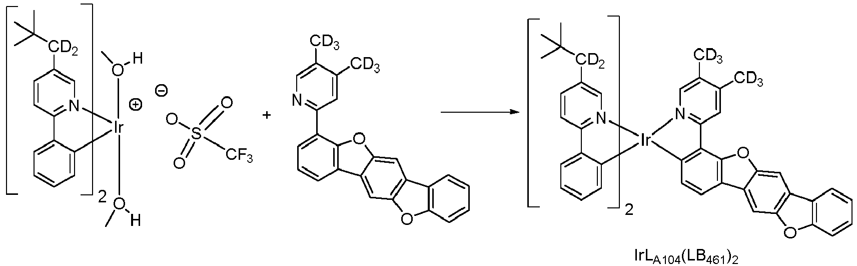

In an oven-dried 500 mL two-necked round-bottomed flask, the triflic iridium salt complex show above (2.170 g, 5.87 mmol) and the ligand from the previous step (2.0 g) were dissolved in DMF (200 ml) and 2-ethoxyethanol (66.7 ml) under nitrogen to give a dark suspension. The reaction flask was immersed in the oil bath at 100 °C and stirred under nitrogen for 12 days. After completion, the reaction mixture was cooled down to room temperature, diluted with water, and extracted with EtOAc. The extract was washed several times with LiCl aq. 10% and evaporated. The resulting solid residue was subjected to column chromatography on a silica gel column and eluted with toluene/EtOAc/heptane 35/5/60 (v/v/v) to yield the target material **IrL_{A104}(L**_{B461}**)₂** (1.6 g, 48% yield).

### Synthesis of IrL_{A110}(L_{B284})₂

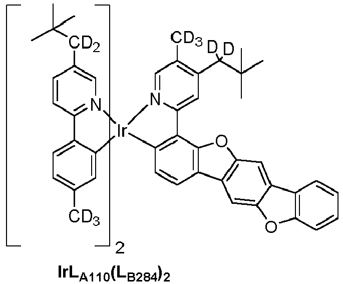

IrL_{A110}(L_{B284})₂ was made in manner similar to IrL_{A104}(L_{B461})₂

### Synthesis of IrL_{A67}(L_{B461})₂

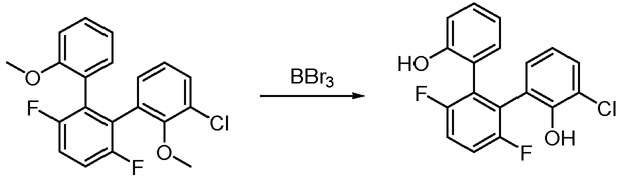

3-Chloro-3',6'-difluoro-2,2"-dimethoxy-1,1':2',1"-terphenyl (10.8 g, 29.9 mmol) was dissolved in DCM (400 ml) and then cooled to 0 °C degree. A IN tribromoborane (BBr₃) solution in DCM (90 ml, 90 mmol) was added dropwise. The reaction mixture was stirred at 20 °C overnight, then quenched with water and extracted with DCM. The combined organic phase was washed with brine. After the solvent was removed, the residue was subjected to column chromatography on a silica gel column eluted with DCM/heptanes gradient mixture to yield 3-chloro-3',6'-difluoro-[1,1':2',1"-terphenyl]-2,2"-diol as white solid (4.9 g, 53% yield).

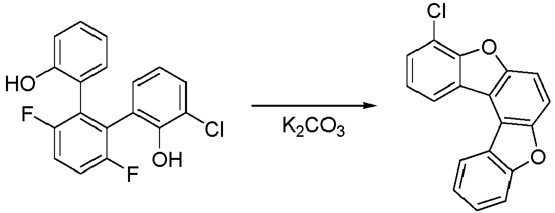

A mixture of 3-chloro-3',6'-difluoro-[1,1':2',1"-terphenyl]-2,2"-diol (5 g, 15.03 mmol) and K₂CO₃ (6.23 g, 45.08 mmol) in 1-methylpyrrolidin-2-one (75 mL) was vacuumed and stored under nitrogen. The mixture was heated at 150 °C overnight. After the reaction was cooled to 20 °C, it was diluted with water and extracted with EtOAc. The combined organic phase was washed with brine. After the solvent was removed, the residue was subjected to column chromatography on a silica gel column eluted with 20% DCM in heptane to yield target chloride as white solid (3.0 g, 68% yield).

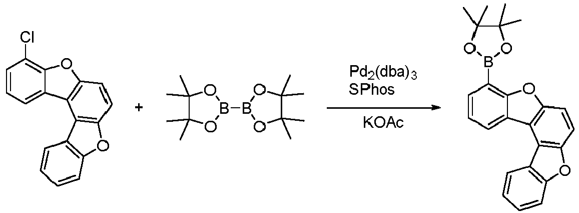

The chloride molecule above (3 g, 10.25 mmol) was mixed with 4,4,4',4',5,5,5',5'-octamethyl-2,2'-bi(1,3,2-dioxaborolane) (5.21 g, 20.50 mmol), tris(dibenzylideneacetone)dipalladium(0) (0.188 g, 0.205 mmol), dicyclohexyl(2',6'-dimethoxy-[1,1'-biphenyl]-2-yl)phosphane (SPhos, 0.337 g, 0.820 mmol), and potassium acetate ("KOAc")(2.012 g, 20.50 mmol) and suspended in 1,4-dioxane (80 ml). The mixture was degassed and heated at 100 °C overnight. After the reaction mixture was then cooled to 20 °C, before being diluted with water and extracted with EtOAc. The combined organic phase was washed with brine. After the solvent was evaporated, the residue was purified on a silica gel column eluted with 2% EtOAc in DCM to yield the target boronic ester as white solid (3.94 g, 99% yield).

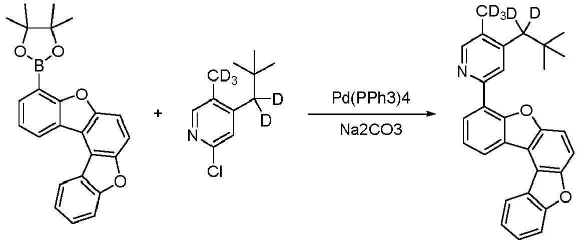

The boronic ester from above (3.94 g, 10.25 mmol), 2-chloro-4-(2,2-dimethylpropyl-1,1-d2)-5-(methyl-d3)pyridine (3.12 g, 15.38 mmol) and sodium carbonate (2.72 g, 25.6 mmol) were suspended in the mixture of DME (80 ml) and water (20 ml). The reaction mixture was degassed and tetrakis(triphenylphosphine)palladium(0) (0.722 g, 0.625 mmol) was added as one portion. The mixture was heated at 100 °C for 14 h. After the reaction was cooled to 20 °C, it was diluted with water and extracted with EtOAc. The combined organic phase was washed with brine. After the solvent was evaporated, the residue was subjected to column chromatography on a silica gel column eluted with 2% EtOAc in DCM to yield the target ligand as white solid (1.6 g, 37% yield)

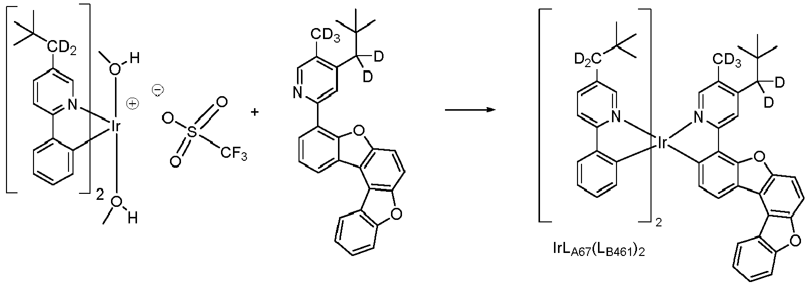

The iridium complex triflic salt shown above (1.7 g) and the target ligand from the previous step (1.5 g, 3.57 mmol) were suspended in the mixture of 2-ethoxyethanol (35 ml) and DMF (35 ml). The mixture was degassed for 20 min and was heated to reflux (90 °C) under nitrogen for 18 h. After the reaction was cooled to 20 °C, the solvent was evaporated. The residue was dissolved in DCM and was filtered through a short silica gel plug. The solvent was evaporated, and the residue was subjected to column chromatography on a silica gel then eluted with a mixture of DCM and heptane (7/3, v/v) to yield the target complex **IrL_{A67}(L_{B461})₂** as yellow crystals (0.8 g, 38% yield).

### Synthesis of IrL_{A109}(L_{B463})₂

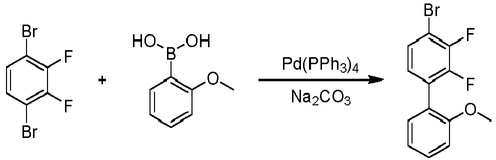

1,4-dibromo-2,3-difluorobenzene (15 g, 55.2 mmol), (2-methoxyphenyl)boronic acid (8.80 g, 57.9 mmol), sodium carbonate (11.69 g, 110 mmol), and tetrakis(triphenylphosphine)palladium(0) (3.19 g, 2.76 mmol) were dissolved in a mixture of water (140 ml) and dioxane (140 ml). The reaction mixture was degassed and heated in an 80 °C oil bath for 20 h. The reaction mixture was cooled to room temperature, mixed with brine and extracted with EtOAc. The extract was washed with water, brine, dried, and evaporated to leave a solid/liquid mixture that was absorbed onto a silica gel plug and chromatographed on silica gel column eluted with heptane to yield 4-bromo-2,3-difluoro-2'-methoxy-1,1'-biphenyl as a colorless oil (12.5 g, 75% yield).

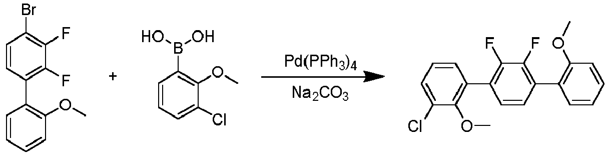

4-Bromo-2,3-difluoro-2'-methoxy-1,1'-biphenyl (12.38 g, 41.4 mmol), (3-chloro-2-methoxyphenyl)boronic acid (8.10 g, 43.5 mmol), sodium carbonate (8.77 g, 83 mmol), and tetrakis(triphenylphosphine)palladium(0) (1.435 g, 1.242 mmol) were dissolved in a mixture of water (125 ml) and dioxane (125 ml). The reaction mixture was degassed and heated in an 80 °C oil bath for 20 h. Gas chromatography-Mass Spectroscopy (GCMS) analysis showed 80% conversion, so additional Pd(PPh₃)₄ (1.435 g, 1.242 mmol) and boronic acid (2.4 g, 0.3 equiv.) were add. The resulting mixture was degassed and heated at 90 °C overnight, then allowed to cool to room temperature. Brine (100 ml) was added to the resulting mixture, which was then extracted with DCM. The extracts were washed with water, brine, dried and evaporated. The residue was chromatographed on silica gel column eluted with heptane to yield 3-chloro-2',3'-difluoro-2,2"-dimethoxy-1,1':4',1"-terphenyl as white solid (9.95 g, 66% yield).

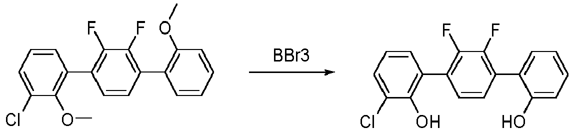

A solution of 3-chloro-2',3'-difluoro-2,2"-dimethoxy-1,1':4',1"-terphenyl (9.95 g, 27.6 mmol) in DCM (150 ml) was cooled in an ice/salt bath and boron tribromide (BBr₃, 1 N solution in DCM, 110 ml, 110 mmol) was added dropwise. The reaction was stirred overnight while slowly warming to room temperature. The resulting mixture was cooled in an ice bath and 125 ml of water was added dropwise. The resulting mixture was stirred for 30 minutes and extracted with DCM. The extracts were washed with water, dried and evaporated, to yield 3-chloro-2',3'-difluoro-[1,1':4',1"-terphenyl]-2,2"-diol (8.35 g, 90% yield) which was used without further purification.

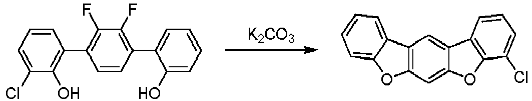

3-Chloro-2',3'-difluoro-[1,1':4',1"-terphenyl]-2,2"-diol (8.35 g, 25.10 mmol) and potassium carbonate (7.63 g, 55.2 mmol) were suspended in N-methyl-2-pyrrolidine (NMP)(100 ml), degassed, and heated under nitrogen in a 130 °C oil bath for 16 h. The reaction mixture was allowed to cool to room temperature and the solvent was distilled off under vacuum. The residue was chromatographed on silica gel column and eluted with heptanes/EtOAc 9/1 (v/v) to provide the target chloride as white solid (6.5 g, 88% yield).

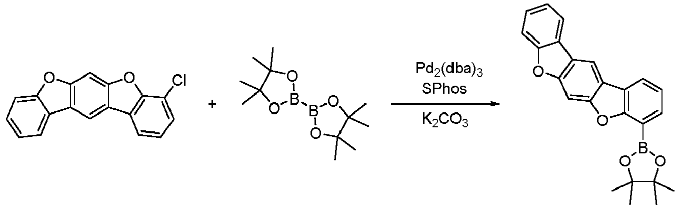

Chloride intermediate from previous step (6.5 g, 22.21 mmol), 4,4,4',4',5,5,5',5'-octamethyl-2,2'-bi(1,3,2-dioxaborolane) (11.28 g, 44.4 mmol), potassium acetate (4.36 g, 44.4 mmol), Pd₂(dba)₃ (0.305 g, 0.333 mmol), and dicyclohexyl(2',6'-dimethoxy-[1,1'-biphenyl]-2-yl)phosphane (Sphos, 0.547 g, 1.332 mmol) were suspended in dioxane (250 ml). The reaction mixture was degassed and heated to reflux under nitrogen for 14 h. The resulting mixture was cooled down to room temperature, diluted with water, and extracted with EtOAc. The extracts were washed with water and dried, then evaporated leaving an orange semi-solid. Trituration with heptane and filtration provided 5.1 g of orange solid, containing 94 % of the target product and 6 % of the de-chlorinated product.

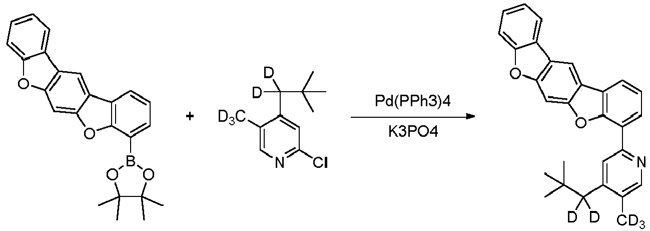

The boronic ester from the previous step (3.6 g, 9.37 mmol), 2-chloro-4-(2,2-dimethylpropyl-1,1-d2)-5-(methyl-d3)pyridine (1.899 g, 9.37 mmol), and tetrakis(triphenyl)phosphine)palladium(0) (0.541 g, 0.468 mmol) were dissolved in dioxane (110 ml). Potassium phosphate tribasic monohydrate (6.46 g, 28.1 mmol) in water (20 ml) was added as one portion. The reaction mixture was degassed and heated to reflux under nitrogen for 24 h. The reaction was allowed to cool to room temperature, mixed with 75 ml of brine, and extracted with EtOAc. The extracts were washed with brine, dried, and evaporated. The resulting solid was chromatographed on silica gel column and eluted with heptane/DCM 50/50 to 0/100 (v/v) gradient mixture to yield the target compound as white solid (3.17 g). Crystallization from heptanes/DCM provided 1.95 g of white solid.

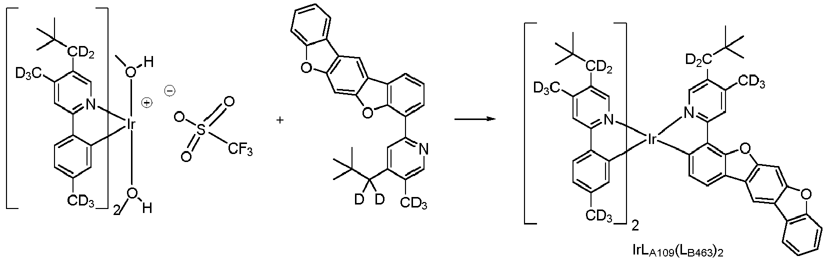

The polycyclic compound from the previous step (1.95 g, 4.59 mmol) was dissolved in a 2-ethoxy ethanol (25 ml) and DMF (25 ml) mixture. The iridium triflic salt complex shown above (2.362 g, 2.55 mmol) was added as one portion. The reaction mixture was degassed and heated to 100 °C in an oil bath under nitrogen for 9 days. The reaction mixture was then cooled down to room temperature and the solvents were evaporated. The residue was triturated with methanol to produce 3.4 g of solid that was chromatographed on silica gel column and eluted with a heptane/toluene/DCM 60/30/10 mixture (v/v/v) to recover 1.1 g of yellow solid (98.8% purity by HPLC). The mixture was then subjected to a second chromatography on a silica gel column eluted with heptanes/toluene 1/1 (v/v) mixture, followed by trituration with methanol to yield the target complex **IrL_{A109}(L**_{B463}**)₂** as a yellow solid (2.0 g).

### Synthesis of IrL_{A111}(L_{B284})₂

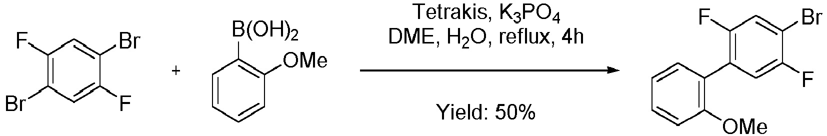

In a 1 L round bottom flask equipped with a reflux condenser under argon, a mixture of 1,4-dibromo-2,5-difluorobenzene (53.7 g, 197 mmol), (2-methoxyphenyl)boronic acid (20 g, 132 mmol), potassium phosphate monohydrate (60.5 g, 263 mmol) in dimethoxyethane (DME) (590 mL) and water (65 ml) was bubbled with argon for 10 min, then tetrakis (1.521 g, 1.316 mmol) was added and the reaction mixture was refluxed at 82 °C for 8 hours. The reaction was monitored by liquid chromatography-mass spectroscopy (LCMS). The reaction mixture was cooled to room temperature and treated with water (200 ml). The aqueous layer was separated and extracted several times with ethyl acetate (300 ml each). The organic layer was washed with brine (200 mL), dried with Na₂SO₄, filtered, concentrated, and dried *in vacuo.* The crude product was chromatographed on a 220 g gold SiO₂ column eluting with 0-40% EtOAc/Hexane to yield 5-bromo-2,4difluoro-2'-methoxy-1,1'byphenyl as clear oil (19.68 g, 50% yield).

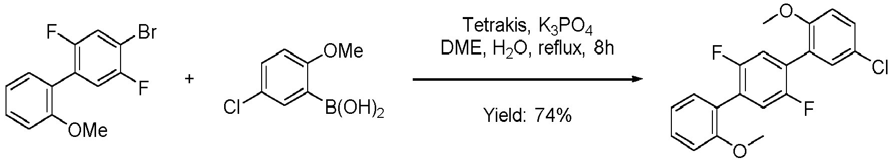

A solution of 5-bromo-2,4-difluoro-2'-methoxy-1,1'biphenyl (20 g, 66.9 mmol) and (4-chloro-2-methoxyphenyl)boronic acid (18.69 g, 100 mmol), and potassium phosphate monohydrate (30.8 g, 134 mmol) in DME (301 ml) and water (33.4 ml) was stored under argon atmosphere with a reflux condenser. The reaction mixture was bubbled with argon for 10 minutes, then tetrakis (1.545, 1.337 mmol) was added and bubbling with argon was continued for 5 more minutes. The reaction mixture was heated to reflux at 82 °C for 12 hours. Reaction was monitored by LCMS. The reaction mixture was then cooled to room temperature and water (450 mL) was added. The solid product was filtered off and washed with water (50 mL) and then dried *in vacuo* to yield 4-chloro-2',5'-difluoro-2,2'-dimethoxy-1,1'4'1"terphenyl as a pale yellow solid (17.85 g, 74% yield).

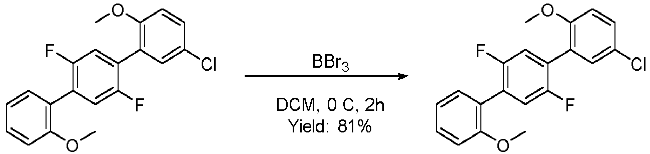

A 500mL round bottom (RB) flask was charged with 4-chloro-2',5'-difluoro-2,2'-dimethoxy-1,1'4'1"terphenyl (T18-224B) (26.5, 73.5 mmol) in dichloromethane (367 mL). Then borontribromide (15.68 mL, 162 mmol) was added. The resulting mixture was stirred at room temperature for about 2 h until liquid chromatography showed that the starting material was fully consumed. Then, the reaction mixture was slowly cooled to 0 °C, and quenched with methanol (5 ml), followed by concentration *in vacuo.* The residue was slowly treated with water (50 mL) and EtOAc (100 mL). The organic and aqueous layers were separated, and the aqueous layer was extracted with EtOAc (100 ml). The combined organic layers were washed with brine (100 ml) and dried with Na₂SO₄, filtered, concentrated and dried *in vacuo.* The crude product was loaded on SiO₂ and chromatographed on a 330 g gold SiO₂ column eluting with 0-30% hexane/EtOAc to give the pure product 4-chloro-2',5'-difluoro-1,1'4'1"teiphenyl-2,2'-diol (17.85 g, 81%).

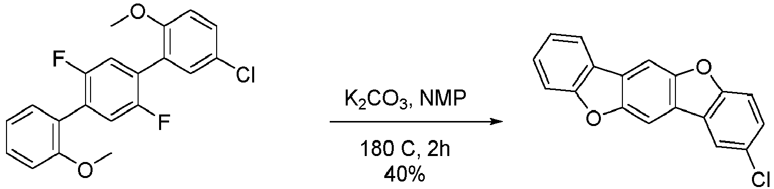

A 250mL RB flask was charged with 4-chloro-2',5'-difluoro-2,2'-dimethoxy-1,1'4'1"terphenyl (T18-224A) (6.5 g, 73.5 mmol) and dissolved in NMP (98 mL) under an argon atmosphere. Then, cesium carbonate (13.37 g, 41 mmol) was added and the resulting mixture was stirred at 150 °C for about 4 h until liquid chromatography confirmed complete consumption of starting material. Then reaction mixture was cooled to room temperature and quenched with water (50 mL) to afford an off-white solid product (90% purity based on LCMS). The purification was conducted via recrystallization in tetrahydrofuran (THF), followed by DME washings under argon atmosphere several times to afford the required purity. The product was further purified via charcoal treatment to afford the white solid of (2.287g, 40%).

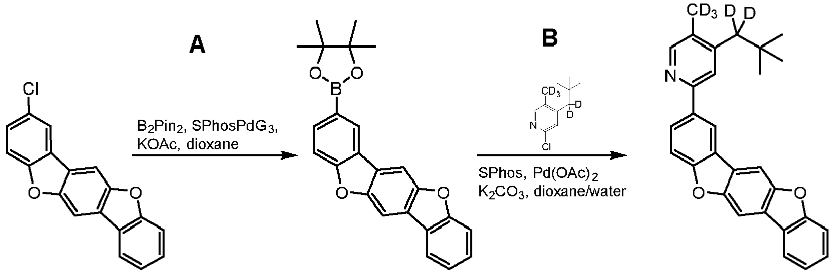

Step A. A 500 mL, 4-neck round bottom flask equipped with a condenser, stir bar, and thermocouple was charged with 2-chloro-bis(benzofuro)[2,3-*b*:2',3'-*e*]benzene (5.81 g, 19.8 mmol, 1.0 equiv), bis(pinacolato)diboron (5.29 g, 20.8 mmol, 1.05 equiv), potassium acetate (4.87 g, 49.6 mmol, 2.5 equiv) and 1,4-dioxane (132 mL). The reaction mixture was sparged with nitrogen for 15 minutes, and SPhosPdG₃ ((2-Dicyclohexylphosphino-2',6'-dimethoxybiphenyl) [2-(2'-amino-1,1'-biphenyl)]palladium(II)methanesulfonate) (0.387 g, 0.496 mmol, 0.025 equiv) was added. Sparging was continued then reaction mixture heated at 100 °C overnight. The cooled reaction mixture was passed through a pad of silica gel (40 g), rinsed with ethyl acetate (200 mL), and the filtrate concentrated under reduced pressure. The crude residue was chromatographed on neutral alumina (150 g), eluting with a gradient of 0-20% ethyl acetate in heptanes (200 mL of solvent mixture for each 10% increase of polarity) to give 2-BPin-bis(benzofuro) [2,3-*b*:2',3'-*e*]benzene (6.3 g, 83% yield) as a white solid.

Step B. In the second step of the synthesis scheme shows above, an 100 mL, 4-neck round bottom flask equipped with a condenser, stir bar and thermocouple was charged 2-BPin-bis(benzofuro) [2,3-*b*:2',3'-*e*]benzene (3.6 g, 9.4 mmol, 1.0 equiv), 2-chloro-4-(2,2-dimethyl-propyl-1,1-*d₂*)-5-(methyl-*d₃*)pyridine (1.99 g, 9.8 mmol, 1.05 equiv), potassium carbonate (2.59 g, 18.7 mmol, 2.0 equiv), and a mixture of 1,4-dioxane (46.8 mL) and deionized, ultrafiltered water (15.6 mL). The reaction mixture was sparged with nitrogen for 15 minutes, then palladium(II) acetate (0.063 g, 0.281 mmol, 0.03 equiv) and 2-dicyclohexyl-phosphino-2',6'-dimethoxy-biphenyl (SPhos) (0.231 g, 0.562 mmol, 0.06 equiv) were added. Sparging was continued while the reaction mixture was heated at reflux overnight. The reaction mixture was cooled to room temperature then filtered and the solid was washed with dichloromethane (50 mL). [***Note:** Due to its low solubility, a small amount of product remained on the filter*.] The filtrate was washed with water (50 mL), dried over sodium sulfate, filtered and concentrated under reduced pressure. The white solid was triturated with hot ethyl acetate (20 mL) and filtered to give2-(4-(2,2-dimethylpropyl-1,1-*d₂*)-5-(methyl-*d₃*)pyridine)-bis(benzofuro) [2,3-*b*:2',3'-*e*]benzene (2.2 g, 55% yield) as a white solid.

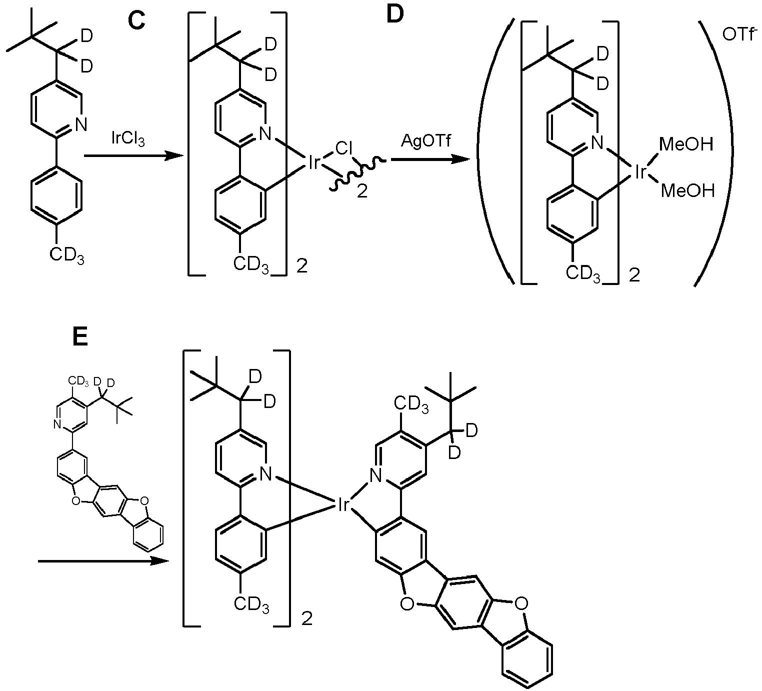

Step C. A 1L 4-neck flask was charged with 2-ethoxyethanol (240 mL) and DIUF water (80 mL) and the mixture sparged with nitrogen for 15 minutes. Iridium(III) chloride hydrate (21.9 g, 69.2 mmol, 1.0 equiv) and 5-(2,2-dimethylpropyl-1,1-*d₂*)-2-(4-(methyl-*d₃*)phenyl)pyridine (37.2 g, 152 mmol, 2.2 equiv) were added and the reaction mixture heated at reflux for 63 hours. The cooled reaction mixture was filtered and the solid washed with methanol then air-dried to give di-*µ*-chloro-tetrakis[*κ2*(C2,N)-5-(2,2-dimethyl-propyl-1,1-*d₂*)-2-(4-methyl-*d₃*)phenyl)pyridine]diiridium(III) (34.2 g, 69% yield), containing ~2.7% ligand, as a dark yellow solid.

Step D. A 4L 4-neck flask was charged with di-*µ*-chloro-tetrakis[*κ2*(C2,N)-5-(2,2-dimethylpropyl-1,1-*d₂*)-2-(4-methyl-*d₃*)phenyl)pyridine]diiridium(III) (34.2 g, ~23.9 mmol, 1.0 equiv) in dichloromethane (830 mL). The flask was wrapped with aluminum foil to exclude light and a solution of silver trifluoromethanesulfonate (14.6 g, 56.7 mmol, 2.37 equiv) in methanol (150 mL) added. The reaction mixture was stirred at room temperature for 24 hours then filtered through a pad of silica gel (100 g) topped with Celite (30 g), rinsing thoroughly with dichloromethane. The filtrate was concentrated under reduced pressure and the residue dried in a vacuum oven to give [Ir(5-(2,2-dimethylpropyl-1,1-*d₂*)-2-(4-methyl-*d*₃-phenyl)pyridine(-1H))₂-(MeOH)₂](trifluoromethanesulfonate) (35.2 g, 83% yield, 96.9% UPLC purity) as a yellow solid.

Step E. A 100 mL 1-neck round bottom flask, equipped with a condenser and stir bar, was charged with [Ir(5-(2,2-dimethylpropyl-1,1-*d₂*)-2-(4-methyl-*d*₃-phenyl)pyridine(-1H))₂-(MeOH)₂](trifluoromethanesulfonate) (1.3 g, 1.46 mmol, 1.0 equiv), 2-(4-(2,2-dimethylpropyl-1,1-*d₂*)-5-(methyl-*d₃*)pyridine)-bis(benzofuro)[2,3-*b*:2',3'-*e*]-benzene (1.3 g, 3.06 mmol, 2.1 equiv) and ethanol (32.4 mL). The flask was wrapped with aluminum foil and the reaction mixture was heated at 85 °C for a total of 14 hours. [***Note:** The reaction mixture was not heated overnight*.]. The reaction mixture was cooled to room temperature and filtered. The crude solid was washed with methanol (50 mL) and the filtrate concentrated under reduced pressure. The residue was dissolved in dichloromethane and passed through a short silica gel pad (30 g), rinsing with dichloromethane (100 mL), and the eluted solution concentrated under reduced pressure. The residue was chromatographed on an Interchim automated system (80 g Sorbtech column, 45 min run), eluting with 40% dichloromethane in heptanes. [***Note:** Each fraction was analyzed for purity by the SA50Long LC method.]* Product fractions were concentrated under reduce pressure to give bis[5-(2,2-dimethyl propyl-1,1-*d₂*)-2-(4-(methyl-*d₃*)-[1'-phenyl]-2'-yl)pyridine-1-yl] [2-(4-(2,2-dimethylpropyl-1,1-*d₂*)-5-(methyl-*d₃*)pyridine-2-yl)-(bisbenzofuro)[2,3-*b*:2',3'-*e*]benzene-3-yl] iridium(III) (0.5 g, 28% yield, 97.5% UHPLC purity), containing ~2% of the wrong heteroleptic complex, as a yellow solid.

### Device Examples

The following compounds were used in the device examples.

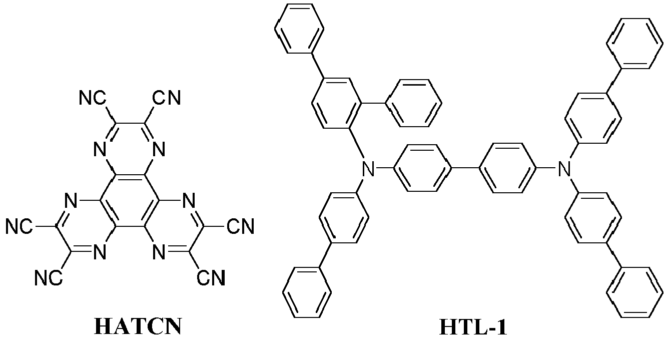

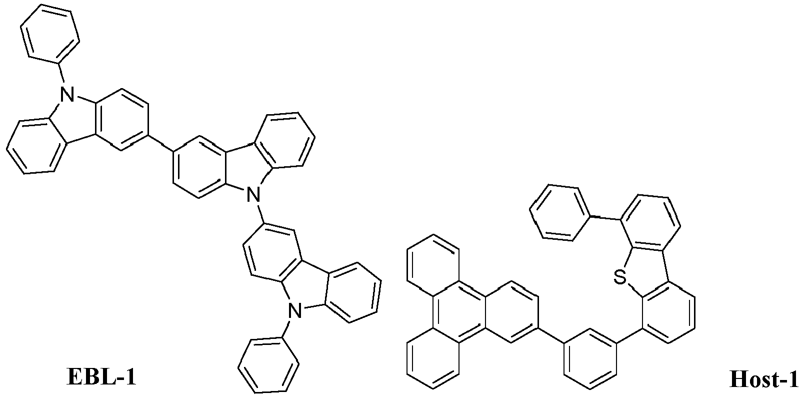

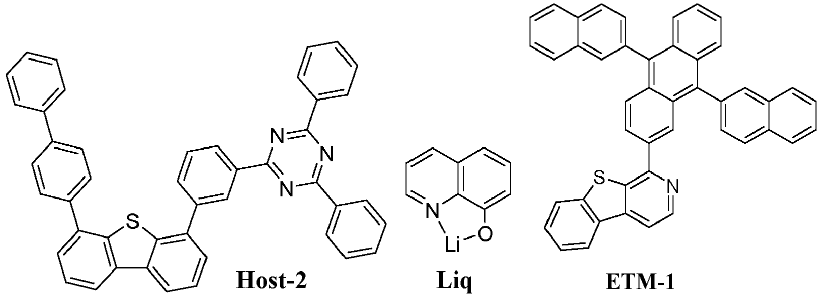

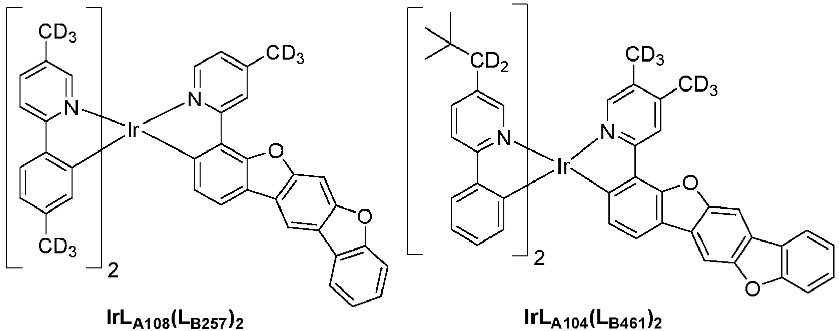

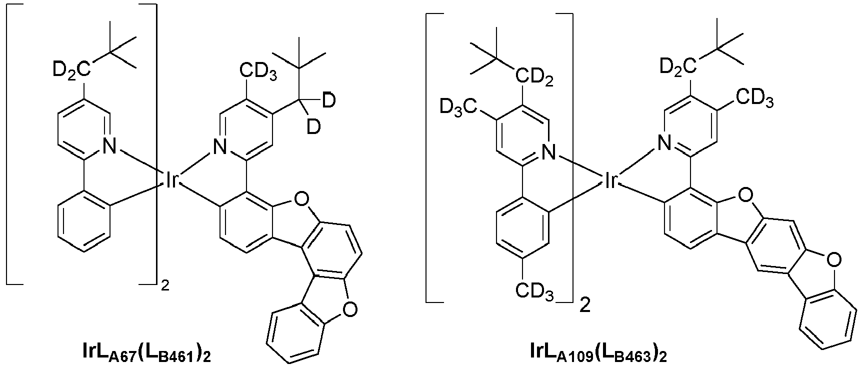

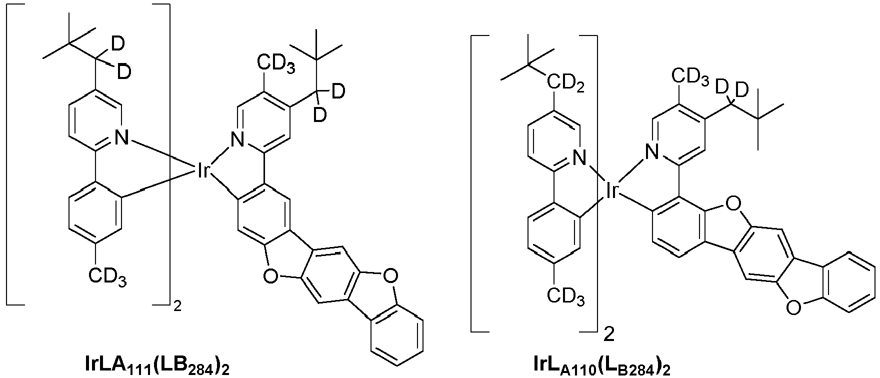

All example devices were fabricated by high vacuum (<10⁻⁷ Torr) thermal evaporation. The anode electrode was 800 Å of indium tin oxide (ITO). The cathode consisted of 1000 Å of Al. All devices were encapsulated with a glass lid sealed with an epoxy resin in a nitrogen glove box (<1 ppm of H₂O and O₂) immediately after fabrication, and a moisture getter was incorporated inside the package. The organic stack of the device examples consisted of sequentially, from the ITO surface, 100Å of HATCN as the hole injection layer (HIL); 400 Å of HTL-1 as the hole transporting layer (HTL); 50 Å of EBL-1 as the electron blocking layer; 400 Å of an emissive layer (EML) comprising 12% of the dopant in a host comprising a 60/40 mixture of Host-1 and Host-2; 350 Å of Liq doped with 35% of ETM-1 as the ETL; and 10 Å of Liq as the electron injection layer (EIL).

Upon fabrication, the electroluminescence (EL) and current density-voltage-luminance (JVL) performance of the devices was measured. The device lifetimes were evaluated at a current density of 80 mA/cm². The device data is summarized in Table 1, and demonstrates that the dopants of the present invention afford green emitting devices with narrow line width and high efficiency.

**Table 1**

| **Device Example** | **Dopant** | **1931 CIE** | | **λ max [nm]** | **FWHM [nm]** | **At 10mA/cm²** | | **At 80 mA/cm2** |
|---|---|---|---|---|---|---|---|---|
| | | **x** | **y** | | | **Voltage [V]** | **EQE [%]** | **LT_{95%} [h]** |
| **1** | IrL_{A104}(L_{B461})₂ | 0.326 | 0.642 | 527 | 32 | 4.71 | 20.1 | 133 |
| **2** | IrL_{A110}(L_{B284})₂ | 0.329 | 0.641 | 527 | 31 | 4.5 | 22.9 | 138 |
| **3** | IrL_{A67}(L_{B461})₂ | 0.306 | 0.647 | 520 | 53 | 4.57 | 21.4 | 10 |
| **4** | IrL_{A109}(L_{B463})₂ | 0.332 | 0.634 | 524 | 57 | 4.52 | 23.2 | 18 |
| **5** | IrL_{A108}(L_{B257})₂ | 0.351 | 0.621 | 530 | 62 | 4.72 | 22.0 | 20 |
| **6** | IrL_{A111}(L_{B284})₂ | 0.288 | 0.624 | 510 | 70 | 4.6 | 16.6 | 36 |

It is understood that the various embodiments described herein are by way of example only, and are not intended to limit the scope of the invention. For example, many of the materials and structures described herein may be substituted with other materials and structures without deviating from the spirit of the invention. The present invention as claimed may therefore include variations from the particular examples and preferred embodiments described herein, as will be apparent to one of skill in the art. It is understood that various theories as to why the invention works are not intended to be limiting.

The invention is further described by the following numbered aspects:
1. A compound comprising a first ligand L_{A} of Formula I 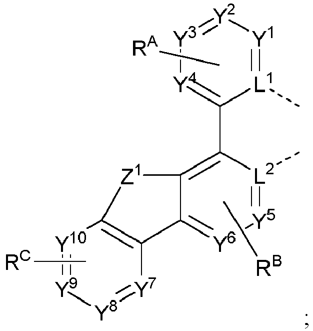 wherein one of L¹ and L² is C, and the other of L¹ and L² is N; wherein Y¹ to Y¹⁰ are each independently selected from the group consisting of C and N; wherein at least two adjacent Y⁷, Y⁸, Y⁹, and Y¹⁰ are carbon atoms that are fused to a structure of Formula II 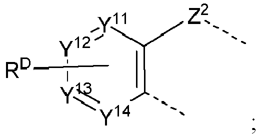 wherein Y¹¹ to Y¹⁴ are each independently selected from the group consisting of C and N; wherein Z¹ and Z² are each independently selected from the group consisting of O, S, Se, NR, CRR', and SiRR'; wherein R^{A}, R^{B}, and R^{D} represent mono to a maximum possible number of substitutions, or no substitution; wherein R^{C} represents di-, tri-, or tetra-substitution; wherein each R, R', R^{A}, R^{B}, R^{C}, and R^{D} is independently hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof; wherein any two substituents may be joined or fused together to form a ring; wherein L_{A} is complexed to a metal M by L¹ and L², and M has an atomic weight greater than 40; wherein M is optionally coordinated to other ligands; and wherein the ligand L_{A} is optionally linked with other ligands to comprise a tridentate, tetradentate, pentadentate, or hexadentate ligand.
2. The compound of aspect 1, wherein each R, R', R^{A}, R^{B}, R^{C}, and R^{D} is independently a hydrogen or a substituent selected from the group consisting of deuterium, fluorine, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof.
3. The compound of aspect 1 or 2, wherein M is selected from the group consisting of Ir, Rh, Re, Ru, Os, Pt, Au, and Cu.
4. The compound of any one of aspects 1 to 3, wherein Y¹ to Y¹⁴ are each C.
5. The compound of any one of aspects 1 to 3, wherein at least one of Y¹ to Y⁴ is N, and/or at least one of Y¹¹ to Y¹⁴ is N.
6. The compound of any one of aspects 1 to 5, wherein Z¹ is O.
7. The compound of any one of aspects 1 to 6, wherein Y⁷ to Y¹⁰ are each C.
8. The compound of any one of aspects 1 to 7, wherein L¹ is N and L² is C.
9. The compound of any one of aspects 1 to 7, wherein Z¹ and Z² are para with respect to one another.
10. The compound of any one of aspects 1 to 9, wherein the first ligand L_{A} is selected from the group consisting of: 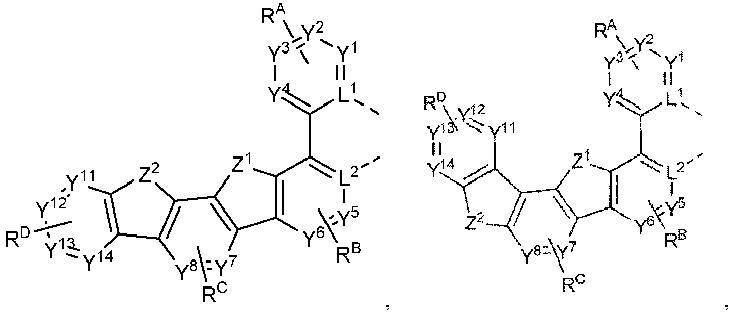 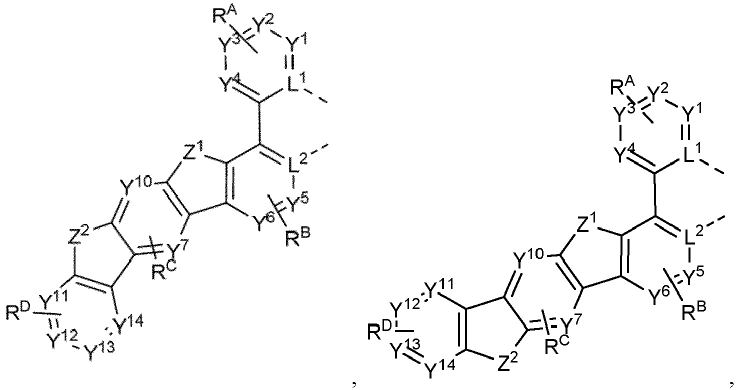 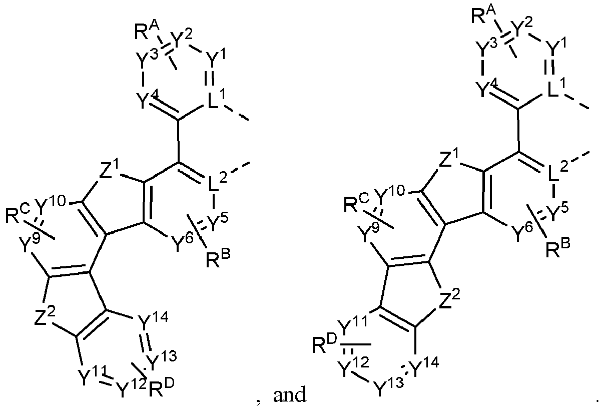
11. The compound of any one of aspects 1 to 10, wherein the first ligand L_{A} is selected from the group consisting of L_{A1} to L_{A111} as described herein before.
12. The compound of any one of aspects 1 to 11, wherein the compound has a formula of M(L_{A})ₓ(L_{B})_{y}(L_{c})_{z} wherein L_{B} and L_{C} are each a different bidentate ligand; and wherein x is 1, 2, or 3; y is 0, 1, or 2; z is 0, 1, or 2; and x+y+z is the oxidation state of the metal M.
13. The compound of aspect 12, wherein L_{B} and L_{C} are each independently selected from the group consisting of: 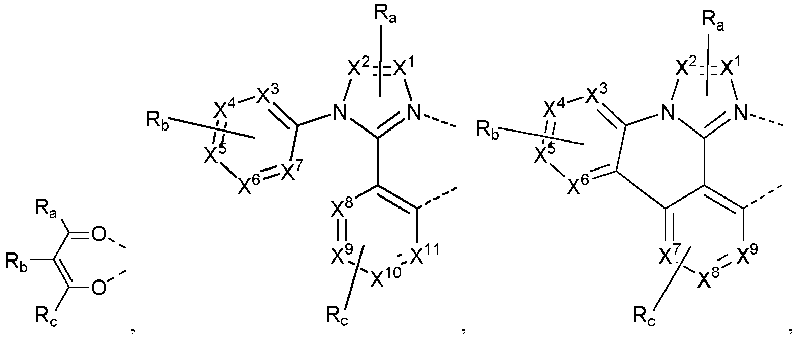 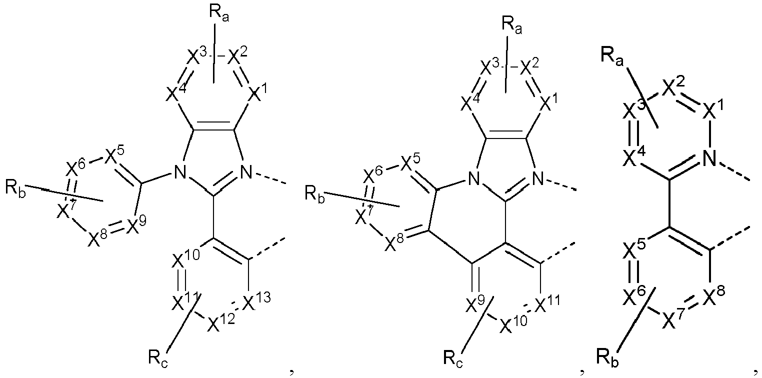 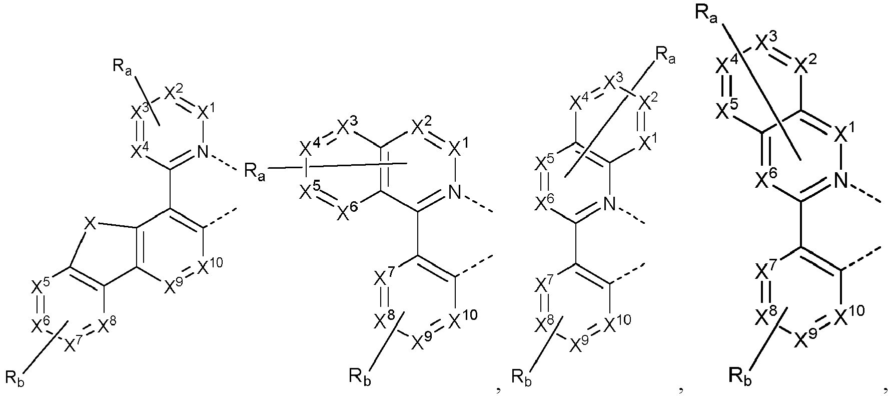 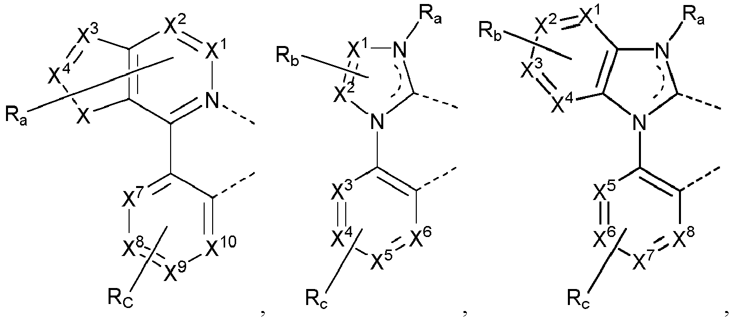 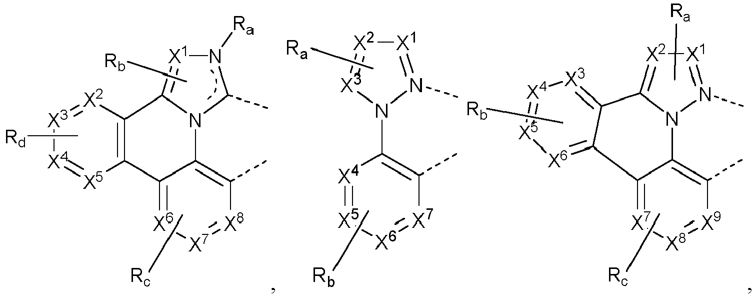 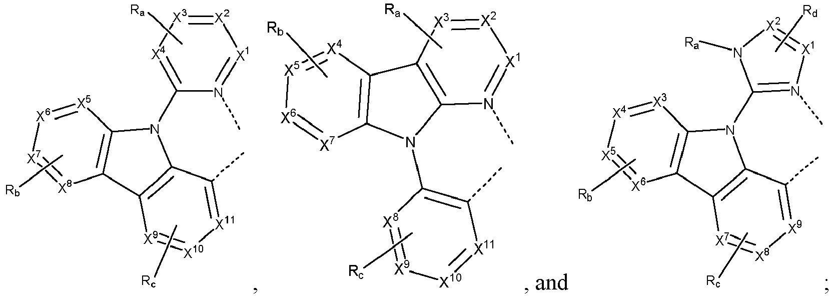 wherein each X¹ to X¹³ is independently selected from the group consisting of carbon and nitrogen; wherein X is selected from the group consisting of BR', NR', PR', O, S, Se, C=O, S=O, SO₂, CR'R", SiR'R", and GeR'R"; wherein R' and R" are optionally fused or joined to form a ring; wherein each Rₐ, R_{b}, R_{c}, and R_{d} represents from mono substitution to a maximum possible number of substitutions, or no substitution; wherein R', R", Rₐ, R_{b}, R_{c}, and R_{d} are each independently a hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof; and wherein any two adjacent substituents of Rₐ, R_{b}, R_{c}, and R_{d} are optionally fused or joined to form a ring or form a multidentate ligand.
14. The compound of aspect 11, wherein the compound is Compound Ax having the formula Ir(L_{A*i*})₃, or Compound B*y* having the formula Ir(L_{A*i*})(L_{B*k*})₂;
   wherein *x* = *i*, and *y* = 468*i*+*k*-468;
   wherein *i* is an integer from 1 to 111, and *k* is an integer from 1 to 468;
   wherein L_{B*k*} have the structures L_{B1} to L_{B468} as described herein before.
15. An organic light emitting device (OLED) comprising:
   an anode; a cathode; and an organic layer, disposed between the anode and the cathode, comprising a compound as described in any one of aspects 1 to 14.
16. The OLED of aspect 15, wherein the organic layer is an emissive layer and the compound is an emissive dopant or a non-emissive dopant.
17. The OLED of aspect 15, wherein the organic layer further comprises a host, wherein host comprises at least one chemical group selected from the group consisting of triphenylene, carbazole, dibenzothiophene, dibenzofuran, dibenzoselenophene, azatriphenylene, azacarbazole, aza-dibenzothiophene, aza-dibenzofuran, and aza-dibenzoselenophene.
18. The OLED of aspect 15, wherein the organic layer further comprises a host, wherein the host is selected from the group consisting of: 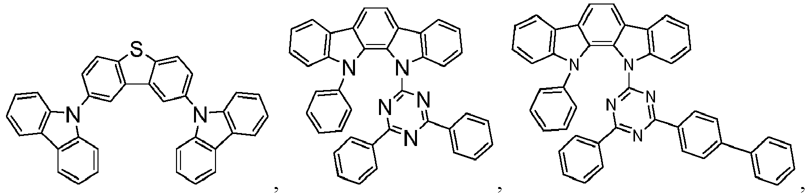 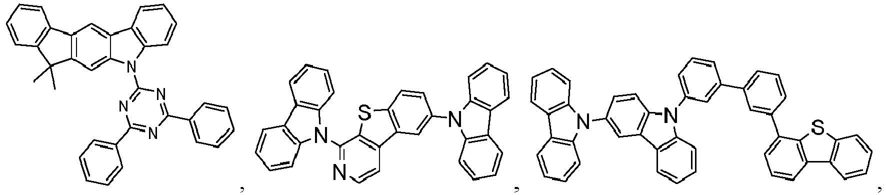 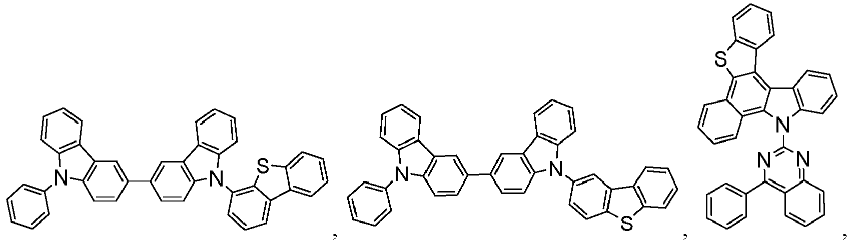 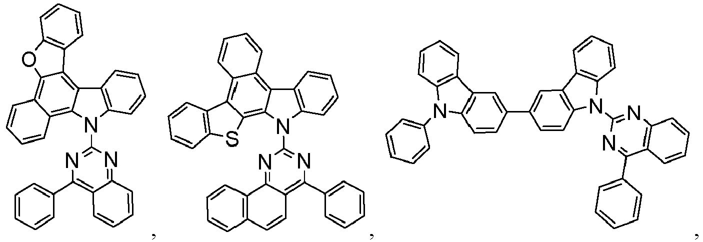 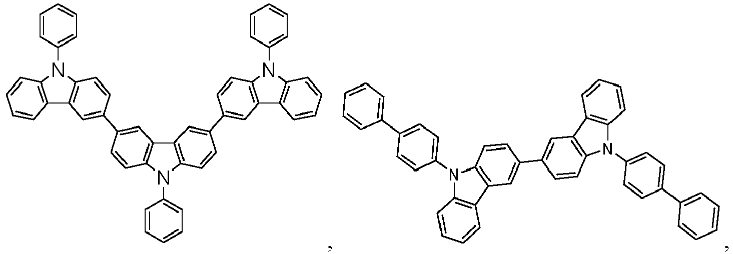 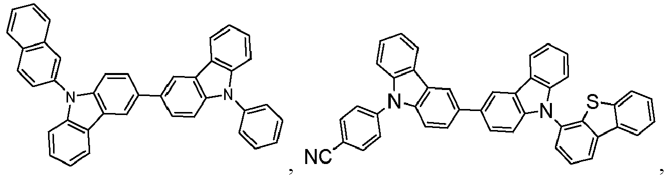 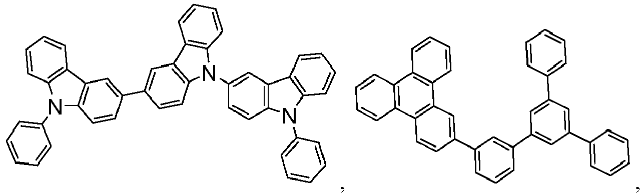 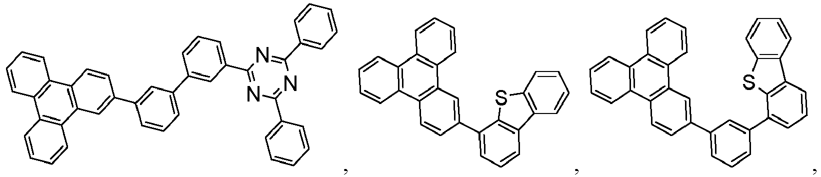 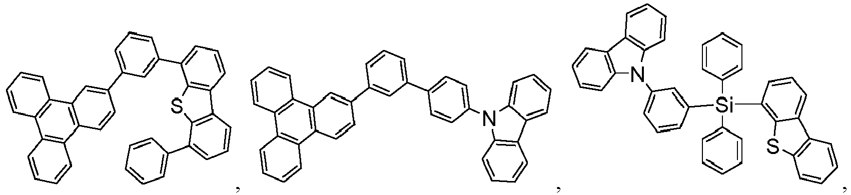 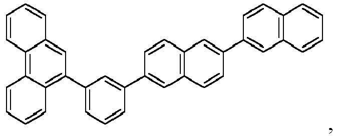 and combinations thereof.
19. A consumer product comprising an organic light-emitting device (OLED) comprising: an anode; a cathode; and an organic layer, disposed between the anode and the cathode, comprising a compound as described in any one of aspects 1 to 14.
20. A formulation comprising a compound as described in any one of aspects 1 to 14.

## Claims

1. A compound comprising a first ligand L_{A} of Formula I 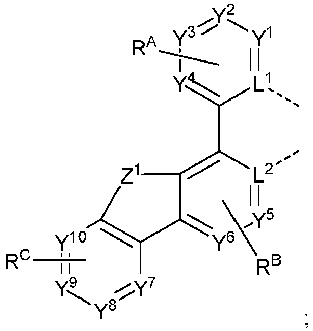
wherein one of L¹ and L² is C, and the other of L¹ and L² is N;
wherein Y¹ to Y¹⁰ are each independently selected from the group consisting of C and N;
wherein at least two adjacent Y⁷, Y⁸, Y⁹, and Y¹⁰ are carbon atoms that are fused to a structure of Formula II 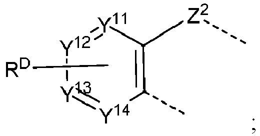
wherein Y¹¹ to Y¹⁴ are each independently selected from the group consisting of C and N;
wherein Z¹ and Z² are each independently selected from the group consisting of O, S, Se, NR, CRR', and SiRR';
wherein R^{A}, R^{B}, and R^{D} represent mono to a maximum possible number of substitutions, or no substitution;
wherein R^{C} represents di-, tri-, or tetra-substitution;
wherein each R, R', R^{A}, R^{B}, R^{C}, and R^{D} is independently hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof;
wherein any two substituents may be joined or fused together to form a ring;
wherein L_{A} is complexed to a metal M by L¹ and L², and M has an atomic weight greater than 40;
wherein M is optionally coordinated to other ligands; and
wherein the ligand L_{A} is optionally linked with other ligands to comprise a tridentate, tetradentate, pentadentate, or hexadentate ligand.

2. The compound of claim 1, wherein M is selected from the group consisting of Ir, Rh, Re, Ru, Os, Pt, Au, and Cu.

3. The compound of claim 1 or 2, wherein Y¹ to Y¹⁴ are each C.

4. The compound of claim 1 or 2, wherein at least one of Y¹ to Y⁴ is N, and/or at least one of Y¹¹ to Y¹⁴ is N.

5. The compound of any one of claims 1 to 4, wherein Z¹ is O.

6. The compound of any one of claims 1 to 5, wherein L¹ is N and L² is C.

7. The compound of any one of claims 1 to 6, wherein Z¹ and Z² are para with respect to one another.

8. The compound of any one of claims 1 to 7, wherein the first ligand L_{A} is selected from the group consisting of:

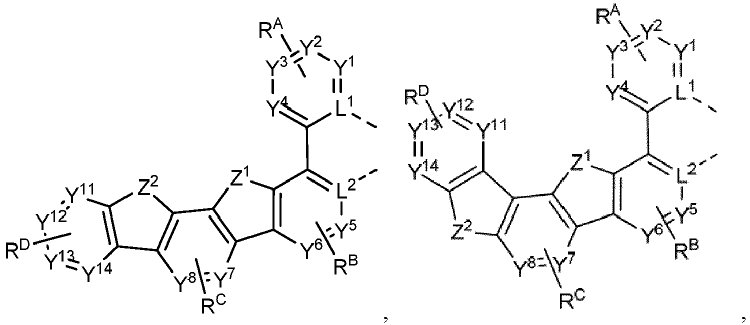

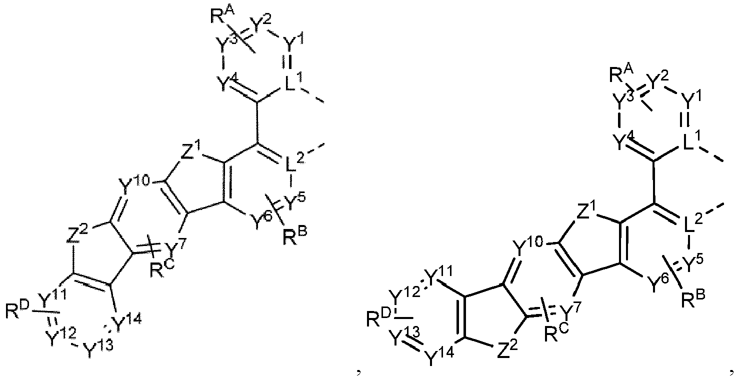

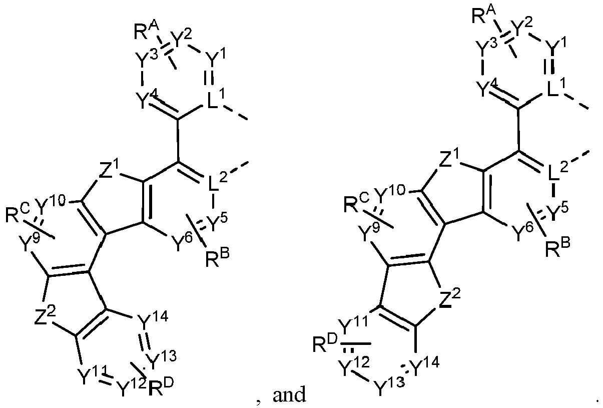

9. The compound of claims 1 or 2, wherein the first ligand L_{A} is selected from the group consisting of:

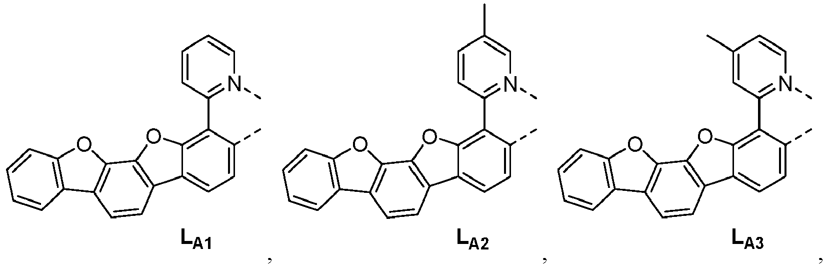

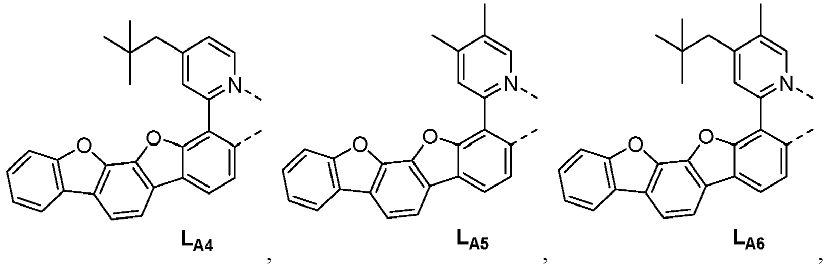

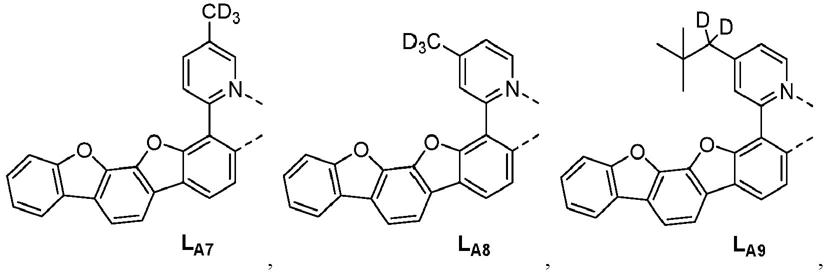

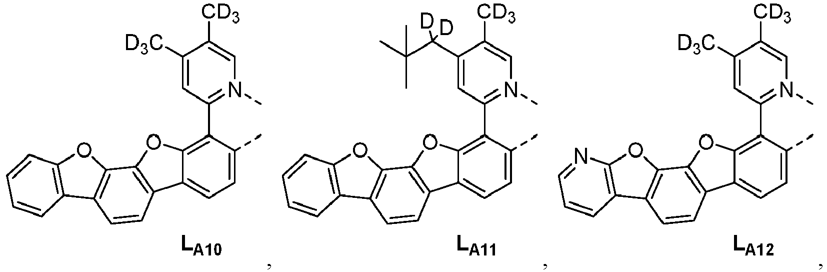

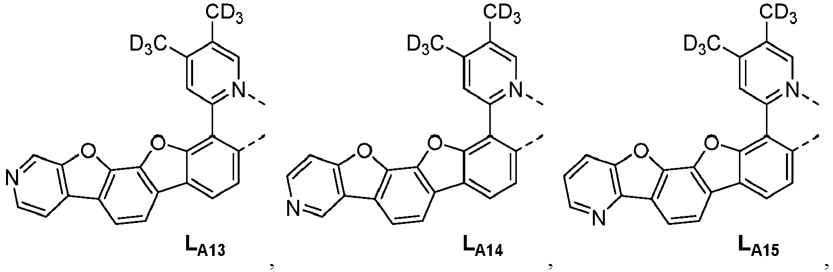

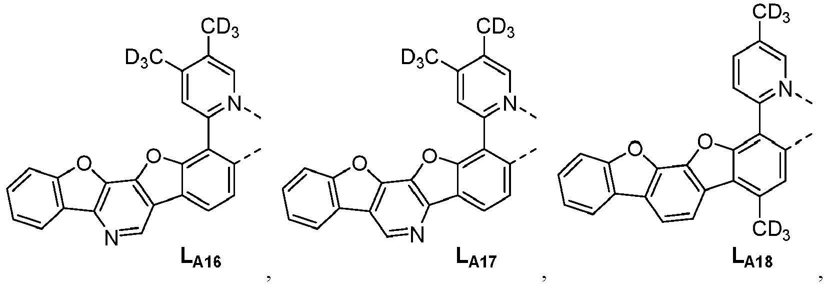

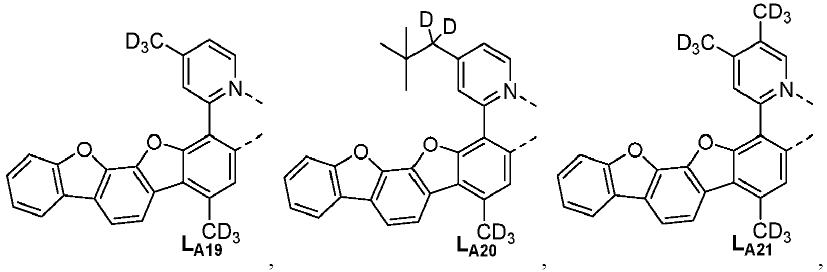

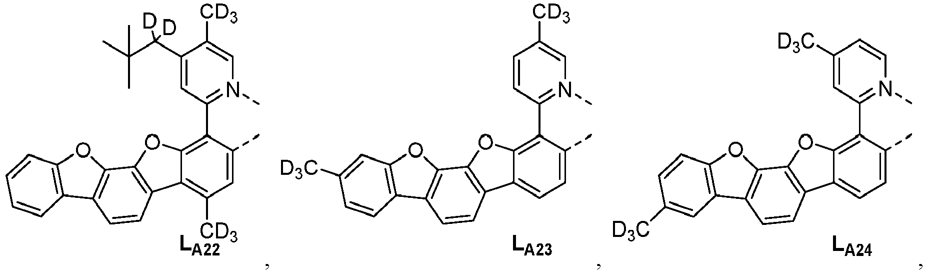

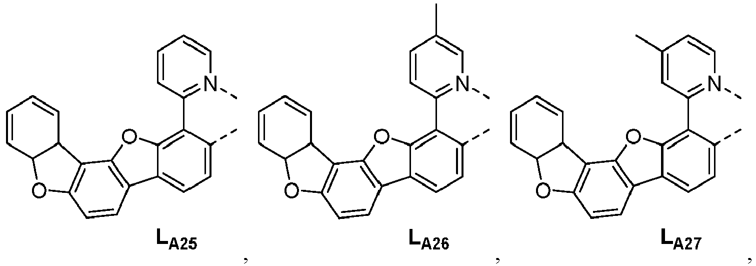

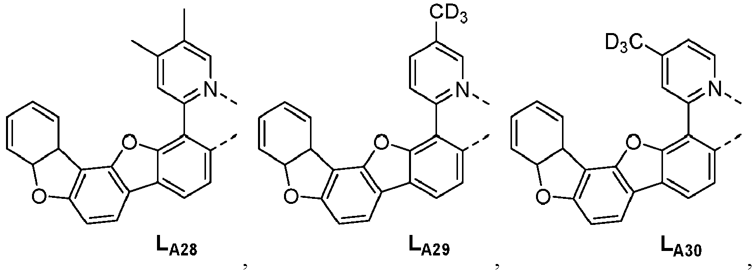

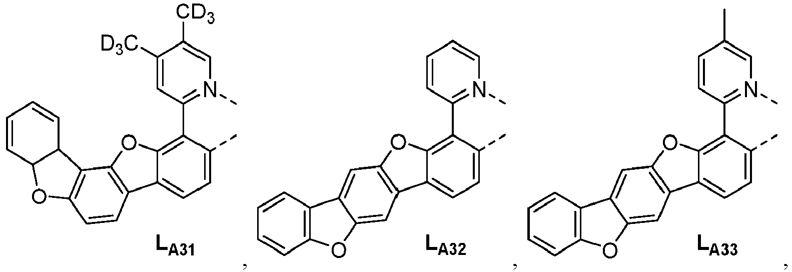

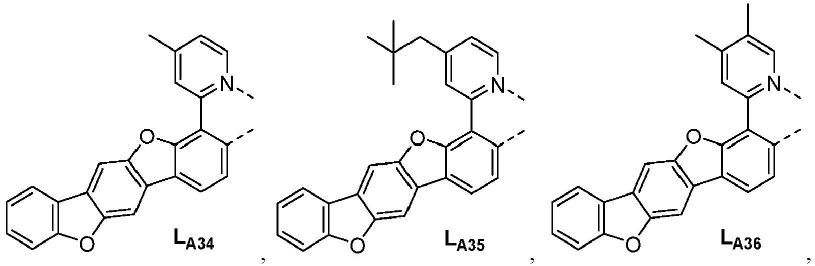

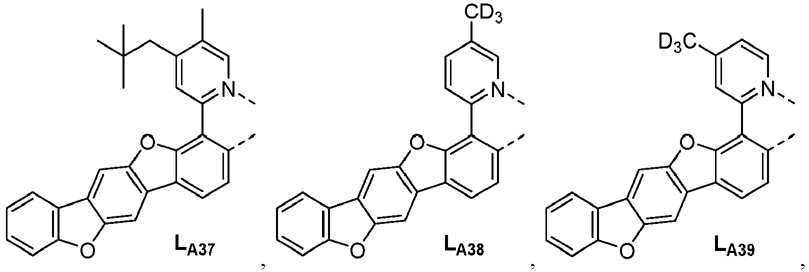

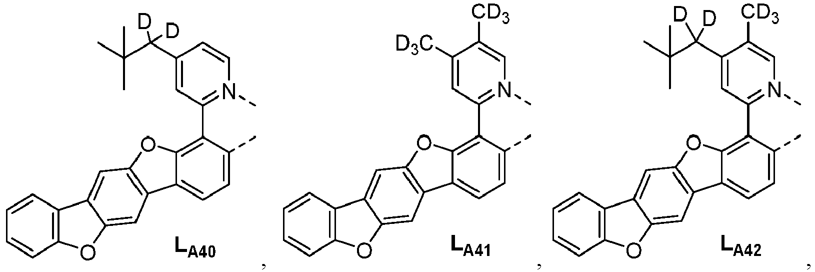

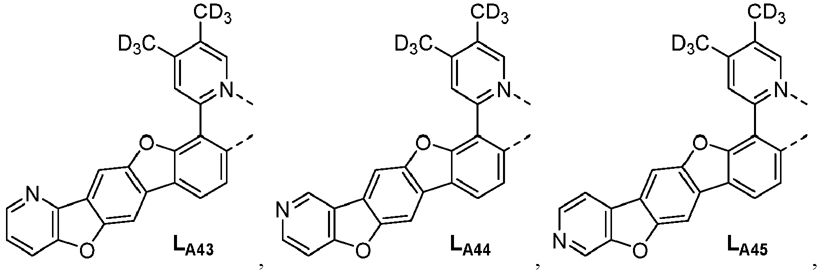

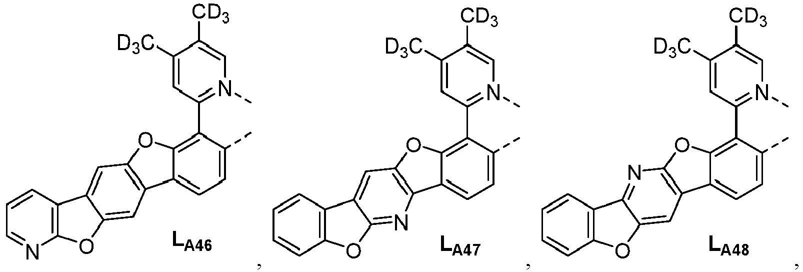

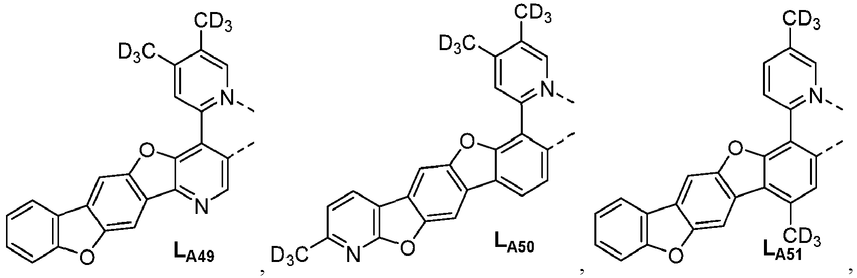

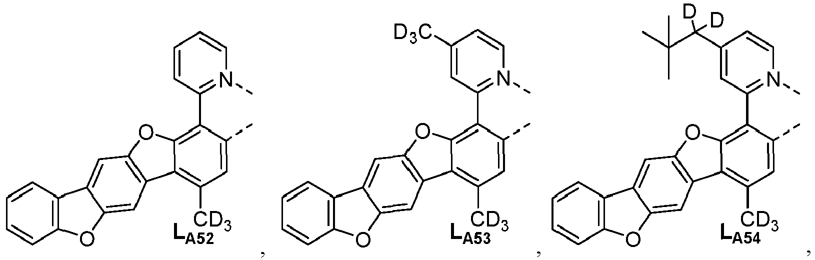

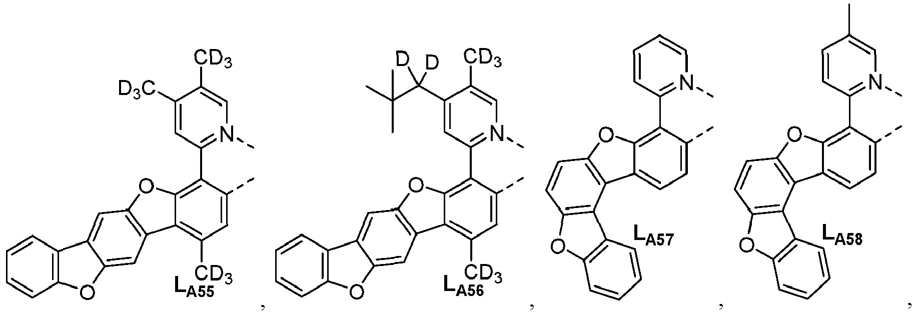

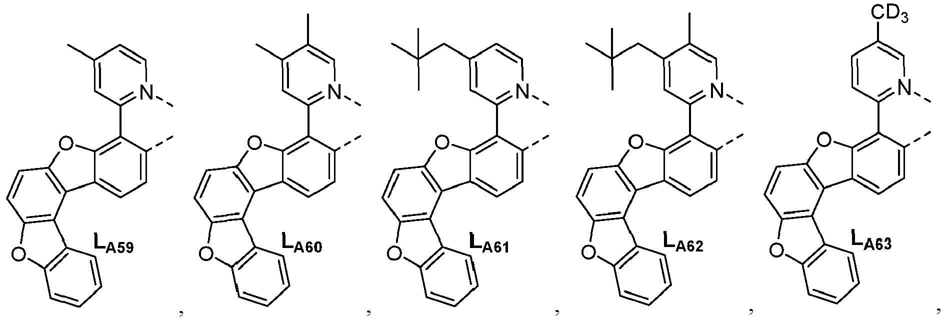

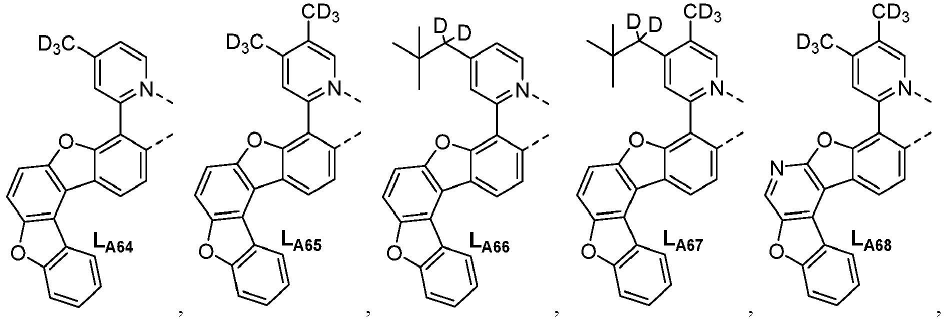

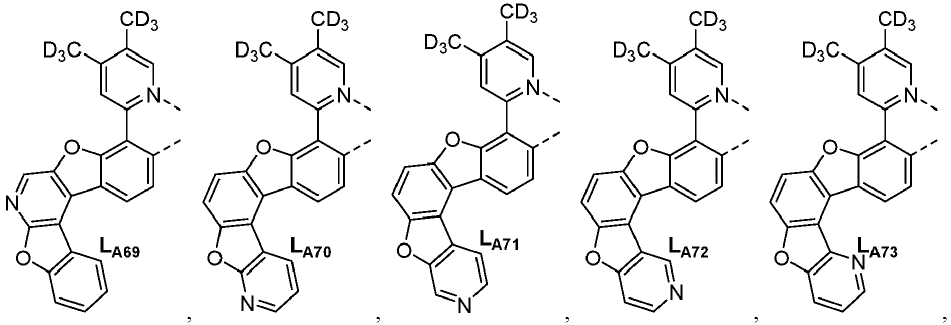

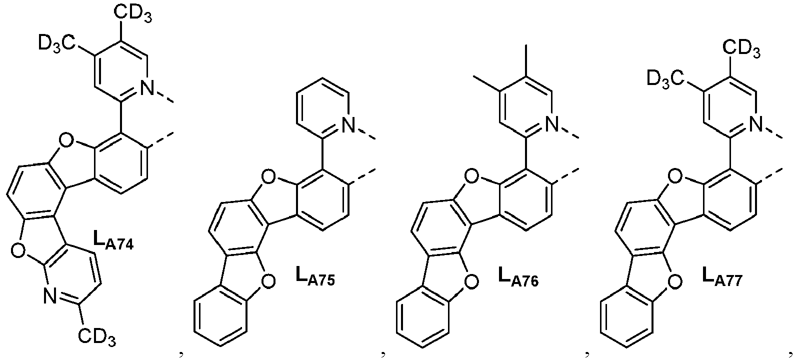

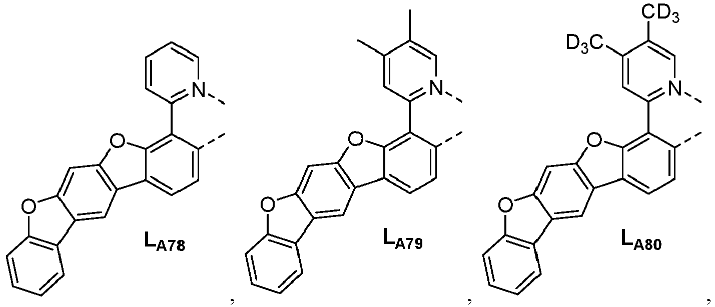

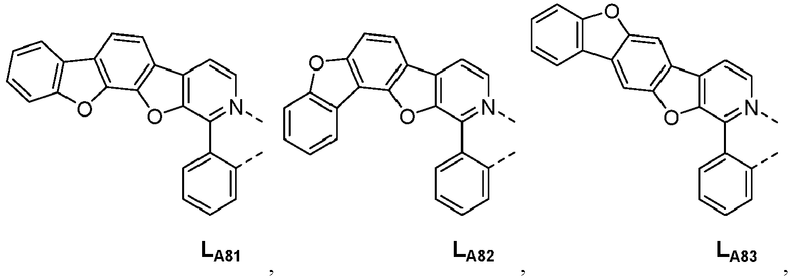

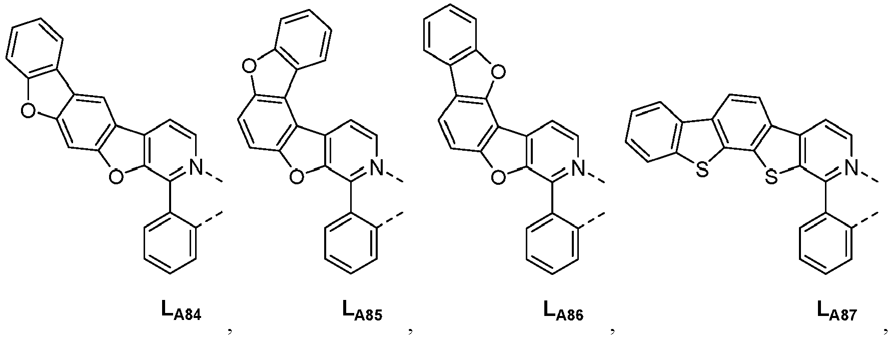

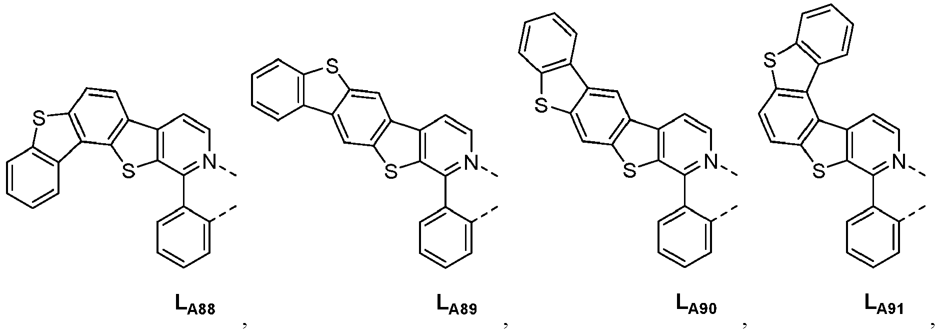

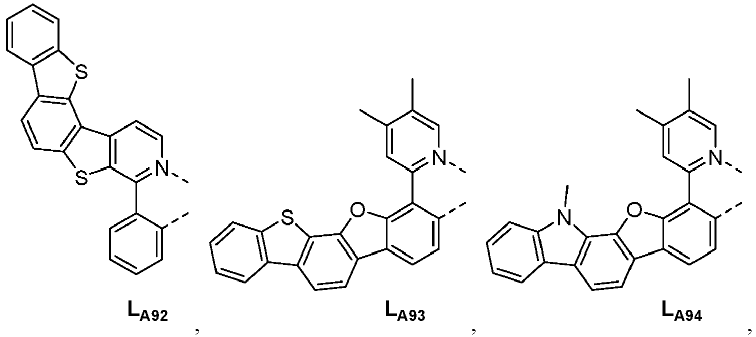

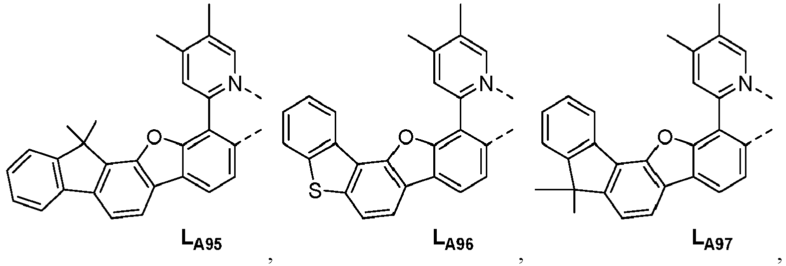

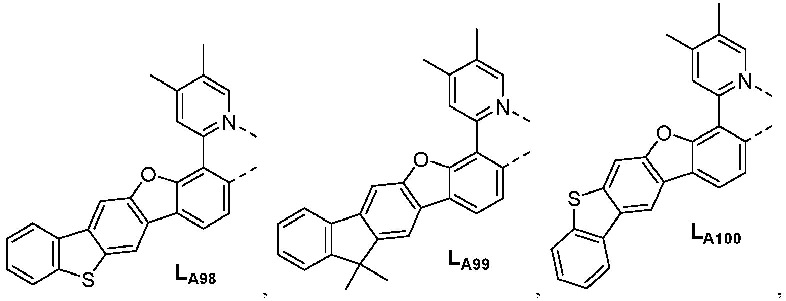

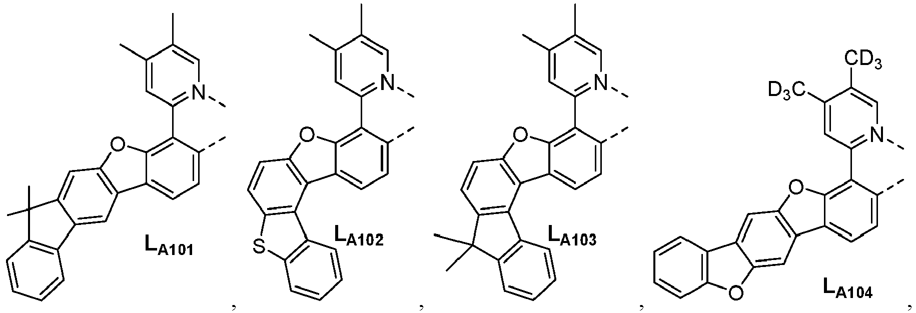

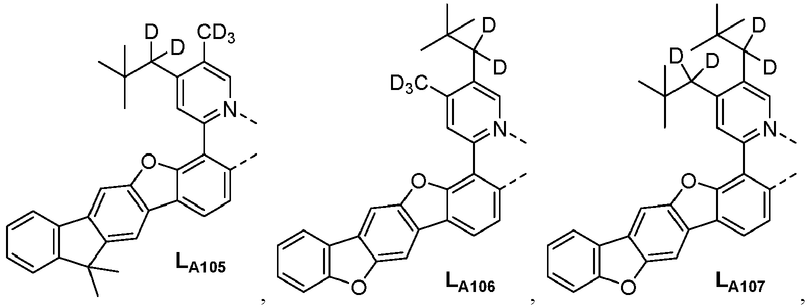

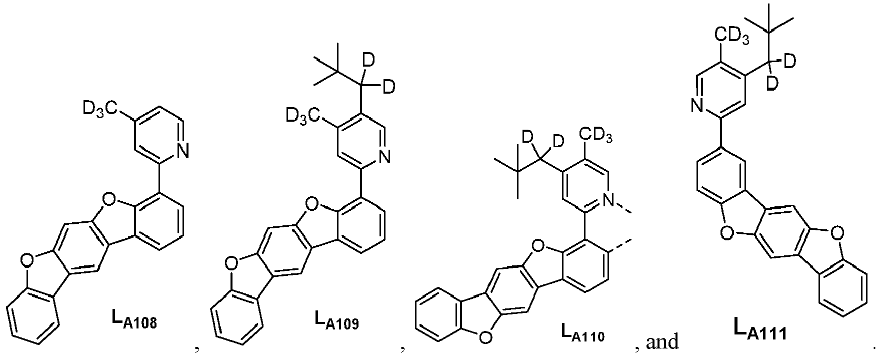

10. The compound of any one of claims 1 to 9, wherein the compound has a formula of M(L_{A})ₓ(L_{B})_{y}(L_{C})_{z} wherein L_{B} and L_{C} are each a different bidentate ligand; and wherein x is 1, 2, or 3; y is 0, 1, or 2; z is 0, 1, or 2; and x+y+z is the oxidation state of the metal M.

11. The compound of claim 10, wherein L_{B} and L_{C} are each independently selected from the group consisting of: 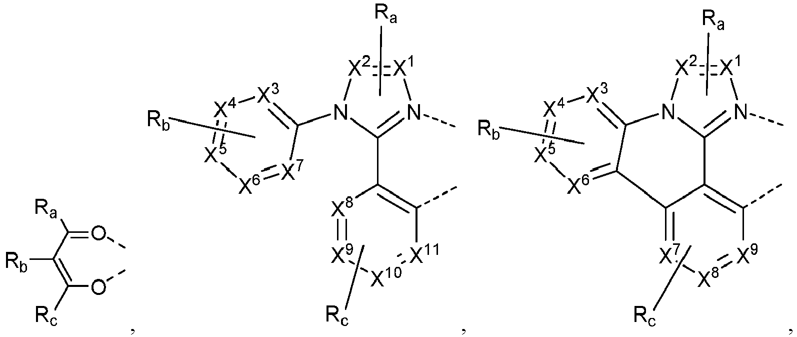 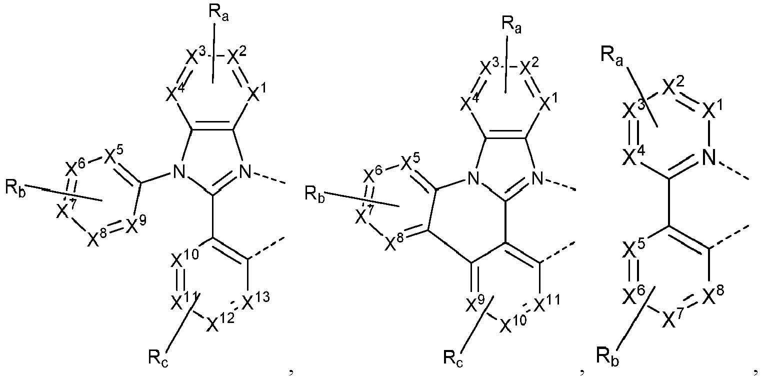 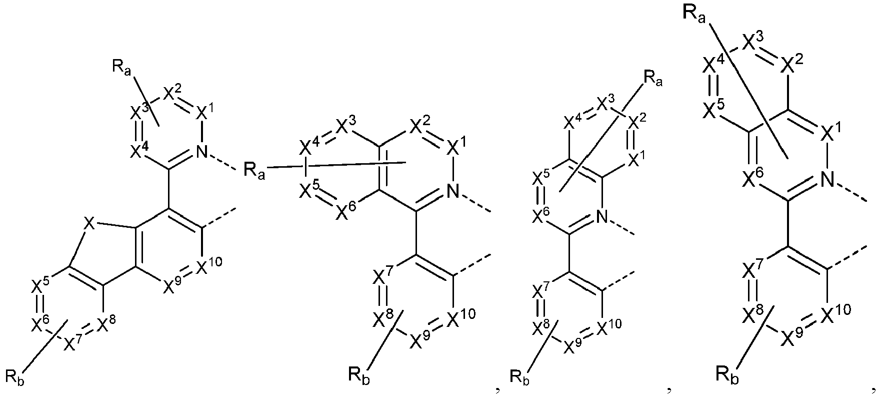 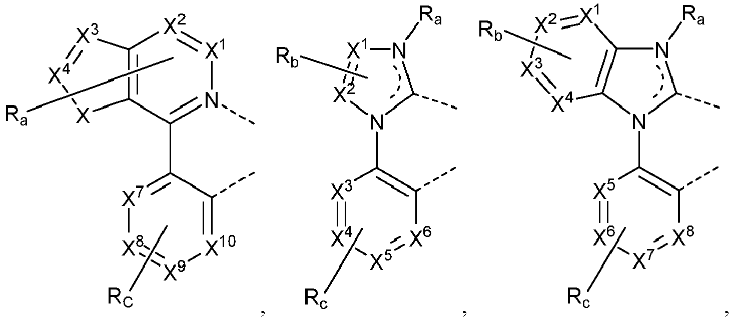 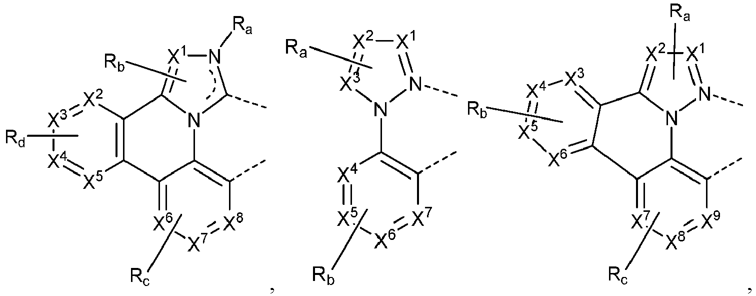 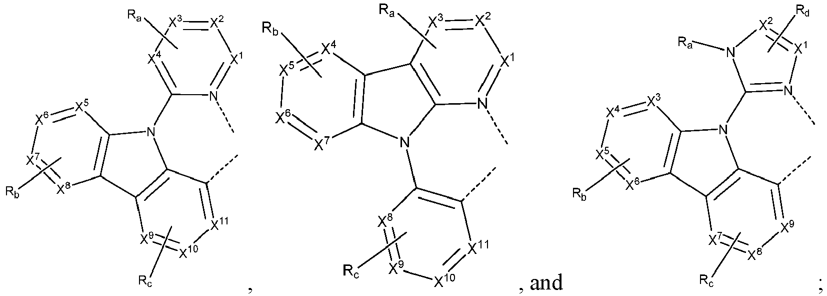
wherein each X¹ to X¹³ is independently selected from the group consisting of carbon and nitrogen;
wherein X is selected from the group consisting of BR', NR', PR', O, S, Se, C=O, S=O, SO₂, CR'R", SiR'R", and GeR'R";
wherein R' and R" are optionally fused or joined to form a ring;
wherein each Rₐ, R_{b}, R_{c}, and R_{d} represents from mono substitution to a maximum possible number of substitutions, or no substitution;
wherein R', R", Rₐ, R_{b}, R_{c}, and R_{d} are each independently a hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof; and
wherein any two adjacent substituents of Rₐ, R_{b}, R_{c}, and R_{d} are optionally fused or joined to form a ring or form a multidentate ligand.

12. The compound of claim 9, wherein the compound is Compound Ax having the formula Ir(L_{A*i*})₃, or Compound B*y* having the formula Ir(L_{A*i*})(L_{Bk})₂;
wherein *x* = *i*, and *y* = 468*i*+*k*-468;
wherein *i* is an integer from 1 to 111, and *k* is an integer from 1 to 468;
wherein L_{B*k*} have the following structures: 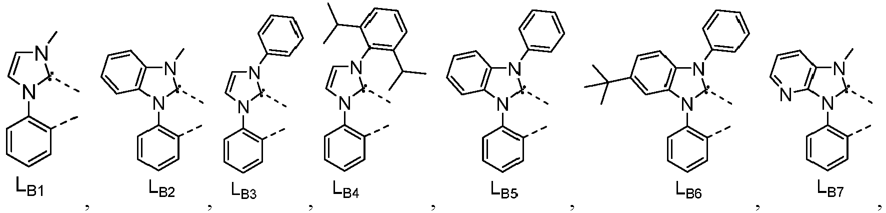 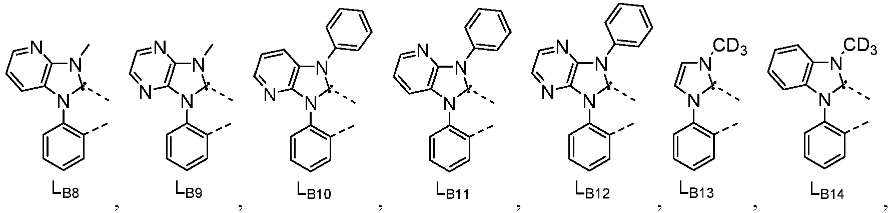 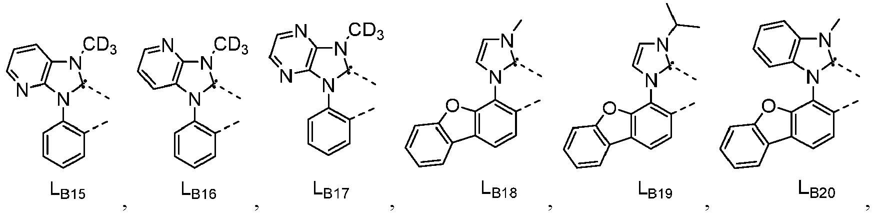 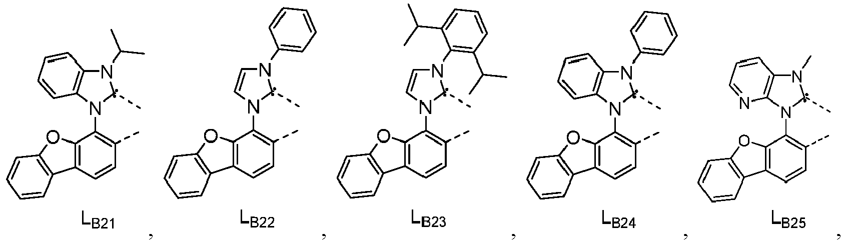 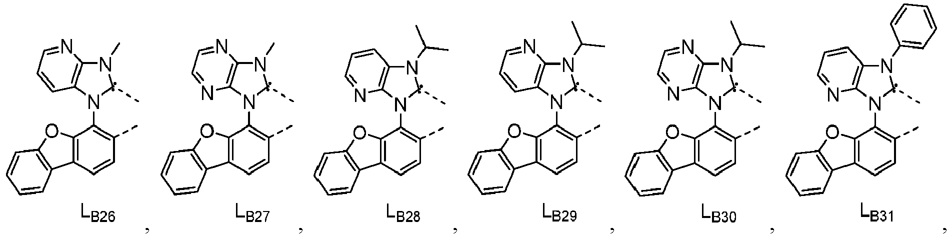 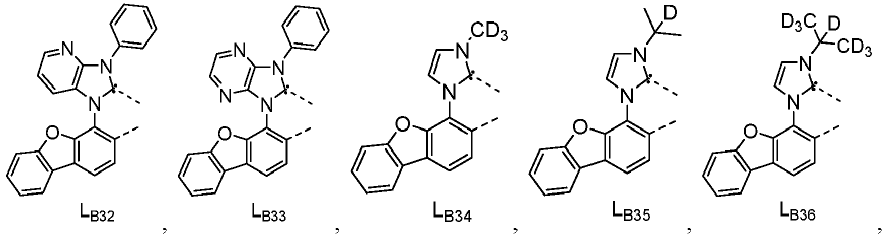 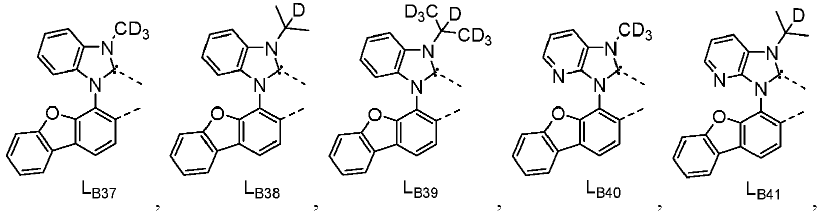 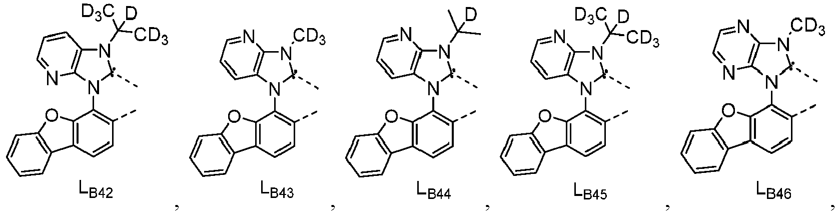 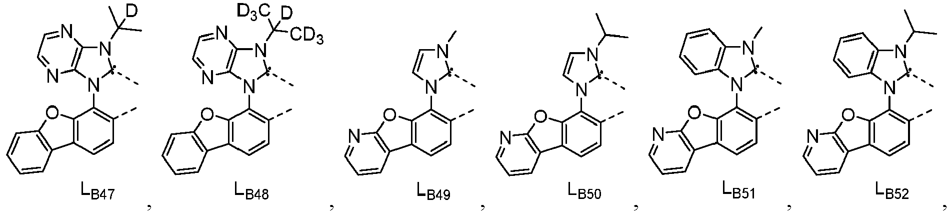 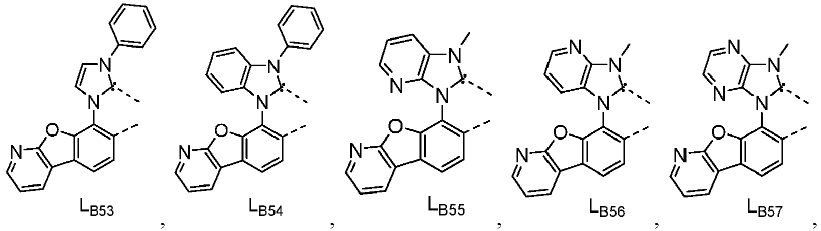 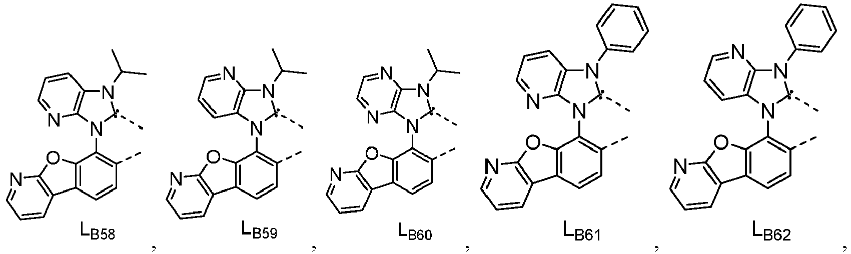 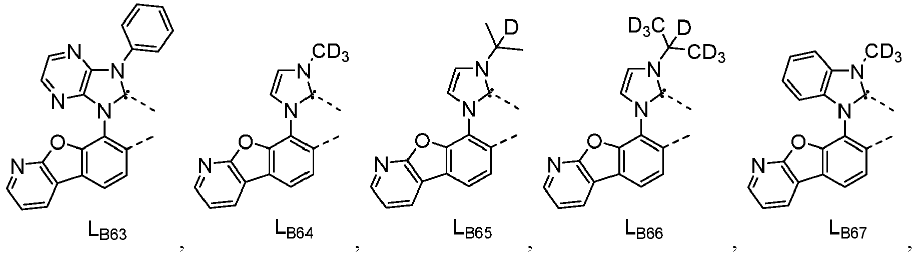 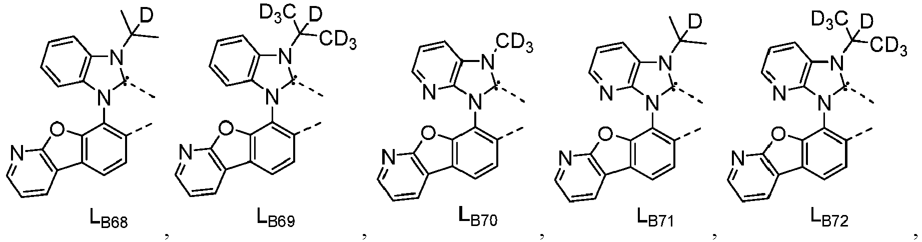 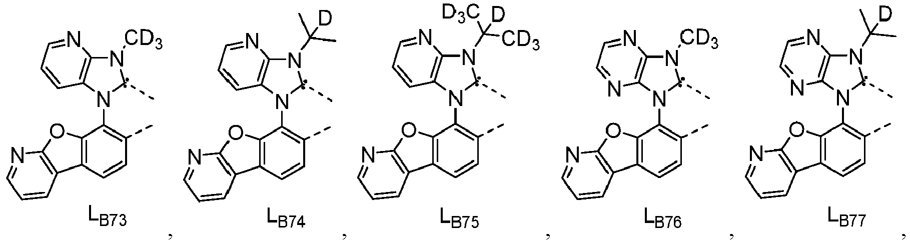 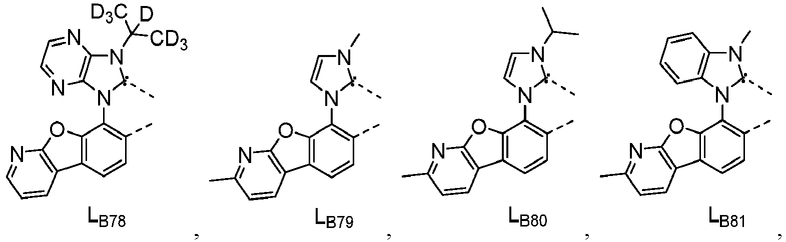 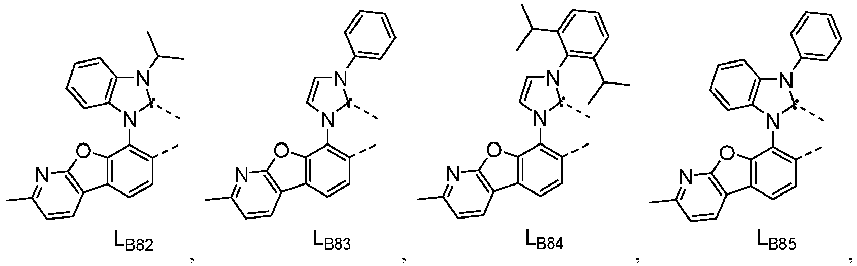 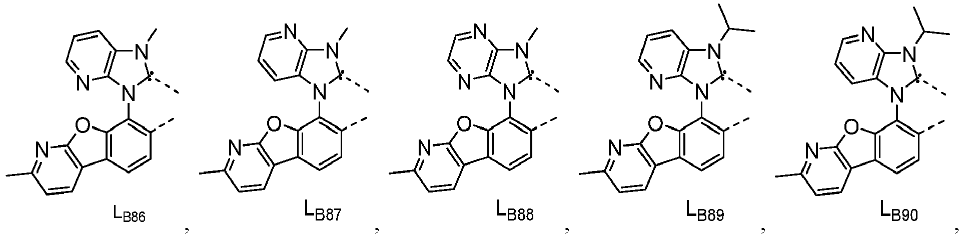 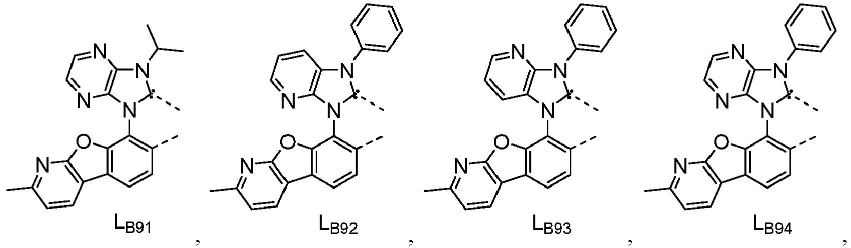 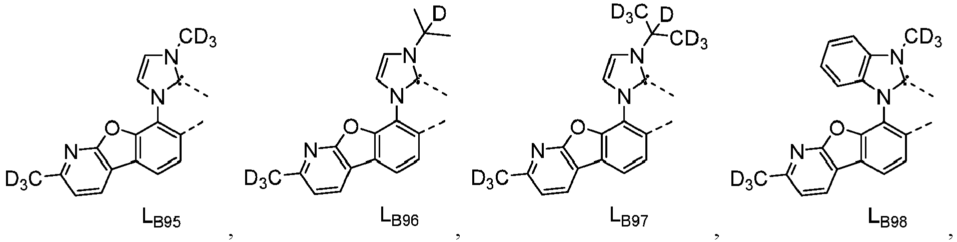 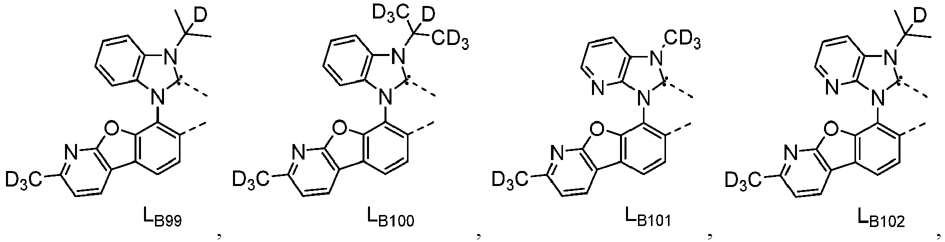 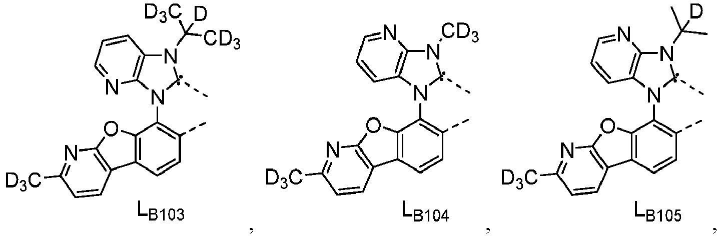 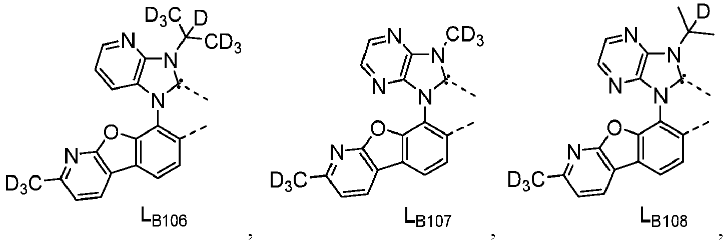 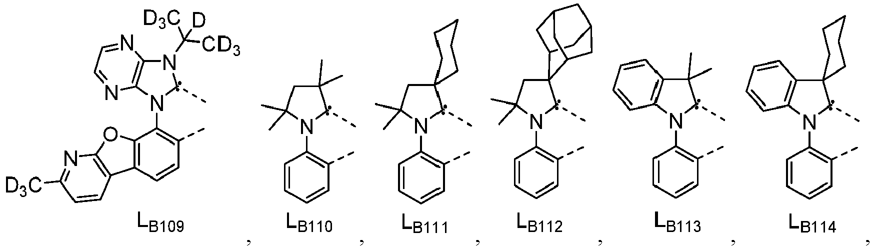 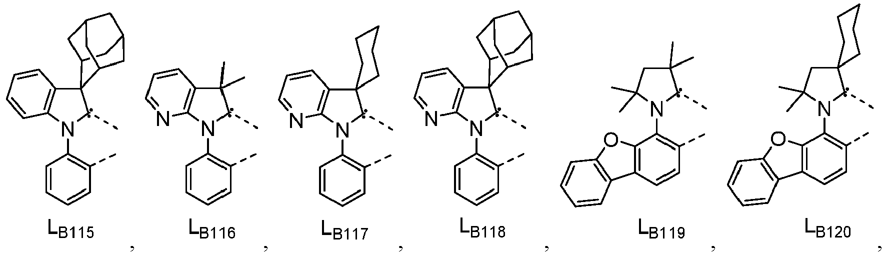 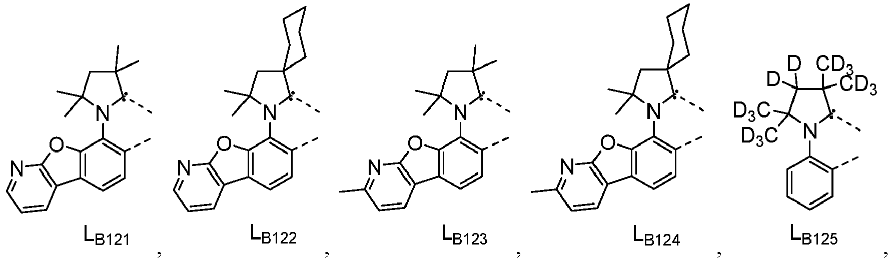 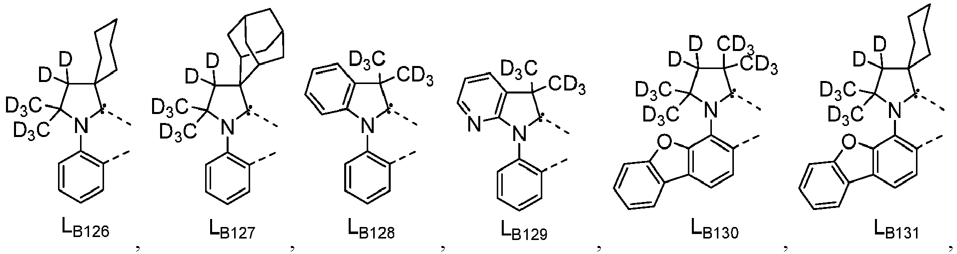 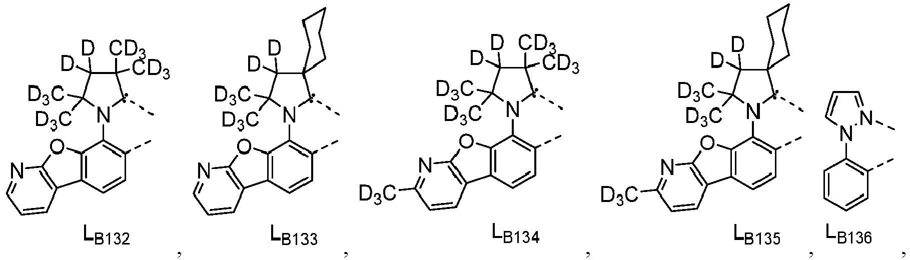 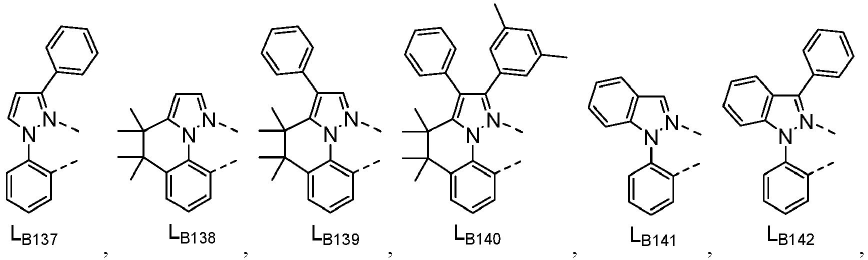 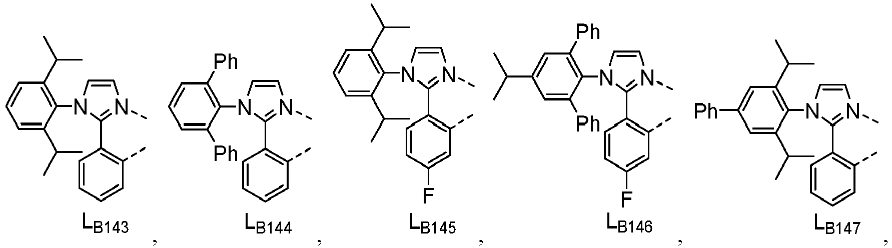 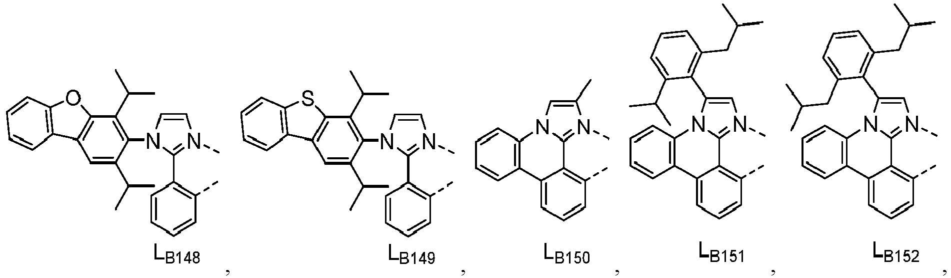 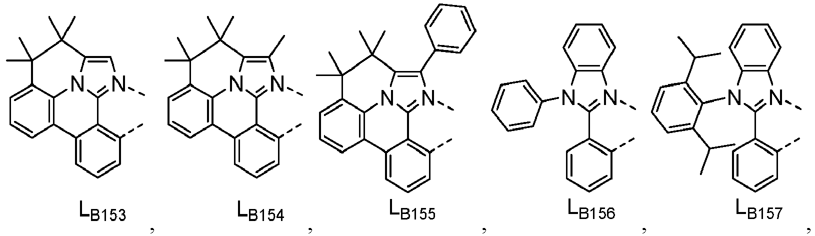 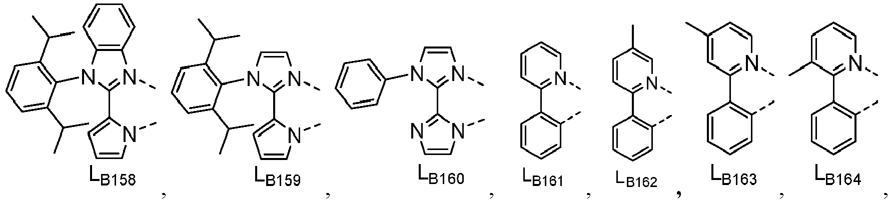 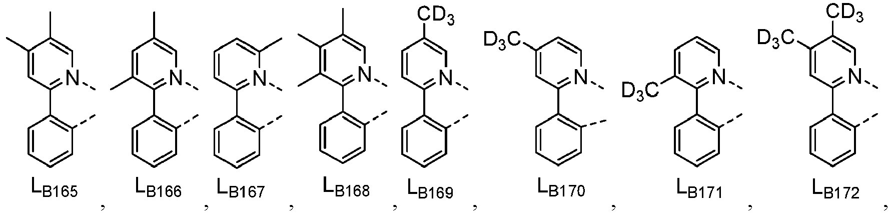 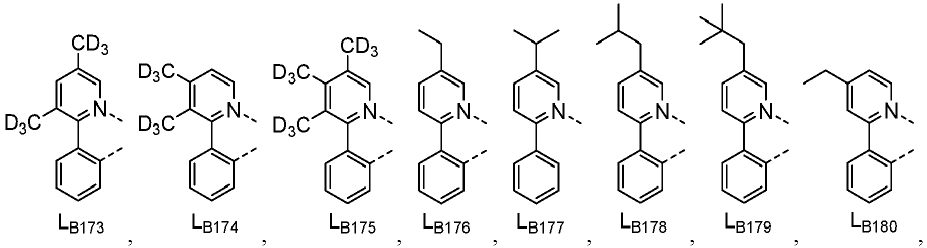 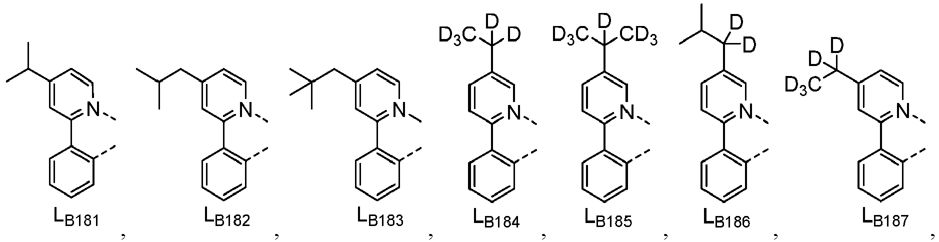 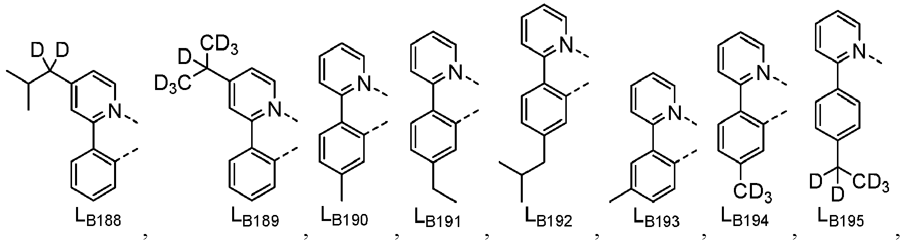 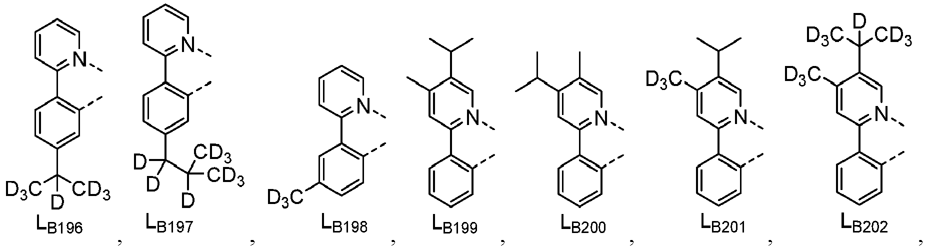 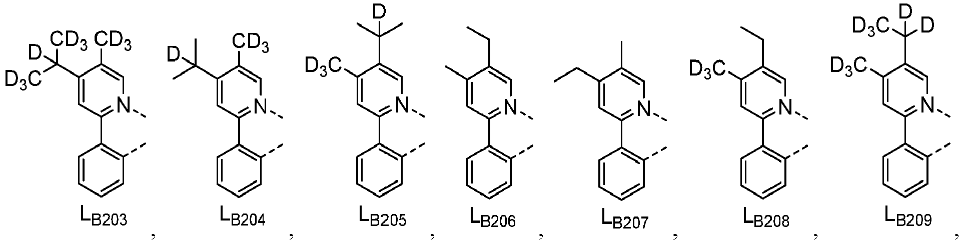 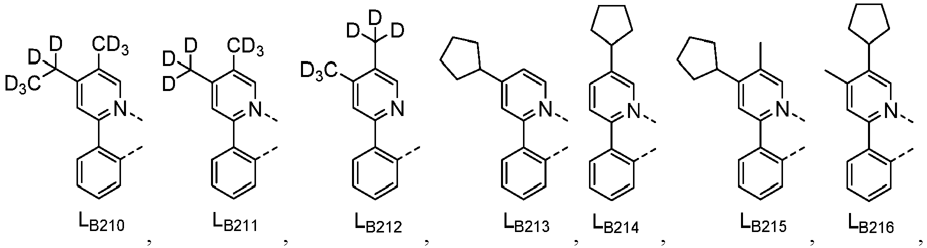 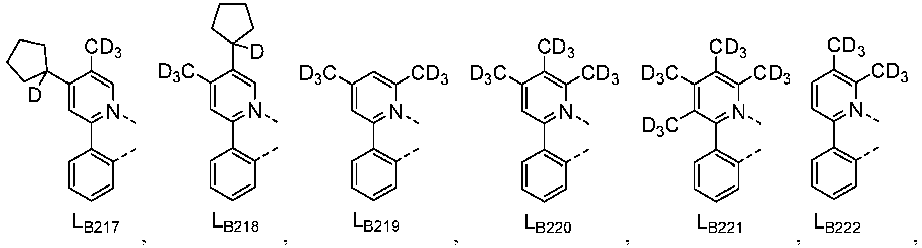 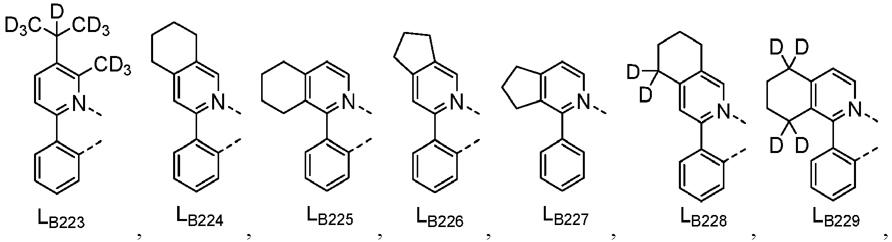 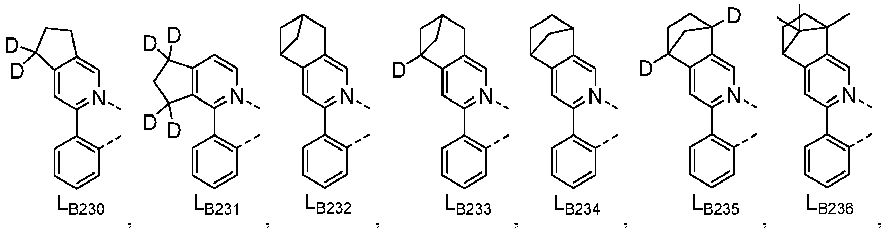 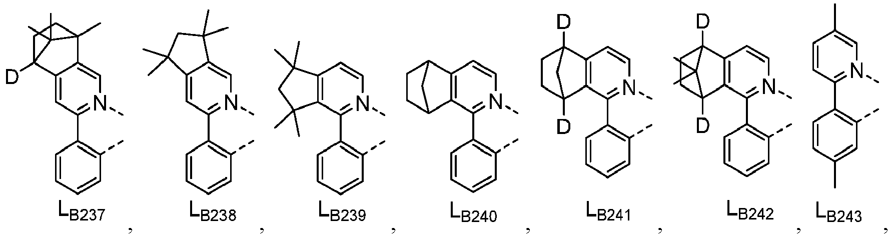 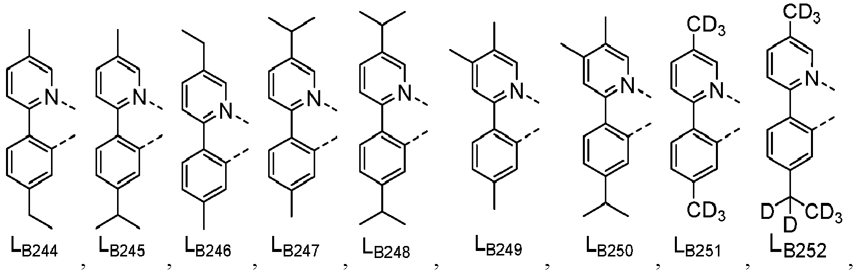 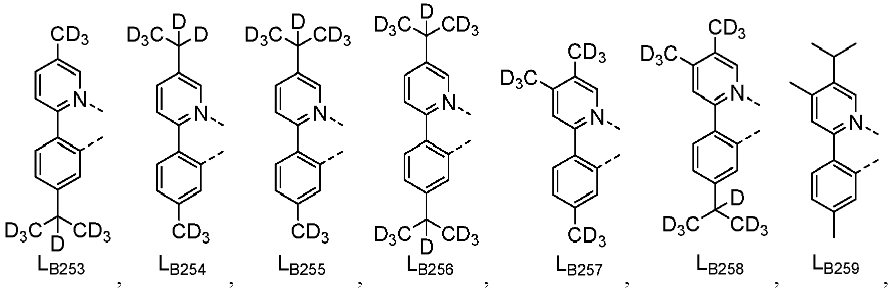 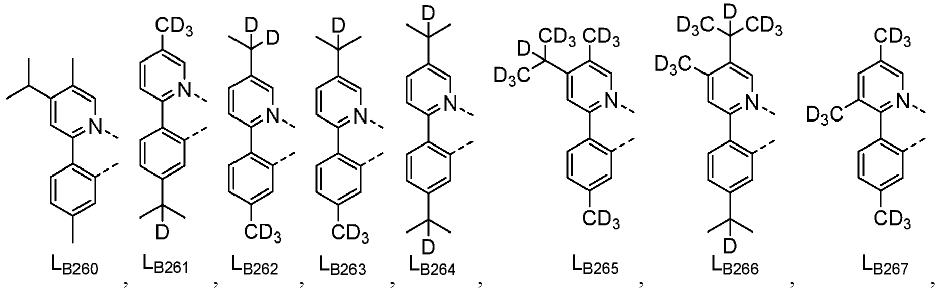 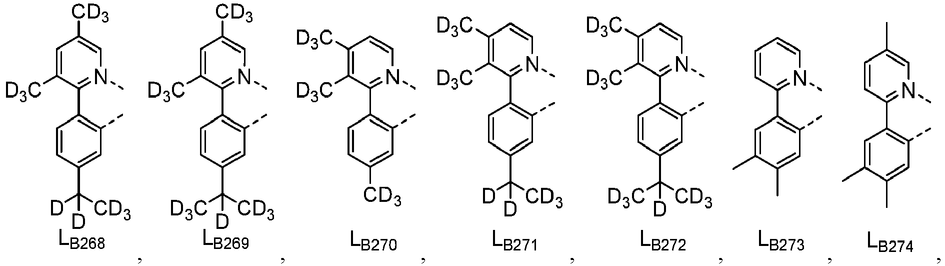 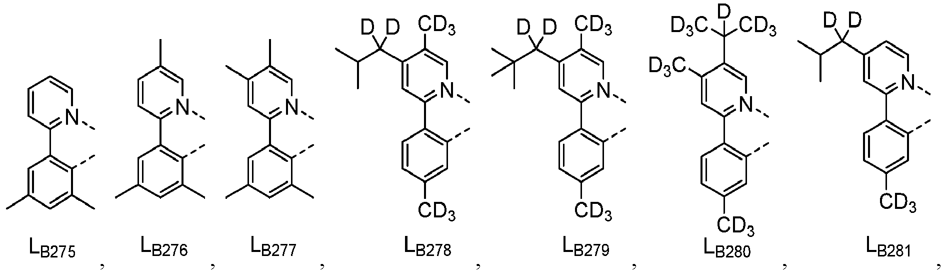 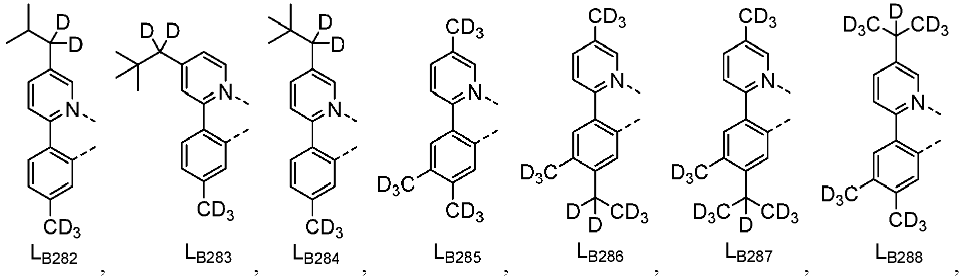 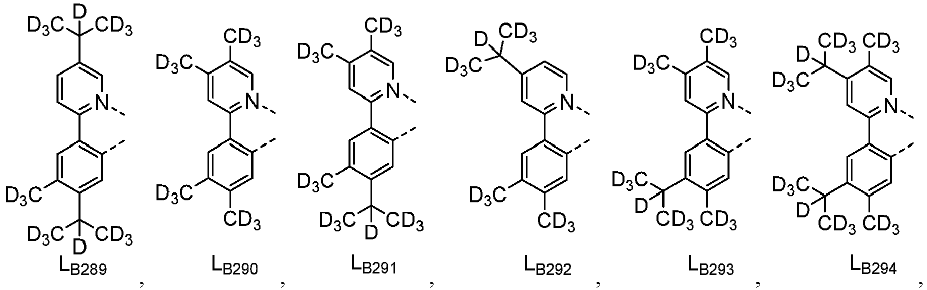 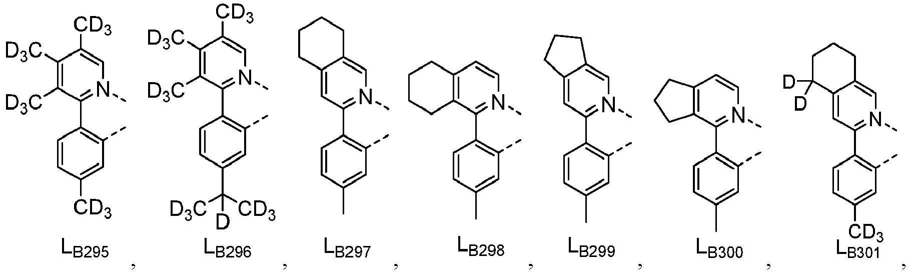 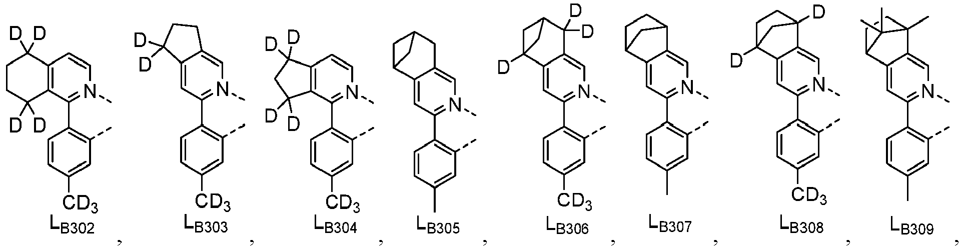 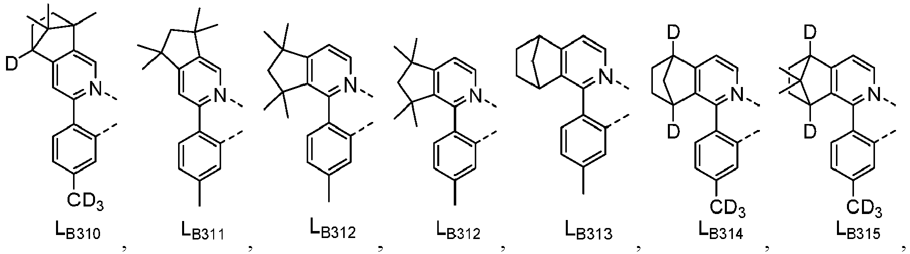 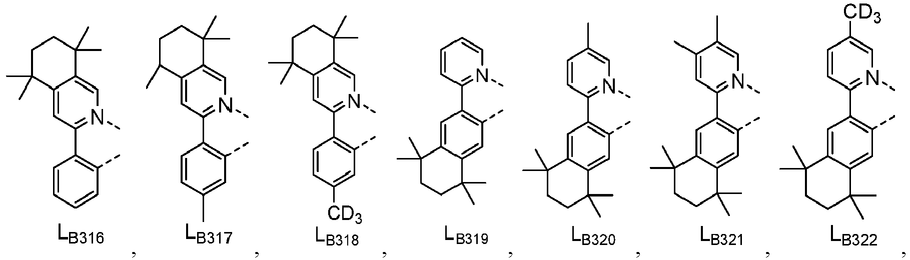 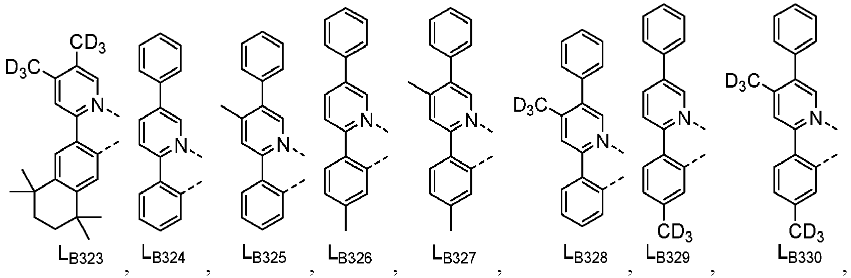 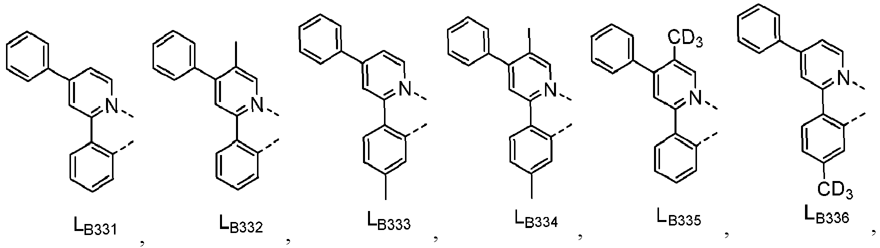 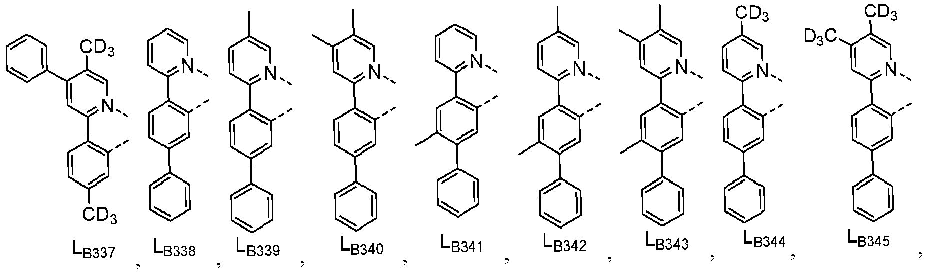 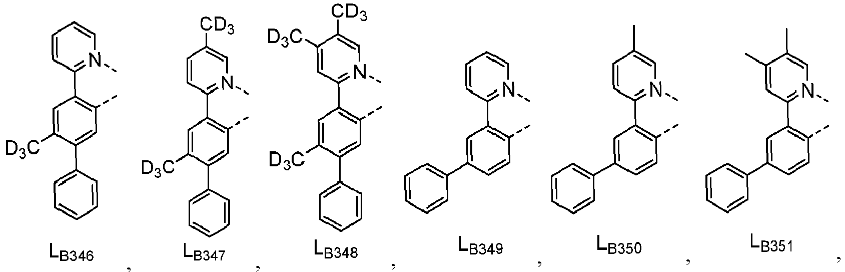 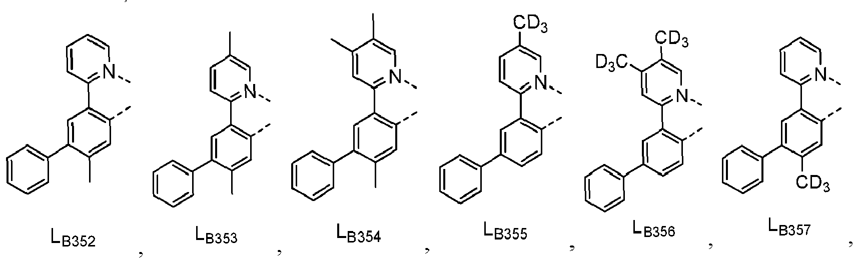 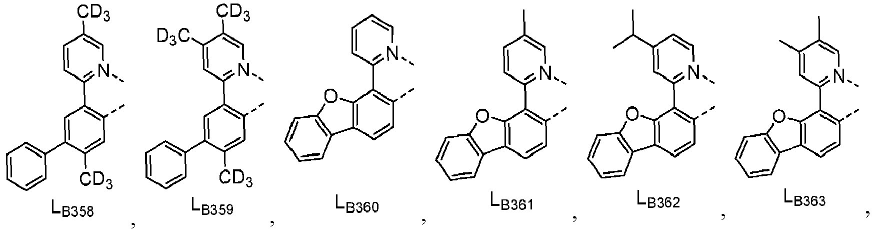 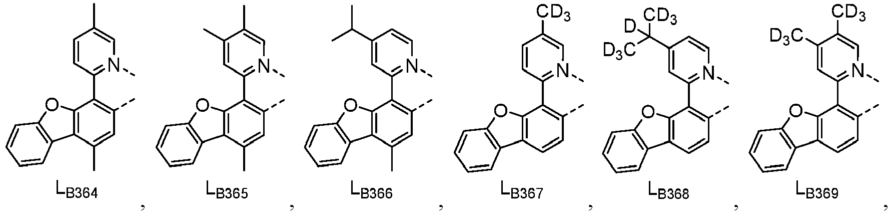 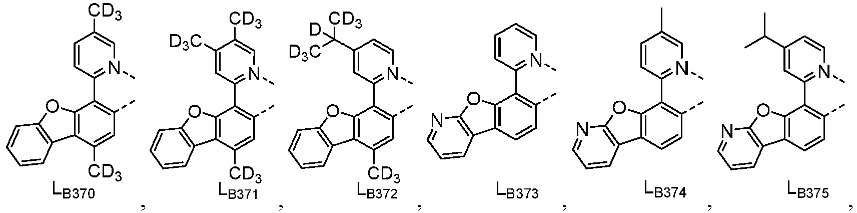 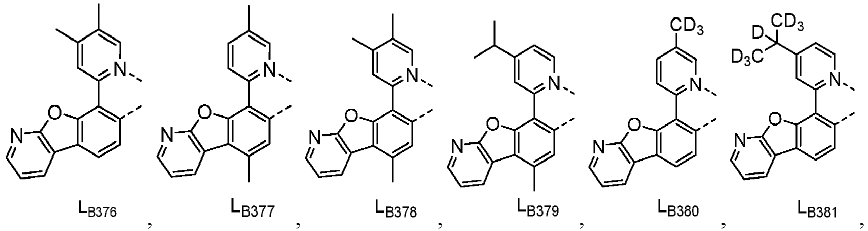 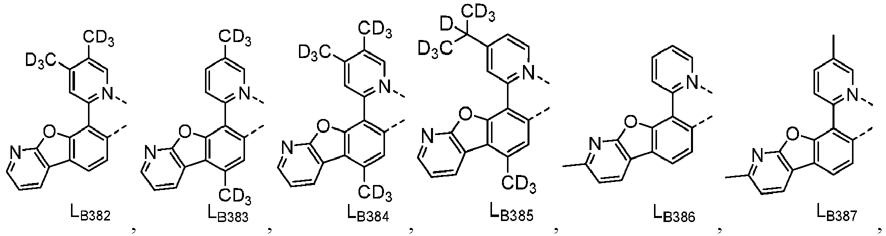 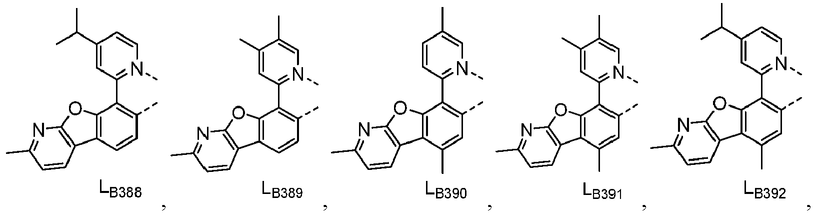 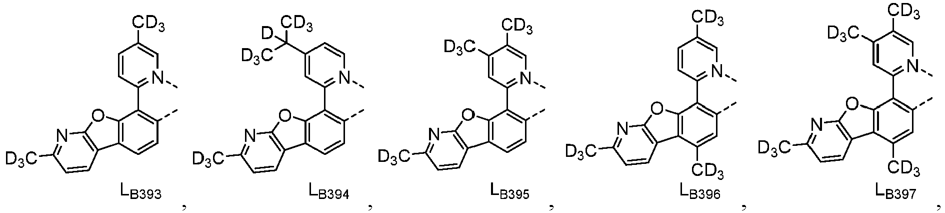 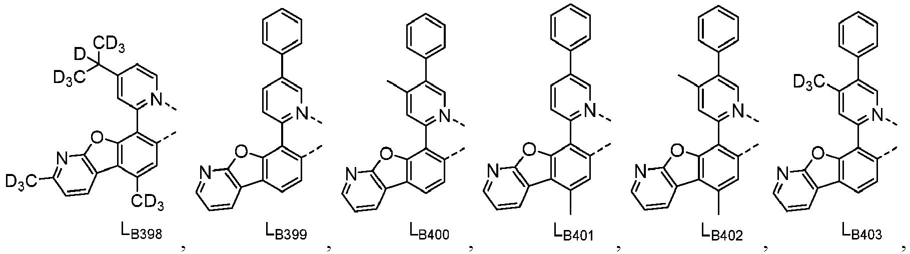 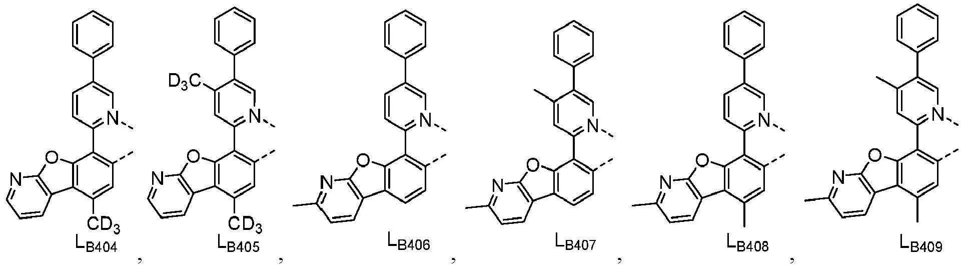 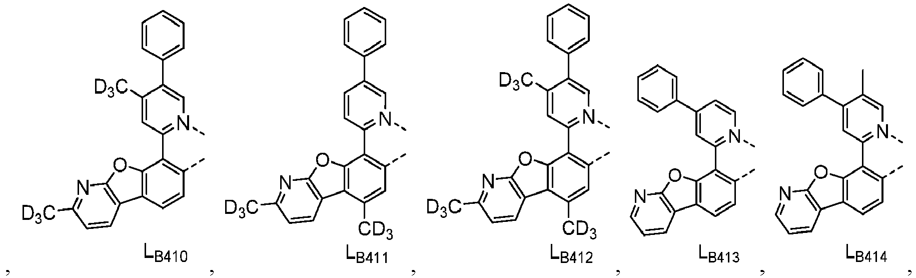 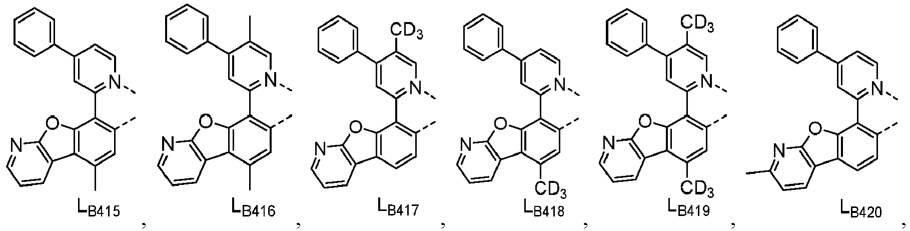 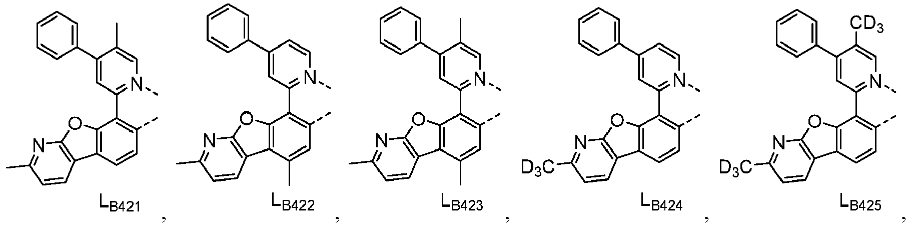 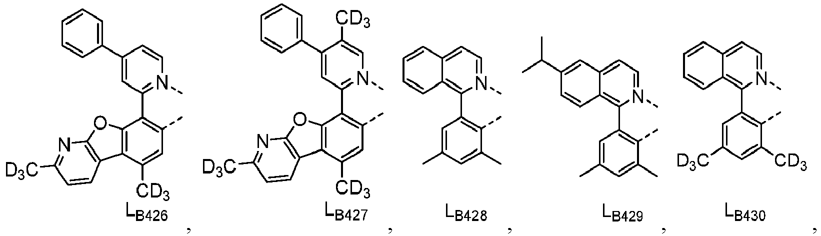 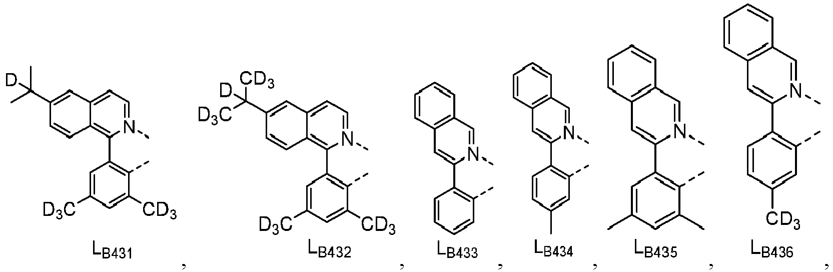 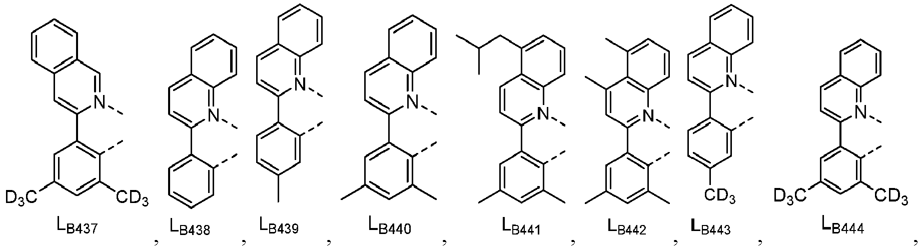 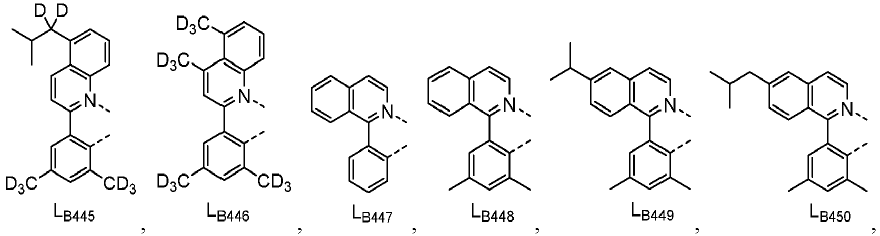 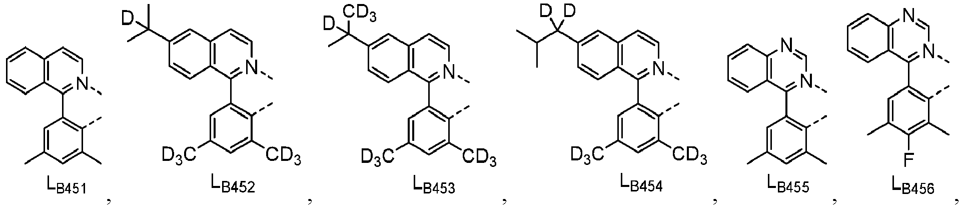 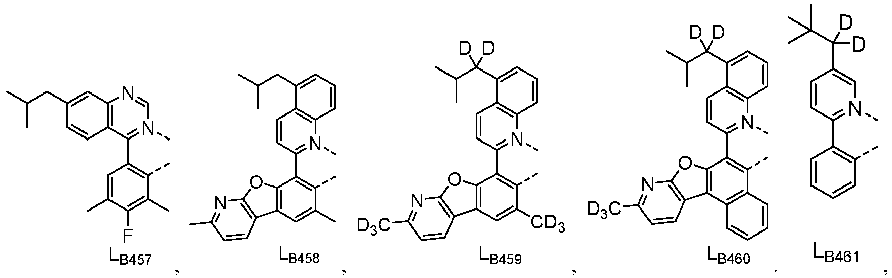 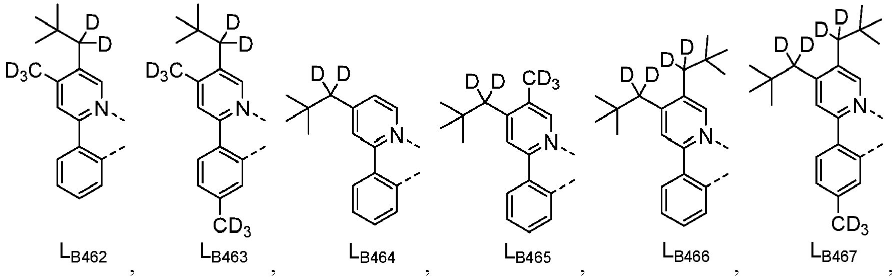 , and

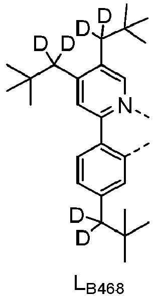

13. An organic light emitting device (OLED) comprising:
an anode;
a cathode; and
an organic layer, disposed between the anode and the cathode, comprising a compound as defined in any one of claims 1 to 12 and comprising a first ligand L_{A} of Formula I 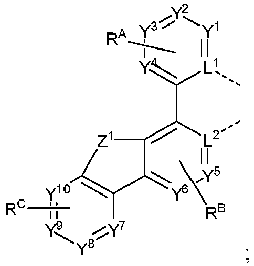
wherein one of L¹ and L² is C, and the other of L¹ and L² is N;
wherein Y¹ to Y¹⁰ are each independently selected from the group consisting of C and N;
wherein at least two adjacent Y⁷, Y⁸, Y⁹, and Y¹⁰ are carbon atoms that are fused to a structure of Formula II 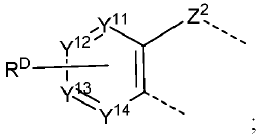
wherein Y¹¹ to Y¹⁴ are each independently selected from the group consisting of C and N;
wherein Z¹ and Z² are each independently selected from the group consisting of O, S, Se, NR, CRR', and SiRR';
wherein R^{A}, R^{B}, and R^{D} represent mono to a maximum possible number of substitutions, or no substitution;
wherein R^{C} represents di-, tri-, or tetra-substitution;
wherein each R, R', R^{A}, R^{B}, R^{C}, and R^{D} is independently a hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof;
wherein any two substituents may be joined or fused together to form a ring;
wherein L_{A} is complexed to a metal M by L¹ and L², and M has an atomic weight greater than 40;
wherein M is optionally coordinated to other ligands; and
wherein the ligand L_{A} is optionally linked with other ligands to comprise a tridentate, tetradentate, pentadentate, or hexadentate ligand.

14. The OLED of claim 13, wherein the organic layer is an emissive layer and the compound is an emissive dopant or a non-emissive dopant.

15. A consumer product comprising an organic light-emitting device (OLED) comprising:
an anode;
a cathode; and
an organic layer, disposed between the anode and the cathode, comprising a compound as defined in any one of claims 1 to 12 and comprising a first ligand L_{A} of Formula I 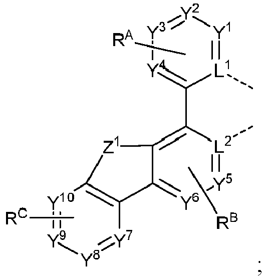
wherein one of L¹ and L² is C, and the other of L¹ and L² is N;
wherein Y¹ to Y¹⁰ are each independently selected from the group consisting of C and N;
wherein at least two adjacent Y⁷, Y⁸, Y⁹, and Y¹⁰ are carbon atoms that are fused to a structure of Formula II 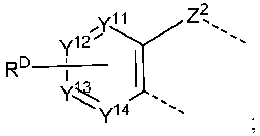
wherein Y¹¹ to Y¹⁴ are each independently selected from the group consisting of C and N;
wherein Z¹ and Z² are each independently selected from the group consisting of O, S, Se, NR, CRR', and SiRR';
wherein R^{A}, R^{B}, and R^{D} represent mono to a maximum possible number of substitutions, or no substitution;
wherein R^{C} represents di-, tri-, or tetra-substitution;
wherein each R, R', R^{A}, R^{B}, R^{C}, and R^{D} is independently a hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof;
wherein any two substituents may be joined or fused together to form a ring;
wherein L_{A} is complexed to a metal M by L¹ and L², and M has an atomic weight greater than 40;
wherein M is optionally coordinated to other ligands; and
wherein the ligand L_{A} is optionally linked with other ligands to comprise a tridentate, tetradentate, pentadentate, or hexadentate ligand.
